# EUROPEAN PATENT APPLICATION

(11) **EP 1 431 290 A1**
(43) Date of publication of application: **23.06.2004**
(21) Application number: 02768118.8
(22) Date of filing: 27.09.2002
(51) Int. Cl.: C07D 213/38, C07D 231/12, C07D 233/64, C07D 235/14, C07D 307/54, C07D 401/12, C07D 401/14, C07D 403/12, C07D 403/14, C07D 409/12, C07D 409/14, A61K 31/341, A61K 31/417, A61P 29/00, A61P 31/12, A61P 35/04, A61P 43/00

(54) **NOVEL NITROGENOUS COMPOUND AND USE THEREOF**

(30) Priority: 28.09.2001 JP 2001302584
(71) Applicant: Kureha Chemical Industry Co., Ltd., Tokyo 103-8552 (JP)
(72) Inventor: YAMAZAKI, Toru, Katsushika-ku, Tokyo 124-0022 (JP); SAITOU, Atsushi, Chuo-ku, Chiba-shi, Chiba 260-0844 (JP); ONO, Masahiro, Tot.-ku, Yokohama-shi, Kanagawa 245-0053 (JP); YOKOYAMA, Sei, Kokubunji-shi, Tokyo 185-0012 (JP); BANNAI, Kenji, Nerima-ku, Tokyo 176-0001 (JP); HIROSE, Kunitaka, Nerima-ku, Tokyo 179-0074 (JP); YANAKA, Mikiro, Matsudo-shi, Chiba 271-0092 (JP)
(74) Representative: Forstmeyer, Dietmar, Dr. rer. nat., Dipl.-Chem.
(86) International application number: PCT/JP2002/010069
(87) International publication number: WO 2003/029218

(57) **Abstract**

A novel nitrogen-containing compound effective against diseases such as HIV viral infectious diseases, rheumatism, and cancerous metastasis. It is a nitrogen-containing compound represented by the following general formula (1). In the formula, A typically represents a group represented by the formula (2) (A₁ is hydrogen or an optionally substituted, mono- or polycyclic, heteroaromatic or aromatic ring; G₁ is a single bond or a hydrocarbon group represented by the following formula (3) wherein R₁, R₂, and R₃ may be optionally substituted hydrocarbon groups); W is an optionally substituted hydrocarbon group or heterocyclic ring; x is -C(=O)NH-; y is -C(=O)-; and D₁ is hydrogen atom, alkyl having a polycyclic aromatic ring, di (substituted alkyl)amine, or alicyclic amine.

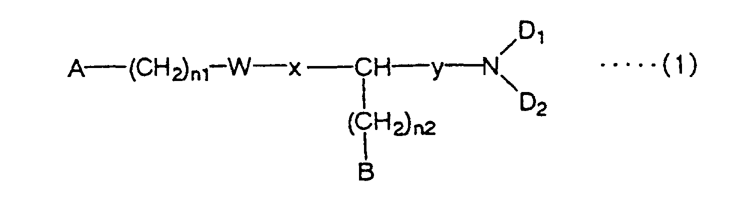

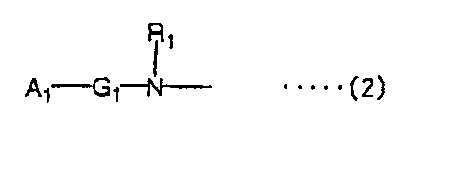

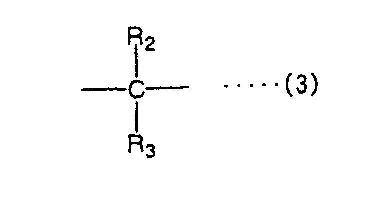

## Description

### TECHNICAL FIELD

The present invention relates to a nitrogen-containing compound or a pharmacologically acceptable salt thereof, and more particularly to a nitrogen-containing compound having an antiviral activity, and a drug for associated diseases such as a rheumatic disease and a cancer metastatic disease, based on antagonism against a chemokine receptor CXCR4.

### BACKGROUND ART

While examples of the drugs against acquired immuno-deficiency syndrome (AIDS) caused by an infection with the human immunodeficiency virus (HIV) include a reverse transcriptase inhibitor and a protease inhibitor, therapeutical effectiveness of those drugs has been lost due to the emergence of drug resistant HIV mutants (Saishin Igaku, Vol. 53, No. 9, p. 2031 (1998)). Also, the polypharmacy using the combination of such drugs has such disadvantages that it requires many conditions to be observed in administration, that it is complex, that it needs many kinds of drugs to be administered, and it causes various side effects (Nikkei Science, Oct, p. 29 (1998)). Moreover, particularly in case of using the protease inhibitor, it is known that the probability of causing emergence and screening of the resistant strain will increase unless the administration of approximately 100% of the drugs is kept, in spite of the complex administration method and many side effects thereof (Molecular Medicine, Vol. 36, No. 9, p. 1012 (1999)). Alternatively, development of vaccine has been attempted because many viral diseases were destroyed or remarkably weakened by vaccines in the past. However, this is considered to be extremely difficult due to frequent occurrence of various HIV mutant (Nikkei Science, Oct. p. 42 (1998)).

Although several kinds of compounds having an anti-HIV effect has been reported as described above, there is now strongly desired to develop a novel antiviral drug with excellent anti-retrovirus effect which is capable of opposing to the expression of the resistance, and which has little toxicity and cause little side effect, thereby allowing long term administration.

Chemokines is one kind of cytokine which renders chemotaxis to leukocytes, and is a secretory protein.
Chemokine is classified into CXC-kemokine, CC-kemokine, C-kemokine, CX3C-kemokine according to the Cys sequence at N-terminal, and the total number thereof is said to be about 30.

The chemokine receptor includes several sub types, including CXCR4. It is known that the CXCR4 which is a ligand for CXC-kemokine SDF-1α is utilized as a coreceptor on infection with a host cell of T cell-directive HIV (Science, 272, 872 (1996); Nature, 382, 829 (1996)). The HIV invades through binding to the CXCR4 on the surface of a host cell of an envelope protein gp120. That is, the drug having antagonism against the CXCR4 is expected as an anti-HIV drug based on a novel mechanism of invasion inhibition, and there have been reported three low molecular compounds as such drugs: AMD3100 (Journal of experimental medicine (J. Exp. Med), 186, 1383 (1997)), T22 (J. Exp. Med, 186, 1389 (1997)), and ALX40-4C (J. Exp. Med, 186, 1395 (1997)).

On the other hand, it has been elucidated that the CRCX4 associates with various diseases other than HIV infection. For example, there has been reported rheumatic disease (WO 00/06086), and cancer metastasis (Nature, 410, 50 (2001)), etc.

As a drug for such diseases , there is strongly desired to develop a novel low-molecular drug which has CXCR4 antagonism, and which further has little toxicity and cause little side effect, thereby allowing long term administration.

### DISCLOSURE OF INVENTION

Therefore, the object of the present invention is to provide a drug having an excellent anti-retrovirus effect, an excellent CXCR4 antagonism against SDF-1α, and a novel chemical structure with high safety.

As a result of researches to develop a compound having an excellent anti-retrovirus effect, and also having a novel chemical structure useful as an excellent CXCR4 antagonist against SDF-1α, the present inventors have found a group of nitrogen-containing compounds which exhibit protection characteristics in a cell vaccinated with HIV-1 and therefore are regarded as having a potentiality for treatments of AIDS, AIDS-associated complication, and so on, and which also exhibit a powerful CXCR4 antagonism and therefore are regarded as having a potentiality for treatments of rheumatic disease, cancer metastatic diseases, and so on. Thus, an obj ect of the present invention is to provide a compound represented by a general formula (1) defined below, which has an anti-virus activity for mainly HIV and a CXCR4 antagonism, and an another object of the invention is to provide a drug composed of the compound represented by the general formula (1) defined below, for treating virus-infected patients and patients suffering from rheumatis, cancer, etc.

Specifically, the present invention relates to a nitrogen-containing compound represented by the following general formula (1) or a pharmacologically acceptable salt thereof: 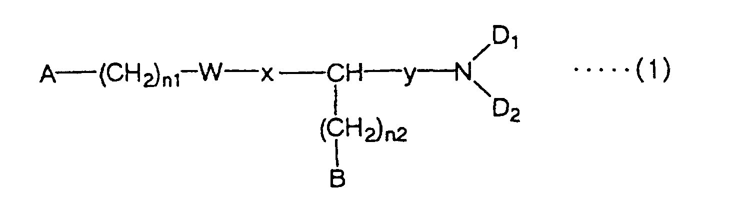 (In the general formula (1),
n₁ represents an integer of 0 to 3 and n₂ represents an integer of 0 to 4.

A represents a group represented by the following general formula (2): 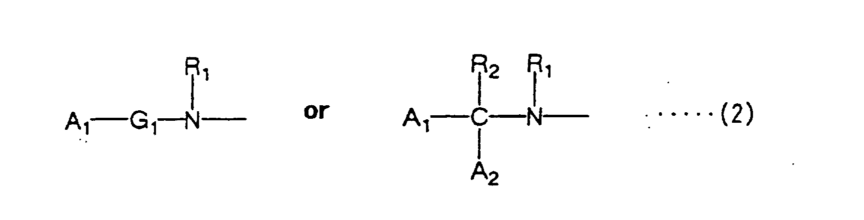 In the general formula (2),
A₁ and A₂ each independently represent a hydrogen atom, an optionally substituted mono- or polycyclic heteroaromatic ring, or an optionally substituted mono- or polycyclic aromatic ring.
G₁ represents a single bond or a group represented by the following general formula (3): 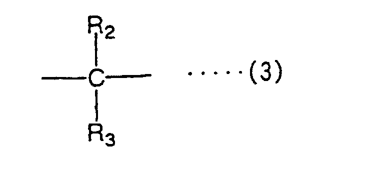 R₁, R₂, and R₃ represent an optionally substituted alkyl group having 1 to 6 carbon atoms, an optionally substituted alkenyl group having 2 to 6 carbon atoms, an optionally substituted alkynyl group having 2 to 6 carbon atoms, or an optionally substituted cyclic alkyl group having 3 to 6 carbon atoms.
W represents an optionally substituted alkylene group having 1 to 7 carbon atoms, an optionally substituted alkenylene group having 2 to 7 carbon atoms, an optionally substituted alkynylene group having 2 to 7 carbon atoms, an optionally substituted cyclic alkylene group having 3 to 10 carbon atoms, an optionally substituted mono- or polycyclic aromatic ring, an optionally substituted mono- or polycyclic heteroaromatic ring, or an optionally substituted mono- or polycyclic saturated heterocyclic ring.
D₁ and D₂ each independently represent a hydrogen atom or a group represented by the following general formula (4):

―G₂―R₄ (4)

In the general formula (4),
G₂ represents an optionally substituted alkylene group having 1 to 15 carbon atoms, an optionally substituted alkenylene group having 2 to 7 carbon atoms, or an optionally substituted alkynylene group having 2 to 7 carbon atoms.
R₄ represents a hydrogen atom, an optionally substituted cyclic alkyl group having 3 to 10 carbon atoms, an optionally substituted mono- or polycyclic aromatic ring, an optionally substituted and partly saturated polycyclic aromatic ring, an optionally substituted mono- or polycyclic heteroaromatic ring, an optionally substituted and partly saturated polycyclic heteroaromatic ring, or an optionally substituted mono- or polycyclic saturated heterocyclic ring.
B represents a group represented by the following general formula (5): 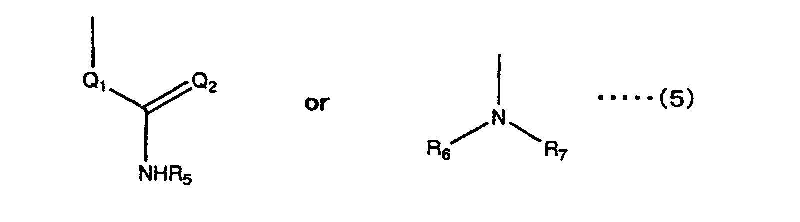 In the general formula (5),
Q₁ represents S, O, or NH, and Q₂ represents S, O, or NR₈ (except for a case of Q₁=NH and Q₂=NR₈).
R₅ and R₈ each independently represent a hydrogen atom, an optionally substituted lower alkyl group, an optionally substituted cyclic alkyl group, or an optionally substituted aromatic ring, and R₅ and R₈ optionally form a ring.
R₆ and R₇ each independently represent a hydrogen atom, a substituent represented by the following general formula (6), an optionally substituted alkyl group having 1 to 15 carbon atoms, an optionally substituted cyclic alkyl group having 3 to 15 carbon atoms, an optionally substituted alkenyl group having 1 to 3 double bonds and 2 to 15 carbon atoms, or an optionally substituted alkynyl group having 1 to 3 triple bonds and 2 to 15 carbon atoms, and R₆ and R₇ optionally form a ring wherein R₆ and R₇ are optionally bonded with each other via a heteroatom, a cyclic alkyl group, an aromatic ring, a heteroaromatic ring, or a heterocyclic ring to form the ring. 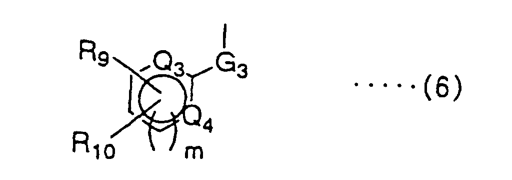 In the formula (6),
m represents 0 or 1, when m=0, Q₃ represents CH or N and Q₄ represents N, S, or O, and when m=1, Q₃ and Q₄ each independently represent CH or N.
G₃ represents an optionally subsituted alkylene group having 1 to 4 carbon atoms, or an optionally substituted alkenylene group having 2 to 4 carbon atoms.
R₉ represents
a lower alkyl group, an alkoxy group, a haloalkyl group, a haloalkoxy group, a hydroxyalkoxy group, a halogen group, an amino group, an alkylamino group, a carboxyl group, an alkoxycarbonyl group, a carbamoyl group, an alkylcarbamoyl group, a saturated heterocyclic ring, or a heteroaromatic ring, which is substituted at any position of carbon atoms forming a ring, and when m=1, Q₃ and Q₄ simultaneously represent CH, R₉ optionally represents a hydrogen atom.
R₁₀ represents a hydrogen atom, or a group similar to R₅, and optionally bonds with G₃ to form a ring;
x represents a group represented by the following general formula (7): 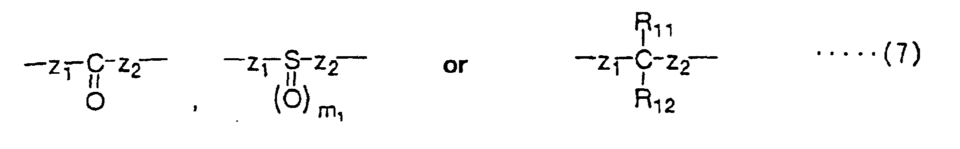 In the general formula (7),
z₁ and z₂ each independently represent a single bond, S, O, or NR₁₃, and m₁ represents an integer of 1 or 2.
R₁₁, R₁₂, and R₁₃ each independently represent a hydrogen atom, an optionally substituted alkyl group having 1 to 6 carbon atoms, an optionally substituted alkenyl group having 2 to 6 carbon atoms, an optionally substituted alkynyl group having 2 to 6 carbon atoms, or an optionally substituted cyclic alkyl group having 3 to 6 carbon atoms.
y represents a group represented by the following general formula (8): 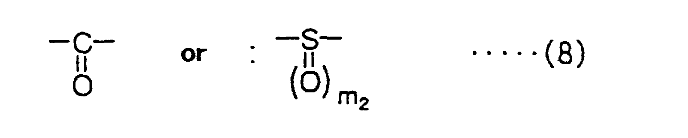 (In the general formula (8),
m₂ represents an integer of 1 or 2.)

When there is one asymmetric carbon atom optionally existing in the compound represented by the general formula (1), the compound is in any form of a pure optical isomer represented as absolute configuration of R or S, a mixture thereof in any ratio, and a racemic modification, and when there are two or more of the asymmetric carbon atoms in the compound, the compound is in any form of an optically pure diastereomer, a racemic modification thereof, or a combination thereof in any ratio.

Further, in the general formula (1) , preferably, x is a group represented by the following general formula (9): 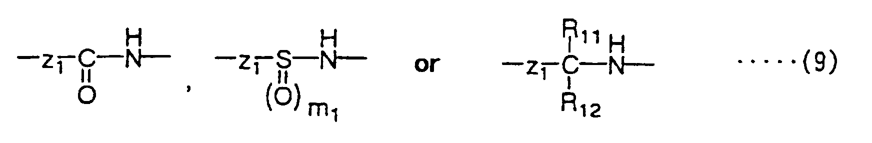 z₁, m₁, R_{11,} and R₁₂ are the same as described above; and
x is preferably represented by the following general formula (13) :

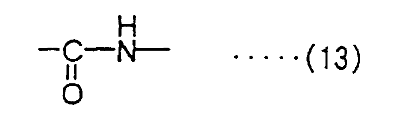

Further, y is preferably a group represented by the following general formula (10): 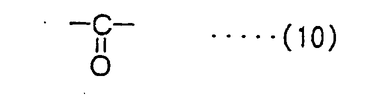 A is preferably a group represented by the following general formula (11) : 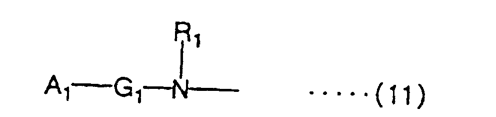 (wherein A₁, G₁, and R₁ are the same as described above).

Preferably, D₁ represents a hydrogen atom; and D₂ represents a group represented by the following general formula (12):

―G₄―R₁₄ (12)

wherein G4 represents an optionally substituted alkylene group having 1 to 4 carbon atoms; and R₁₄ represents an optionally substituted mono- or polycyclic aromatic ring, or an optionally substituted mono- or polycyclic heteroaromatic ring).

Preferably, B is represented by the following general formula (14): 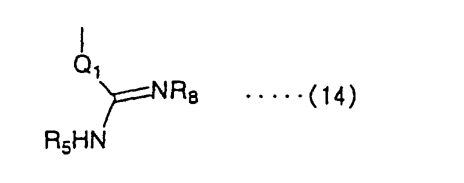 (wherein Q₁, R₅, and R₈ are the same as described above (except for when Q₁ = NH)) or
B is represented by the following general formula (15): 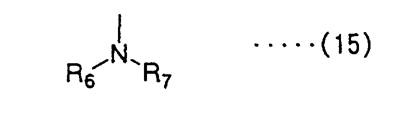 (wherein R₆ and R₇ are the same as described above).

The terms as used in this specification are defined as described below, and they are used singly or in combination.

An alkyl group represents a saturated hydrocarbon group with any structure of a straight chain, a branched chain, or a ring. Examples of the alkyl group include a methyl group, an ethyl group, an n-propyl group, an isopropyl group, an n-butyl group, an isobutyl group, a pentyl group, and a neopentyl group.

An alkenyl group represents a hydrocarbon group with any structure of a straight chain, a branched chain, or a ring having a double bond. Examples of the alkenyl group include an allyl group, a 1-butenyl group, a 2-butenyl group, an isobutenyl group, and a cyclohexenyl group.

An alkynyl group represents a hydrocarbon group with any structure of a straight chain, a branched chain, or a ring having a triple bond. Examples of the alkynyl group include a propynyl group and a 1-butynyl group.

A cyclic alkyl group represents a cyclic hydrocarbon group. Examples of the cyclic alkyl group include a cyclopropyl group, a cyclobutyl group, a cyclopentyl group, a cyclohexyl group, and a cycloheptyl group.

An aromatic ring represents an aromatic ring formed of a hydrocarbon. Examples of a monocyclic aromatic ring include a benzene ring; and examples of a polycyclic ring include a naphthalene ring and an anthracene ring.

Examples of a partly saturated polycylic aromatic ring include a dihydronaphthalene ring, a tetralin ring, and an indan ring. A heteroaromatic ring represents an aromatic ring having a nitrogen atom, an oxygen atom, or a sulfur atom in the ring. Examples of a monocyclic heteroaromatic ring include a pyrrole ring, a furan ring, a thiophene ring, a pyridine ring, a pyrimidine ring, a pyridazine ring, a pyrazine ring, an imidazole ring, a thiazole ring, an oxazole ring, and a triazole ring. Examples of a polycyclic heteroaromatic ring include a quinoline ring, an isoquinoline ring, a benzimidazole ring, a benzthiazole ring, a benzoxazole ring, an indole ring, a benzfuran ring, and a benzthiophene ring. Examples of a partly saturated polycyclic heteroaromatic ring include a tetrahydroisoquinoline ring and a tetrahydroquinoline ring. A heterocyclic ring represents a saturated ring having a nitrogen atom, an oxygen atom, or a sulfur atom in the ring. Examples of the heterocyclic ring include pyrrolidine, piperidine, piperazine, morpholine, and thiomorpholine.

An alkylene group represents a hydrocarbon group being capable of bonding with two groups at the ends. Examples of the alkylene group include an ethylene group, a propylene group, an isopropylene group, a butylene group, an isobutylene group, and a 2,2-dimethylethylene group.

An alkenylene group represents a group having a double bond in an alkylene group. Examples of the alkenylene group include a propenylene group, a 2-butenylene group, and a 1,3-butadienylene group.

An alkynylene group represents a group having a triple bond in an alkylene group. Examples of the alkynylene group include a propynylene group and a butynylene group.

The cyclic alkylene group in W represents a cyclic hydrocarbon group that can be connected with two groups at an optional position. Examples of the cyclic alkylene group include a cyclopropylene group, a cyclopentylene group, a cyclohexylene group, and a tetralinylene group. An aromatic ring also represents an aromatic ring that can be connected with two groups at an optional position. Examples of the aromatic ring include a phenylene ring and a naphthalene ring. A heteroaromatic ring also represents a heteroaromatic ring that can be connected with two groups at an optional position.
Examples of the heteroaromatic ring to be used include a pyrrole ring, a furan ring, a thiophene ring, a pyridine ring, a pyrimidine ring, a pyrazine ring, a pyridazine ring,an imidazole ring, a thiazole ring, an oxazole ring, a triazole ring, a quinoline ring, an isoquinoline ring, a benzoimidazole ring, a benzothiazole ring, a benzooxazole ring, an indole ring, a benzofuran ring, and a benzothiophene ring.

B represents R₆(R₇)-, where R₆ and R₇ may form a ring. R₆ and R₇ are directly binded, and formed a ring together with a nitrogen atom to which they are bound. Examples of such a ring include a pyrrolidine ring, a piperidine ring, a hexamethyleneimine ring, and a heptamethyleneimine ring. Examples of a ring formed by binding R₆ and R₇ are binded through a heteroatom, and formed a ring together with a nitrogen atom to which they are bound. Examples of such a ring include a morpholine ring and a piperadine ring. Further, examples of a ring formed by binding R₆ and R₇ through an aromatic ring include a tetrahydroisoquinoline ring and a tetrahydroindole ring.

Examples of a ring formed of R₁₀ and G₃ include a tetralinyl group, an indanyl group, a tetrahydroquinolyl group, and a tetrahydroisoquinolyl group.

Examples of the groups that may be "optionally substituted" in the expressions for each substituent include a hydroxyl group, a thiol group, a formyl group, a carboxyl group, a sulfonyl group, an amino group, an amide group, a carbamoyl group, a cyano group, an alkoxy group, an alkoxycarbonoyl group, an alkylamino group, an acylamino group, an alkoxycarbonylamino group, alkylthio group, and a phenyl group. The alkoxy group represents a group in which an alkyl group binds through an oxygen atom, and the acylamino group represents a group in which an alkyl group or a phenyl group binds to an amino group through a carbonyl group.

### BRIEF DESCRIPTION OF THE DRAWINGS

Fig. 1 is a diagram showing reaction steps of Production Method Example 1.
Fig. 2 is a diagram showing reaction steps of Production Method Example 2.

### BEST MODE FOR CARRYING OUT THE INVENTION

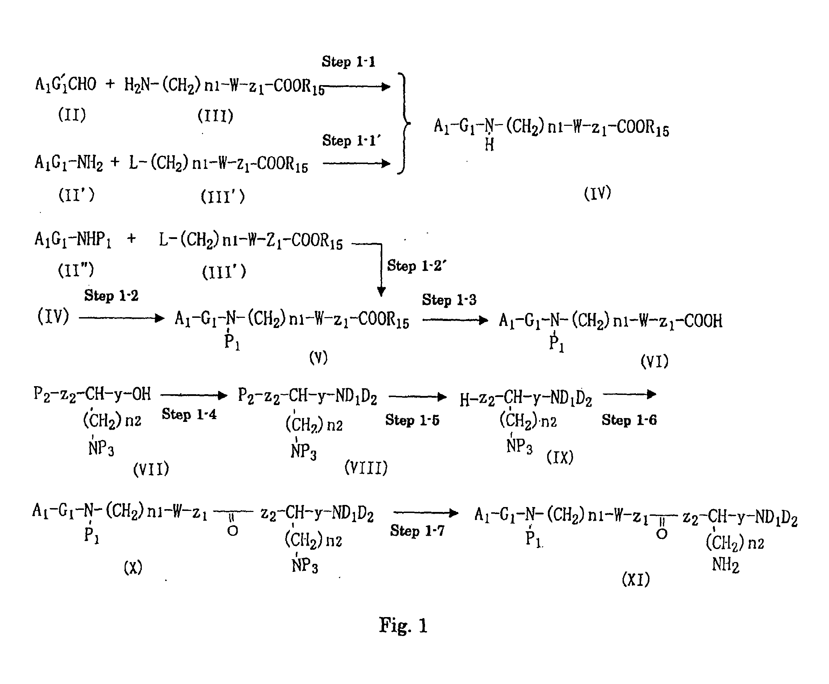

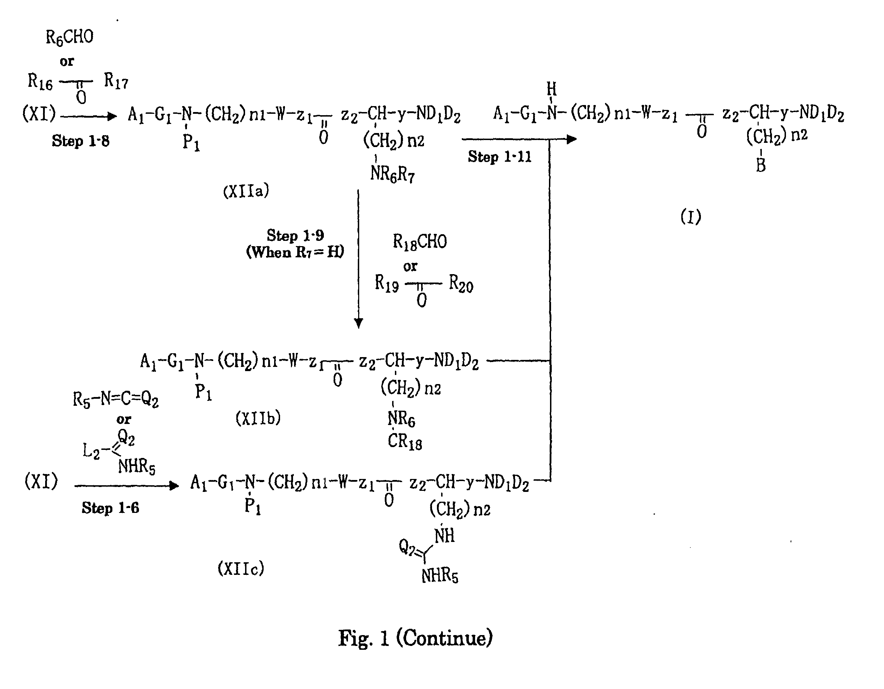

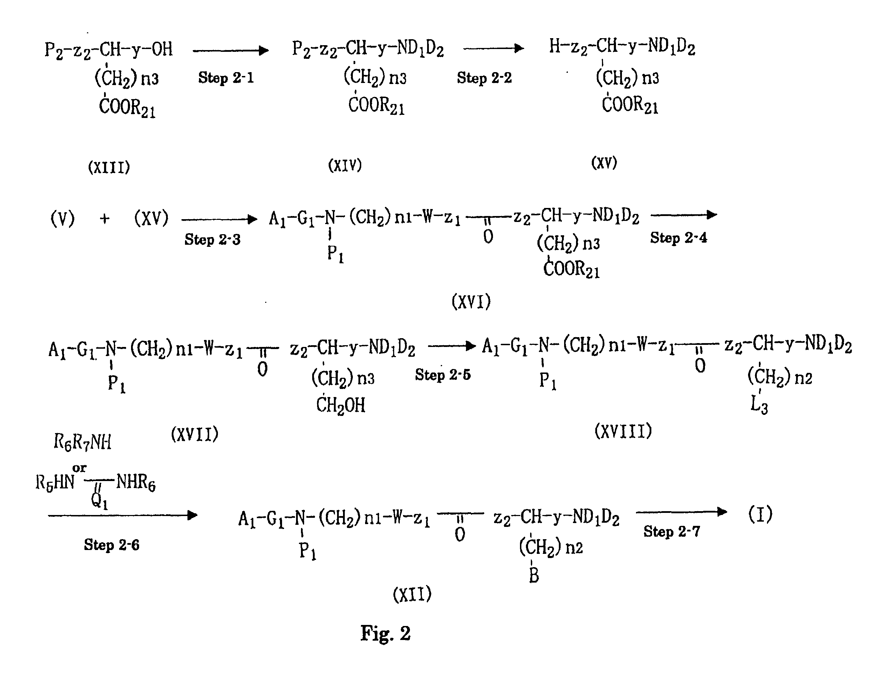

The compound of the present invention may be manufactured by a combination of organic chemical reactions generally known. While the reaction schemes are illustrated in Fig. 1 and 2 to explain production methods, the production method of the compound of the present invention is not limited thereto.

### Production Method Example 1

Fig. 1 shows a reaction scheme of Production Method Example 1.

### Step 1-1

A commercially available aldehyde (II) (A₁ is the same as described above; and G_{1'} represents a group same as G₁ except that it has one carbon atom fewer than G₁) , and an amine (III) (n₁, W, and Z₁ are the same as described above; and R₁₅ represents a group such as an alkyl group, an benzyl group, etc.) or a salt thereof may be added to any organic solvent (e.g. , methanol, ethanol, toluene, etc.) , withacatalyst (acidorbase, adehydratingagent) if necessary, to react at -20°C to 150°C. A reducing agent (e.g., sodium borohydride) may then be added for reaction, thereby obtaining a compound (IV). In this process, a reducing agent (e.g., sodium cyano borohydride) may be used simultaneously in the presence of the aldehyde (II) and the amine (III), thereby also obtaining the compound (IV).

### Step 1-1'

A commercially available amine (II') (A₁ and G₁ are the same as describe above) or a salt thereof and a compound (III') having a leaving group (n₁, W, and z₁ are the same as described above; R₁₅ represents a group such as an alkyl group, a benzyl group, etc.; and L represents a leaving group such as a halogen atom, a p-toluenesulfonyloxy group, a methanesulfonyloxy group, etc.) may be added to any organic solvent (e.g. , methanol, ethanol, toluene, etc.) to react in the presence of a base (e.g., triethylamine, potassium carbonate, etc.) at -20°C to 150°C, thereby obtaining the compound (IV).

### Step 1-2

The compound (IV) obtained in the previous step may be reacted in an organic solvent (e.g., DMF, chloroform, toluene, etc.) with any protecting reagent P₁X (P₁ represents a protecting group such as a t-butoxycarbonyl group (hereinafter, a Boc group), a benzyloxycarbonyl group (hereinafter, a Cbz gruop), 9-fluorenyloxycarbonyl group (hereinafter, an Fmoc group), etc.; and X represents a leaving group such as a halogen atom, imidazole, etc. or a group capable of forming an anhydride with P₁) at room temperature or under a heating condition, thereby obtaining a compound (V).

### Step 1-2'

A reagent (II') obtained by previously protecting a commercially available amine with P₁ (A₁, G₁, and P₁ are the same as describe above) and the compound (III') having a leaving group (n₁, W, and z₁ are the same as described above; R₁₅ represents a group such as an alkyl group, a benzyl group, etc.; and L represents a leaving group such as a halogen atom, a p-toluenesulfonyloxy group, a methanesulfonyloxy group, etc.) may be added to and reacted in any organic solvent (e.g., dimethylformamide (hereinafter, DMF), tetrahydrofuran (hereinafter, THF), chloroform, etc.) in the presence of a base (e.g., sodium hydride, potassium t-butoxide, etc.) at -20°C to 150°C, thereby obtaining the compound (V).

### Step 1-3

The compound (V) obtained in the previous step may be reacted in a mixed solution consisting of an basic aqueous solution (e.g., sodium hydroxide aqueous solution) and an organic solvent (e.g., THF, methanol, or a mixed solution thereof) at room temperature or under a heating condition, thereby obtaining a compound (VI).

### Step 1-4

A commercially available or known compound (VII) (z₂, n₂ and y are the same as described above; P₂ represents a protecting group such as a Boc group, a Cbz group, an Fmoc group, etc. ; and P₃ represents independently a protecting group similar to that of P₂ or a phthalimide group, etc.) and an amine compound HD₁D₂ may be reacted in an organic solvent, while adding a known condensation agent (e.g., dicyclohexylcarbodiimide, N-ethyl-N-(3-dimethylaminopropyl)carbodiimide (hereinafter, WSCI), chloroformate, etc.), and, if necessary, a catalyst (1-hydroxybenzotriazole (hereinafter, HOBt), dimethylaminopyridine (hereinafter, DMAP), triethylamine, etc.), at -20°C to 120°C, thereby obtaining the compound (VIII).

### Step 1-5

A compound (IX) may be obtained by removing the protecting group P₂ in the compound (VIII). For example, when P₂ is a Boc group, the reaction is terminated by adding a strong acid such as a hydrochloric acid, a trifluoroacetic acid, or the like or a weak acid such as an acetic acid, or the like to act in any solvent.

### Step 1-6

The compound obtained in Step 1-3 and the compound obtained in Step 1-5 may be reacted in an organic solvent such as DMF, chloroform, methylene chloride, etc. , while adding a condensation agent (similar to the reagent used in Step 1-4) and, if necessary, a catalyst (similar to the reagent used in Step 1-4) , at -20°C to 120°C to thereby obtain a compound (X).

### Step 1-7

A compound (XI) may be obtained by removing the protecting group P₃ in the compound (X). For example, when P₃ is a phthalimide group, the reaction is terminated by using methylamine or hydrazine hydrate to act in an organic solvent (e.g., methanol or DMF) at room temperature or under a heating condition.

### Step 1-8

The compound (XI) may be added in an organic solvent (e.g., methanol or ethanol) with any aldehyde (R₆CHO) (R₆ is the same as described above) or ketone (R₁₆R₁₇CO) (R₁₆ and R₁₇ represent groups which form R₆ in combination) and, if necessary, a catalyst (acetic acid, pyridiniumparatoluenesulfonate, a molecular sieve, etc.) to react at room temperature or under a heating condition. A reducing agent (sodium borohydride, borane-dimethylamine complex, etc.) may then be added and reacted under cooling condition of room temperature to -20°C, thereby obtaining a compound (XIIa) in which R₇ is a hydrogen atom.

Alternatively, the compound (XI) may be added in an organic solvent (e.g. , methanol or ethanol) with 2 or more equivalent amount of any aldehyde (R₆CHO) or ketone (R₁₆R₁₇CO) and, if necessary, a catalyst (acetic acid, pyridiniumparatoluenesufonate, or a molecular seive) and further added with a reducing agent (such as sodium borohydride, borane-dimethylamine complex, etc.) under cooling condition of room temperature to -20°C for reaction, thereby obtaining the compound (XIIa) in which R₆ and R₇ (R₇ is the same as described above) are the same.

### Step 1-9

When R₇ in the compound (XIIa) is a hydrogen atom, the compound may further be added in an organic solvent (e.g., methanol or ethanol) with any aldehyde (R₁₈CHO) (R₁₈ represents a same group as R₇ other than a hydrogen atom) or ketone (R₁₉R₂₀CO) (R₁₉ and R₂₀ represent groups which form R₁₈ in combination) and, if necessary, a catalyst (acetic acid, pyridiniumparatoluenesufonate, or a molecular seive) and further added with a reducing agent (such as sodium borohydride, sodium cyano borohydride, borane-dimethylamine complex, etc.) under room temperature to cooling condition of -20°C for reaction, thereby obtaining a compound (XIIb).

### Step 1-10

The compound (XI) may be added in an organic solvent (e.g., methanol or chloroform) with any isocyanate (R-N=C=Q₂), acylating agent (L₂-C (NR₅) =Q₂) (R₅ is the same as described above; and L₂ represents a leaving group such as a halogen atom, a toluenesulfonyloxy group, a methanesulfonyloxy group, 1,3-dimethylpyrazole, etc.), and, if necessary, a catalyst (such as trimehylamine, DMAP, etc.) for reaction at roometemperature or under a heating condition, thereby obtaining a compound (XIIc).

### Step 1-11

The subject compound (I) or a salt thereof may be obtained by removing the protecting group P₁ from the compound (XII). For example, when P₁ is a Boc group, a strong acid such as a hydrochloric acid, methanesulfonic acid, etc. or a weak acid such as an acetic acid, etc. may be caused to act in any solvent to terminate the reaction.

### Production Method Example 2

Fig. 2 shows a reaction scheme of Production Method Example 2.

### Step 2-1

A commercially available or known compound (XIII) (P₂, Z₂, and y are the same as described above; n₃ represents an integer of 0 to 3; and R₂₁ represents an alkyl group, an benzyl group, etc.) and an amine compound HND₁D₂ (the same as described above) may be reacted in an organic solvent, while adding a known condensation agent (e.g., carbodiimide or chloroformate) and, if necessary, a catalyst (HOBt, DMAP or triethylamine) at -20°C to 120°C, thereby obtaining a compound (XIV).

### Step 2-2

A compound (XV) may be obtained by removing the protecting group P₂ from the compound (XIV). For example, when P₂ is a Boc group, a strong acid such as a hydrochloric acid, a trifluoroacetic acid, etc. or a weak acid such as an acetic acid, etc. may be caused to act in any solvent to complete the reaction.

### Step 2-3

The compound (XV) obtained in Step 2-2 and the compound (V) obtained in Step 1-2 may be reacted in an organic solvent while adding a condensation agent (the same as the reagent used in Step 1-4) and, if necessary, a catalyst (the same as the reagent used in Step 1-4) at -20°C to 120°C, thereby obtaining a compound (XVI).

### Step 2-4

The compound (XVI) may be dissolved in an organic solvent (such as methanol, ethanol, THF, or a mixture thereof), and then be added with a reducing agent (e.g., lithium aluminum hydride, sodium borohydride-calcium chloride, etc.) to react at -20°C to 120°C, thereby obtaining a compound (XVII).

### Step 2-5

The hydroxyl group in the compound (XVII) may be converted to a leaving group L₃ (L₃ represents a halogen atom, methanesulfonyl group, toluenesulfonyl group, etc.), thereby obtaining the compound (XVI). For example, when L₃ is a sulfonic acid derivative, the compound (XVI) may be obtained by reacting the compound (XVII) with a sulfonic acid anhydride or a sulfonic acid chloride in the presence of a base, or when L₃ is a halogen atom, the compound (XVI) may be obtained by reacting the compound (XVII) with phosphorous tribromide, sulfuryl chloride, etc.

### Step 2-6

The compound (XVI) may be reacted in an organic solvent with an amine R₆R₇NH (R₆ and R₇ are the same as described above) or R₅NHC=Q₁NHR₆ (R₅, Q₁, and R₆ are the same as described above), if necessary, in the presence of a catalyst of a base, etc., thereby obtaining the compound (XII).

### Step 2-7

The subject compound (I) may be obtained by same operations as in Step 1-11.

As a nitrogen-containing compound of the present invention the following compounds can be given:
N-[(S)-1-(1-naphthyl)ethyl]-5-(2-methylbenzyl)amino-2-(S) -[4-[N-(imidazol-2-ylmethyl)aminomethyl]benzoyl]aminopentanoyl amide [Compound No. 1];
N-[(S)-1-(1-naphthyl)ethyl]-5-(2-methoxybenzyl)amino-2-(S )-[4-[N-(imidazol-2-ylmethyl)aminomethyl]benzoyl]aminopentanoy lamide [Compound No. 2];
N-[(S)-1-(1-naphthyl)ethyl]-5-isobutyl-2-(S)-[4-[N-(imida zol-2-ylmethyl)aminomethyl]benzoyl]aminopentanoylamide [Compound No. 3];
N-[(S)-1-(1-naphthyl)ethyl]-5-(2,2-dimethylpropyl)amino-2 -(S)-[4-[N-(imidazol-2-ylmethyl)aminomethyl]benzoyl]aminopentanoylamide [Compound No. 4];
N-[(S)-1-(1-naphthyl)ethyl]-5-(2-trifluoromethylbenzyl)amino-2-(S)-[4-[N-(imidazol-2-ylmethyl)aminomethyl]benzoyl]aminopentanoylamide [Compound No. 5];
N-[(S)-1-(1-naphthyl)ethyl]-5-(2-chlorobenzyl)amino-2-(S) -[4-[N-(imidazol-2-ylmethyl)aminomethyl]benzoyl]aminopentanoylamide [Compound No. 6];
N-[(S)-1-(1-naphthyl)ethyl]-5-(3-methylbenzyl)amino-2-(S) -[4-[N-(imidazol-2-ylmethyl)aminomethyl]benzoyl]aminopentanoylamide [Compound No. 7];
N-[(S)-1-(1-naphthyl)ethyl]-5-(4-methylbenzyl)amino-2-(S) -[4-[N-(imidazol-2-ylmethyl)aminomethyl]benzoyl]aminopentanoylamide [Compound No. 8];
N-[(S)-1-(1-naphthyl)ethyl]-5-(3-methylpyridin-2-yl)methylamino-2-(S)-[4-[N-(imidazol-2-ylmethyl)aminomethyl]benzoyl]aminopentanoylamide [Compound No. 9];
N-[(S)-1-(1-naphthyl)ethyl]-5-(5-methylpyridin-2-yl)methylamino-2-(S)-[4-[N-(imidazol-2-ylmethyl)aminomethyl]benzoyl]aminopentanoylamide [Compound No. 10];
N-[(S)-1-(1-naphthyl)ethyl]-5-(4-methylpyridin-2-yl)methylamino-2-(S)-[4-[N-(imidazol-2-ylmethyl)aminomethyl]benzoyl]aminopentanoylamide [Compound No. 11];
N-[(S)-1-(1-naphthyl)ethyl]-5-(2,6-dimethoxybenzyl)amino-2-(S)-[4-[N-(imidazol-2-ylmethyl)aminomethyl]benzoyl]aminopentanoylamide [Compound No. 12];
N-[(S)-1-(1-naphthyl)ethyl]-5-(3-methylthiophen-2-yl)methylamino-2-(S)-[4-[N-(imidazol-2-ylmethyl)aminomethyl]benzoyl]aminopentanoylamide [Compound No. 13];
N-[(S)-1-(1-naphthyl)ethyl]-5-(3-methoxypyridin-2-yl)methylamino-2-(S)-[4-[N-(imidazol-2-ylmethyl)aminomethyl]benzoyl]aminopentanoylamide [Compound No. 14];
N-[(S)-1-(1-naphthyl)ethyl]-5-(2-dimethylaminobenzyl)amino-2-(S)-[4-[N-(imidazol-2-ylmethyl)aminomethyl]benzoyl]aminopentanoylamide [Compound No. 15];
N-[(S)-1-(1-naphthyl)ethyl]-5-(3-n-propyloxypyridin-2-yl) methylamino-2-(S)-[4-[N-(imidazol-2-ylmethyl)aminomethyl]benzoyl]aminopentanoylamide [Compound No. 16];
N-[(S)-1-(1-naphthyl)ethyl]-5-(3-ethoxypyridin-2-yl)methylamino-2-(S)-[4-[N-(imidazol-2-ylmethyl)aminomethyl]benzoyl]aminopentanoylamide [Compound No. 17];
N-[(S)-1-(1-naphthyl)ethyl]-5-[2-(2-hydroxyethoxy)benzyl] amino-2-(S)-(4-[N-(imidazol-2-ylmethyl)aminomethyl]benzoyl]aminopentanoylamide [Compound No. 18];
N-[(S)-1-(1-naphthyl)ethyl]-5-(2-trifluoromethoxybenzyl)amino-2-(S)-[4-[N-(imidazol-2-ylmethyl)aminomethyl]benzoyl]aminopentanoylamide [Compound No. 19];
N-[(S)-1-(1-naphthyl)ethyl]-5-(2-hydroxybenzyl)amino-2-(S )-[4-[N-(imidazol-2-ylrnethyl)aminomethyl]benzoyl]aminopentanoylamide [Compound No. 20];
N-[(S)-1-(1-naphthyl)ethyl]-5-(3-isopropyloxypyridin-2-yl )methylamino-2-(S)-[4-[N-(imidazol-2-ylmethyl)aminomethyl]benzoyl]aminopentanoylamide [Compound No. 21];
N-[(S)-1-(1-naphthyl)ethyl]-5-(2-ethylbenzyl)amino-2-(S)-[4-[N-(imidazol-2-ylmethyl)aminomethyl]benzoyl]aminopentanoylamide [Compound No. 22];
N-[(S)-1-(1-naphthyl)ethyl]-5-(2-isopropyloxybenzyl)amino-2-(S)-[4-[N-(imidazol-2-ylmethyl)aminomethyl]benzoyl]aminopentanoylamide [Compound No. 23];
N-[(S)-1-(1-naphthyl)ethyl]-5-(2-morpholinobenzyl)amino-2 -(S)-[4-[N-(imidazol-2-ylmethyl)aminomethyl)benzoyl]aminopentanoylamide [Compound No. 24];
N-[(S)-1-(1-naphthyl)ethyl]-5-(2-(4-methylpiperazino)benzyl)amino-2-(S)-[4-[N-(imidazol-2-ylmethyl)aminomethyl]benzoyl] aminopentanoylamide [Compound No. 25];
N-[(S)-1-(1-naphthyl)ethyl]-5-(3-methylpyrrol-2-yl)methylamino-2-(S)-[4-[N-(imidazol-2-ylmethyl)aminomethyl]benzoyl]aminopentanoylamide [Compound No. 26];
N-[(S)-1-(1-naphthyl)ethyl]-5-cyclohexylamino-2-(S)-[4-[N -(imidazol-2-ylmethyl)aminomethyl]benzoyl]aminopentanoylamide[Compound No. 27];
N-[(S)-1-(1-naphthyl)ethyl]-5-(1,2,3,4-tetrahydronaphthalen-1-yl)amino-2-(S)-[4-[N-(imidazol-2-ylmethyl)aminomethyl]benzoyl]aminopentanoylamide [Compound No. 28];
N-[(S)-1-(1-naphthyl)ethyl]-5-(indan-1-yl)amino-2-(S)-[4-[N-(imidazol-2-ylmethyl)aminomethyl]benzoyl)aminopentanoylamide [Compound No. 29];
N-[(S)-1-(1-naphthyl)ethyl]-5-(1-methylpiperidin-4-yl)amino-2-(S)-[4-[N-(imidazol-2-ylmethyl)aminomethyl]benzoyl]aminopentanoylamide [Compound No. 30];
N-[(S)-1-(1-naphthyl)ethyl]-5-(pentan-3-yl)amino-2-(S)-[4-[N-(imidazol-2-ylmethyl)aminomethyl]benzoyl]aminopentanoylamide [Compound No. 31];
N-[(S)-1-(1-naphthyl)ethyl]-5-dimethylamino-2-(S)-[4-[N-(imidazol-2-ylmethyl)aminomethyl]benzoyl]aminopentanoylamide [Compound No. 32];
N-[(S)-1-(1-naphthyl)ethyl]-5-diisobutylamino-2-(S)-[4-[N-(imidazol-2-ylmethyl)aminomethyl]benzoyl]aminopentanoylamide [Compound No. 33];
N-[(S)-1-(1-naphthyl)ethyl]-5-di-n-propylamino-2-(S)-[4-[ N-(imidazol-2-ylmethyl)aminomethyl]benzoyl]aminopentanoylamide [Compound No. 34];
N-[(S)-1-(1-naphthyl)ethyl]-5-di-n-butylamino-2-(S)-[4-[N-(imidazol-2-ylmethyl)aminomethyl]benzoyl]aminopentanoylamide [Compound No. 35];
N-[(S)-1-(1-naphthyl)ethyl]-5-[N-methyl-(3-methylpyridin-2-yl)methylamino]-2-(S)-[4-[N-(imidazol-2-ylmethyl)arninomethyl ]benzoyl]aminopentanoylamide [Compound No. 36];
N-[(S)-1-(1-naphthyl)ethyl]-5-[N-methyl-(2-methoxybenzyl) amino]-2-(S)-[4-[N-(imidazol-2-ylmethyl)aminomethyl]benzoyl]aminopentanoylamide [Compound No. 37];
N-[(S)-1-(1-naphthyl)ethyl]-5-(N-methyl-isobutylamino)-2-(S)-[4-[N-(imidazol-2-ylmethyl)aminvmethyl]benzoyl]aminopentanoylamide [Compound No. 38];
N-[(S)-1-(1-naphthyl)ethyl]-5-(N-n-propyl-isobutylamino)-2-(S)-[4-[N-(imidazol-2-ylmethyl)aminomethyl]benzoyl]aminopentanoylamide [Compound No. 39];
N-[(S)-1-(1-naphthyl)ethyl]-5-(N-ethyl-isobutylamino)-2-(S)-[4-[N-(imidazol-2-ylmethyl)aminomethyl]benzoyl]aminopentanoylamide [Compound No. 40];
N-[(S)-1-(1-naphthyl)ethyl]-5-(N-isopropyl-isobutylamino) -2-(S)-[4-[N-(imidazol-2-ylmethyl)aminomethyl]benzoyl]aminopentanoylamide [Compound No. 41];
N-[(S)-1-(1-naphthyl)ethyl]-5-(N-methyl-cyclohexylamino)-2-(S)-[4-[N-(imidazol-2-ylmethyl)aminomethyl]benzoyl]aminopentanoylamide [Compound No. 42];
N-[(S)-1-(1-naphthyl)ethyl]-5-(N-methyl-cyclopentylamino)-2-(S)-[4-[N-(imidazol-2-ylmethyl)aminomethyl]benzoyl]aminopentanoylamide [Compound No. 43];
N-[(S)-1-(1-naphthyl)ethyl]-5-(N-n-propyl-isopropylamino)-2-(S)-[4-[N-(imidazol-2-ylmethyl)aminomethyl]benzoyl]aminopentanoylamide [Compound No. 44];
N-[(S)-1-(1-naphthyl)ethyl]-5-di-n-propylamino-2-(S)-[4-[N-(pyridin-2-ylmethyl)aminomethyl]benzoyl]aminopentanoylamide[Compound No. 45];
N-[(S)-1-(1-naphthyl)ethyl]-5-diisobutylamino-2-(S)-[4-[N-(pyridin-2-ylmethyl)aminomethyl]benzoyl]aminopentanoylamide [Compound No. 46];
N-(1-naphthylmethyl)-5-(3-methylpyridin-2-yl)methylamino-2-(S)-[4-[N-(imidazol-2-ylmethyl)aminomethyl]benzoyl]aminopentanoylamide [Compound No. 47];
N-(1-naphthylmethyl)-5-diisobutylamino-2-(S)-[4-[N-(imidazol-2-ylmethyl)aminomethyl]benzoyl]aminopentanoylamide [Compound No. 48];
N-(1-naphthylmethyl)-5-(N-methyl-cyclohexylamino)-2-(S)-[ 4-[N-(imidazol-2-ylmethyl)aminomethyl]benzoyl]aminopentanoylamide [Compound No. 49];
N-[(S)-1-(1-naphthyl)ethyl]-5-(2-ethoxybenzyl)amino-2-(S) -[4-[N-(imidazol-2-ylmethyl)aminomethyl]benzoyl]aminopentanoylamide [Compound No. 50];
N-[(S)-1-(1-naphthyl)ethyl]-5-diethylamino-2-(S)-[4-[N-(imidazol-2-ylmethyl)aminomethyl]benzoyl]aminopentanoylamide [Compound No. 51];
N-[(S)-1-(1-naphthyl)ethyl]-5-(2-phenylpropan-2-ylamino)-2-(S)-[4-[N-(imidazol-2-ylmethyl)aminomethyl]benzoyl]aminopentanoylamide [Compound No. 52];
N-[(S)-1-(1-naphthyl)ethyl]-5-[2-(2-methoxyphenyl)ethyl]amino-2-(S)-[4-[N-(imidazol-2-ylmethyl)aminomethyl]benzoyl]aminopentanoylamide [Compound No. 53];
N-[(S)-1-(1-naphthyl)ethyl]-5-(1,2,3,4-tetrahydroisoquinolin-2-yl)-2-(S)-[4-[N-(imidazol-2-ylmethyl)aminomethyl]benzoyl ]aminopentanoylamide [Compound No. 54];
N-[(S)-1-(1-naphthyl)ethyl]-5-(hexamethyleneimin-1-yl)-2-(S)-[4-[N-(imidazol-2-ylmethyl)aminomethyl]benzoyl]aminopentanoylamide [Compound No. 55];
N-[(S)-1-(1-naphthyl)ethyl]-5-(heptamethyleneimin-1-yl)-2 -(S)-[4-[N-(imidazol-2-ylmethyl)aminomethyl]benzoyl]aminopentanoylamide [Compound No. 56];
N-[(S)-1-(1-naphthyl)ethyl]-5-morpholino-2-(S)-[4-[N-(imidazol-2-ylmethyl)aminomethyl]benzoyl]aminopentanoylamide [Compound No. 57];
N-[(S)-1-(1-naphthyl)ethyl]-5-piperidino-2-(S)-[4-[N-(imidazol-2-ylmethyl)aminomethyl]benzoyl]aminopentanoylamide [Compound No. 58];
N-[(S)-1-(1-naphthyl)ethyl]-5-(pyrrolidin-1-yl)-2-(S)-[4-[N-(imidazol-2-ylmethyl)an)inomethyl]benzoyl]aminopentanoylamide [Compound No. 59];
N-[(S)-1-(1-naphthyl)ethyl]-5-bis(2-methoxyethyl)amino-2-(S)-[4-[N-(imidazol-2-ylmethyl)aminomethyl]benzoyl]aminopentanoylamide [Compound No. 60];
N-[(S)-1-(1-naphthyl)ethyl]-5-bis(2-hydroxyethyl)amino-2-(S)-[4-[N-(imidazol-2-ylmethyl)aminomethyl]benzoyl]aminopentanoylamide [Compound No. 61];
N-[(S)-1-(1-naphthyl)ethyl]-5-(N-n-propyl-(2-methoxyethyl )amino)-2-(S)-[4-[N-(imidazol-2-ylmethyl)aminomethyl]benzoyl]aminopentanoylamide [Compound No. 62];
N-[(S)-1-(1-naphthyl)ethyl]-5-(N-isobutyl-(2-methoxyethyl )amino)-2-(S)-[4-[N-(imidazol-2-ylmethyl)aminomethyl]benzoyl]aminopentanoylamide [Compound No. 63];
N-[(S)-1-(1-naphthyl)ethyl]-5-(N-isopropyl-(2-methoxyethyl)amino)-2-(S)-[4-[N-(imidazol-2-ylmethyl)aminomethyl]benzoyl] aminopentanoylamide [Compound No. 64];
N-[(S)-1-(1-naphthyl)ethyl]-5-(N-n-propyl-(2-hydroxyethyl )amino)-2-(S)-[4-[N-(imidazol-2-ylmethyl)aminomethyl]benzoyl]aminopentanoylamide [Compound No. 65];
N-[(S)-1-(1-naphthyl)ethyl]-5-(N-isobutyl-(2-hydroxyethyl )amino)-2-(S)-[4-[N-(imidazol-2-ylmethyl)aminomethyl]benzoyl]aminopentanoylamide [Compound No. 66];
N-[(S)-1-(1-naphthyl)ethyl]-5-(N-isopropyl-(2-hydroxyethyl)amino)-2-(S)-[4-[N-(imidazol-2-ylmethyl)aminomethyl]benzoyl] aminopentanoylamide [Compound No. 67];
N-[(S)-1-(1-naphthyl)ethyl]-5-(hexamethyleneimin-1-yl)-2-(S)-[4-[N-(pyridin-2-ylmethyl)aminomethyl]benzoyl]aminopentanoylamide [Compound No. 68];
N-[(S)-1-(1-naphthyl)ethyl]-5-(bis(2-hydroxyethyl)amino)-2-(S)-[4-[N-(pyridin-2-ylmethyl)aminomethyl]benzoyl]aminopentanoylamide [Compound No. 69];
N-[(S)-1-(1-naphthyl)ethyl]-5-(N-isobutyl(2-hydroxyethyl)amino)-2-(S)-[4-[N-(pyridin-2-ylmethyl)aminomethyl]benzoyl]aminopentanoylamide [Compound No. 70];
N-[(S)-1-(1-naphthyl)ethyl]-5-ureide-2-(S)-[4-[N-(imidazol-2-ylmethyl)aminomethyl]benzoyl]aminopentanoylamide [Compound No. 71];
N-[ (S)-1-(1-naphthyl)ethyl]-5-(3-phenylthioureide)-2-(S)-[4-[N-(pyridin-2-ylmethyl)aminomethyl]benzoyl]aminopentanoylamide [Compound No. 72];
N-[(S)-1-(1-naphthyl)ethyl]-5-sulfinamidino-2-(S)-[4-[N-(pyridin-2-ylmethyl)aminomethyl]benzoyl]aminopentanoylamide [Compound No. 73];
N-[(S)-1-(1-naphthyl)ethyl]-5-(N-methylsulfinamidino)-2-(S)-[4-[N-(pyridin-2-ylmethyl)aminomethyl]benzoyl]aminopentanoylamide [Compound No. 74];
N-[(S)-1-(1-naphthyl)ethyl]-5-(N,N'-dimethylsulfinamidino )-2-(S)-[4-[N-(pyridin-2-ylmethyl)aminomethyl]benzoyl]aminopentanoylamide [Compound No. 75];
N-[(S)-1-(1-naphthyl)ethyl]-5-sulfinamidino-2-(S)-[4-[N-(imidazol-2-ylmethyl)aminomethyl]benzoyl]aminopentanoylamide [Compound No. 76];
N-[(S)-1-(1-naphthyl)ethyl]-5-(N-methylsulfinamidino)-2-(S)-[4-[N-(imidazol-2-ylmethyl)aminomethyl]benzoyl]aminopentanoylamide [Compound No. 77];
N-[(S)-1-(1-naphthyl)ethyl]-5-(N,N'-dimethylsulfinamidino )-2-(S)-[4-[N-(imidazol-2-ylmethyl)aminomethyl]benzoyl]aminopentanoylamide [Compound No. 78];
N-[(S)-1-(naphthyl)ethyl]-5-[N-methyl-(pentan-3-yl)amino]-2-(S)-[4-[N-(imidazol-2-ylmethyl)aminomethyl]benzoyl]aminopentanoylamide [Compound No. 79];
N-[(S)-1-(naphthyl)ethyl]-5-[N-ethyl-(pentan-3-yl)amino]-2-(S)-[4-[N-(imidazol-2-ylmethyl)aminomethyl]benzoyl]aminopentanoylamide [Compound No. 80];
N-[(S)-1-(naphthyl)ethyl]-5-[N-n-propyl-(pentan-3-yl)amino]-2-(S)-[4-[N-(imidazol-2-ylmethyl)aminomethyl]benzoyl]aminopentanoylamide [Compound No. 81];
N-[(S)-1-(naphthyl)ethyl]-5-[N-carboxymethyl(isobutyl)amino]-2-(S)-{4-[N-(1H-imidazol-2-ylmethyl)aminomethyl]benzoyl}aminopentanoylamide [Compound No. 82];
N-[(S)-1-(naphthyl)ethyl]-5-[N-carbamoylmethyl(isobutyl)amino]-2-(S)-{4-[N-(1H-imidazol-2-ylmethyl)aminomethyl]benzoyl} aminopentanoylamide [Compound No. 83];
N-[(S)-1-(naphthyl)ethyl]-5-[N-methoxycarbonylmethyl(isobutyl)amino]-2-(S)-{4-[N-(1H-imidazol-2-ylmethyl)aminomethyl]benzoyl}aminopentanoylamide [Compound No. 84];
N-[(1S)-4-[(2-hydroxyethyl)isobutylamino]-1-{[(S)-1-(naphthalen-1-yl)ethyl]carbamoyl}butyl]-4-{[(1-methyl-1H-imidazol-2 -ylmethyl)amino]methyl}benzamide [Compound No. 85];
N-[(1S)-4-dipropylamino-1-{[(S)-1-(naphthalen-1-yl)ethyl] carbamoyl}butyl]-4-{[(1-methyl-1H-imidazol-2-ylmethyl)amino]methyl}benzamide [Compound No. 86];
N-[(1S)-4-dipropylamino-1-{[(S)-1-(naphthalen-1-yl)ethyl] carbamoyl}butyl]-4-{[(1-methyl-1H-benzoimidazol-2-ylmethyl)amino]methyl}benzamide [Compound No. 87];
N-[(1S)-4-dipropylamino-1-{[(S)-1-(naphthalen-1-yl)ethyl] carbamoyl}butyl]-4-{[(1-methyl-1H-imidazol-2-ylmethyl)methylamino]methyl}benzamide [Compound No. 88];
N-[(1S)-4-[(2-hydroxyethyl)isobutylamino]-1-{[(S)-1-(naphthalen-1-yl)ethyl]carbamoyl}butyl]-4-{[(1-methyl-1H-imidazol-2 -ylmethyl)methylamino]methyl}benzantide [Compound No. 89];
N-[(1S)-4-[(2-hydroxyethyl)isobutylamino]-1-{[(S)-1-(naphthalen-1-yl)ethyl]carbamoyl}butyl]-4-{[(1-methyl-1H-benzoimidazol-2-ylmethyl)amino]methyl}benzamide [Compound No. 90];
N-[(1S)-4-[(2-hydroxyethyl)isobutylamino]-1-{[(S)-1-(naphthalen-1-yl)ethyl]carbamoyl}butyl]-4-([(1-methyl-1H-imidazol-2 -ylmethyl)methylamino]methyl}benzamide [Compound No. 91];
N-[(1S)-4-dipropylamino-1-{[(S)-1-(naphthalen-1-yl)ethyl] carbamoyl}butyl]-4-{[(1-methyl-1H-imidazol-2-ylmethyl)methylamino]methyl}benzamide [Compound No. 92];
N-[(1S)-4-[(2-hydroxyethyl)isobutylamino]-1-([(S)-1-(naphthalen-1-yl)ethyl]carbamoyl}butyl]-4-{[(furan-2-ylmethyl)amino ]methyl)benzamide [Compound No. 93];
N-[(1S)-4-isobutylmethanesulfonylamino-1-{[(S)-1-(naphthalen-1-yl)ethyl]carbamoyl}butyl]-4-{[(1H-imidazol-2-ylmethyl)amino]methyl}benzamide [Compound No. 94];
N-[(1S)-4-dipropylamino-1-{[(S)-1-(naphthalen-1-yl)ethyl]carbamoyl}butyl]-4-{[(1-ethyl-1H-imidazol-2-ylmethyl)amino]methyl}benzamide [Compound No. 95];
N-[(1S)-4-dipropylamino-1-{[(S)-1-(naphthalen-1-yl)ethyl]carbamoyl}butyl]-4-{[(1-propyl-1H-imidazol-2-ylmethyl)amino]methyl}benzamide [Compound No. 96];
5-{[(1-methyl-1H-imidazol-2-ylmethyl)amino]methyl}thiophene-2-carboxylic-[(1S)-4-[(3-methylpyridin-2-ylmethyl)amino]-1-{[(S)-1-(naphthalen-1-yl)ethyl]carbamoyl}butyl]amide [Compound No. 97];
5-{[(1-methyl-1H-imidazol-2-ylmethyl)amino]methyl}thiophene-2-carboxylic-[(1S)-4-[(2-methyoxybenzylamino)-1-{[(S)-1-(naphthalen-1-yl)ethyl]carbamoyl}butyl]amide [Compound No. 98];
5-{[(1-methyl-1H-imidazol-2-ylmethyl)amino]methyl}thiophene-2-carboxylic-[(1S)-4-(1-ethylpropylamino)-1-{[(S)-1-(naphthalen-1-yl)ethyl]carbamoyl}butyl]amide [Compound No. 99];
N-{(1S)-4-dipropylamino-1-[2-(1H-indol-3-yl)ethylcarbamoyl]butyl}-4-{[(1H-imidazol-2-ylmethyl)amino]methyl}benzamide [Compound No. 100];
4-{[(1H-imidazol-2-ylmethyl)amino]methyl}naphthalen-1-carboxylic-{(1S)-4-dipropylamino-1-[2-(1H-indol-3-yl)ethylcarbamoyl]butyl}amide [Compound No. 101];
N-{(1S)-4-dipropylamino-1-[2-(1H-indol-3-yl)ethylcarbamoyl]butyl}-4-{[(1-methyl-1H-imidazol-2-ylmethyl)amino]methyl}benzamide [Compound No. 102];
5-{[(1-methyl-1H-imidazol-2-ylmethyl)amino]methyl}thiophene-2-carboxylic-{(1S)-4-dipropylamino-1-[2-(1H-indol-3-yl)ethylcarbamoyl]butyl}amide [Compound No. 103];
N-{(1S)-4-diprapylamino-1-[2-(1H-indol-3-yl)ethylcarbamoyl]butyl}-4-{[(pyridin-2-ylmethyl)amino]methyl}benzamide [Compound No. 104];
4-{[(pyridin-2-ylmethyl)amino]methyl}naphthalen-1-carboxylic-{(1S)-4-dipropylamino-1-[2-(1H-indol-3-yl)ethylcarbamoyl]butyl}amide [Compound No. 105];
N-{(1S)-4-dipropylamino-1-[2-(1H-indol-3-yl)ethylcarbamoyl]butyl}-6-{[(pyridin-2-ylmethyl)amino]methyl}nicotinamide [Compound No. 106];
8-{[(pyridin-2-ylmethyl)amino]methyl}isoquinoline-5-carboxylic-{(1S)-4-dipropylamino-1-[2-(1H-indol-3-yl)ethylcarbamoyl ]butyl}amide [Compound No. 107];
N-[(1S)-4-dipropylamino-1-{[(S)-1-(naphthalen-1-yl)ethyl]carbamoyl}butyl]-4-({[1-(2-hydroxyethyl)-1H-imidazol-2-ylmethyl]amino}methyl)benzamide [Compound No. 108];
4-{[(1-methyl-1H-imidazol-2-ylmethyl)amino]methyl}naphthalen-1-carboxylic-[(1S)-4-[(3-methylpyridin-2-ylmethyl)amino]-1 -{[(S)-1-(naphthalen-1-yl)ethyl]carbamoyl}butyl]amide [Compound No. 109] ;
4-{[(1-methyl-1H-imidazol-2-ylmethyl)amino]methyl}naphthalen-1-carboxylic-[(1S)-4-(2-methoxybenzylamino)-1-{[(S)-1-(naphthalen-1-yl)ethyl]carbamoyl}butyl]amide [Compound No. 110];
4-{[(1-methyl-1H-imidazol-2-ylmethyl)amino]methyl}naphthalen-1-carboxylic-[(1S)-4-(1-ethylpropylamino)-1-{[(S)-1-(naphthalen-1-yl)ethyl]carbamoyl}butyl]amide [Compound No. 111];
4-{[bis(pyridin-2-ylmethyl)amino]methyl}-N-[(1S)-4-[2-hydroxyethyl)isobutylamino]-1-{[(S)-1-(naphthalen-1-yl)ethyl]carbamoyl}butyl]benzamide [Compound No. 112];
N-[(1S)-4-dipropylamino-1-(3-isopropoxypropylcarbamoyl)butyl]-4-{[(1-methyl-1H-imidazol-2-ylmethyl)amino]methyl}benzamide [Compound No. 113];
4-{[(1-methyl-1H-imidazol-2-ylmethyl)amino]methyl}naphthalen-1-carboxylic-[(1S)-4-dipropylamino-1-(3-isopropoxypropylcarbamoyl)butyl]amide [Compound No. 114];
N-[(1S)-4-dipropylamino-1-(3-isopropoxypropylcarbamoyl)butyl)-4-{[(1H-imidazol-2-ylmethyl)amino]methyl}benzamide [Compound No. 115];
5-{[(1H-imidazol-2-ylmethyl)amino]methyl}thiophene-2-carboxylic-{(1S)-4-dipropylamino-1-[2-(1H-indol-3-yl)ethylcarbamoyl]butyl}amide [Compound No. 116];
5-{[(pyridin-2-ylmethyl)amino]methyl}thiophene-2-carboxylic-{(1S)-4-dipropylamino-1-[2-(1H-indol-3-yl)ethylcarbamoyl]butyl}amide [Compound No. 117];
N-{(1S)-4-dipropylamino-1-[2-(1H-indol-3-yl)ethylcarbamoyl]butyl}-4-{[(pyridin-2-ylmethyl)amino]methyl}benzamide [Compound No. 118);
4-{[(1-methyl-1H-imidazol-2-ylmethyl)amino]methyl}naphthalen-1-carboxylic-{(1S)-4-dipropylamino-1-[2-(1H-indol-3-yl)ethylcarbamoyl]butyl}amide [Compound No. 119];
N-[(1S)-4-(4,5-dihydro-1H-imidazol-2-ylsulfanyl)-1-{[(S)-1-(naphthalen-1-yl)ethyl]carbamoyl}butyl]-4-{[(pyridin-2-ylmethyl)amino]methyl}benzamide [Compound No. 120];
N-[(1S)-4-(1H-imidazol-2-ylsulfanyl)-1-{[(S)-1-(naphthalen-1-yl)ethyl]carbamoyl}butyl]-4-{[(1H-imidazol-2-ylmethyl)amino]methyl}benzamide [Compound No. 121];
N-[(1S)-4-dipropylamino-1-(isopropylcarbamoyl)butyl]-4-{((1-methyl-1H-imidazol-2-ylmethyl)amino]methyl}benzamide [Compound No. 122];
N-[(1S)-4-dipropylamino-1-(isopropylcarbamoyl)butyl]-4-{[ (pyridin-2-ylmethyl)amino]methyl}benzamide [Compound No. 123];
4-{[bis(1H-imidazol-2-ylmethyl}amino]methyl}-N-[(1S)-4-[(2-hydroxyethyl)isobutylamino]-1-{[(S)-1-(naphthalen-1-yl)ethyl ]carbamoyl}butyl]benzamide [Compound No. 124];
N-[(1S)-4-dipropylamino-1-(isopropylcarbamoyl)butyl]-4-{[(1H-imidazol-2-ylmethyl)amino]methyl}benzamide [Compound No. 125];
4-{[(1H-imidazol-2-ylmethyl)amino]methyl}-N-[(1S)-2-(3H-imidazol-4-yl)-1-{[(S)-1-(naphthalen-1-yl)ethyl]carbamoyl}ethyl ]benzamide [Compound No. 126];
4-{[(1H-imidazol-2-ylmethyl)amino]methyl}-N-[(1S)-2-(1H-indol-2-yl)-1-{[(S)-1-(naphthalen-1-yl)ethyl]carbamoyl}ethyl]benzamide [Compound No. 127];
N-[(1S)-4-dipropylamino-1-(3-phenylpropylcarbamoyl)butyl]-4-{[(1H-imidazol-2-ylmethyl)amino]methyl}benzamide [Compound No. 128];
N-[(1S)-4-dipropylamino-1-(3-phenylpropylcarbamoyl)butyl]-4-{[(1-methyl-1H-imidazol-2-ylmethyl)amino]methyl}benzamide [Compound No. 129];
N-[(1S)-4-dipropylamino-1-(3-phenylpropylcarbamoyl)butyl]-4-{[(pyridin-2-ylmethyl)amino]methyl}benzamide [Compound No. 130];
4-{[(1H-imidazol-2-ylmethyl)amino]methyl}-N-({[(S)-1-(naphthalen-1-yl)ethyl]carbamoyl}methyl)benzamide [Compound No. 131];
4-{[(1H-imidazol-2-ylmethyl)amino]methyl}-N-[(1S)-2-methyl-1-{[(S)-1-(naphthalen-1-yl)ethyl]carbamoyl}propyl]benzamide [Compound No. 132];
N-[(1S)-4-dipropylamino-1-{[(naphthalen-1-ylmethyl)carbamoyl]butyl}-4-{[(1H-imidazol-2-ylmethyl)amino]methyl}benzamide [Compound No. 133];
4-{[(1H-imidazol-2-ylmethyl)amino]methyl}-N-[(1S)-4-[(1H-imidazol-2-ylmethyl)amino]-1-{[(S)-1-(naphthalen-1-yl)ethyl]carbamoyl}butyl]benzamide [Compound No. 134];
4-{[(pyridin-2-ylmethyl)amino]methyl}-N-[(1S)-4-[(1H-imidazol-2-ylmethyl)amino]-1-{[(S)-1-(naphthalen-1-yl)ethyl]carbamoyl}butyl]benzamide [Compound No. 135];
N-[(1S)-4-(2-hydroxyethylamino)-1-{[(S)-1-(naphthalen-1-yl)ethyl]carbamoyl}butyl]-4-{[(1-methyl-1H-imidazol-2-ylmethyl) amino]methyl}benzamide [Compound No. 136];
N-[(1S)-4-(2-hydroxyethylamino)-1-{[(S)-1-(naphthalen-1-yl)ethyl]carbamoyl}butyl]-4-{[(1H-imidazol-2-ylmethyl)amino]methyl}benzamide [Compound No. 137];
4-{[(pyridin-2-ylmethyl)amino]methyl}naphthalen-1-carboxylic-[(1S)-4-(4,5-dihydro-1H-imidazol-2-ylamino)-1-{[(S)-1-(naphthalen-1-yl)ethyl]carbamoyl}butyl]amide [Compound No. 138];
N-[(1S)-4-(4,5-dihydro-1H-imidazol-2-ylamio)-1-{[(S)-1-(naphthalen-1-yl)ethyl]carbamoyl}butyl]-4-({[1-methyl-1H-imidazol-2-ylmethyl]amino}methyl)benzamide [Compound No. 139];
4-{[(1H-imidazol-2-ylmethyl)amino]methyl}naphthalen-1-carboxylic-[(1S)-4-cyclohexylamino-1-{[(S)-1-(naphthalen-1-yl)ethyl]carbamoyl}butyl]amide [Compound No. 140];
N-[(1S)-4-cyclohexylamino-1-{[(S)-1-(naphthalen-1-yl)ethyl]carbamoyl}butyl]-4-({[1-(2-hydroxyethyl)-1H-imidazol-2-ylmethyl]amino}methyl)benzamide [Compound No. 141·];
4-{[(1-methyl-1H-imidazol-2-ylmethyl)amino]methyl}naphthalen-1-carboxylic-[(1S)-4-methylcarbamimidoylsulfanyl-1-{[(S)-1 -(naphthalen-1-yl)ethyl]carbamoyl}butyl]amide [Compound No. 142];
4-{[(1H-imidazol-2-ylmethyl)amino]methyl}-N-[(1S)-1-{[(S)-1-(naphthalen-1-yl)ethyl]carbamoyl}-(2-phenyl)ethyl]benzamide [Compound No. 143];
4-{[(1-methyl-1H-imidazol-2-ylmethyl)amino]methyl}-N-[(1S )-4-[(1H-imidazol-2-ylmethyl)amino]-1-{[(S)-1-(naphthalen-1-yl )ethyl]carbamoyl}butyl]benzamide [Compound No. 144];
4-{[(1-methyl-1H-imidazol-2-ylmethyl)amino]methyl}-N-[(1S)-4-[(1-methyl-1H-imidazol-2-ylmethyl)amino]-1-{[(S)-1-(naphthalen-1-yl)ethyl]carbamoyl}butyl]benzamide [Compound No. 145];
4-({[1-(2-hydroxyethyl)-1H-imidazol-2-ylmethyl]amino}methyl)naphthalen-1-carboxylic-[(1S)-4-cyclohexylamino-1-{[(S)-1-(naphthalen-1-yl)ethyl]carbamoyl}butyl]amide [Compound No. 146];
4-{[(1-methyl-1H-imidazol-2-ylmethyl)amino]methyl}-N-[(1S )-4-methylcarbamimidoylsulfanyl-1-{[(S)-1-(naphthalen-1-yl)ethyl]carbamoyl}butyl]benzamide [Compound No. 147];
4-{[(1H-imidazol-2-ylmethyl)amino]methyl}-N-[(1S)-4-[(1-methyl-1H-imidazol-2-ylmethyl)amino]-1-{[(S)-1-(naphthalen-1-yl )ethyl]carbamoyl}butyl]benzamide [Compound No. 148];
N-[(1S)-4-[(3-methylpyridin-2-ylmethyl)amino-1-{[(S)-1-(naphthalen-1-yl)ethyl]carbamoyl}butyl]-4-({[1-(2-hydroxyethyl)-1H-imidazol-2-ylmethyl]amino}methyl)benzamide[Compound No.149];
N-{(1S)-4-dipropylamino-1-[(1H-indol-3-ylmethyl)carbamoyl ]butyl}-4-{[(1H-imidazol-2-ylmethyl)amino]methyl}benzamide [Compound No. 150];
N-[(1S)-4-dipropylamino-1-{[(S)-1-(naphthalen-1-yl)ethyl]carbamoyl}butyl]-4-{[(1-oxypyridin-2-ylmethyl)amino]methyl}benzamide [Compound No. 151];
N-[(1S)-4-(4,5-dihydro-1H-imidazol-2-ylamino)-1-{[(S)-1-(naphthalen-1-yl)ethyl]carbamoyl}butyl]-4-({[1-(2-hydroxyethyl) -1H-imidazol-2-ylmethyl]amino}methyl)benzamide [Compound No. 152];
(2S)-5-dipropylamino-2-(4-{[(1-methyl-1H-imidazol-2-ylmethyl)amino]methyl}benzenesulfonylamino)pentanoylic-[(S)-1-(naphthalen-1-yl)ethyl]amide [Compound No. 153];
(2S)-5-dipropylamino-2-(4-{[(1H-imidazol-2-ylmethyl)amino ]methyl}benzenesulfonylamino)pentanoylic-[(S)-1-(naphthalen-1-yl)ethyl]amide [Compound No. 154];
N-[(1S)-4-dipropylamino-1-{[1-(4-fluoronaphthalen-1-yl)ethyl]carbamoyl}butyl]-4-{[(1H-imidazol-2-ylmethyl)amino]methyl} benzamide [Compound No. 155];
N-[(1S)-4-dipropylamino-1-{[1-(4-fluoronaphthalen-1-yl)ethyl]carbamoyl}butyl]-4-{[(1-methyl-1H-imidazol-2-ylmethyl)amino]methyl}benzamide [Compound No. 156];
N-[(1S)-4-dipropylamino-1-((S)-1-naphthalen-2-ylethylcarbamoyl)butyl]-4-{[(1H-imidazol-2-ylmethyl)amino]methyl}benzamide [Compound No. 157];
4-{[bis(1H-imidazol-2-ylmethyl)amino]methyl}-N-{(1S)-4-dipropylamino-1-[2-(1H-indol-3-yl)ethylcarbamoyl]butyl}benzamide [Compound No. 158];
N-{(1S)-4-dipropylamino-1-[(2-methoxynaphthalen-1-ylmethyl)carbamoyl]butyl}-4-{[(1H-imidazol-2-ylmethyl)amino]methyl}benzamide [Compound No. 159];
N-{(1S)-4-dipropylamino-1-[(4-methoxynaphthalen-1-ylmethyl)carbamoyl]butyl}-4-{[(1H-imidazol-2-ylmethyl)amino]methyl}benzamide [Compound No. 160]; and
4-{[bis(1H-imidazol-2-ylmethyl)amino]methyl}-N-((1S)-4-dipropylamino-1-isopropylcarbamoylbutyl)benzamide [Compound No. 161].

The present invention relates to a CXCR4 antagonist including the above-described compounds or a pharmacologically acceptable salt thereof as an effective ingredient.

The CXCR4 antagonist or salt thereof may be used in treatment or prevention of viral disease such as AIDS, cancer treatment, or treatment or prevention of rheumatis, etc.

Those compounds may optionally form a hydrate or a solvate. The pharmacologically acceptable salt is a salt which may be formed by the nitrogen-containing compound represented by the above described formula (I) , and may be any salt that is pharmacologically acceptable. For example, trifluoroacetate, hydrochloride, acetate, sulfate, nitrate, lactate, maleate, methanesulfonate, toluenesulfonnate, tartrate, citrate, oxalate, malonate, succinate, fumarate, propionate, butyrate, etc. may be given.
Those compounds may form a hydrate or a solvate in some cases. One or two or more asymmetric carbon atoms may exist in the compound represented by the general formula (I) ; when one asymmetric carbon atoms exists, the compound may be in any form of a pure optical isomer represented as absolute configuration of R or S, a mixture thereof in any ratio, and a racemic mixture thereof, and when two or more of asymmetric carbon atoms exist in the compound, the compound may be in any form of an optically pure diastereomer, a racemic mixture thereof, and a combination thereof in any ratio. The medical preparation including the compound of the present invention represented by the general formula (I) or pharmacologically acceptable salt as an effective ingredientmay be administered orally or parenterally in a form of tablet, powder, granule, capsule, pill, suppository, injection, eye-drops, liquid drug, troche, aerosol, suspension, emulsion, syrup, etc., mixed with a well-known pharmacologically acceptable carrier, excipient, diluent, extender, disintegrator, stabilizer, preservative, buffer, emulsifier, flavoring agent, colorant, edulcorant, thickening agent, corrigent, solubilizer, and other additives, specific examples thereof including: water; vegetable oil; alcohol such as ethanol or benzyl alcohol; glycol, glycerol triacetate, gelatin, lactose, carbohydrate such as starch; magnesium stearate; potassium stearate; tarc; lanoline; petrolatum; macrogall; crystalline cellulose; hydroxypropyl cellulose; etc. While the dose may vary depending on the kind and degree of disease, the kind of the compound to be administered, the administration path, and age, sex, and weight of the patient, in general, 0.1 to 5,000 mg, particularly 1 to 3,000 mg per one adult is preferably administered.

A production method of CXCR4 antagonist of the present invention will now be described in more detail with reference to ProductionExamplesandExamples. Hereinafter, unless particularly described, reagents used are commercially available products (e.g., Tokyo Kasei Kogyo Co. Ltd. (Tokyo), KANTO KAGAKU (Tokyo), etc.) readily available to a person skilled in the art.

### Examples

### Production Example 1: Synthesis of N-[(S)-1-(1-naphthyl)ethyl]-5-(2-methylbenzyl)amino-2-(S)-[4-[N-(imidazol-2-ylmethyl)aminomethyl]benzoyl]aminopentanoylamide [Compound No. 1]

### Example 1-1: Synthesis of 4-(N-Boc-N-(imidazol-2-ylmethyl)aminomethyl)benzoic acid (Compound VI-1)

Commercially available methyl bromomethylbenzoate (10.01 g) was dissolved in DMF (100 ml), and the solution was added with potassium phthalimide (9.70 g) and stirred at room temperature for 1.5 hours. After completion of reaction, the solution was concentrated, and water was added to the concentrate. Then, extraction was performed with chloroform. The resultant solution was washed with brine and dried with anhydrous sodium sulfate, and the solvent was distilled of f , to thereby obtain a white solid (12.91 g). Subsequently, a portion (7.56 g) of the obtained solid was dissolved in methanol (100 ml), and the solution was added with hydrazine monohydrate (6.25 ml) and stirred at 60°C for 1.5 hours. After completion of reaction, the precipitated solid was separated by filtration, and the solvent was distilled off. Water was added to the residue, and extraction was performed with chloroform. The resultant solution was washed with 0.3 mol/l of a sodium hydroxide aqueous solution and brine and dried with anhydrous sodium sulfate, and the solvent was distilled off. Methanol (120 ml) and 2-imidazolecarboaldehyde (2.35 g) were added to the residue, followed by stirring at roomtemperature for 2 days. After completion of reaction, the precipitated solid was separated by filtration. The liquid layer was exsiccated by concentration, and washing was performed by adding anhydrous methanol (30 ml). Then, the solid was separated by filtration. The resultant solid and the solid that had been previously separated by filtration were suspended in methanol (86 ml) , and sodium borohydride (1.42 g) was added under ice-cooling. The solution was stirred at room temperature for 1 hour, and the solvent was distilled off. After addition of water, extraction was performed with chloroform, and the organic layer was washed with brine and dried with anhydrous sodium sulfate, followed by concentration under reduced pressure and drying, to thereby obtain colorless viscous liquid (4.32 g). A portion (4.28 g) of the liquid was dissolved in DMF (65 ml), and the solution was added with di-t-butyldicarbonate (8.9 ml) and stirred at room temperature for 1 hour. After completion of reaction, the solvent was distilled off, and the residue was dissolved in chloroform, followed by washing with brine. After drying with anhydrous sodium sulfate, the solvent was distilled off, and THF (43 ml), methanol (43 ml), and 1 mol/l of a sodium hydroxide aqueous solution (43 ml) were added to the residue, followedby stirring at room temperature for 14 hours. After completion of reaction, the solvent was distilled off, and water (5 ml) was added to the residue. Further, 1 mol/l of a hydrochloric acid aqueous solution was carefully added to the solution, and the acid-precipitate was separated by filtration and dried, to thereby obtain the subject compound (4.87 g) as a white solid.
MS(FAB,Pos.):m/z=332[M+1]⁺

### Example 1-2: Synthesis of (S)-5-phthalimide-2-Boc-aminovaleric acid (Compound VII-1)

Commercially available ornithine hydrochloride (13.35 g) was dissolved in water (135 ml). Basic copper (II) carbonate (10.4 g) was gradually added to the solution while stirring was performed with heating in an oil bath (100°C), and stirring with heating was performed for 10 minutes, followed by filtration of the reaction suspension. The filtrate was diluted by adding water so as to have the total volume of 270 ml, and the solution was added with sodium carbonate (13.2 g) and carboethoxyphthalimide (19.1 g) and stirred at room temperature for 2 hours. The reaction suspension was cooled to 2°C and allowed to stand overnight, and a pale blue precipitate was separated by filtration and dried under reduced pressure. The residue was dissolved by adding a mixed solution of 4 mol/l of hydrochloric acid (80 ml) and methanol (80 ml), and the aqueous layer was washed with diethyl ether, followed by allowing it to stand overnight under cooling condition of 2°C. The resultant white precipitate was separated by filtration and dried under reduced pressure.

The precipitate was dissolved in DMF (100 ml), and triethylamine (23.7 ml) and di-t-butyldicarbonate (18.8 ml) were added to the solution. After reaction at room temperature overnight, the reaction solution was concentrated, and the concentrate was diluted with chloroform. The resultant solution was washed with brine twice and dried with anhydrous sodium sulfate, and the solvent was distilled off. The resultant residue was purified by silica gel column chromatography (200 g, chloroform/methanol = 10/1), to thereby obtain the subject compound (26.93 g) as colorless viscous liquid.
MS (FAB,Pos.) :m/z=363 [M+1]⁺

### Example 1-3: Synthesis of N-[(1S)-1-(1-naphthyl)ethyl]-5-phthalimide-(S)-2-(Boc-amino)pentanoylamide (Compound VIII-1)

The compound (19.4 g) obtained in Example 1-2 was dissolved in DMF (194 ml), and (S)-1-(1-naphthyl)ethylamine (9.17 g), WSCI hydrochloride (15.4 g), and HOBt (10.9 g) were added to the solution. After reaction all day and night, the reaction solution was concentrated, and the resultant residue was diluted with chloroform. A saturated sodium carbonate aqueous solution was added to the solution, and extraction was performed with chloroform. The organic layer was washed with brine, followed by drying and concentration with anhydrous sodium sulfate. The resultant residue was recrystallized from ethyl acetate, to thereby obtain the subject compound (20.96 g).
MS (FAB,Pos.) :m/z=516 [M+1]⁺
¹H-NMR(500MHz,CDCl₃): δ=1.44(9H,s),1.50-1.70(4H,m),1.71(3H,d,J=6 Hz),3.66-3.71(1H,m),3.81-3.90(1H,m),4.37-4.43(1H,m),5.29(1H,d, J=8.3Hz),5.85(1H,dq,J=6.8,7.1Hz),7.32(1H,t,J=7.1Hz),7.41(2H,t, J=7.3Hz),7.52(1H,d,J=7.3Hz),7.56-7.62(2H,m),7.63-7.70(2H,m),7. 71(1H,d,J=7.8Hz),7.74(1H,d,J=8.3Hz),7.96(1H,d,J=8.5Hz).

### Example 1-4: Synthesis of N-[(1S)-1-(1-naphthyl)ethyl]-5-phthalimide-(S)-2-[4-[N-Boc-N-(imidazol-2-ylmethyl)aminomethyl]benzoyl]aminopentanoylamide

The compound (19.3 g) obtained in Example 1-3 was dissolved in methanol (100 ml), and the solution was added with dioxane (100 ml) and concentrated hydrochloric acid (20 ml) and stirred at 45°C for 4 hours. After completion of reaction, the solvent was distilled off, and the residue was dissolved in DMF (200 ml). The solution was added with the compound (13.64 g) obtained in Example 1-1, WSCI hydrochloride (18.8 g), DMAP (9.40 g), and HOBt (7.60 g) and stirred at room temperature for 24 hours. After completion of reaction, the solvent was distilled off, and chloroform was added to the solution. The resultant solution was washed with a saturated sodium hydrogencarbonate aqueous solution and brine. After drying with anhydrous sodium sulfate, the solvent was distilled off, and the residue was purified by silica gel column chromatography (chloroform/methanol = 20/1), to thereby obtain the subj ect compound (26.24 g) as a white solid.
MS (FAB,Pos.) :m/z=729 [M+1]⁺
¹H-NMR(500MHz,CDCl₃) : δ=1.50-1.82(16H,m) ,3.69-3.74(1H,m) ,3.93-4.02(1H,m),4.01(2H,s),4.50(2H,s),5.00-5.07(1H,m),5.83-5.90(1H,m),6.94(1H,d,J=8.3Hz) ,6.98(2H,s) ,7.12(1H,d,J=8.3Hz) ,7.24(1H,d,J= 8.5Hz) ,7.37-7.43(2H,m) ,7.52-7.58(3H,m) ,7.64-7.69(2H,m) ,7.72(1H ,d,J=8.1Hz) ,7.75(1H,d,J=8.3Hz) ,7.79(2H,d,J=8.3Hz) ,7.98(1H,d,J= 8.3Hz) ,8.02(1H,s).

### Example 1-5: Synthesis of N-[(1S)-1-(1-naphthyl)ethyl]-5-amino-(S)-2-[4-[N-Boc-N-(imidazol-2-ylmethyl)aminomethyl]benzoyl]aminopentanoylamide (Compound XI-1)

The compound (26.13 g) obtained in Example 1-4 was dissolved in 40% methylamine/methanol solution, followed by stirring at room temperature for 24 hours. After completion of reaction, the solution was concentrated under reduced pressure, and the residue was dissolved in chloroform. The resultant solution was washed with distilledwaterandbrine. Theorganic layerwas dried with anhydrous sodium sulfate, and the solvent was distilled off. The residue was purified by silica gel column chromatography (chloroform/methanol/water=7/3/0.5), to thereby obtain the subject compound (12.6 g) as a white solid.
MS(FAB,Pos.):m/z=599[M+1]⁺
¹H-NMR(500MHz,DMSO-d₆) : δ=1.40(9H,brs), 1.30-1.45(2H,m), 1.51(3H,s ,J=6.8Hz),1.62-1.78(2H,m),2.45-2.55(2H,m),4.33(2H,brs),4.43(2H ,s),4.40-4.52(1H,m),5.71(1H,quint.,J=6.8Hz),6.84(1H,s),7.05(1H ,s),7.20-7.32(2H,m),7.47-7.57(4H,m),7.82(1H,d,J=8.1Hz),7.84(2H ,d,J=8.1Hz),7.94(1H,d,J=7.6Hz),8.10(1H,d,J=8.1Hz),8.48(1H,d,J= 7.8Hz),8.64(1H,d,J=7.8Hz),11.8-12.0(1H,br).

### Example 1-6: Synthesis of N-[(S)-1-(1-naphthyl)ethyl]-5-(2-methylbenzyl)amino-2-(S)-[4-[N-Boc-N-(imidazol-2-ylmethyl)aminomethyl]benzoyl]aminopentanoylamide (Compound XIIa-1)

The compound (299.7 mg) obtained in Example 1-5 was dissolved in methanol (6 ml), and the solution was added with 2-toluylaldehyde (69.5 µl) and stirred at room temperature for 2 hours. After completion of reaction, the solvent was distilledoff, and the residue was dried in vacuum and re-dissolved in methanol (6 ml). The solution was ice-cooled, and sodium borohydride (39.4 mg) was added to the solution. The resultant solution was returned to room temperature and stirred for 20 minutes. After completion of reaction, the solvent was distilled off, and the residue was dissolved in chloroform. The resultant solution was washed with 1N of sodium hydroxide and brine and dried with anhydrous sodium sulfate. The solvent was distilled off, and the residue was purified by silica gel column chromatography (chloroform/methanol = 10/1), to thereby obtain the subject compound (268.1 mg) as a white solid.
MS (FAB,Pos.) :m/z=703 [M+1]⁺

### Example 1-7: synthesis of N-[(S)-1-(1-naphthyl)ethyl]-5-(2-methylbenzyl)amino-2-(S)-[4-[N-(imidazol-2-ylmethyl)aminomethyl]benzoyl]aminopentanoylamide [Compound No. 1]

The compound (200.1 mg) obtained in Example 1-6 was dissolved in chloroform (6 ml), and methanesulfonic acid (92.4 µl) was added to the solution under ice-cooling, followed by stirring at room temperature for 4 hours. After completion of reaction, the solvent was distilled off, and the residue was purified by silica gel column chromatography (chloroform/methanol/water = 7/3/0.5), to thereby obtain a methanesulfonate (196.3 mg) of the subject compound as a white solid.
MS (FAB,Pos.) :m/z=603 [M+1]⁺
¹H-NMR(500MHz,DMSO-d₆) : δ=1.52(3H,d,J=6.8Hz) ,1.65-1.82(4H,m) ,2.3 2(3H,s) ,2.34(9H,s) ,2.92-3.05(2H,m) ,4.08(2H,d,J=5.9Hz) ,4.33(2H, brs) ,4.44(2H,brs) ,4.55-4.62(1H,m) ,5.72(1H,quint. ,J=6.8Hz) ,7.22 -7.35(3H,m) ,7.38(1H,d,7.6Hz) ,7.49(1H,t,J=7.6Hz) ,7.50-7.71(7H,m ) ,7.83(1H,d,J=8.3Hz) ,7.93(1H,d,J=8.5Hz) ,7.98(2H,d,J=8.3Hz) ,8.1 0(1H,d,J=8.3Hz) ,8.56(1H,d,J=6.8Hz) ,8.63(2H,brs) ,8

### Production Example 2: Synthesis of N-[(S)-1-(1-naphthyl)ethyl]-5-(2-methoxybenzyl)amino-2-(S)-[4-[N-(imidazol-2-ylmethyl)aminomethyl]benzoyl]aminopentanoylamide [Compound No. 2]

The compound (106.0 mg) obtained in Example 1-5 was dissolved in methanol (2 ml) , and the solution was added with 2-anisaldehyde (24.2 µl) and stirred at room temperature for 70 minutes. After completion of reaction, the solvent was distilledoff, and the residue was dried in vacuum and re-dissolved in methanol (2 ml). The solution was ice-cooled, and sodium borohydride (12.7 mg) was added to the solution. The resultant solution was returned to room temperature and stirred for 30 minutes. After completion of reaction, the solvent was distilled off, and the residue was dissolved in chloroform. The resultant solution was washed with 1 N sodium hydroxide and brine and dried with anhydrous sodium sulfate, and the solvent was distilled off. The residue was dissolved in chloroform (3 ml), and methanesulfonic acid (74 µl) was added under ice-cooling, followed by stirring at room temperature for 10 hours. After completion of reaction, the solvent was distilled off, and the residue was purified by silica gel column chromatography (chloroform/methanol/water = 7/3/0.5), to thereby obtain a methanesulfonate of the subject compound (115.5 mg) as a white solid.
MS (FAB,Pos.) :m/z=619 [M+1]⁺
¹H-NMR(500MHz,DMSO-d₆) : δ=1.52(3H,d,J=7.0Hz) ,1.62-1.82(4H,m) ,2.3 5 (9H,s) ,2.85-3.97 (2H,m) ,3.79 (3H,s) ,4.03 (2H,d,J=6.3Hz) ,4.31 (2H, brs) ,4.40(2H,brs) ,4.54-4.61(1H,m) ,5.72(1H,quint. ,J=7.0Hz) ,6.98 (1H,dd,J=7.3,1.0Hz) ,7.08(1H,d,J=7.6Hz) , 7.36(1H,dd,J=7.6,1.7Hz) ,7.42(1H,td,J=7.3,1.7Hz) ,7.46-7.61(8H,m) ,7.83(1H,d,J=8.1Hz) ,7. 94(1H,d,J=7.8Hz) ,7.97(2H,d,J=8.3Hz) ,8.10(1H,d,J=8.3Hz) ,8.48-8. 55(2H,m) ,8.54(1H,d,J=8.1Hz) ,8.72(1H,d,J=7.8Hz) .

### Production Example 3: Synthesis of N-[(S)-1-(1-naphthyl)ethyl]-5-isobutylamino-2-(S)-[4-[N-(imidazol-2-ylmethyl)aminomethyl)benzoyl]aminopentanoylamide [Compound No. 3]

### Example 3-1: Synthesis of N-[(S)-1-(1-naphthyl)ethyl]-5-isobutylamino-2-(S)-[4-[N-Boc-N-(imidazol-2-ylmethyl)aminomethyl]benzoyl]aminopentanoylamide

The compound (0.600 g) obtained in Example 1-5 was dissolved in methanol (10 ml). Molecular Sieve 3A powder (1g), acetic acid (0.12ml), and isobutylaldehyde (0.137ml) were added to the solution, and the resultant solution was stirred at room temperature overnight. Sodium cyano borohydride (0.189g) was added to the solution, followed by further stirring at room temperature overnight. After completion of reaction, the reaction solution was filtered, and the solvent was distilled off under reduced pressure. Chloroform (10 ml) and 10% sodium hydroxide aqueous solution were added to the residue, and the solution was stirred for 30 minutes, followed by extraction three times with chloroform. The organic layer was dried with anhydrous sodium sulfate. After filtration, the solvent was distilled off under reduced pressure, and the residue was purified by silica gel column chromatography (chloroform/methanol/water = 7/3/0.5) , to thereby obtain the subject compound (326 mg) as a white solid.
MS (FAB,Pos.) :m/z=655 [M+1]⁺
¹H-NMR(500MHz,DMSO-d₆) : δ=0.82(6H,d,J=6.6Hz) ,1.35-1.38(9H,br) ,1. 41-1.49(2H,m) ,1.52(3H,d,J=6,8Hz) ,1.59-1.75(3H,m) ,2.29(2H,d,J=6 .6Hz) ,4.12(1H,brs) ,4.33(1H,brs) ,4.42-4.43(2H,br) ,4.49-4.53(2H, m),4.54-4.57(1H,br),5.71(1H,t,J=7.3Hz),6.84(1H,brs),7.04(1H,br s),7.24-7.29(2H,br),7.46-7.56(4H,m),7.82-7.85(3H,m),7.93(1H,d, J=7.6Hz),8.10(1H,d,J=8.1Hz),8.42(1H,d,J=8.1Hz),8.66(1H,d,J=7.8 Hz).

### Example 3-2: Synthesis of N-[(S)-1-(1-naphthyl)ethyl]-5-isobutylamino-2-(S)-[4-[N-(imidazol-2-ylmethyl)aminomethyl]benzoyl]aminopentanoylamide [Compound No. 3]

The compound (0.241 g) obtained in Example 3-1 was dissolved in chloroform (5 ml), and the solution was added with methanesulfonic acid (0.17 ml) and stirred at room temperature overnight. After completion of reaction, the solvent was distilled off under reduced pressure, and the residue was purified by silica gel column chromatography (chloroform/methanol/water = 7/3/0.5), to thereby obtain a methanesulfonate of the subject compound (0.312 g) as a white solid.
MS (FAB,Pos.) :m/z=555 [M+1]⁺
¹H-NMR(500MHz,DMSO-d₆) : δ=0.89 (6H,d,J=6.6Hz) ,1.35-1.38(9H,br) ,1. 52(3H,d,J=6.8Hz) ,1.61-1.74(4H,m) ,1.84-1.89(1H,m) ,2.36(9H,s) ,2. 63-2.68(2H,m) ,2.87(2H,m) ,4.14-4.19(3H,br) ,4.58(1H,dd,J=14.0,8. 4Hz) ,5.72(1H,quint. ,J=6.8Hz) ,7.31(2H,brs) ,7.48-7.58(6H,m) ,7.84 (1H,d,J=8.1Hz) ,7.96(3H,d,J=7.6Hz) ,8.10(1H,d,J=8.1Hz), 8.14(2H,b rs) ,8.53(1H,d,J=8.3Hz) ,8.73(1H,d,J=7.8Hz) .

### Production Example 4: Synthesis of N-[(S)-1-(1-naphthyl)ethyl]-5-(2,2-dimethylpropyl)amino-2-(S)-[4-[N-(imidazol-2-ylmethyl)aminomethyl]benzoyl]aminopentanoylamide [Compound No. 4]

### Example 4-1: Synthesis of N-[(S)-1-(1-naphthyl)ethyl]-5-(2,2-dimethylpropyl)amino-2-(S)-[4-[N-Boc-N-(imidazol-2-ylmethyl)aminomethyl]benzoyl]aminopentanoylamide

The compound (0.600 g) obtained in Example 1-5 was dissolved in methanol (10 ml). Molecular Sieve 3A powder (1g), acetic acid (0.12 ml), and pivalaldehyde (0. 137 ml) were added to the solution, and the resultant solution was stirred at room temperature overnight. Sodium cyano borohydride (0.189 g) was added to the solution, followed by further stirring at room temperature overnight. After completion of reaction, the reaction solution was filtered, and the solvent was distilled off under reduced pressure. Chloroform (10 ml) and 10% sodium hydroxide aqueous solution were added to the residue, and the solution was stirred for 30 minutes, followed by extraction three times with chloroform. The organic layer was dried with anhydrous sodium sulfate. After filtration, the solvent was distilled off under reduced pressure, and the residue was purified by silica gel column chromatography (chloroform/methanol/water = 7/3/0.5) , to thereby obtain the subject compound (296 mg) as a white solid.
MS (FAB,Pos.) :m/z=669 [M+1]⁺
¹H-NMR(500MHz,DMSO-d₆) : δ=0.91(9H,s) ,1.35-1.37(9H,br) ,1.52(3H,d, J=6.8Hz) ,1.61-1.73(4H,m) ,2.58(2H,m) ,2.79(2H,m) ,4.33(1H,brs) ,4. 42(2H,d,J=8.1Hz) ,4.49(1H,brs) ,4.54-4.57(1H,br) ,5.73(1H,quint., J=6.8Hz) ,6.84(1H,brs) ,7.05(1H,brs) ,7.25-7.29(1H,m) ,7.47-7.57(4 H,m) ,7.83(1H,d,J=8.1Hz) ,7.88(2H,d,J=8.3Hz) ,7.95(1H,d,J=7.8Hz) , 8.10(1H,d,J=8.1Hz) ,8.46(1H,d,J=7.8Hz) ,8.72(1H,d,J=7.6Hz) .

### Example 4-2: Synthesis of N-[(S)-1-(1-naphthyl)ethyl]-5-(2,2-dimethylpropyl)amino-2-(S)-[4-[N-(imidazol-2-ylmethyl)aminomethyl]benzoyl]aminopentanoylamide [Compound No. 4]

The compound (0.200 g) obtained in Example 4-1 was dissolved in chloroform (5ml), and the solution was added with methanesulfonic acid (0.118 ml) and stirred at room temperature overnight. After completion of reaction, the solvent was distilled off under reduced pressure, and the residue was purified by silica gel column chromatography (chloroform/methanol/water = 7/3/0.5), to thereby obtain a methanesulfonate (0.201 g) of the subject compound as a white solid.
MS (FAB,Pos.) :m/z=569 [M+1]⁺
¹H-NMR(500MHz,DMSO-d₆) : δ=0 .94 (9H,s) , 1.52 (3H,d,J=6.8Hz) , 1.65 (1H, brs) ,1.73(3H,brs) ,2.66-2.68(2H,m) ,2.89(2H,brs) ,4.29-4.33(3H,br), 4.58(1H,d,J=8.1Hz) ,5.72(1H,quint. ,J=6.8Hz) ,7.47-7.60(8H,m) ,7 .84(1H,d,J=8.1Hz) ,7.94-7.99(5H,m) ,8.10(1H,d,J=8.1Hz) ,8.56(1H,d ,J=7.8Hz) ,8.74(1H,d,J=7.6Hz) .

### Production Example 5: Synthesis of N-[(S)-1-(1-naphthyl)ethyl]-5-(2-trifluoromethylbenzyl)amino-2 -(S)-[4-[N-(imidazol-2-ylmethyl)aminomethyl]benzoyl]aminopentanoylamide [Compound No. 5]

The compound (99.3 mg) obtained in Example 1-5 was dissolved in methanol (2 ml), and the solution was added with 2-trifluoromethyl benzaldehyde (26.4 µl) and stirred at room temperature for 2 hours. After completion of reaction, the solvent was distilled off, and the residue was dried in vacuum and re-dissolved in methanol (2 ml). Thesolutionwas ice-cooled, andsodiumcyanoborohydride (12.9 mg) was added to the solution. The resultant solution was returned to room temperature and stirred for 20 minutes. After completion of reaction, the solvent was distilled off, and the residue was dissolved in chloroform. The resultant solution was washed with 1 mol/l sodium hydroxide and brine and dried with anhydrous sodium sulfate, and the solvent was distilled off. The residue was dissolved in chloroform (6 ml), and methanesulfonic acid (92.4 µl) was added under ice-cooling, followed by stirring at room temperature for 15 hours. After completion of reaction, the solvent was distilled off, and the residue was purified by silica gel column chromatography (chloroform/methanol/water = 7/3/0.5), to thereby obtain a methanesulfonate (144.5 mg) of the subject compound as a white solid.
MS (FAB,Pos.) :m/z=657 [M+1]⁺
¹H-NMR(500MHz,DMSO-d₆) : δ=1.52(3H,d,J=7.1Hz) , 1.63-1.82(4H,m) ,2.3 4(9H,s) ,2.95-3.12(2H,m) ,4.27(2H,d,J=5.6Hz) ,4.32(2H,brs) ,4.42(2 H,brs) ,4.55-4.62(1H,m) ,5.71(1H,quint. ,J=7.1Hz) ,7.45-7.68(9H,m) ,7.76(1H,d,7.6Hz) ,7.79(1H,d,J=6.8Hz) ,7.83(2H,d,J=8.3Hz) ,7.95(1 H,d,J=7.8Hz) ,7.98(2H,d,J=8.3Hz) ,8.10(1H,d,J=8.3Hz) ,8.56(1H,d,J =8.1Hz) ,8.72(1H,d,J=7.6Hz) ,8.95(2H,brs) .

### Production Example 6: Synthesis of N-[(S)-1-(1-naphthyl)ethyl]-5-(2-chlorobenzyl)amino-2-(S)-[4-[N-(imidazol-2-ylmethyl)aminomethyl]benzoyl]aminopentanoylamide [Compound No. 6]

The compound (108.0 mg) obtained in Example 1-5 was dissolved in methanol (2 ml), and the solution was added with 2-chlorobenzaldehyde (22.6 µl) and stirred at room temperature for 2 hours. After completion of reaction, the solvent was distilled off, and the residue was dried in vacuum and re-dissolved in methanol (2 ml). The solution was ice-cooled, and sodium borohydride (14.1 mg) was added to the solution. The resultant solution was returned to room temperature and stirred for 20 minutes. After completion of reaction, the solvent was distilled off, and the residue was dissolved in chloroform. The resultant solution was washed with 1 N sodium hydroxide and brine and dried with anhydrous sodiumsulfate, and the solvent was distilled off. The residue was dissolved in chloroform (6ml) , and methanesulfonic acid (92.4 µl) was added under ice-cooling, followed by stirring at room temperature for 15 hours. After completion of reaction, the solvent was distilled off, and the residue was purified by silica gel column chromatography (chloroform/methanol/water = 7/3/0.5), to thereby obtain a methanesulfonate (133.1mg) of the subject compound as a white solid.
MS(FAB,Pos.):m/z=624,626[M+1]⁺
¹H-NMR(500MHz,DMSO-d₆):δ=1.52(3H,d,J=7.1Hz),1.63-1.81(4H,m),2.3 5(9H,s),2.95-3.05(2H,m),4.21(2H,d,J=5.6Hz),4.30(2H,brs),4.40(2 H,brs),4.57-4.61(1H,m),5.72(1H,quint.,J=7.1Hz),7.45-7.70(11H,m ),7.83(1H,d,J=8.3Hz),7.94(1H,d,J=6.8Hz),7.83(2H,d,J=8.3Hz),7.9 4(1H,d,J=7.5Hz),7.98(2H,d,J=8.1Hz),8.10(1H,d,J=8.1Hz),8.54(1H, d,J=8.5Hz),8.72(1H,d,J=7.8Hz),8.81(2H,brs).

### Production Example 7: Synthesis of N-[(S)-1-(1-naphthyl)ethyl]-5-(3-methylbenzyl)amino-2-(S)-[4-[N-(imidazol-2-ylmethyl)aminomethyl]benzoyl]aminopentanoylamide [Compound No. 7]

The compound (101.2 mg) obtained in Example 1-5 was dissolved in methanol (2 ml), and the solution was added with 3-toluyl aldehyde (23.6 µl) and stirred at room temperature for 2 hours. After completion of reaction, the solvent was distilled off, and the residue was dried in vacuum and re-dissolved in methanol (2 ml). The solution was ice-cooled, and sodium borohydride (12.6 mg) was added to the solution. The resultant solution was returned to room temperature and stirred for 20 minutes. After completion of reaction, the solvent was distilled off, and the residue was dissolved in chloroform. The resultant solution was washed with 1N sodium hydroxide and brine and dried with anhydrous sodium sulfate, and the solvent was distilled off. The residue was dissolved in chloroform (6 ml), and methanesulfonic acid (92.4µl) was added under ice-cooling, followed by stirring at room temperature for 12 hours. After completion of reaction, the solvent was distilled off, and the residue was purified by silica gel column chromatography (chloroform/methanol/water = 7/3/0.5), to thereby obtain a methanesulfonate (133.4 mg) of the subject compound as a white solid.
MS (FAB,Pos.) :m/z=603 [M+1]⁺
¹H-NMR(500MHz,DMSO-d₆) : δ=1.52(3H,d,J=6.8Hz) ,1.59-1.79(4H,m) ,2.3 1(3H,s) ,2.35(9H,s) ,2.80-2.95(2H,m) ,4.04(2H,d,J=5.6Hz) ,4.31(2H, brs) ,4.41(2H,brs) ,4.51-4.60(1H,m) ,5.71(1H,quint. ,J=6.8Hz) ,7.20 -7.28(3H,m) ,7.31(1H,t,J=7.8Hz) ,7.46-7.65(BH,m) ,7.83(1H,d,J=8.1 Hz) ,7.93-7.95(1H,m) ,7.97(2H,d,J=7.8Hz) ,8.10(1H,d,J=8.1Hz) ,8.54 (1H,d,J=7.8Hz) ,8.68(2H,brs) ,8.72(1H,d,J=7.8Hz) .

### Production Example 8: Synthesis of N-[(S)-1-(1-naphthyl)ethyl]-5-(4-methylbenzyl)amino-2-(S)-[4-[N-(imidazol-2-ylmethyl)aminomethyl]benzoyl]aminopentanoylamide [Compound No. 8]

The compound (103.6 mg) obtained in Example 1-5 was dissolved in methanol (2 ml) , and the solution was added with 4-toluyl aldehyde (23.6 µl) and stirred at room temperature for 2 hours. After completion of reaction, the solvent was distilled off, and the residue was dried in vacuum and re-dissolved in methanol (2ml). The solution was ice-cooled, and sodium borohydride (12.6 mg) was added to the solution. The resultant solution was returned to room temperature and stirred for 120 minutes. After completion of reaction, the solvent was distilled off, and the residue was dissolved in chloroform. The resultant solution was washed with 1N sodium hydroxide and brine and driedwith anhydrous sodium sulfate, and the solvent was distilled off. The residue was dissolved in chloroform (6 ml), and methanesulfonic acid (92.4 µl) was added under ice-cooling, followed by stirring at room temperature for 12 hours. After completion of reaction, the solvent was distilled off, and the residue was purified by silica gel column chromatography (chloroform/methanol/water = 7/3/0.5), to thereby obtain a methanesulfonate (133.8 mg) of the subject compound as a white solid.
MS (FAB,Pos.) :m/z=603 [M+1]⁺
¹H-NMR(500MHz,DMSO-d₆) : δ=1.52(3H,d,J=6.8Hz) ,1.59-1.79(4H,m) ,2.0 9 (3H,s) ,2.35 (9H,s) ,2.80-2.95 (2H,m) ,4.03 (2H,d,J=5.6Hz) ,4.30 (2H, brs) ,4.37(2H,brs) ,4.51-4.60(1H,m) ,5.71(1H,quint. ,J=6.8Hz) ,7.22 (2H,d,J=7.8Hz) ,7.32(2H,d,J=7.8Hz) ,7.46-7.62(8H,m) ,7.83(1H,d,J= 8.1Hz) ,7.93-7.96(1H,m) ,7.97(2H,d,J=8.5Hz) ,8.10(1H,d,J=7.8Hz) ,8 .54(1H,d,J=7.8Hz) ,8.68(2H,brs) ,8.72(1H,d,J=7.8Hz) .

### Production Example 9: Synthesis of N-[(S)-1-(1-naphthyl)ethyl]-5-(3-methylpyridin-2-yl)methylamino-2-(S)-[4-[N-(imidazol-2-ylmethyl)aminomethyl]benzoyl]aminopentanoylamide [Compound No. 9]

### Example 9-1: Synthesis of 3-methyl-2-pyridinealdehyde

Commercially available 2,3-lutidine (5.00 g) was dissolved in methylene chloride (50 ml), and the solution was cooled to 0°C. Subsequently, mCPBA (12.1 g) was added, followed by stirring at room temperature for 2 hours. After completion of reaction, methylene chloride was added to the solution, and the resultant solution was washed with 1 mol/l sodium hydroxide aqueous solution and brine and dried with anhydrous sodium sulfate. The solvent was distilled off to obtain crude 2,3-lutidine-N-oxide (3.16 g).

A portion (2.00g) of the obtained crude product was dissolved in methylene chloride (40 ml), followed by cooling to 0°C, then trifluoroacetic acid anhydride (4.49 ml) was added to the solution, followed by stirring at room temperature for 4 hours and then at 45°C for 3 hours. After completion of reaction, the solvent was distilled off and the residue was dissolved in methanol (30 ml), followed by addition of a sodium methoxide/methanol solution till pH of the solution became 10. After stirring the solution at room temperature for 1 hour, the solvent was distilled off and then the residue was extracted with methylene chloride. After drying with anhydrous sodium sulfate, the solvent was distilled off, to thereby obtain crude 3-methyl-2-hydroxymethylpyridine (1.3 g).

A portion (605.3 mg) of the obtained product was dissolved in chloroform (30 ml) and then added with manganese dioxide (3.03 g) (chemicals treated, manufactured by Wako Pure Chemical Industries, Ltd.), followed by stirring at 70°C for 2 hours. After completion of reaction, the catalyst was removed by filtration with Celite and the solvent was concetrated. The residue was purified by silica gel column chromatography (chloroform/ethyl acetate=1:1), to thereby obtain a light orange liquid of the subject compound (419.8 mg).
MS (FAB,Pos.) :m/z= 122 [M+1]⁺
¹H-NMR(500MHz,CDCl₃) : δ=2.67(3H,s),7.40(1H,dd,J=7.8,4.6Hz),7.64(1H,d,J=7.8Hz) ,8.67(1H,d,J=4.6Hz) ,10.2(1H,s) .

### Example 9-2: Synthesis of N-[(S)-1-(1-naphthyl)ethyl]-5-(3-methylpyridin-2-yl)methylamino-2-(S)-[4-[N-(imidazol-2-ylmethyl)aminomethyl]benzoyl]aminopentanoylamide [Compound No. 9)

The compound (50.1 mg) obtained in Example 1-5 was dissolved in methanol (1 ml), and the solution was added with the compound (17.7 mg) obtained in Example 9-1 and stirred at room temperature for 2 hours. After completion of reaction, the solvent was distilled off, and the residue was dried in vacuum and re-dissolved in methanol (1 ml). The solution was ice-cooled, and sodium borohydride (6.3 mg) was added to the solution. The resultant solution was returned to room temperature and stirred for 30 minutes. After completion of reaction, the solvent was distilled off, and the residue was dissolved in chloroform. The resultant solution was washed with 0.5 mol/l sodium hydroxide and brine and dried with anhydrous sodium sulfate, andthesolventwasdistilledoff. Theresiduewasdissolved in chloroform (1.5 ml), and methanesulfonic acid (33.7 µl) and methanol (33.7µl) were added under ice-cooling, followed by stirring at room temperature for 16 hours. After completion of reaction, the solvent was distilled off, and the residue was purified by silica gel column chromatography (chloroform/methanol/water = 7/3/0.5), to thereby obtain a methanesulfonate (54.9 mg) of the subj ect compound as a white solid.
MS(FAB,Pos.):m/z=604[M+1]⁺
¹H-NMR(500MHz,DMSO-d₆):δ=1.52(3H,d,J=6.8Hz),1.68-1.88(4H,m),2.2 5(3H,s),2.37(9H,s),2.99-3.09(2H,m),4.30(2H,t,J=5.6Hz),4.36(2H, brs),4.52(2H,brs),4.55-4.61(1H,m),5.71(1H,quint.,J=6.8Hz),7.35 (1H,dd,J=7.8,4.9Hz),7.45-7.58(4H,m),7.61(2H,d,J=8.3Hz),7.69(1H ,d,J=7.6Hz),7.72(2H,s),7.83(1H,d,J=8.1Hz),7.94(1H,d,J=7.8Hz),7 .99(2H,d,J=8.3Hz),8.10(1H,d,J=8.3Hz),8.43(1H,d,J=4.9Hz),8.57(1 H,d,J=8.1Hz),8.73(1H,d,J=7.6Hz),8.97(2H,brs),9.3-9.9(1H,br).

### Production Example 10: Synthesis of N-[(S)-1-(1-naphthyl)ethyl]-5-(5-methylpyridin-2-yl)methylamino-2-(S)-[4-[N-(imidazol-2-ylmethyl)aminomethyl]benzoyl]aminopentanoylamide [Compound No. 10]

### Example 10-1: Synthesis of 5-methyl-2-pyridinealdehyde

The same procedure as in Example 9-1 was performed by using 2,5-lutidine as a raw material, to thereby obtain 5-methylpyridine-2-aldehyde (439.9 mg).
MS(FAB,Pos.):m/z= 122[M+1]⁺
¹H-NMR(500MHz,CDCl₃) : δ=2.46(3H,s),7.67(1H,dd,J=7.9,1.4Hz),7.89(1H,d,J=7.9Hz),8.62(1H,d,J=1.4Hz),10.05(1H,s).

### Example 10-2: Synthesis of N-[(S)-1-(1-naphthyl)ethyl]-5-(5-methylpyridin-2-yl)methylamino-2-(S)-[4-[N-(imidazol-2-ylmethyl)aminomethyl]benzoyl]aminopentanoylamide [Compound No. 10]

The compound (49.2 mg) obtained in Example 1-5 was dissolved in methanol (1 ml), and the solution was added with the compound (16.2 mg) obtained in Example 10-1 and stirred at room temperature for 2 hours. After completion of reaction, the solvent was distilled off, and the residue was dried in vacuum and re-dissolved in methanol (1 ml). The solution was ice-cooled, and sodium borohydride (6.2 mg) was added to the solution. The resultant solution was returned to room temperature and stirred for 30 minutes. After completion of reaction, the solvent was distilled off, and the residue was dissolved in chloroform. The resultant solution was washed with 0. 5 mol/l sodium hydroxide and brine and dried with anhydrous sodium sulfate, and the solvent was distilled off. The residuewas dissolved in chloroform (1.5 ml), and methanesulfonic acid (33.7 µl) and methanol (33.7 µl) were added under ice-cooling, followed by stirring at room temperature for 18 hours. After completion of reaction, the solvent was distilled off, and the residue was purified by silica gel column chromatography (chloroform/methanol/water = 7/3/0.5), to thereby obtain a methanesulfonate (61.6mg) of the subj ect compound as a white solid.
MS (FAB,Pos.) :m/z=604 [M+1]⁺
¹H-NMR(500MHz,DMSO-d₆) : δ=1.52(3H,d,J=6.8Hz) ,1.63-1.80(4H,m) ,2.2 5(3H,s) ,2.37(9H,s) ,2.99-3.09(2H,m) ,4.30(2H,t,J=5.3Hz) ,4.36(2H, brs) ,4.52(2H,brs) ,4.55-4.61(1H,m) ,5.70(1H,quint. ,J=6.8Hz) ,7.37 (1H,d,J=7.8Hz) ,7.46-7.59(4H,m) ,7.61(2H,d,J=8.3Hz) ,7.69(1H,d,J= 7.8Hz) ,7.73(2H,s) ,7.83(1H,d,J=8.1Hz) ,7.94(1H,d,J=7.8Hz) ,7.98(2 H,d,J=8.3Hz) ,8.10(1H,d,J=8.3Hz) ,8.46(1H,s) ,8.55(1H,d,J=8.1Hz) , 8.71(1H,d,J=7.8Hz) ,8.96(2H,brs) ,9.4-9.9(1H,br) .

### Production Example 11: Synthesis of N-[(S)-1-(1-naphthyl)ethyl]-5-(4-methylpyridin-2-yl)methylamino-2-(S)-[4-[N-(imidazol-2-ylmethyl)aminomethyl]benzoyl]aminopentanoylamide [Compound No. 11]

### Example 11-1: Synthesis of 4-methyl-2-pyridinealdehyde

The same procedure as in Example 9-1 was performed by using 2,4-lutidine as a raw material, to thereby obtain 4-methylpyridine-2-aldehyde (40.5 mg).
1H-NMR(500MHz,CDCl₃) : δ=2.46(3H,s),7.35(1H,d,J=4.9Hz),7.80(1H,s) ,8.65(1H,d,J=4.9Hz),10.08(1H,s).
MS(FAB,Pos.):m/z= 122[M+1]⁺

### Example 11-2: Synthesis of N-[(S)-1-(1-naphthyl)ethyl]-5-(4-methylpyridin-2-yl)methylamino-2-(S)-[4-[N-(imidazol-2-ylmethyl)aminomethyl]benzoyl]aminope ntanoylamide [Compound No. 11]

The compound (50.0 mg) obtained in Example 1-5 was dissolved in methanol (1 ml), and the solution was added with the compound (20.3 mg) obtained in Example 11-1 and stirred at room temperature for 3 hours. After completion of reaction, the solvent was distilled off, and the residue was dried in vacuum and re-dissolved in methanol. (1 ml). The solution was ice-cooled, and sodium borohydride (12.2 mg) was added to the solution. The resultant solution was returned to room temperature and stirred for 30 minutes. After completion of reaction, the solvent was distilled off, and the residue was dissolved in chloroform. The resultant solution was washed with 0.5 mol/l sodium hydroxide and brine and dried with anhydrous sodium sulfate, and the solvent was distilled off. Theresiduewasdissolved in chloroform (1.5 ml), and methanesulfonic acid (50.0 µl) and methanol (50.0 µl) were added under ice-cooling, followed by stirring at room temperature for 12 hours. After completion of reaction, the solvent was distilled off, and the residue was purified by silica gel column chromatography (chloroform/methanol/water = 7/3/0.5), to thereby obtain a methanesulfonate (68.8mg) of the subject compound as a white solid.
MS (FAB,Pos.) :m/z=604 [M+1]⁺
¹H-NMR (500MHz,DMSO-d₆) : δ=1.52(3H,d,J=6.8Hz) ,1.63-1.81(4H,m) ,2.3 5(3H,s) ,2.37(9H,s) ,2.91-3.01(2H,m) ,4.22(2H,t,J=5.3Hz) ,4.36 (2H, brs) ,4.52(2H,brs) ,4.50-4.60(1H,m), 5.71(1H,quint. ,J=6.8Hz) ,7.29 (1h,d,J=4.9Hz) ,7.30(1H,s) ,7.46-7.58(4H,m) ,7.61(2H,d,J=8.3Hz) ,7 .72(2H,s) ,7.83(1H,d,J=8.1Hz) ,7.94(1H,d,J=7.8Hz) ,7.98(2H,d,J=8. 3Hz) ,8.10(1H,d,J=8.5Hz) ,8.46(1H,d,J=4.9Hz) ,8.55(1H,d,J=8.3Hz), 8.71(1H,d,J=7.8Hz) ,8.97(2H,brs) .

### Production Example 12: Synthesis of N-[(S)-1-(1-naphthyl)ethyl]-5-(2,6-dimethoxybenzyl)amino-2-(S)-[4-[N-(imidazol-2-ylmethyl)aminomethyl]benzoyl]aminopentanoylamide [Compound No. 12]

The compound (30.3 mg) obtained in Example 1-5 was dissolved in methanol (0.3 ml), and the solution was added with 2,6-dimethoxybenzaldehyde (8.7 mg) and stirred at room temperature for 2 hours. After completion of reaction, the solvent was distilled off, and the residue was dried in vacuum and re-dissolved in methanol (0.6 ml). The solution was ice-cooled, and sodium borohydride (3.8 mg) was added to the solution. The resultant solution was returned to room temperature and stirred for 30 minutes. After completion of reaction, the solvent was distilled off, and the residue was dissolved in chloroform. The resultant solution was washed with 0.5 mol/l sodium hydroxide and brine and dried with anhydrous sodium sulfate, andthesolventwasdistilledoff. Theresiduewasdissolved in chloroform (0.9 ml), and methanesulfonic acid (29 µl) and methanol (29 µl) were added under ice-cooling, followed by stirring at room temperature for 12 hours. After completion of reaction, the solvent was distilled off, and the residue was purified by silica gel column chromatography (chloroform/methanol/water = 7/3/0.5), to thereby obtain a methanesulfonate (32.6 mg) of the subject compound as a white solid.
MS (FAB,Pos.) :m/z=649 [M+1]⁺
¹H-NMR(500MHz,DMSO-d₆) : δ=1.53(3H,d,J=6.8Hz) ,1.61-1.80(4H,m) ,2.2 7(9H,s) ,2.81-2.99(2H,m) ,3.77(3H,s) ,3.98(2H,t,J=5.6Hz) ,4.29(2H, s) ,4.39(2H,brs) ,4.54-4.61(1H,m) ,5.71(1H,quint. ,J=6.8Hz) ,6.71(2 H,d,J=8.5Hz) ,7.39(1H,t,J=8.5Hz) ,7.47-7.63(8H,m) ,7.82(1H,d,J=8. 3Hz) ,7.94(1H,d,J=7.8Hz) ,7.97(2H,d,J=8.3Hz) ,8.10(1H,d,J=8.3Hz), 8.32(2H,brs) ,8.54(1H,d,J=8.1Hz) ,8.71(1H,d,J=7.8Hz) .

### Production Example 13: Synthesis of N-[(S)-1-(1-naphthyl)ethyl]-5-(3-methylthiophen-2-yl)methylamino-2-(S)-[4-[N-(imidazol-2-ylmethyl)aminomethyl]benzoyl]aminopentanoylamide [Compound No. 13]

The compound (30.9 mg) obtained in Example 1-5 was dissolved in methanol (0.3 ml), and the solution was added with 3-methylthiophene-2-aldehyde (5.5 µl) and stirred at room temperature for 2 hours. After completion of reaction, the solvent was distilled off, and the residue was dried in vacuum and re-dissolved in methanol (0.6 ml). The solution was ice-cooled, and sodium borohydride (3.8 mg) was added to the solution. The resultant solution was returned to room temperature and stirred for 30 minutes. After completion of reaction, the solvent was distilled off, and the residue was dissolved in chloroform. The resultant solution was washed with 0.5 mol/l sodium hydroxide and brine and dried with anhydrous sodium sulfate, and the solvent was distilled off. The residue was dissolved in chloroform (0.9 ml), and methanesulfonic acid (29 µl) and methanol (29 µl) were added under ice-cooling, followed by stirring at room temperature for 12 hours. After completion of reaction, the solvent was distilled off, and the residue was purified by silica gel column chromatography (chloroform/methanol/water = 7/3/0.5), to thereby obtain a methanesulfonate (31.1 mg) of the subject compound as a white solid.
MS (FAB,Pos.) :m/z=609 [M+1]⁺
¹H-NMR(500MHz,DMSO-d₆) : δ=1.52(3H,d,J=6.8Hz) ,1.59-1.80(4H,m) ,2.2 1(3H,s) ,2.35(9H,s) ,2.82-3.01(2H,m) ,4.24(2H,t,J=5.6Hz) ,4.30(2H, s) ,4.48(2H,br) ,4.53-4.60(1H,m) ,5.71(1H,quint. ,J=6.8Hz) ,6.94(1H ,d,J=5.1Hz) ,7.47-7.64(9H,m) ,7.83(1H,d,J=8.1Hz) ,7.94(1H,d,J=7.6 Hz) ,7.96(2H,d,J=8.1Hz) ,8.10(1H,d,J=8.1Hz) ,8.54(1H,d,J=7.6Hz) ,8 .66(2H,brs) ,8.70(1H,d,J=7.8Hz) .

### Production Example 14: Synthesis of N-[(S)-1-(1-naphthyl)ethyl]-5-(3-methoxypyridin-2-yl)methylamino-2-(S)-[4-[N-(imidazol-2-ylmethyl)aminomethyl]benzoyl]aminopentanoylamide [Compound No. 14]

The compound (31.6 mg) obtained in Example 1-5 was dissolved in methanol (0.3 ml), and the solution was added with 3-methoxy-2-pyridinealdehyde (11.3 mg) that had been synthesized in accordance with a method described in Cominus D. et al ., Tetrahedron Lett., 29 (7), 773 (1988) and stirred at room temperature for 4.5 hours. After completion of reaction, the solvent was distilled off, and the residue was dried in vacuum and re-dissolved in methanol (0.6 ml). The solution was ice-cooled, and sodium borohydride (9 mg) was added to the solution. The resultant solution was returned to room temperature and stirred for 30 minutes. After completion of reaction, the solvent was distilled off, and the residue was dissolved in chloroform. The resultant solution was washed with 0.5 mol/l sodium hydroxide and brine and dried with anhydrous sodium sulfate, and the solvent was distilled off. The residuewas dissolved in chloroform (0.9ml), and methanesulfonic acid (40µl) and methanol (40 µl) were added under ice-cooling, followed by stirring at room temperature for 1.5 hours. After completion of reaction, the solvent was distilled off, and the residue was purified by silica gel column chromatography (chloroform/methanol/water = 7/3/0.5), to thereby obtain a methanesulfonate (36.1 mg) of the subject compound as a white solid.
MS(FAB,Pos.):m/z=620[M+1]⁺
¹H-NMR(500MHz,DMSO-d₆):δ=1.52(3H,d,J=7.1Hz),1.65-1.82(6H,m),2.3 6(12H,s),2.97-3.05(2H,m),3.86(3H,s),4.22(2H,t,J=5.9Hz),4.34(2H ,s),4.46(2H,br),4.55-4.62(1H,m),5.71(1H,quint.,J=7.1Hz),7.43-7 .60(6H,m),7.61(2H,d,J=8.1Hz),7.70(2H,brs),7.83(1H,d,J=7.8Hz),7 .94(1H,d,J=7.1Hz),7.98(2H,d,J=8.1Hz),8.10(1H,d,J=8.5Hz),8.18(1 H,d,J=4.6Hz),8.55(1H,br),8.72(1H,d,J=7.8Hz),8.94(2H,brs).

### Production Example 15: Synthesis of N-[(S)-1-(1-naphthyl)ethyl]-5-(2-dimethylaminobenzyl)amino-2-(S)-[4-[N-(imidazol-2-ylmethyl)aminomethyl]benzoyl]aminopentanoylamide [Compound No. 15]

The compound (28.7 mg) obtained in Example 1-5 was dissolved in methanol (0.3 ml), and the solution was added with 2-(N,N-dimethylamino)benzaldehyde (13.5 mg) that had been synthesized in accordance with a method described in Frank K., Tetrahedron Lett., 24 (21), 2213 (1983) and stirred at room temperature for 0.5 hours. After completion of reaction, the solvent was distilled off, and the residue was dried in vacuum and re-dissolved in methanol (0.6 ml). The solution was ice-cooled, and sodium borohydride (9 mg) was added to the solution. The resultant solution was returned to room temperature and stirred for 30 minutes. After completion of reaction, the solvent was distilled off, and the residue was dissolved in chloroform. The resultant solution was washed with 0.5 mol/l sodium hydroxide and brine and dried with anhydrous sodium sulfate, andthesolventwasdistilledoff. Theresiduewasdissolved in chloroform (0.5ml), and methanesulfonic acid (40 µl) and methanol (40 µl) were added under ice-cooling, followed by stirring at room temperature for 3 hours. After completion of reaction, the solvent was distilled off, and the residue was purified by silica gel column chromatography (chloroform/methanol/water = 7/3/0.5), to thereby obtain a methanesulfonate (17.9 mg) of the subject compound as a white solid.
MS (FAB,Pos.) :m/z=632 [M+1]⁺
¹H-NMR(500MHz,DMSO-d₆) : δ=1.52(3H,d,J=6.8Hz) ,1.62-1.81(4H,m) ,2.4 0 (9H,s) ,2.57(6H,s) ,2.88-2.97 (2H,m) ,4.18 (2H,br) ,4.34 (2H,br) ,4.4 9(2H,br) ,4.51-4.61(1H,m) ,5.71(1H,quint. ,J=6.8Hz) ,7.12-7.19(1H, m) ,7.25-7.32(1H,m) ,7.36-7.44(2H,m) ,7.43-7.62(8H,m) ,7.67(2H,br) ,7.83(1H,d,J=8.1Hz) ,7.94(1H,d,J=7.1Hz) ,7.98(2H,d,J=7.8Hz) ,8.10 (1H,d,J=7.6Hz) ,8.32-8.38(1H,br) ,8.65(2H,br) ,8.70(1H,d,J=7.8Hz)

### Production Example 16: Synthesis of N-[(S)-1-(1-naphthyl)ethyl]-5-(3-n-propyloxypyridin-2-yl)methylamino-2-(S)-[4-[N-(imidazol-2-ylmethyl)aminomethyl]benzoyl]aminopentanoylamide [Compound No. 16]

### Example 16-1: Synthesis of 3-n-propyloxy-2-pyridinealdehyde

Commercially available 3-hydroxy-2-methylpyridine (2.5311g) was dissolved in DMF (25 ml) and added with cesium carbonate (7.54 g), followed by dropping n-propyliodide (2.35 ml). After stirring for 3 hours at room temperature for completing the reaction, the solvent was distilled off and then chloroform was added, followed by washing with distilled water. After the solvent was distilled off, the residue was purified by silica gel column chromatography (chloroform/methanol=20/1), to thereby obtain a brown liquid of 3-n-propyloxy-2-methylpyridine (2.57 g).

A portion (1.00 g) of the obtained product was dissolved in methylene chloride (10 ml), followed by cooling to 0°C, and then mCPBA (1.50 g) was added, followed by stirring at room temperature for 2.5 hours. After completion of reaction, methylene chloride was added, and then the solution was washed with 2 mol/l sodium hydroxide aqueous solution and brine, followed by drying with anhydrous sodium sulfate. Then, the solvent was distilled off to obtain a crude N-oxide product (958.3 mg).

A portion (500 mg) of the obtained product was dissolved in methylene chloride (10 ml), followed by cooling to 0°C, and then trifluoroacetic acid anhydride (0. 826 ml) was added to the solution, followed by stirring at 45°C for 3 hours. After completion of reaction, the solvent was distilled off and the residue was dissolved in methanol (10 ml), followed by adding 1 mol/l sodium hydroxide aqueous solution till pH of the solution became 10. After stirring at room temperature for 1 hour, the solvent was distilled off, and then chloroform was added, followed by washing with distilled water and brine. After drying with anhydrous sodium sulfate, the solvent was distilled off to obtain a brown liquid of the subject product (482.9mg). The obtained product (248.5mg, 1.45 mmol) was dissolved in chloroform (12.5 ml) and then added with manganese dioxide (1.25 g) (chemicals treated, Wako Pure Chemical Industries Ltd.), followed by stirring at 70°C for 1.5 hour. After completion of reaction, the catalyst was removed by filtration with Celite and the solvent was concentrated. The residue was purified by silica gel column chromatography (12.5 g, chloroform/ethyl acetate=1/1), to thereby obtain a light brown liquid of the subject compound (216.9 mg).
MS (EI,Pos.) :m/z= 165 [M⁺]
¹H-NMR(500MHz,CDCl₃) : δ=1.10(3H,t,J=7.5Hz) ,1.92(2H,qd,J=7.5, 6.6H z) ,4.08(2H,t,J=6.6Hz) ,7.41(1H,dd,J=8.5,1.2Hz) ,7.47(1H,dd,J=8.5 ,4.6Hz) ,8.39(1H,dd,J=4.6,1.2Hz) ,10.44(1H,s) .

### Example 16-2: Synthesis of N-[(S)-1-(1-naphthyl)ethyl]-5-(3-n-propyloxypyridin-2-yl)methylamino-2-(S)-[4-[N-(imidazol-2-ylmethyl)aminomethyl]benzoyl]aminopentanoylamide [Compound No. 16]

The compound (29.3 mg) obtained in Example 1-5 was dissolved in methanol (0.3 ml), and the solution was added with 3-n-propoxy-2-pyridinealdehyde (15.6 mg) and stirred at room temperature for 0.5 hours. After completion of reaction, the solvent was distilled off, and the residue was dried in vacuum and re-dissolved in methanol (0.6 ml). The solution was ice-cooled, and sodium borohydride (10mg) wasaddedtothesolution. The resultant solution was returned to room temperature and stirred for 20 minutes. After completion of reaction, the solvent was distilled off, and the residue was dissolved in chloroform. The resultant solution was washed with 0.5 mol/l sodium hydroxide and brine and dried with anhydrous sodium sulfate, and the solvent was distilled off. The residue was dissolved in chloroform (0.9 ml), andmethanesulfonic acid (40 µl) and methanol (40 µl) were added under ice-cooling, followed by stirring at room temperature for 3 hours. After completion of reaction, the solvent was distilled off, and the residue was purified by silica gel column chromatography (chloroform/methanol/water = 7/3/0.5), to thereby obtain a methanesulfonate (36.2 mg) of the subject compound as a white solid.
MS(FAB,Pos.):m/z=648[M+1]⁺
¹H-NMR(500MHz,DMSO-d₆):δ=0.99(3H,t,J=7.1Hz),1.51(3H,d,J=6.8Hz), 1.65-1.83(6H,m),2.36(12H,s),2.98-3.11(2H,m),4.01(2H,t,J=6.6Hz) ,4.24(2H,t,J=5.9Hz),4.35(2H,s),4.49(2H,br),4.52-4.61(1H,m),5.7 1(1H,quint .,J=6.8Hz) ,7.40-7.44(1H,m) ,7.45-7.58(6H,m) ,7.61(2H,d, J=8.5Hz) ,7.70(2H,brs) ,7.82(1H,d,J=8.1Hz) ,7.94(1H,d,J=7.8Hz) ,7 .98(2H,d,J=8.5Hz),8.10(1H,d,J=8.1Hz),8.17(1H,d,J=4.9Hz),8.55(1 H,d,J=8.3Hz),8.72(1H,d,J=7.8Hz),8.94(2H,brs).

### Production Example 17: Synthesis of N-[(S)-1-(1-naphthyl)ethyl]-5-(3-ethoxypyridin-2-yl)methylamino-2-(S)-[4-[N-(imidazol-2-ylmethyl)aminomethyl]benzoyl]aminopentanoylamide [Compound No. 17]

The compound (30.7 mg) obtained in Example 1-5 was dissolved in methanol (0.3 ml), and the solution was added with 3-ethoxy-2-pyridinealdehyde (11.7 mg) that had been synthesized in accordance with a method described in Marsais F. et al., Synthesis, 235 (1982) and stirred at room temperature for 0.5 hours. After completion of reaction, the solvent was distilled off, and the residue was dried in vacuum and re-dissolved in methanol (0.6 ml). The solution was ice-cooled, and sodium borohydride (10 mg) was added to the solution. The resultant solution was returned to room temperature and stirred for 30 minutes. After completion of reaction, the solvent was distilled off, and the residue was dissolved in chloroform. The resultant solution was washed with 0.5 mol/l sodium hydroxide and brine and dried with anhydrous sodium sulfate, and the solvent was distilled off. The residue was dissolved in chloroform (0.9 ml), and methanesulfonic acid (39 µl) and methanol (39 µl) were added under ice-cooling, followed by stirring at room temperature for 3 hours. After completion of reaction, the solvent was distilled off, and the residue was purified by silica gel column chromatography (chloroform/methanol/water = 7/3/0.5), to thereby obtain a methanesulfonate (38.7 mg) of the subject compound as a white solid.
MS (FAB,Pos.) :m/z=634 [M+1]⁺
¹H-NMR(500MHz,DMSO-d₆) : δ=1.35(3H,t,J=7.1Hz) ,1.52(3H,d,J=7.1Hz), 1.64-1.82(4H,m) ,2.35(12H,s) ,2.98-3.10(2H,m) ,4.13(2H,q,J=7.1Hz) ,4.22(2H,t,J=5.8Hz) ,4.29(2H,s) ,4.45(2H,br) ,4.55-4.62(1H,m) ,5.7 1(1H,quint. ,J=7.1Hz) ,7.41-7.43(1H,m) ,7.45-7.70(9H,m) ,7.82(1H,d ,J=8.1Hz) ,7.94(1H,d,J=7.6Hz) ,7.97(2H,d,J=5.9Hz) ,8.09(1H,d,J=8. 3Hz) ,8.17(1H,d,J=4.9Hz) ,8.55(1H,d,J=8.1Hz) ,8.73(1H,d,J=7.8Hz) , 8.93 (2H,brs) .

### Production Example 18: Synthesis of N-[(S)-1-(1-naphthyl)ethyl]-5-[2-(2-hydroxyethoxy)benzyl]amino -2-(S)-[4-[N-(imidazol-2-ylmethyl)aminomethyl]benzoyl]aminopentanoylamide [Compound No. 18]

The compound (31.3 mg) obtained in Example 1-5 was dissolved in methanol (0.3 ml), and the solution was added with 2-(2-hydroxyethoxy)benzaldehyde (10.6 mg) and stirred at room temperature for 0.5 hours. After completion of reaction, the solvent wasdistilledoff, and the residue was dried in vacuum and re-dissolved in methanol (0.6 ml). The solution was ice-cooled, and sodium borohydride (10 mg) was added to the solution. The resultant solution was returned to room temperature and stirred for 60 minutes.

After completion of reaction, the solvent was distilled off, and the residue was dissolved in chloroform. The resultant solution was washed with 0.5 mol/l sodium hydroxide and brine and dried with anhydrous sodium sulfate, and the solvent was distilled off. The residue was dissolved in chloroform (0.9 ml), and methanesulfonic acid (20 µl) and methanol (20 µl) were added under ice-cooling, followed by stirring at room temperature for 3 hours. After completion of reaction, the solvent was distilled off, and the residue was purified by silica gel column chromatography (chloroform/methanol/water = 7/3/0.5), to thereby obtain a methanesulfonate (25.6 mg) of the subject compound as a white solid.
MS (FAB,Pos.) :m/z=649 [M+1]⁺
¹H-NMR(500MHz,DMSO-d₆) :δ=1.52(3H,d,J=6.8Hz) ,1.61-1.81(4H,m) ,2.4 0 (9H,s) ,2.85-2.99 (2H,m) ,3.72 (2H,t,J=5.4Hz) ,4.03-4.12 (4H,m) , 4.2 7(4H,br) ,4.52-4.61(1H,m) ,5.71(1H,quint. ,J=6.8Hz) ,6.99(1H,t,J=7 .6Hz) ,7.09(1H,d,J=8.1Hz) ,7.30-7.41(2H,m) ,7.39-7.62(8H,m),7.82(1H,d,J=8.1Hz) ,7.94(1H,d,J=8.6Hz) ,7.96(2H,d,J=7.7Hz),8.09(1H,d, J=8.5Hz) ,8.49(2H,brs) ,8.51(1H,d,J=8.1Hz) ,8.70(1H,d,J=7.8Hz) .

### Production Example 19: Synthesis of N-[(S)-1-(1-naphthyl)ethyl]-5-(2-trifluoromethoxybenzyl)amino-2-(S)-[4-[N-(imidazol-2-ylmethyl)aminomethyl]benzoyl]aminopentanoylamide [Compound No. 19]

The compound (31.6 mg) obtained in Example 1-5 was dissolved in methanol (0.3 ml), and the solution was added with 2-trifluoromethoxybenzaldehyde (9.0 µl) and stirred at room temperature for 1.5 hours. After completion of reaction, the solvent wasdistilledoff, and the residue was dried in vacuum and re-dissolved in methanol (0.6 ml). The solution was ice-cooled, and sodium borohydride (10mg) was added to the solution. The resultant solution was returned to room temperature and stirred for 60 minutes. After completion of reaction, the solvent was distilled off, and the residue was dissolved in chloroform. The resultant solution was washed with 0.5 mol/l sodium hydroxide and brine and dried with anhydrous sodium sulfate, and the solvent was distilled off. The residuewas dissolved in chloroform (0.9 ml), and methanesulfonic acid (40 µl) and methanol (40 µl) were added under ice-cooling, followed by stirring at room temperature for 2.5 hours. After completion of reaction, the solvent was distilled off, and the residue was purified by silica gel column chromatography (chloroform/methanol/water = 7/3/0.5), to thereby obtain a methanesulfonate (29.9 mg) of the subject compound as a white solid.
MS (FAB,Pos.) :m/z=673 [M+1]⁺
¹H-NMR(500MHz,DMSO-d₆) : δ=1.52(3H,d,J=6.8Hz) ,1.60-1.81(4H,m) ,2.3 4(9H,s) ,2.92-3.07(2H,m) ,4.19(2H,t,J=5.6Hz) ,4.30(2H,br) ,4.42(2H ,br) ,4.53-4.61(1H,m) ,5.71(1H,quint. ,J=6.8Hz) ,7.45-7.68(12H,m), 7.83(1H,d,J=8.1Hz) ,7.95(1H,d,J=7.8Hz),7.97(2H,d,J=8.0Hz),8.09(1H,d,J=7.8Hz) ,8.54(1H,d,J=8.1Hz) ,8.71(1H,d,J=7.8Hz) ,8.84(2H,br s) .

### Production Example 20: Synthesis of N-[(S)-1-(1-naphthyl)ethyl]-5-(2-hydroxybenzyl)amino-2-(S)-[4-[N-(imidazol-2-ylmethyl)aminomethyl]benzoyl]aminopentanoylamide [Compound No. 20]

The compound (30.6 mg) obtained in Example 1-5 was dissolved in methanol (0.3 ml), and the solution was added with 2-trimethylsilyloxybenzaldehyde (11.7 mg) and stirred at room temperature for 1 hour. After completion of reaction, the solvent was distilled off, and the residue was dried in vacuum and re-dissolved in methanol (0.6 ml). The solution was ice-cooled, and sodium borohydride (10mg) was added to the solution. The resultant solution was returned to room temperature and stirred for 30 minutes. After completion of reaction, the solvent was distilled off, and the residue was dissolved in chloroform. The resultant solution was washed with 0.5 mol/l sodium hydroxide and brine and dried with anhydrous sodium sulfate, and the solventwas distilled off. The residuewas dissolved in chloroform (0.9 ml), and methanesulfonic acid (39 µl) and methanol (39 µl) were added under ice-cooling, followed by stirring at room temperature for 1.5 hours. After completion of reaction, the solvent was distilled off, and the residue was purified by silica gel column chromatography (chloroform/methanol/water = 7/3/0.5), to thereby obtain a methanesulfonate (24.5 mg) of the subject compound as a white solid.
MS (FAB,Pos.) :m/z=605 [M+1]⁺
¹H-NMR(500MHz,DMSO-d₆) : δ=1.51 (3H,d,J=6.8Hz) ,1.61-1.81 (4H,m) ,2.3 5 (9H,s) 2.84-2.98 (2H,m) ,4.01 (2H,brs) ,4.31(2H,s) ,4.41 (2H,s) ,4.5 2-4.59(1H,m) ,5.71(1H,quint. ,J=6.8Hz) ,6.83(1H,t,J=7.6Hz) ,6.91(1 H,d,J=8.3Hz) ,7.21-7.30(2H,m) ,7.45-7.65(8H,m) ,7.82(1H,d,J=8.3Hz ) ,7.94 (1H,d,J=7.8Hz) ,7.97 (2H,d,J=8.3Hz) ,8.09(1H,d,J=8.1Hz) ,8.4 9-8.60(3H,m) ,8.71(1H,d,J=7.8Hz) ,10.18(1H,s) .

### Production Example 21: Synthesis of N-[(S)-1-(1-naphthyl)ethyl]-5-(3-isopropyloxypyridin-2-yl)methylamino-2-(S)-[4-[N-(imidazol-2-ylmethyl)aminomethyl]benzoyl]aminopentanoylamide [Compound No. 21]

### Example 21-1: Synthesis of 3-isopropyloxy-2-pyridine aldehyde

A brown liquid of the subject product (96.6 mg) was obtained by a reaction using 2-iodopropane in a same manner as in Example 16-1.
MS (EI,Pos.) :m/z= 165 [M⁺]
¹H-NMR(500MHz,CDCl₃) : δ=1.44(6H,d,J=7.5Hz) ,4.71(1H,sex,J=6.0Hz). 7.42(1H,dd,J=8.7,1.5Hz) ,7.45(1H,dd,J=8.7,4.1Hz) ,8.38(1H,dd,J=4 .1,1.5Hz) ,10.45(1H,s) .

### Example 21-2: Synthesis of N-[(S)-1-(1-naphthyl)ethyl]-5-(3-isopropyloxypyridin-2-yl)methylamino-2-(S)-[4-[N-(imidazol-2-ylmethyl)aminomethyl]benzoyl]aminopentanoylamide [Compound No. 21]

The compound (28.4 mg) obtained in Example 1-5 was dissolved in methanol (0.3 ml), and the solution was added with the compound (12.5 mg) obtained in Example 21-1 and stirred at room temperature for 2 hours. After completion of reaction, the solvent was distilled off, and the residue was dried in vacuum and re-dissolved in methanol (0.6 ml). The solution was ice-cooled, and sodium borohydride (10 mg) was added to the solution. The resultant solution was returned to room temperature and stirred for 60 minutes. After completion of reaction, the solvent was distilled off, and the residue was dissolved in chloroform. The resultant solution was washed with 0.5 mol/l sodium hydroxide and brine and dried with anhydrous sodium sulfate, and the solvent was distilled off. The residuewas dissolved in chloroform (0.9 ml), and methanesulfonic acid (40 µl) andmethanol (40 µl) were added under ice-cooling, followed by stirring at room temperature for 1.5 hours. After completion of reaction, the solvent was distilled off, and the residue was purified by silica gel column chromatography (chloroform/methanol/water = 7/3/0.5), to thereby obtain a methanesulfonate (24.4 mg) of the subject compound as a white solid.
MS(FAB,Pos.):m/z=648[M+1]⁺
¹H-NMR(500MHz,DMSO-d₆) : δ=1.29(6H,d,J=5.9Hz),1.51(3H,d,J=7.1Hz), 1.65-1.82(4H,m),2.35(9H,s),2.98-3.10(2H,m),4.20(2H,t,J=5.9Hz), 4.33(2H,s),4.43(2H,s),4.53-4.61(1H,m),4.72(1H,sex,J=5.9Hz),5.7 1(1H,quint.,J=7.1Hz),7.39-7.43(1H,m),7.45-7.70(9H,m),7.82(1H,d ,J=8.1Hz),7.94(1H,d,J=7.8Hz),7.98(2H,d,J=8.3Hz),8.09(1H,d,J=7. 8Hz) ,8.16(1H,d,J=4.6Hz) ,8.55(1H,d,J=8.1Hz) ,8.72(1H,d,J=7.8Hz), 8.91 (2H,brs) .

### Production Example 22: Synthesis of N-[(S)-1-(1-naphthyl)ethyl]-5-(2-ethylbenzyl)amino-2-(S)-[4-[N -(imidazol-2-ylmethyl)aminomethyl]benzoyl]aminopentanoylamide [Compound No. 22]

The compound (29.6 mg) obtained in Example 1-5 was dissolved in methanol (0.3 ml), and the solution was added with 2-ethylbenzaldehyde (14.9 mg) and stirred at room temperature for 2 hours. After completion of reaction, the solvent was distilled off, and the residue was dried in vacuum and re-dissolved in methanol (0.6 ml). The solution was ice-cooled, and sodium borohydride (10 mg) was added to the solution. The resultant solution was returned to room temperature and stirred for 60 minutes. After completion of reaction, the solvent was distilled off, and the residue was dissolved in chloroform. The resultant solution was washed with 0.5 mol/l sodium hydroxide and brine and dried with anhydrous sodium sulfate, and the solvent was distilledoff. The residuewas dissolved in chloroform (0.9 ml), and methanesulfonic acid (33 µl) and methanol (33 µl) were added under ice-cooling, followed by stirring at room temperature for 1.5 hours. After completion of reaction, the solvent was distilled off, and the residue was purified by silica gel column chromatography (chloroform/methanol/water = 7/3/0.5), to thereby obtain a methanesulfonate (24.4 mg) of the subject compound as a white solid.
MS(FAB,Pos.):m/z=617[M+1]⁺
¹H-NMR(500MHz,DMSO-d₆) : δ=1.13(3H,t,J=7.6Hz) ,1.52(3H,d,J=6.8Hz), 1.64-1.81(4H,m),2.35(9H,s),2.66(2H,q,J=7.6Hz),2.95-3.05(2H,m), 4.10 (2H,t,J=5.1Hz) ,4.30(2H,s) ,4.37(2H,brs) ,4.56-4.62(1H,m) ,5.7 2(1H,quint. ,J=6.8Hz) ,7.23-7.30(2H,m) ,7.36(1H,td,J=7.6,1.2Hz) ,7 .40 (1H,d,J=7.8Hz) ,7.45-7.65 (8H,m) ,7.83 (1H,d,J=8.3Hz) ,7.94 (1H,d ,J=7.8Hz) ,7.98(2H,d,J=8.1Hz) ,8.10(1H,d,J=8.3Hz) ,8.55(1H,d,J=7. 3Hz) ,8.64(2H,brs) ,8.73(1H,d,J=7.8Hz) .

### Production Example 23: Synthesis of N-[(S)-1-(1-naphthyl)ethyl]-5-(2-isopropyloxybenzyl)amino-2-(S)-[4-[N-(imidazol-2-ylmethyl)aminomethyl]benzoyl]aminopentanoylamide [Compound No. 23]

The compound (31.0 mg) obtained in Example 1-5 was dissolved in methanol (0.3 ml), and the solution was added with 2-isopropyloxybenzaldehyde (14.7mg) and stirred at room temperature for 1.5 hours. After completion of reaction, the solvent was distilled off, and the residue was dried in vacuum and re-dissolved in methanol (0.6 ml). The solution was ice-cooled, and sodium borohydride (10mg) was added to the solution. The resultant solution was returned to room temperature and stirred for 90 minutes. After completion of reaction, the solvent was distilled off, and the residue was dissolved in chloroform. The resultant solution was washed with 0.5 mol/l sodium hydroxide and brine and dried with anhydrous sodium sulfate, andthesolventwasdistilledoff. Theresiduewasdissolved in chloroform (0.9 ml), and methanesulfonic acid (33 µl) and methanol (33 µl) were added under ice-cooling, followed by stirring at room temperature for 2 hours. After completion of reaction, the solvent was distilled off, and the residue was purified by silica gel column chromatography (chloroform/methanol/water = 7/3/0.5), to thereby obtain a methanesulfonate (18.8 mg) of the subject compound as a white solid.
MS (FAB,Pos.) :m/z=647 [M+1]⁺
¹H-NMR(500MHz,DMSO-d₆) : δ=1.25(3H,d,J=6.1Hz) ,1.26(3H,d,J=6.1Hz), 1.52(3H,d,J=6.8Hz) ,1.60-1.80(4H,m) ,2.33(9H,s) ,2.84-2.98(2H,m), 4.02(2H,t,J=5.9Hz) ,4.23(4H,s) ,4.54-4.60(1H,m) ,4.65(1H,sex,J=6. 1Hz) ,5.71(1H,quint. ,J=6.8Hz) ,6.96(1H,t,J=7.6Hz) ,7.09(1H,d,J=8. 1Hz) ,7.36-7.40(2H,m) ,7.40-7.61(8H,m) ,7.83(1H,d,J=7.8Hz) ,7.95(1 H,d,J=7.6Hz) ,7.96(2H,d,J=8.5Hz) ,8.09(1H,d,J=7.8Hz),8.50(2H,brs),8.53(1H,d,J=8.3Hz) ,8.70(1H,d,J=7.8Hz).

### Production Example 24: Synthesis of N-[(S)-1-(1-naphthyl)ethyl]-5-(2-morpholinobenzyl)amino-2-(S)-[4-[N-(imidazol-2-ylmethyl)aminomethyl]benzoyl]aminopentanoylamide [Compound No. 24]

The compound (29.9 mg) obtained in Example 1-5 was dissolved in methanol (0.3 ml), and the solution was added with 2-morpholinobenzaldehyde (15.4 mg) and stirred at room temperature for 1.5 hours. After completion of reaction, the solvent was distilled off, and the residue was dried in vacuum and re-dissolved in methanol (0.6 ml). The solution was ice-cooled, and sodium borohydride (10mg) wasaddedtothesolution. Theresultantsolution was returned to room temperature and stirred for 90 minutes. After completion of reaction, the solvent was distilled off, and the residue was dissolved in chloroform. The resultant solution was washed with 0.5 mol/l sodium hydroxide and brine and dried with anhydrous sodium sulfate, andthesolventwasdistilledoff. Theresiduewasdissolved in chloroform (0.9 ml), and methanesulfonic acid (44 µl) and methanol (44 µl) were added under ice-cooling, followed by stirring at room temperature for 1 hour. After completion of reaction, the solvent was distilled off, and the residue was purified by silica gel column chromatography (chloroform/methanol/water = 7/3/0.5), to thereby obtain a methanesulfonate (34.2 mg) of the subject compound as a white solid.
MS(FAB,Pos.):m/z=674[M+1]⁺
¹H-NMR(500MHz,DMSO-d₆):δ=1.52(3H,d,J=7.1Hz),1.63-1.81(4H,m),2.3 6(9H,s),2.80(4H,t,J=4.6Hz),2.91-3.03(2H,m),3.68(4H,t,J=4.6Hz), 4.17(2H,t,J=5.6Hz),4.31(2H,s),4.41(2H,s),4.56-4.61(1H,m),5.72(1H,quint.,J=7.1Hz),7.20(1H,td,J=7.3,1.0Hz),7.28(1H,d,J=8.0Hz), 7.40-7.61(10H,m),7.83(1H,d,J=8.3Hz),7.95(1H,d,J=7.6Hz),7.99(2H ,d,J=8.3Hz),8.10(1H,d,J=8.1Hz),8.56(1H,d,J=8.3Hz),8.63(2H,brs) ,8.72(1H,d,J=7.8Hz).

### Production Example 25: Synthesis of N-[(S)-1-(1-naphthyl)ethyl]-5-(2-(4-methylpiperazino)benzyl)amino-2-(S)-[4-[N-(imidazol-2-ylmethyl)aminomethyl]benzoyl]aminopentanoylamide [Compound No. 25]

The compound (30.2 mg) obtained in Example 1-5 was dissolved in methanol (0.3 ml), and the solution was added with 2-(4-methylpiperazino)benzaldehyde (25.2 mg) and stirred at room temperature for 1.5 hours. After completion of reaction, the solvent wasdistilledoff, and the residue was dried in vacuum and re-dissolved in methanol (0.6 ml). The solution was ice-cooled, and sodium borohydride (10 mg) was added to the solution. The resultant solution was returned to room temperature and stirred for 90 minutes. After completion of reaction, the solvent was distilled off, and the residue was dissolved in chloroform. The resultant solution was washed with 0.5 mol/l sodium hydroxide and brine and dried with anhydrous sodium sulfate, andthe solventwas distilled off. The residuewas dissolved in chloroform (0.9 ml), and methanesulfonic acid (55 µl) and methanol (55 µl) were added under ice-cooling, followed by stirring at room temperature for 1.5 hours. After completion of reaction, the solvent was distilled off, and the residue was purified by silica gel column chromatography (chloroform/methanol/water = 7/3/0.5), to thereby obtain a methanesulfonate (37.5 mg) of the subject compound as a white solid.
MS (FAB,Pos.) :m/z=687 [M+1]⁺
¹H-NMR(500MHz,DMSO-d₆) : δ=1.52 (3H,d,J=6.8Hz) ,1.65-1.83 (4H,m) ,2.3 7(12H,s) ,2.84(3H,s) ,2.91-3.01(4H,m) ,3.02-3.14(2H,m),3.14-3.27(2H,m),3.29-3.50(2H,m) ,4.15(2H,t,J=5.9Hz) ,4.32(2H,s) ,4.44(2H,s) ,4.56-4.62(1H,m) ,5.72(1H,quint.,J=6.8Hz) ,7.25(1H,td,J=7.5,1.0H z) ,7.29(1H,d,J=8.1Hz) ,7.44(1H,td,J=7.5,1.5Hz) ,7.45-7.65(9H,m), 7.83(1H,d,J=8.1Hz) ,7.95(1H,d,J=7.5Hz),7.98(2H,d,J=8.3Hz),8.10(1H,d,J=8.1Hz) ,8.57(1H,d,J=8.3Hz) ,8.58(2H,brs) ,8.73(1H,d,J=7.3H z) ,9.60(1H,brs) .

### Production Example 26: Synthesis of N-[(S)-1-(1-naphthyl)ethyl]-5-(3-methylpyrrol-2-yl)methylamino -2-(S)-[4-[N-(imidazol-2-ylmethyl)aminomethyl]benzoyl]aminopentanoylamide [Compound No. 26]

The compound (40.2 mg) obtained in Example 1-5 was dissolved in methanol (0.3 ml), and the solution was added with 3-methylpyrrol-2-yl aldehyde (12.7 mg), followed by stirring at room temperature for 1 hour. After completion of reaction, the solvent was distilled off and dried in vacuum, followed by re-dissolving in methanol (0.6 ml). After ice-cooling the solution, sodiumborohydride (10 mg) was added. Then, the solution was returned to room temperature and stirred for 60 minutes. After completion of reaction, the solvent was distilled off and the residue was dissolved in chloroform, followed by washing with 0.5 mol/l sodium hydroxide and brine and then drying with anhydrous sodium sulfate. After drying, the solvent was distilled off and the residue was dissolved in methanol (0.9 ml). Under ice-cooling, 4 mol/l hydrochloric acid/dioxane (0.4 ml) was gradually added to the solution and stirred for 70 minutes under ice-cooling. After completion of reaction, the solvent was distilled off, followed by distilling azeotropically with methanol, to thereby obtain a white violet solid of the hydrochloride (15.4 mg) of the subject compound.
MS (FAB,Pos.) :m/z=592 [M+1]⁺
¹H-NMR(500MHz,DMSO-d₆) : δ=1.52 (3H,d,J=6.8Hz) ,1.62-1.83 (4H,m) ,2.0 1(3H,s) ,2.78-2.89(2H,m) ,3.99(2H,t,J=5.6Hz) ,4.33(2H,s) ,4.44(2H, s) ,4.54-4.62(1H,m) ,5.70(1H,quint. ,J=6.8Hz) ,5.86(1H,d,J=2.6Hz), 6.02(1H,d,J=2.6Hz) ,7.46-7.57 (4H,m) ,7.61 (2H,s) ,7.66 (2H,d,J=8.3H z) ,7.82(1H,d,J=8.1Hz) ,7.94(1H,d,J=7.8Hz) ,7.98(2H,d,J=8.3Hz) ,,8 .10(1H,d,J=8.5Hz) ,8.59(1H,d,J=8.3Hz) ,8.85(1H,d,J=7.8Hz) ,8.89(2 H,brs) ,10.71(1H,s).

### Production Example 27: Synthesis of N-[(S)-1-(1-naphthyl)ethyl]-5-cyclohexylamino-2-(S)-[4-[N-(imidazol-2-ylmethyl)aminomethyl]benzoyl]aminopentanoylamide [Compound No. 27]

The compound (307.6 mg) obtained in Example 1-5 was dissolved in methanol (6 ml), and then added with cyclohexanone (62.3 µl), sodium cyano borohydride (65.0 mg), and acetic acid (0.3 ml), followed by stirring at room temperature for 24 hours. After completion of reaction, the resultant solution was concentrated and added with chloroform, followed by washing with 1N sodium hydroxide and brine. The solvent was distilled off, and the residue was purified by silica gel column chromatography (chloroform/methanol/water=7/3/0.5) to obtain an intermediate. The intermediate was dissolved in methanol (1 ml) and then added with 4M hydrochloric acid/dioxane (1 ml), followed by stirring at room temperature for 5.5 hours. After completion of reaction, the solvent was concentrated, and the residue was purified by silica gel column chromatography (chloroform/methanol/water=7/3/0.5), followed by concentrating the obj ective fraction. The concentrate was dissolved in a solution of chloroform/ethnol=10/2, and then the solution was filtrated and concentrated, to thereby obtain a white solid of a hydrochloride (99.4 mg) of the subject compound.
MS(FAB,Pos.):m/z=581[M+1]⁺
¹H-NMR(500MHz,DMSO-d₆) : δ=1.02-1.12(1H,m) ,1.14-1.32(4H,m), 1.52(3 H,d,J=6.8Hz),1.55-1.61(1H,m),1.62-1.89(6H,m),1.95-2.05(2H,m),2 .81-2.95(3H,m),4.33(2H,s),4.42(2H,s),4.58-4.64(1H,m),5.71(1H,q uint.,J=6.8Hz),7.47-7.60(6H,m),7.66(2H,d,J=8.3Hz),7.82(1H,d,J= 7.8Hz),7.94(1H,d,J=7.8Hz),7.99(2H,d,J=8.3Hz),8.11(1H,d,J=8.5Hz ),8.63(1H,d,J=8.5Hz),8.74(1H,br),8.81(1H,br),8.88(1H,d,J=7.6Hz).

### Production Example 28: Synthesis of N-[(S)-1-(1-naphthyl)ethyl]-5-(1,2,3,4-tetrahydronaphthalen-1-yl)amino-2-(S)-[4-[N-(imidazol-2-ylmethyl)aminomethyl]benzoyl]aminopentanoylamide [Compound No. 28]

### Example 28-1: Synthesis of N-[(S)-1-(1-naphthyl)ethyl]-5-(1,2,3,4-tetrahydronaphthalen-1-yl)amino-2-(S)-[4-[N-Boc-N-(imidazol-2-ylmethyl)aminomethyl]benzoyl]aminopentanoylamide (Compound XIIa-4)

The compound (201.4 mg) obtained in Example 1-5 was dissolved in methanol (6 ml), and the solution was added with α-tetralone (88.9 µl), sodium cyano borohydride (42.1 mg), and acetic acid (0.2 ml) and stirred at room temperature for 3 days. After completion of reaction, the solution was concentrated, and chloroform was added to the concentrate. The resultant solution was washed with 1N sodium hydroxide and brine. After drying with anhydrous sodium sulfate, the solvent was distilled off, and the residue was purified by silica gel column chromatography (chloroform/methanol/water = 7/3/0.5), to thereby obtain the subject compound (11.0 mg) as a pale orange solid.
MS (FAB,Pos.) :m/z=729 [M+1]⁺
¹H-NMR(500MHz,DMSO-d₆) : δ=1.30-1.62(2H,m) ,1.35 and 1.38(9H,2s) ,1.5 1(3H,d,J=6.8Hz) ,1.63-1.88(6H,m) ,2.50-2.78(4H,m) ,3.57-3.66(1H,m ) ,4.28-4.59(5H,m) ,5.71(1H,quint. ,J=6.8Hz) ,6.84(2H,s) ,7.00-7.16 (4H,m) ,7.20-7.37(3H,m) ,7.45(1H,td,J=7.6,2.7Hz) ,7.49-7.59(3H,m) ,7.80-7.88(3H,m) ,7.93(1H,d,J=7.8Hz) ,8.10(1H,d,J=7.3Hz) ,8.38(1H ,d,J=6.8Hz) ,8.65(1H,d,J=5.1Hz) ,11.83 and 11.95(1H,2brs) .

### Example 28-2: Synthesis of N-[(S)-1-(1-naphthyl)ethyl]-5-(1,2,3,4-tetrahydronaphthalen-1-yl)amino-2-(S)-[4-[N-(imidazol-2-ylmethyl)aminomethyl]benzoyl] aminopentanoylamide [Compound No. 28]

The compound (9.5 mg) obtained in Example 28-1 was dissolved in methanol (0.2 ml), and the solution was added with 4 mol/l dioxane (0.2 ml) and stirred at room temperature for 1.5 hours. After completion of reaction, the solvent was distilled off, and the residue was purified by silica gel column chromatography (chloroform/methanol/water = 7/3/0.5), to thereby obtain a hydrochloride (8.5 mg) of the subject compound as a pale orange solid.
MS (FAB,Pos.) :m/z=629 (M+1)⁺
¹H-NMR(500MHz,DMSO-d₆) : δ=1.52(3H,d,J=6.8Hz) ,1.71-1.1.82(5H,m) ,1 .82-2.00(2H,m) ,2.03-2.14(1H,m) ,2.69-2.77(1H,m) ,2.79-2.84(1H,m) ,2.85-3.01(2H,m) ,4.30(2H,s) ,4.37(2H,s) ,3.41-3.47(1H,m) ,4.55-4. 62(1H,m) ,5.71(1H,quint. ,J=6.8Hz) ,7.18-7.25(2H,m) ,7.30(1H,t,J=7 .3Hz) ,7.48-7.59(7H,m) ,7.64(2H,d,J=8.1Hz) ,7.82(1H,d,J=7.8Hz) ,7. 94(1H,d,J=7.6Hz) ,7.99(2H,d,J=8.1Hz) ,8.11(1H,d,J=7.8Hz) ,8.61(1H ,d,J=8.1Hz) ,8.82 (1H,d,J=7.6Hz) ,8.88 (1H,brs) ,9.01 (1H,brs) .

### Production Example 29: Synthesis of N-[(S)-1-(1-naphthyl)ethyl]-5-(indan-1-yl)amino-2-(S)-[4-[N-(imidazol-2-ylmethyl)aminomethyl]benzoyl]aminopentanoylamide [Compound No. 29]

### Example 29-1: Synthesis of N-[(S)-1-(1-naphthyl)ethyl]-5-(indan-1-yl)amino-2-(S)-[4-[N-Boc-N-(imidazol-2-ylmethyl)aminomethyl]benzoyl]aminopentanoylamide (Compound XIIa-5)

The compound (201.4 mg) obtained in Example 1-5 was dissolved in methanol (6ml), and the solution was added with 1-indanone (409.4 mg), sodium cyano borohydride (266.2 mg), and acetic acid (0.3 ml) and stirred at room temperature for 10 days. After completion of reaction, the solution was concentrated, and chloroform was added to the concentrate. The resultant solution was washed with 1N sodium hydroxide and brine. After drying with anhydrous sodium sulfate, the solvent was distilled off, and the residue was purified by silica gel column chromatography (chloroform/methanol = 20/1), to thereby obtain the subject compound (233.5 mg) as a white solid.
MS (FAB,Pos.) :m/z=715 [M+1]⁺
¹H-NMR(500MHz,DMSO-d₆) : δ=1.35 and 1.37(9H,2s) ,1.52(3H,d,J=7.0Hz), 1.55-1.70(2H,m) ,1.65-1.83(2H,m) ,1.87-2.06(1H,m) ,2.08-2.21(1H,m ) ,2.72-2.90(3H,m) ,3.96-3.08(1H,m) ,4.30-4.61(6H,m) ,5.72(1H,quin t. ,J=7.0Hz) ,6.85(1H,brs) ,7.04(1H,brs) ,7.19-7.35(5H,m) ,7.42-7.5 7(5H,m) ,7.82(1H,d,J=8.1Hz) ,7.87(2H,d,J=8.2Hz) ,7.94(1H,d,J=7.5H z) ,8.11(1H,d,J=8.1Hz) ,8.45(1H,dd,J=8.1,2.7Hz) ,8.70(1H,brs) ,11. 86 and 11.97 (1H,2br).

### Example 29-2: Synthesis of N-[(S)-1-(1-naphthyl)ethyl]-5-(indan-1-yl)amino-2-(S)-[4-[N-(imidazol-2-ylmethyl)aminomethyl]benzoyl]aminopentanoylamide [Compound No. 29]

The compound (228.1 mg) obtained in Example 29-1 was dissolved in chloroform (6.6 ml), and methanesulfonic acid (144 µl) was added to the solution under ice-cooling, followed by stirring at room temperature for 9 hours. After completion of reaction, the solvent was distilled off, and the residue was purified by silica gel column chromatography (chloroform/methanol/water = 7/3/0.5), to thereby obtain a methanesulfonate (214.8 mg) of the subject compound as a white solid.
MS (FAB,Pos.) :m/z=615 [M+1]⁺
¹H-NMR(500MHz,DMSO-d₆) : δ=1.52(3H,d,J=6.8Hz) ,1.62-1.85(4H,m) ,2.0 5-2.17(1H,m) ,2.30-2.47(1H,m) ,2.36(9H,s) ,2.80-2.90(1H,m) ,2.90-3 .00(2H,m) ,3.00-3.10(1H,m) ,4.32(2H,br) ,4.41(2H,br) ,4.55-4.63(1H ,m) ,4.67-4.75(1H,m) ,5.72(1H,quint. ,J=6.8Hz) ,7.25-7.31(1H,m) ,7. 36(2H,s) ,7.48-7.72(9H,m) ,7.83(1H,d,J=8.1Hz) ,7.95(1H,d,J=7.6Hz) ,7.98(2H,d,J=8.1Hz) ,8.11(1H,d,J=8.1Hz) ,8.55(1H,d,J=7.8Hz) ,8.66-8.82(3H,m) .

### Production Example 30: Synthesis of N-[(S)-1-(1-naphthyl)ethyl]-5-(1-methylpiperidin-4-yl)amino-2-(S)-[4-[N-(imidazol-2-ylmethyl)aminomethyl]benzoyl]aminopentanoylamide [Compound No. 30]

The compound (0.050 g) obtained in Example 1-5 was dissolved in methanol (2 ml), and then 1-methyl-4-piperidone (15 µl) was added to the solution, followed by stirring at room temperature for 3 hours. The solution was further added with molecular sieve 3A powder (0.1 g) and acetic acid (10 µl) and stirred for additional 2 hours. The reaction solution was filtrated and the solvent in the filtrate wasdistilledoffunderreducedpressure. Afterdryingusingavacuum pump, the residue was dissolved in methanol, and the solution was added with sodium cyano borohydride (0.016 mg) and then stirred at room temperature for 5 hours. After completion of reaction, the residuewasdissolvedinchloroform, followedbyaddingwater. After extracting with chloroform three times, the organic layer was washed with brine and then dried with anhydrous sodium sulfate. After filtration, the solvent was distilled off under reduced pressure. The residue was dissolved in chloroform (2 ml), and then the solution was added with methanesulfonate (45 µl) and stirred at room temperature for 2.5 hours. After completion of reaction, the solvent was distilled off under reduced pressure, and the residue was purified by silica gel column chromatography (chloroform/methanol/water=7/3/0.5), to thereby obtain a methanesulfonate (0.032 g) of the subject compound as a white solid.
MS (FAB,Pos.) :m/z=596 [M+1]⁺
¹H-NMR(500MHz,DMSO-d₆) : δ=1.52(3H,d,J=6.8Hz) ,1.64-1.78(6H,m) ,2.1 9(2H,d,J=13.7Hz) ,2.39(15H,s) ,2.77(3H,s) ,2.95-2.99(4H,br),3.24(1H,m),4.36(2H,brs),4.53(2H,brs),4.57(1H,qui,J=6.8Hz),5.74(1H,q uint.,J=6.8Hz),7.47-7.59(4H,m),7.62(2H,d,J=8.3Hz),7.83(1H,d,J= 8.3Hz),7.95(1H,d,J=9.0Hz),7.99(2H,d,J=8.1Hz),8.10(3H,d,J=8.1Hz ),8.57(1H,d,J=8.1Hz),8.63(2H,br),8.71(1H,d,J=7.8Hz),9.43(1H,br s).

### Production Example 31: Synthesis of N-[(S)-1-(1-naphthyl)ethyl]-5-(pentan-3-yl)amino-2-(S)-[4-[N-(imidazol-2-ylmethyl)aminomethyl]benzoyl]aminopentanoylamide [Compound No. 31]

The compound (0.052 g) obtained in Example 1-5 was dissolved in methanol (2 ml), and the solution was added with 3-pentanone (0.036 ml), acetic acid (0.086 ml), and sodium cyano borohydride (24 mg) and stirred at room temperature overnight. After completion of reaction, the solvent was distilled off under reduced pressure, and the residue was dissolved in chloroform. Then, 0.5 mol/l sodium hydroxide aqueous solution was added to the solution for washing. The aqueous layer was subjected to extraction with chloroform three times, and the organic layer was washed with brine, followed by drying with anhydrous sodium sulfate. After filtration, the solvent was distilled off under reduced pressure, and the residue was purified by silica gel column chromatography (chloroform/methanol = 5/1). The purified product was dissolved in chloroform (2 ml), and the solution was added with methanesulfonic acid (0.031 ml) and stirred at room temperature overnight. After completion of reaction, the solvent was distilled off under reduced pressure, and the residue was purified by silica gel column chromatography (chloroform/methanol/water = 7/3/0.5), to thereby obtain a methanesulfonate (0.032 g) of the subject compound as a white solid.
MS (FAB,Pos.) :m/z=569 [M+1]⁺
¹H-NMR(500MHz,DMSO-d₆) : δ=0.84(6H,t,J=7.3Hz) , 1.50-1.77(8H,m) ,1.5 2 (3H,d,J=6.8Hz) ,2.32 (9H,s) ,2.85-2.93 (3H,br) ,4.25 (4H,brs) ,4.58-4.62(1H,m) ,5.72(1H,t,J=7.3Hz) ,7.48-7.59(8H,m) ,7.84(1H,d,J=8.3H z) ,4.34(2H,brs) ,7.95(3H,t,J=8.1Hz) ,8.10(3H,d,J=7.6Hz) ,8.53(1H, d,J=8.1Hz) ,8.72(1H,d,J=7.8Hz) .

### Production Example 32: Synthesis of N-[(S)-1-(1-naphthyl)ethyl]-5-dimethylamino-2-(S)-[4-[N-(imidazol-2-ylmethyl)aminomethyl]benzoyl]aminopentanoylamide [Compound No. 32]

### Example 32-1: Synthesis of N-[(S)-1-(1-naphthyl)ethyl]-5-dimethylamino-2-(S)-[4-[N-(imidazol-2-ylmethyl)aminomethyl]benzoyl]aminopentanoylamide (Compound XIIa-6)

The compound (0.200 g) obtained in Example 1-5 was dissolved in methanol (2 ml), and the solution was added with 36 % formaldehyde aqueous solution (0.550 ml) and stirred at room temperature for 30 minutes. After cooled to 0°C, the solution was added with sodium borohydride (0.103 g) and stirred at 0°C for 15 minutes and then at room temperature overnight. After completion of reaction, the solvent was distilled off under reduced pressure. The residue was dissolved in chloroform and washed with 1 mol/l sodium hydroxide aqueous solution. Then, the solution was extracted with chloroform, washed with brine and dried with sodium anhydrous sulfate. After filtration, the solvent was distilled off under reduced pressure, and then the residue was purified by silica gel column chromatography (chloroform/methanol/water=7/3/0.5), to obtain the subject compound (0.173 g).
MS(FAB,Pos.) :m/z=627[M+1]⁺
¹H-NMR(500MHz,DMSO-d₆) : δ=1.35(9H,s) ,1.51(3H,d,J=6.8Hz) ,1.63-1.7 2(2H,br) ,2.06(6H,s) ,2.19-2.20(2H,br) ,4.33-4.52(5H,m) ,5.72(1H,q uint. ,J=6.8Hz) ,6.85(1H,brs) ,7.04(1H,brs) ,7.25(2H,br) ,7.46-7.56 (4H,m) ,7.82-7.85(3H,m) ,7.94(1H,d,J=7.3Hz) ,8.11(1H,d,J=7.8Hz) ,8 .45-8.47(1H,br) ,8.65(1H,d,J=7.6Hz) ,11.83-11.95(1H,br)

### Example 32-2: Synthesis of N-[(S)-1-(1-naphthyl)ethyl]-5-dimethylamino-2-(S)-[4-[N-(imidazol-2-ylmethyl)aminomethyl]benzoyl]aminopentanoylamide [Compound No. 32]

The compound (0.173 g) obtained in Example 1-5 was dissolved in chloroform (3 ml), and the solution was added with methanesulfonic acid (0.159 ml) and stirred at room temperature for 3 hours. After completion of reaction, the solvent was distilled off under reduced pressure, and the residue was purified by silica gel column chromatography (chloroform/methanol/water = 7/3/0.5), to thereby obtain a methanesulfonate (0.139 g) of the subject compound as a white solid.
MS(FAB,Pos.):m/z=527[M+1)⁺
¹H-NMR(500MHz,DMSO-d₆):δ=1.52(3H,d,J=6.8Hz),1.55-1.73(4H,m),2.3 0(9H,s),2.67(6H,m),3.01(2H,dd,J=13.4,7.6Hz),4.33(2H,brs),4.45(2H,brs),5.56(1H,quint.,J=6.8Hz),7.48-7.58(4H,m),7.60(2H,d,J=8. 5Hz),7.65(2H,brs),7.84(1H,d,J=8.1Hz),7.95(1H,d,J=8.1Hz),7.98(2 H,d,J=8.5Hz),8.11(1H,d,J=8.3Hz),8.56(1H,d,J=7.8Hz),8.74(1H,d,J =8.1Hz) ,9.25(1H,brs) .

### Production Example 33: Synthesis of N-[(S)-1-(1-naphthyl)ethyl]-5-diisobutylamino-2-(S)-[4-[N-(imidazol-2-ylmethyl)aminomethyl)benzoyl]aminopentanoylamide [Compound No. 33]

The compound (0.300 g) obtained in Example 1-5 was dissolved in methanol (5 ml), and the solution was added with isobutyl aldehyde (0.160 ml), acetic acid (0.5 ml), and sodium cyano borohydride (0.195 g) and stirred at room temperature for 2 days. The solvent was distilled off under reduced pressure, and the residue was dissolved in chloroform. Then, 0.5 mol/l sodium hydroxide aqueous solution was added to the solution for washing. The aqueous layer was subj ected to extraction with chloroform three times, and the organic layer was washed with brine, followed by drying with anhydrous sodium sulfate. After filtration, the solvent was distilled off under reduced pressure, and the residue was purified by silica gel column chromatography (chloroform/methanol = 5/1). The purified product was dissolved in chloroform (3 ml), and the solution was added with methanesulfonic acid (0.077 ml) and stirred at room temperature overnight. After completion of reaction, the solvent was distilled off under reduced pressure, and the residue was purified by silica gel column chromatography (chloroform/methanol/water = 7/3/0.5), to thereby obtain amethanesulfonate (0.129 g) of the subject compound as a white solid.
MS(FAB,Pos.):m/z=611[M+1]⁺
¹H-NMR(500MHz,DMSO-d₆):δ=0.86-1.07(12H,m),1.53(3H,d,J=6.8Hz),1. 57-1.80(4H,m),1.92-2.02(2H,m),2.36(9H,s),2.74-2.89(4H,m),3.03-3.11(2H,br),4.32(2H,s),4.43(2H,s),4.59(1H,dd,J=14.2,7.9Hz),5.7 3(1H,quint. ,J=6.8Hz) , ,7.48-7.61(8H,m) ,7.84(1H,d,J=8.3Hz) ,7.95 -7.99(3H,m) ,8.12(1H,d,J=8.1Hz) ,8.59(1H,d,J=8.1Hz) ,8.75(1H,d,J= 7.8Hz).

### Production Example 34: Synthesis of N-[(S)-1-(1-naphthyl)ethyl]-5-di-n-propylamino-2-(S)-[4-[N-(imidazol-2-ylmethyl)aminomethyl]benzoyl]aminopentanoylamide [Compound No. 34]

The compound (52.4 mg) obtained in Example 1-5 was dissolved in methanol (1.5 ml), and the solution was added with propionaldehyde (0.015 ml) and sodium cyano borohydride (11 mg) and stirred at room temperature for 13 hours. After completion of reaction, the solvent was distilled off, and the residue was dissolved in chloroform. The resultant solvent was washed with 0.5 mol/l sodium hydroxide and brine and dried with anhydrous sodium sulfate.

The resultant was dissolved in chloroform (1.0 ml), and the solution was added with methanesulfonic acid (0.022 ml) and methanol (0.022 ml) and stirred at room temperature for 14 hours. After completion of reaction, the solventwas distilledoff, and the residue was purified by silica gel column chromatography (1.2 g, chloroform/methanol/water = 7/3/0.5), to thereby obtain a methanesulfonate (33. 6 mg) of the subject compound as a white solid.
MS (FAB,Pos.) :m/z=583 [M+1]⁺
¹H-NMR(500MHz,DMSO-d₆) : δ=0.83(3H,t,J=7.4Hz) ,0.84(3H,t,J=7.4Hz), 1.52(3H,d,J=6.8Hz) ,1.50-1.80(8H,m) ,2.36(9H,s) ,2.82-2.95(4H,m), 2.96-3.08(2H,m) ,4.31(2H,s) ,4.40(2H,s) ,4.55-4.61(1H,m) ,5.73(1H, quint.) ,7.48-7.63(8H,m) ,7.83(1H,d,J=8.3Hz) ,7.95(1H,d,J=9.0Hz), 7.97(2H,d,J=8.1Hz) ,8.11(1H,d,J=8.1Hz),8.57(1H,d,J=8.3Hz),8.73(1H,d,J=7.8Hz) ,8.89(1H,brs) .

### Production Example 35: Synthesis of N-[(S)-1-(1-naphthyl)ethyl]-5-di-n-butylamino-2-(S)-[4-[N-(imidazol-2-ylmethyl)aminomethyl]benzoyl]aminopentanoylamide [Compound No. 35]

The compound (0.05 g) obtained in Example 1-5 was dissolved in methanol (2 ml) , and the solution was added with n-butyl aldehyde (22 µl), acetic acid (50 µl), and sodium cyano borohydride (32 mg) and stirred at room temperature overnight. After completion of reaction, the solvent was distilled off under reduced pressure, and the residue was dissolved in chloroform. Then, 0.5 mol/l sodium hydroxide aqueous solution was added to the solution. The aqueous layer was subjected to extraction with chloroform three times, and the organic layer was washed with brine, followed by drying with anhydrous sodium sulfate. After filtration, the solvent was distilled off under reduced pressure, and the residue was purified by silica gel column chromatography (chloroform/methanol = 20/1). The purified product was dissolved in chloroform (2 ml), and the solution was added with methanesulfonic acid (18 µl) and stirred at room temperature overnight. After completion of reaction, the solvent was distilled off under reduced pressure, and the residue was purified by silica gel column chromatography (chloroform/methanol/water = 7/3/0.5), to thereby obtain a methanesulfonate (0.010 g) of the subject compound as a white solid.
MS (FAB,Pos.) :m/z=597 [M+1]⁺
¹H-NMR(500MHz, DMSO-d₆) : δ=0.82 (6H,t,J=7.3Hz) ,1.20 (2H,q,J=7.3Hz), 1.24(4H,brs) ,1.34(2H,brs) ,1.51(3H,d,J=7.1Hz) ,1.65-1.73(2H,br), 2.47(2H,brs) ,3.69(2H,s) ,3.75(2H,s) ,4.54(1H,dd,J=8.5,5.9Hz) ,5.7 3(1H,quint. ,J=7.1Hz) ,6.92(2H,s) ,7.43-7.49(3H,m) ,7.51-7.56(3H,m ) ,7.82(1H,d,J=8.1Hz) ,7.85(3H,d,J=8.3Hz) ,7.94(1H,d,J=7.3Hz) ,8.1 1(1H,d,J=7.6Hz) ,8.37(1H,d,J=8.1Hz) ,8.66(1H,d,J=7.8Hz) .

### Production Example 36: Synthesis of N-[(S)-1-(1-naphthyl)ethyl]-5-[N-methyl-(3-methylpyridin-2-yl)methylamino]-2-(S)-[4-[N-(imidazol-2-ylmethyl)aminomethyl]benzoyl]aminopentanoylamide [Compound No. 36]

The compound (301.5 mg) obtained in Example 1-5 was dissolved in methanol (3 ml), and the solution was added with 3-methylpyridine-2-aldehyde (120.1 mg) and stirred at room temperature for 2 hours. After completion of reaction, the solvent was distilled off, and the residue was dried in vacuum and re-dissolved in methanol (1ml). The solution was ice-cooled and added with sodium borohydride (52.5 mg). The solution was returned room temperature and then stirred for 30 minutes. After completion of reaction, the solvent was distilled off and the residue was dissolved in chloroform. The solution was washed with 0.5 mol/l sodium hydroxide and brine, and dried with anhydrous sodium sulfate.

A portion (19.9 mg) of the obtained product was dissolved in THF (0.3 ml), and the solution was added with 36% formaldehyde aqueous solution (3.3 µl), 2.4 mmol/g MP-cyano borohydride (17.8 mg, manufactured by Argonaut Technologies Inc.) and acetic acid (0. 0284 ml) and stirred at room temperature for 1 hour. After completion of reaction, the solvent was distilled off and the residue was dissolved in chloroform. The solution was washed with 0.5 mol/l sodium hydroxide and brine and dried with anhydrous sodium sulfate. The solution was dissolved in chloroform (0.6 ml) and added with methanesulfonate (0.015 ml) and methanol (0.015 ml), followed by stirring at room temperature for 4 hours. After completion of reaction, the solvent was distilled off, and the residue was purified by silica gel column chromatography (0.9 g, chloroform/methanol/water=7/3/0.5) to, thereby obtain a methanesulfonate (15.8 mg) of the subject compound as a white solid.
MS (FAB,Pos.) :m/z=618 [M+1]⁺
¹H-NMR (500MHz ,DMSO-d₆+D₂O) : δ=1.52 (3H,d,J=7.1Hz) ,1.60-1.85 (4H,m) ,2.25 (3H,s) ,2.41(12H,s) ,2.78 (3H, s) ,3.10-3.21 (2H,m) ,4.34 (2H,s), 4.43(2H,2),4.49(2H,s),4.52-4.57(1H,m),5.72(1H,q,J=7.1Hz),7.36(1H,dd,J=7.8,4.9Hz),7.43-7.49(1H,m),7.50-7.59(3H,m),7.61(2H,d,J =8.5Hz),7.64(2H,s),7.71(1H,d,J=6.8Hz),7.82(1H,d,J=8.1Hz),7.92-7.98(3H,m),8.10(1H,d,J=8.1Hz),8.39(1H,d,J=4.9Hz).

### Production Example 37: Synthesis of N-[(S)-1-(1-naphthyl)ethyl]-5-[N-methyl-(2-methoxybenzyl)amino]-2-(S)-[4-[N-(imidazol-2-ylmethyl)aminomethyl]benzoyl]aminopentanoylamide [Compound No. 37]

The compound (301.5 mg) obtained in Example 1-5 was dissolved in methanol (3 ml), and the solution was added with 2-anisaldehyde (0.120 ml) and stirred at room temperature for 1 hour. After completion of reaction, the sol vent was distilled off, and the residue was dried in vacuum and re-dissolved in methanol (1 ml). The solution was ice-cooled, and added with sodium borohydride (52.5 mg). The solution was returned room temperature, and then stirred for 30 minutes. After completion of reaction, the solvent was distilled off and the residue was dissolved in chloroform. The solution was washed with 0.5 mol/l sodium hydroxide and brine, and dried with anhydrous sodium sulfate. The obtained product was dissolved in THF (0. 3 ml), and the solution was added with 36% formaldehyde aqueous solution (4.9 µl), 2.4 mmol/g MP-cyano borohydride (26.5 mg, manufactured by Argonaut Technologies Inc.) and acetic acid (0.0423 ml) and stirred at room temperature for 1 hour. After completion of reaction, the solvent was distilled off and the residue was dissolved in chloroform. The solution was washed with 0.5 mol/l sodium hydroxide and brine and dried with anhydrous sodium sulfate.

The resultant was dissolved in chloroform (0.6 ml) and added withmethanesulfonate (0.0165ml) andmethanol (0.0165ml), followed by stirring at room temperature for 4 hours. After completion of reaction, the solvent was distilled off, and the residue was purified by silica gel column chromatography (1.2 g, chloroform/methanol/water=7/3/0.5) to obtain a white solid of a methanesulfonate (24.2 mg) of the subject compound.
MS(FAB,Pos.):m/z=632[M+1]⁺
¹H-NMR(500MHz,DMSO-d₆+D₂O,70°C) : δ=1.55(3H,d,J=6.8Hz),1.68-1.85(4H,m),2.41(12H,s),2.58(3H,s),2.98-3.08(1H,m),3.03-3.15(1H,m),3 .78(3H,s),3.98-4.05(1H,m),4.15-4.23(1H,m),4.21(2H,s),4.31(2H,s ),4.55-4.61(1H,m),5.74(1H,q,J=6.8Hz),6.99(1H,t,J=7.1Hz),7.08(1 H,d,J=8.5Hz),7.34(1H,d,J=7.3Hz),7.42-7.60(9H,m),7.80(1H,d,J=8. 3Hz),7.88-7.93(3H,m),8.11(1H,d,J=8.3Hz).

### Production Example 38: Synthesis of N-[(S)-1-(1-naphthyl)ethyl]-5-(N-methyl-isobutylamino)-2-(S)-[4-[N-(imidazol-2-ylmethyl)aminomethyl]benzoyl]aminopentanoylamide [Compound No. 38]

The compound (30.2 mg) obtained in Example 3-1 was dissolved in methanol (0.9 ml), and the solution was added with 36% formaldehyde aqueous solution (5.2 µl), sodium cyano borohydride (5.4 mg), and acetic acid (3 drops) and stirred at room temperature for 13 hours. After completion of reaction, the solvent was distilled off, and the residue was dissolved in chloroform. The resultant solvent was washed with 0.5 mol/l sodium hydroxide and brine and dried with anhydrous sodium sulfate.

The resultant was dissolved in chloroform (1 ml), and the solution was added with methanesulfonic acid (0.018 ml) and methanol (0.018 ml) and stirred at room temperature for 4 hours. After completion of reaction, the solvent was distilled off, and the residue was purified by silica gel column chromatography (1.5 g, chloroform/methanol/water = 7/3/0.5), to thereby obtain a methanesulfonate (27.3 mg) of the subj ect compound as a white solid.
MS(FAB,Pos.):m/z=569[M+1]⁺
¹H-NMR(500MHz,DMSO-d₆) : δ=0.6-0.90(6H,m) ,1.53(3H,d,J=6.8Hz) ,1.55 -1.78(8H,m),1.92-2.02(1H,m),2.35(9H,s),2.64(3H,q,J=2.4Hz),2.75 -2.86(2H,m),2.82-3.01(1H,m),3.02-3.14(1H,m),4.31(2H,s),4.40(2H ,s) , 4.55-4.60(1H,m) ,5.73(1H,quint.) ,7.48-7. 63(8H,m) ,7.83(1H,d, J=8.1Hz) ,7.95(1H,d,J=7.6Hz) ,7.98(2H,d,J=7.8Hz) ,8.11(1H,d,J=8.3 Hz) ,8.56(1H,d,J=7.8Hz) ,8.71(1H,d,J=7.8Hz) ,8.75(1H,d,J=8.1Hz) .

### Production Example 39: Synthesis of N-[(S)-1-(1-naphthyl)ethyl]-5-(N-n-propyl-isobutylamino)-2-(S)-[4-[N-(imidazol-2-ylmethyl)aminomethyl]benzoyl]aminopentanoylamide [Compound No. 39]

The compound (30.2 mg) obtained in Example 3-1 was dissolved in methanol (0.9 ml), and the solution was added with propionaldehyde (5.0 µl), sodium cyano borohydride (5.5 mg), and acetic acid (3 drops) and stirred at room temperature for 13 hours. After completion of reaction, the solvent was distilled off, and the residue was dissolved in chloroform. The resultant solventwas washedwith 0.5mol/l sodium hydroxide and brine and dried with anhydrous sodium sulfate.

The intermediate was dissolved in chloroform (1 ml), and the solution was added with methanesulfonic acid (0.018 ml) and methanol (0.018 ml) and stirred at room temperature for 4 hours. After completion of reaction, the solvent was distilled off, and the residue was purified by silica gel column chromatography (1.5 g, chloroform/methanol/water = 7/3/0.5), to thereby obtain a methanesulfonate (24.5 mg) of the subject compound as a white solid.
MS (FAB,Pos.) :m/z=597 [M+1]⁺
¹H-NMR(500MHz,DMSO-d₆) : δ=0.80-0.92(9H,m) ,1.52(3H,d,J=6.8Hz) ,1.5 0-1.80(6H,m) ,1.88-2.01(1H,m) ,2.36(9H,s) ,2.73-2.95(4H,m) ,2.96-3 .10(2H,m) ,3.02-3.14(1H,m) ,4.32(2H,s) ,4.43(2H,s) ,4.55-4.62(1H,m ) ,5.73 (1H,quint.) ,7.47-7.64 (8H,m) 7. 84 (1H,d,J=8.1Hz) , 7.95 (1H,d ,J=7.6Hz) ,7.98(2H,d,J=8.1Hz) ,8.11(1H,d,J=7.8Hz) ,8.58(1H,d,J=7. 1Hz) ,8.64(1H,brs) ,8.74(1H,d,J=7.6Hz) .

### Production Example 40: Synthesis of N-[(S)-1-(1-naphthyl)ethyl]-5-(N-ethyl-isobutylamino)-2-(S)-[4-[N-(imidazol-2-ylmethyl)aminomethyl]benzoyl]aminopentanoylamide [Compound No. 40]

The compound (30.2 mg) obtained in Example 3-1 was dissolved in methanol (0.9 ml), and the solution was added with acetaldehyde (18 mg), sodium cyano borohydride (4.4mg), and acetic acid (3 drops) and stirred at room temperature for 17 hours. After completion of reaction, the solvent was distilled off, and the residue was dissolved in chloroform. The resultant sol vent was washed with 0.5mol/l sodium hydroxide and brine and dried with anhydrous sodium sulfate. The solvent was distilled off, and the residue was purified by silica gel column chromatography (1.5 g, chloroform/methanol/water = 7/3/0.5), to thereby obtain an intermediate (12.7 mg). The intermediate was dissolved in chloroform (1 ml), and the solution was added with methanesulfonic acid (7 µl) and methanol (7 µl) and stirred at room temperature for 4 hours. After completion of reaction, the solvent was distilled off, and the residue was purified by silica gel column chromatography (0.5 g, chloroform/methanol/water = 7/3/0.5), to thereby obtain a methanesulfonate (12.0 mg) of the subject compound as a white solid.
MS(FAB,Pos.):m/z=583[M+1]⁺
¹H-NMR(500MHz,DMSO-d₆+D₂O): δ=0.81-0.92 (6H,m) , 1.11-1.17(3H,m),1. 52(3H,d,J=6.8Hz),1.50-1.71(2H,m),1.70-1.81(2H,m),1.84-1.96(1H, m),2.40(9H,s),2.71-2.77(1H,m),2.81(1H,dd,J=12.9,7.4Hz),2.93-3. 08(4H,m),4.29(2H,s),4.39(2H,s),4.56-4.62(1H,m),5.72(1H,q),7.46 -7.60(8H,m),7.85(1H,d,J=8.1Hz),7.95(3H,d,J=8.3Hz),8.11(1H,d,J= 8.3Hz).

### Production Example 41: Synthesis of N-[(S)-1-(1-naphthyl)ethyl]-5-(N-isopropyl-isobutylamino)-2-(S)-[4-[N-(imidazol-2-ylmethyl)aminomethyl]benzoyl]aminopentanoylamide [Compound No. 41]

The compound (31.4 mg) obtained in Example 3-1 was dissolved in methanol (0.9 ml), and the solution was added with acetone (40.6 mg), sodium cyano borohydride (6.6 mg), and acetic acid (3 drops) and stirred at room temperature for 6 days. After completion of reaction, thesolventwasdistilledoff, and the residue was dissolved in chloroform. The resultant solvent was washed with 0.5mol/l sodium hydroxide and brine and dried with anhydrous sodium sulfate. The solvent was distilled off, and the residue was purified by silica gel column chromatography (1.5 g, chloroform/methanol/water = 7/3/0.5), to thereby obtain an intermediate.

The intermediate was dissolved in chloroform (1 ml), and the solution was added with methanesulfonic acid (7 µl) and methanol (7 µl) and stirred at room temperature for 4 hours. After completion of reaction, the solvent was distilled off, and the residue was purified by silica gel column chromatography (0.5 g, chloroform/methanol/water = 7/3/0.5), to thereby obtain a methanesulfonate (11.8 mg) of the subject compound as a white solid.
MS (FAB,Pos.) :m/z=597 [M+1]⁺
¹H-NMR(500MHz,DMSO-d₆) : δ=0.95(6H,m) ,1.11-1.23(6H,m) , 1.52(3H,d,J =6.8Hz) ,1.57-1.82(4H,m) ,1.83-1.96(1H,m) ,2.36(9H,s) ,2.62-2.69(1 H,m) ,2.85-2.92(4H,m) ,2.93-3.08(2H,m) ,3.41-3.48(1H,m) ,4.31(2H,s ),4.42(2H,s),4.56-4.63(1H,m),5.73(1H,quint.),7.43-7.65(8H,m),7 .84(1H,d,J=8.1Hz),7.95(1H,d,J=7.1Hz),7.98(2H,d,J=8.3Hz),8.08-8 .12(1H,m),8.21(1H,brs),8.56-8.60(1H,m),8.70-8.76(1H,m).

### Production Example 42: Synthesis of N-[(S)-1-(1-naphthyl)ethyl]-5-(N-methyl-cyclohexylamino)-2-(S)-[4-[N-(imidazol-2-ylmethyl)aminomethyl]benzoyl]aminopentanoylamide [Compound No. 42]

### Example 42-1: Synthesis of N-[(S)-1-(1-naphthyl)ethyl]-5-(N-methyl-cyclohexylamino)-2-(S)-[4-[N-Boc-N-(imidazol-2-ylmethyl)aminomethyl]benzoyl]aminopentanoylamide (Compound XIIb-1)

The compound (0.300 g) obtained in Example 1-5 was dissolved in methanol (5 ml), and the solution was added with cyclohexanone (77 µl) and acetic acid (126 µl) and stirred at room temperature overnight. After completion of reaction, the solvent was distilled off under reduced pressure, and the residue was dissolved in THF (4 ml). The resultant solution was added with 36% formaldehyde aqueous solution (19 µl), 2.42 mmol/g MP-cyano borohydride (282 mg, manufactured by Argonaut Technologies Inc.) and acetic acid (450 µl) and stirred at room temperature for 3 hours. After completion of reaction, the solution was filtrated and the solvent in the filtrate was distilled off under reduced pressure, and the residue was dissolved in chloroform, followed by adding 0.5 mol/l sodium hydroxide aqueous solution. The aqueous layer was extracted with chloroform three times, and the organic layer was washed with brine, followed by drying with anhydrous sodium sulfate. After filtration, the solvent was distilled off under reduced pressure, and the residue was purified by silica gel column chromatography (chloroform/methanol/=20/1), to thereby obtain the subject compound (295 mg) as a white solid.
MS(FAB,Pos.):m/z=695[M+1]⁺

### Example 42-2:

### N-[(S)-1-(1-naphthyl)ethyl]-5-(N-methyl-cyclohexylamino)-2-(S)-[4-[N-(imidazol-2-ylmethyl)aminomethyl]benzoyl]aminopentanoylamide [Compound No. 42]

The compound (0.020 g) obtained in Example 42-1 was dissolved in chloroform (2 ml), and the solution was added with methanesulfonic acid (15 µl) and stirred at room temperature for 2 hours. After completion of reaction, the solvent was distilled off under reduced pressure, and the residue was purified by silica gel column chromatography (chloroform/methanol/water = 7/3/0.5), to thereby obtain a methanesulfonate (0.015 g) of the subject compound as a white solid.
MS(FAB,Pos.):m/z=595[M+1]⁺
¹H-NMR(500MHz,DMSO-d₆) : δ=1.15-1.22(2H,m),1.24-1.33(2H,m),1.53(3 H,d,J=6.8Hz),1.54-1.59(4H,m),1.72-1.75(4H,m),1.86(2H,br),2.31(9H,s),2.56(3H,dd,J=8.1,5.1Hz),2.94-2.95(1H,br),3.09(2H,brs),4. 23(4H,brs),4.56-4.60(1H,m),5.74(1H,quint.,J=6.8Hz),7.40(2H,brs ), 7.48-7.59(6H,m), 7.84(1H,d,J=8.3Hz), 7.96(3H,d,J=7.6Hz), 8.11(1 H,d,J=8.1Hz), 8.55 (1H,d,J=8.8Hz), 8.74 (1H,dd,J=5.1,7.9Hz), 8.96 (1H,brs).

### Production Example 43: Synthesis of N-[(S)-1-(1-naphthyl)ethyl]-5-(N-methyl-cyclopentylamino)-2-(S)-[4-[N-(imidazol-2-ylmethyl)aminomethyl]benzoyl]aminopentanoylamide [Compound No. 43]

The compound (0.051 g) obtained in Example 1-5 was dissolved in methanol (2 ml) , and the solution was added with cyclopentanone (9 µl), acetic acid (84 µl), and sodium cyano borohydride (0.011 g) and stirred at room temperature overnight. Subsequently, the solution was added with 36% formaldehyde aqueous solution (20 µl) and stirred at room temperature overnight. After completion of reaction, the solvent was distilled off under reduced pressure, and the residue was dissolved in chloroform. Then, 0.5 mol/l sodium hydroxide aqueous solution was added to the solution for washing. The aqueous layer was subjected to extraction with chloroform three times, and the organic layer was washed with brine, followed by drying with anhydrous sodium sulfate. After filtration, the solvent was distilled off under reduced pressure, and the residue was purified by silica gel column chromatography (silica gel: 3 g, chloroform/methanol = 5/1). The purified product was dissolved in chloroform (2 ml), and the solution was added with methanesulfonic acid (0.027 ml) and stirred at room temperature overnight. After completion of reaction, the solvent was distilled off under reduced pressure, and the residue was purified by silica gel column chromatography (chloroform/methanol/water = 7/3/0.5), to thereby obtain a methanesulfonate (0.059 g) of the subject compound as a white solid.
MS (FAB,Pos.) :m/z=581 [M+1]⁺
¹H-NMR(500MHz,DMSO-d₆) : δ=1.46-1.80 (10H,m) ,1.53 (3H,d,J=6.8Hz) ,1. 86-1.97(2H,br) ,2.35(9H,s) ,2.59-2.61(3H,m) ,2.89-3.00(2H,m) ,4.29 (2H,brs) ,4.37(2H,brs) ,4.58(1H,d,J=7.1Hz) ,5.71-5.77(1H,m) ,7.48-7.66(BH,m) ,7.84(1H,d,J=8.3Hz) ,7.97(3H,t,J=8.1Hz) ,8.12(1H,d,J=8 .3Hz) ,8.56(1H,t,J=7.6Hz) ,8.72-8.77(1H,m) .

### Production Example 44: Synthesis of N-[(S)-1-(1-naphthyl)ethyl]-5-(N-n-propyl-isopropylamino)-2-(S)-[4-[N-(imidazol-2-ylmethyl)aminomethyl]benzoyl]aminopentanoylamide [Compound No. 44]

### Example 44-1: Synthesis of N-[(S)-1-(1-naphthyl)ethyl]-5-(N-isopropylamino)-2-(S)-[4-[N-Boc-N-(imidazol-2-ylmethyl)aminomethyl]benzoyl]aminopentanoylamide (Compound XIIa-7)

The compound (0.500 g) obtained in Example 1-5 was dissolved in methanol (10 ml), and the solution was added with acetone (0.092 ml) and acetic acid (0.500 ml), and stirred at room temperature for 30 minutes. Then, the solution was added with sodium cyano borohydride (0.158 g) and stirred at room temperature overnight. After completion of reaction, the solvent was distilled off under reduced pressure. The residue was dissolved in chloroform and the solution was washed with 0.5 mol/l sodium hydroxide and brine and dried with anhydrous sodium sulfate. After filtration, the solvent was distilled off under reduced pressure, and the residue was dried in vacuum, to thereby obtain the subject compound (0.531 g) as a white solid.
MS (FAB,Pos.) :m/z=641 [M+1]⁺
¹H-NMR(500MHz,DMSO-d₆) : δ=0.91(6H,d,J=6.3Hz) ,1.35-1.47(11H,m) ,1. 51(3H,d,J=6.8Hz),1.70(2H,d,J=7.8Hz),2.43(2H,t,J=7.0Hz),2.60(1H ,quint.,J=6.2Hz),4.33(1H,brs),4.43-4.51(4H,m),5.71(1H,quint.,J =7.1Hz), 6.84(1H,s),7.05(1H,s), 7.24-7.27(2H,br), 7.47-7.56(4H,m) ,7.83(1H,d,J=8.1Hz),7.85(2H,d,J=8.3Hz),7.94(1H,d,J=7.6Hz),8.10 (1H,d,J=7.8Hz),8.41(1H,d,J=7.1Hz),8.64(1H,d,J=8.1Hz),11.83-11. 96(1H,brd).

### Example 44-2: Synthesis of N-[(S)-1-(1-naphthyl)ethyl]-5-(N-n-propyl-isopropylamino)-2-(S)-[4-[N-(imidazol-2-ylmethyl)aminomethyl]benzoyl]aminopentanoylamide [Compound No. 44]

The compound (0.050 g) obtained in Example 44-1 was dissolved in methanol (2 ml) and the solution was added with propionaldehyde (0.020 ml), acetic acid (0.078 ml) and sodium cyano borohydride (0.015g) and stirred at room temperature overnight. Afer completion of reaction, the solvent was distilled off under reduced pressure and the residue was dissolved in chloroform. The solution was washed with 0.5 mol/l sodium hydroxide aqueous solution and saturate saline solution and dried with anhydrous sodium sulfate. After filtration, the solvent was distilled off under reduced pressure, and the residue was purified by silica gel column chromatography (chloroform/methanol=5/1). The purified product was dissolved in chloroform (2 ml), and the solution was added with methanesulfonate (0.016 ml) and stirred at room temperature overnight. After completion of reaction, the solvent was distilled off under reduced pressure, and the residue was purified by silica gel column chromatography (chloroform/methanol/water=7/3/0.5), to thereby obtain a methanesulfonate (0.032 g) of the subject compound as a white solid.
MS (FAB,Pos.) :m/z=583 [M+1)⁺
¹H-NMR(500MHz,DMSO-d₆) : δ=0.84(3H,m) , 1.14-1.19(6H,m), 1.53(3H,d,J =6.8Hz) ,1.53-1.73(4H,m) ,1.74(2H,m) ,2.34(9H,s) ,2.76-2.82(2H,m), 2.95 (2H,brs) ,3.04-3.07 (1H,br) ,4.28 (2H,brs) ,4.34 (2H,brs) ,4.60 (1 H,m) ,5.74(1H,quint. ,J=7.1Hz) ,7.48-7.60(1H,m) ,7.84(1H,d,J=8.1Hz ) ,7.96(3H,t,J=6.8Hz) ,8.12 (1H,d,J=7.8Hz) ,8.55-8.57(1H,m) ,8.61 (1 H,brs) ,8.73(1H,d,J=7.6Hz).

### Production Example 45: Synthesis of N-[(S)-1-(1-naphthyl)ethyl]-5-di-n-propylamino-2-(S)-[4-[N-(pyridin-2-ylmethyl)aminomethyl]benzoyl]aminopentanoylamide [Compound No. 45]

### Example 45-1: Synthesis of methyl 4-[N-Boc-N-(pyridin-2-ylmethyl)aminomethyl]benzoate (Compound V-1)

Commercially available 2-picolylamine (1.08 g) was dissolved in DMF (2.5 ml) and the solution was added with triethylamine (1.55 ml) and cooled to 0°C. A solution obtained by dissolving di-t-butyldicarbonate (2.52 ml) in DMF (7.5 ml) was dropped to the solution over 10 minutes. After warming to room temperature and then stirring for 2 hours, the solvent was distilled off under reduced pressure. The residue was purified by silica gel column chromatography (30 g, chloroform), to thereby obtain a light yellow liquid (1.71 g).

The obtained product (1.199 g) was dissolved in THF (6 ml) and sodium hydride (46.1 mg) (60% paraffin mixture) was suspended therein. After stirring at room temperature for 15 minutes, commercially available methyl-4-bromomethylbenzoate (241 mg) was added to the suspension followed by stirring at room temperature for more 2 days. After completion of reaction, pH of the resultant solution was adjusted to 5 to 7 using 1 mol/l hydrochloric acid aqueous solution and the solution was concentrated. Chloroform (40 ml) was added to the concentrate, and the solution was washed with water and then concentrated. The residue was purified by silica gel column chromatography (6 g, chloroform/ethyl acetate=10/1), to thereby obtain the subject compound (2.21 g) as light yellow liquid.
MS(FAB,Pos.):m/z=357[M+1]⁺
¹H-NMR(500MHz,CDCl₃):δ=1.45(9H,s)3.91(3H,s)4.47(1H,brs)4.52(1H,brs),4.60(2H,s),7.17(1H,dd,J=7.6,4.1Hz) ,7.2-7.4(3H,m) ,7.65(1H,t,J=7.6Hz),8.52(1H,d,J=4.1Hz).

### Example 45-2: Synthesis of 4-[N-Boc-N-(pyridin-2-ylmethyl)aminomethyl]benzoic acid (Compound VI-2)

The compound (200.6 mg) obtained in Example 45-1 was added with methanol (2 ml), THF (2 ml), and 1 mol/l sodium hydroxide (2 ml) and stirred at room temperature for one day. After completion of reaction, the solvent was distilled off and then water (5ml) was added to the residue. Then, 1 mol/l hydrochloric acid aqueous solution was dropped to the solution to adjust pH of the solution to 3. Precipitated crystal was collected by filtration and dried, to thereby obtain the subject compound (123.3 mg) as colorless crystals.
MS(FAB,Pos.):m/z=343[M+1)⁺
¹H-NMR(500MHz,DMSO-d₆) : δ=1.35 and 1.54(9H,brs),4.41(1H,brs),4.51(2H,s),4.58(1H,brs),7.2-7.4(4H,m),7.77(1H,td,J=7.6,1.8Hz),8.52(1H,dd,J=4.9,1.7Hz),12.9(1H,s).

### Example 45-3: Synthesis of [(1S)-1-(1-naphthyl)ethyl]-5-amino-(S)-2-[4-[N-Boc-N-(pyridin-2-ylmethyl)aminomethyl]benzoyl]aminopentanoylamide (Compound XI-2)

The compound (9.2827 g) obtained in Example 1-2 was dissolved in DMF (93 ml) and added with (1S)-1-(1-naphthyl)ethylamine (4.940 g), WSCI hydrochloride (7.4057 g), and HOBt (5.336 g), followed by stirring at room temperature for 16 hours. After completion of reaction, the solvent was distilled off, and then saturated sodium hydrogen carbonate aqueous solution and chloroform was added to the residue. After extracting the aqueous layer with chloroform, the organic layer was combined to the extract and washed with brine. The resultant solution was dried with anhydrous magnesium sulphate and then the solvent was distllated off under reduced pressure. The residue was dissolved in dioxane (80 ml) and added with methanol (80 ml) and concentrated hydrochloric acid (8 ml), followed by stirring at 45°C for 2 hours. The solvent was distilled off under reduced pressure and then the residue was suspended in chloroform and washed with 1 mol/l sodium hydroxide aqueous solution. After drying with anhydrous magnesium sulfate, the solvent was distilled off under reduced pressure. The obtained residue was dissolved in DMF (110 ml) and added with the compound (9.6129 g) obtained in Example 1-2, WSCI hydrochloride (7.4937 g), and HOBt (5.2943 g), followed by stirring at room temperature for 16 hours. After completion of reaction, the solvent was distilled off under reduced pressure, and a saturated sodium hydrogen carbonate aqueous solution and chloroform were added to the residue. After extracting the aqueous layer with chloroform, the extract was combined with the organic layer previously obtained, followed by washing with brine. After drying with anhydrous magnesium sulfate, the solvent was distilled off under reduced pressure. The residue was purified by silica gel column chromatography (294 g, chloroform/ethyl acetate=2/1) . The obtained compound was added with a 40% methylamine/methanol solution (100 ml) and then stirred at room temperature for 40 hours.

After completion of reaction, the solvent was distilled off under reduced pressure, and the residue was purified by silica gel column chromatography (572 g, chloroform/methanol/water=7/3/0.5), to thereby obtain the subject compound (6.8058 g) as a white solid.
MS(FAB,Pos.):m/z=610[M+1]⁺
¹H-NMR(500MHz,CDCl₃) : δ=1.34-1.42(2H,m) ,1.35(9H,br) ,1.51(3H,d,J= 7.1Hz),1.65-1.73(2H,m),3.16-3.44(2H,m),4.40(1H,brs),4.49-4.56(4H,m),5.68-5.72(1H,quint.J=7.1Hz) ,7.20-7.34(4H,m) ,7.42-7.57(4 H,m),7.76-7.80(1H,m),7.82(1H,d,J=7.8Hz),7.86(2H,d,J=8.3Hz),7.9 4(1H,d,J=7.6Hz),8.10(1H,d,J=8.1Hz),8.52(1H,d,J=4.9Hz).

### Example 45-4: Synthesis of N-[(S)-1-(1-naphthyl)ethyl]-5-di-n-propylamino)-2-(S)-[4-[N-Boc-N-(pyridin-2-ylmethyl)aminomethyl]benzoyl]aminopentanoylamide (Compound XIIa-8)

The compound (110 mg) obtained in Example 45-3 was dissolved in methanol (2 ml), and then added with molecular sieves 3A powder (1 g), propionaldehyde (0.0326 ml) and sodium cyano borohydride (34.1 g). The pH of the reaction solution was adjusted to 5 using acetic acid and the solution was then stirred at room temperature overnight. After completion of reaction, the resultant solution was filtrated with Celite and then added with saturated sodium hydrogen carbonate aqueous solution, followed by extracting with chloroform. The organic layer was washed with a saturated sodium hydrogen carbonate aqueous solution and brine, and then dried with anhydrous sodium sulfate. The solvent was distilled off and the residue was purified by silica gel column chromatography (chloroform/methanol=10/1) , to thereby obtain the subject compound (134 mg) as a colorless oily product.
MS (FAB,Pos.) :m/z=694 [M+1]⁺
¹H-NMR(500MHz,DMSO-d₆) : δ=0.77(6H,t,J-6.8Hz) ,1.34(17H,m) ,1.51(3H ,d,J=6.8Hz) ,1.69(2H,br) ,2.51(4H,br) ,4.38(1H,s) ,4.50(3H,m) ,4.56 (1H,s) ,5.72(1H,quint. ,J=7.3Hz) ,7.28(4H,m) ,7.43(1H,t,J=7.6Hz), 7.53(3H,m) ,7.78(1H,dt,J=1.7,7.5Hz) ,7.83(1H,d,J=8.1Hz) ,7.86(2H, d,J=8.3Hz) ,7.94(1H,dd,J=7.3,2.2Hz) ,8.12(1H,d,J=8.1Hz) ,8.37(1H, brs) ,8.52(1H,d,J=4.2Hz) ,8.66(1H,d,J=7.1Hz) .

### Example 45-5: Synthesis of N-[(S)-1-(1-naphthyl)ethyl]-5-di-n-propylamino-2-(S)-[4-[N-(pyridin-2-ylmethyl)aminomethyl]benzoyl]aminopentanoylamide [Compound No. 45]

The compound (131 mg) obtained in Example 45-4 was dissolved in chloroform (1ml), and the solution was added with methanesulfonic acid (0.0735 ml) and stirred at room temperature for 2.5 hours. After completion of reaction, the resultant solution was concentrated, and the residue was purified by silica gel column chromatography (chloroform/methanol = 5/1), to thereby obtain a methanesulfonate (112.8 mg) of the subj ect compound as a white solid.
MS (FAB,Pos.) :m/z=594 [M+1]⁺
¹H-NMR(500MHz,DMSO-d₆) : δ=0.83(3H,t,J=7.3Hz) ,0.84(3H,t,J=7.5Hz), 1.55(7H,m) ,1.74(2H,m) ,2.31(9H,m) ,2.89(5H,m) ,3.02(3H,m) ,4.33(4H ,s) ,4.60(1H,m) ,5.73(1H,quint. ,J=7.3Hz) ,7.52(6H,m) ,7.63(2H,d,J= 8.3Hz) ,7.84(1H,d,J=8.1Hz) ,7.91(1H,dt,J=7.6,1.7Hz) ,7.95(1H,dd,J =7.6,1.7Hz) ,7.97(2H,d,J=8.3Hz) ,8.12(1H,d,J=8.1Hz) ,8.57(1H,d,J= 8.1Hz) ,8.67(1H,d,J=4.9Hz) ,8.74(1H,d,J=7.8Hz) ,8.97(1H,brs) .

### Production Example 46: Synthesis of N-[(S)-1-(1-naphthyl)ethyl]-5-diisobutylamino-2-(S)-[4-[N-(pyridin-2-ylmethyl)aminomethyl]benzoyl]aminopentanoylamide [Compound No. 46]

### Example 46-1: Synthesis of N-[(S)-1-(1-naphthyl)ethyl]-5-diisobutylamino-2-(S)-[4-[N-(pyridin-2-ylmethyl)aminomethyl]benzoyl]aminopentanoylamide (Compound XIIa-9)

The compound (105 mg) obtained in Example 45-3 was dissolved in methanol (2 ml) and then added with isobutylaldehyde (0.0391 ml), acetic acid (0.170 ml), and sodium cyano borohydride (34.1 mg), followed by stirring at room temperature for 4 days. After completion of reaction, the resultant solution was filtrated with Celite, and then added with a saturated sodium hydrogen carbonate aqueous solution followed by extracting with chloroform. The organic layer was washed with a saturated sodium hydrogen carbonate aqueous solution and brine, and then dried with anhydrous sodium sulfate. The solvent was distilled off and the residue was purified by silica gel column chromatography (chloroform/methanol=10/1), to thereby obtain the subject compound (138 mg) as a white solid.
MS (FAB,Pos.) :m/z=722 [M+1]⁺
¹H-NMR(500MHz,DMSO-d₆) : δ=0.77(6H,t,J=6.3Hz) ,0.78(6H,t,J=6.6Hz), 1.34(11H,m) ,1.51(3H,d,J=6.8Hz) ,1.60(3H,m) ,1.73(1H,m) ,1.95(2H,d ,J=6.8Hz) ,1.96(2H,d,J=7.6Hz) ,2.27(2H,m) ,4.40(1H,s) ,4.50(4H,m), 5.71(1H,quint.,J=7.3Hz),7.29(4H,m),7.45(1H,t,J=7.6Hz),7.53(3H, m),7.78(1H,dt,J=7.6,1.7Hz),7.82(1H,d,J=8.1Hz).,7.85(2H,d,J=8.3 Hz),7.94(1H,dd,J=7.1,2.2Hz),8.11(1H,d,J=7.6Hz),8.33(1H,d,J=8.3 Hz),8.52(1H,d,J=4.6Hz),8.65(1H,d,J=7.8Hz)

### Example 46-2: Synthesis of N-[(S)-1-(1-naphthyl)ethyl]-5-diisobutylamino)-2-(S)-[4-[N-(pyridin-2-ylmethyl)aminomethyl]benzoyl]aminopentanoylamide [Compound No. 46]

The compound (33.0 mg) obtained in Example 46-1 was dissolved in chloroform (1 ml), and the solution was added with methanesulfonic acid (0.0356 ml) and stirred at room temperature for 4 hours. After completion of reaction, the resultant solution was concentrated, and the residue was purified by silica gel column chromatography (chloroform/methanol = 5/1), to thereby obtain a methanesulfonate (10.0 mg) of the subject compound as a white solid.
MS(FAB,Pos.):m/z=622[M+1]⁺
¹H-NMR(500MHz,DMSO-d₆):δ=0.77(6H,d,J=6.1Hz),0.78(6H,d,J=6.1Hz), 1.24(1H,s),1.38(3H,br),1.51(3H,d,J=6.8Hz),1.60(3H,m),1.73(1H,b r),1.96(4H,br),2.26(1H,m),4.11(4H,s),4.53(1H,m),5.71(1H,quint. ,J=7.3Hz),7.52(9H,m),7.83(2H,m),7.91(2H,d,J=8.3Hz),7.94(1H,dd, J=7.1,2.2Hz),8.11(1H,d,J=9.0Hz),8.39(1H,d,J=8.3Hz),8.60(1H,br) 8.68(1H,d,J=7.8Hz).

### Production Example 47: Synthesis of N-(1-naphthylmethyl)-5-(3-methylpyridin-2-yl)methylamino-2-(S)-[4-[N-(imidazol-2-ylmethyl)aminomethyl]benzoyl]aminopentanoylamide [Compound No. 47]

### Example 47-1: Synthesis of N-(1-naphthylmethyl)-5-phthalimide-(S)-2-(Boc-amino)pentanoylamide (Compound VIII-2)

The compound (26.93 g) obtained in Example 1-2 was dissolved in DMF (250 ml) and then added with 1-naphthalenemethylamine (12.0 ml), WSCI hydrochloride (19.6 g), and HOBt (13.8 g). The resultant solution was stirred at room temperature for 1.5 hours and then concentrated. The obtained residue was diluted with chloroform and then washed with a 1 mol/l sodium hydroxide aqueous solution, a 1 mol/l hydrochloric acid solution, and brine. The organic layer was dried with anhydrous sodium sulfate and concentrated, and then the obtained residue was subjected to recrystallization using methanol, to thereby obtain the subject compound (21.34 g).
MS(FAB,Pos.):m/z=502[M+1]⁺
¹H-NMR(500MHz,DMSO-d₆) : δ=1.40(9H,s) ,1.60-1.80(4H,m) ,3.68-3.75(1 H,m),3.78-3.67(1H,m),4.33-4.40(1H,m),4.80(1H,d,J=14.4,4.7Hz),4 .98(1H,dd,J=14.4,5.9Hz),5.21(1H,d,J=8.5Hz),6.62(1H,br),77.39-7 .49(4H,m),7.57-7.69(4H,m),7.81(2H,t,J=9.5Hz),7.93(1H,d,J=8.5Hz).

### Example 47-2: Synthesis of N-(1-naphthylmethyl)-5-phthalimide-(S)-2-[4-[N-Boc-N-(imidazol -2-ylmethyl)aminomethyl]benzoylamino]pentanoylamide (Compound X-2)

The compound (11.56 g) obtained in Example 47-1 was dissolved in dioxane (116 ml) and then added with concentrated hydrochloric acid (11.6 ml), followed by stirring at room temperature for 4 hours. After completion of reaction, the solvent was distilled off, and the residue was dissolved in chloroform and washed with a 1 mol/l sodium hydroxide aqueous solution and brine. The solution was dried with anhydrous sodium sulfate and then the solvent was distilled off. The residue was dissolved in DMF (200 ml). The DMF solution was added with the compound (7.64 g) obtained in Example 1-1, WSCI hydrochloride (6.63 g), and HOBt (4.67 g) and then stirred at room temperature for 2 hours. After completion of reaction, the solvent was distilled off, and the residue was added with chloroform and washed with a 1 mol/l sodium hydroxide aqueous solution and brine. After drying with anhydrous sodium sulfate, the solvent was distilled off and the residue was purified by silica gel column chromatography (chloroform/methanol=10/1), to thereby obtain a white solid of the subject compound (11.71 g) as a white solid.
MS(FAB,Pos.):m/z=715[M+1]⁺
¹H-NMR(500MHz,CDCl₃):δ=1.65(9H,s),1.70-1.82(4H,m),3.69-3.97(2H, m),3.92-3.98(1H,m),4.40(2H,s),4.50(2H,s),4.77(1H,dd,J=14.6,4.4 Hz),4.95-5.02(1H,m),5.05(1H,dd,J=14.6.6.5Hz),6.84(1H,t,J=5.2Hz ),6.98(2H,d,J=14.8Hz),7.08(1H,d,J=8.2Hz),7.24(2H,d,J=7.8Hz),7. 40-7.47(4H,m),7.53-7.59(2H,m),7.64-7.68(2H,m),7.76(2H,d,J=8.4H z),7.81(2H,dd,J=9.0,7.9Hz),7.94(1H,dd,J=8.2,0.9Hz).

### Example 47-3: Synthesis of N-(1-naphthylmethyl)-5-amino-(S)-2-[4-[N-Boc-N-(imidazol-2-ylmethyl)aminomethyl]benzoylamino]pentanoylamide (Compound XI-3)

The compound (7.76 g) obtained in Example 47-2 was dissolved in methanol (78 ml), and then added with hydrazine monohydrate (2.63 ml), followed by stirring at 60°C for 2 hours. After completion of reaction, the solvent was distilled off, and chloroform was added to the residue to filtrate off the generated solid. The mother solution was washed with brine and then dried with anhydrous sodium sulfate. Then, the solvent was distilled off and the residue was dried under reduced pressure, to thereby obtain the subj ect compound (6.74 g) as a light yellow solid.
MS(FAB,Pos.):m/z=585[M+1]⁺
¹H-NMR(500MHz,DMSO-d₆) : δ=1.45-1.65(2H,m) ,1.65(9H,brs) ,1.90-1.97 (2H,m) ,2.66-2.78(2H,m) ,4.38(2H,s) ,4.48(2H,s) ,4.68(1H,dd,J=13.5, 6.9Hz) ,4.97(2H,m) ,6,97(2H,brs) ,7.21(2H,d,J=8.1Hz) ,7.35(1H,t,J =4.9Hz) ,7.39-7.52(4H,m) ,7.73(2H,d,J=8.3Hz) ,7.80(1H,d,J=7.8Hz), 7.82-7.89(2H,m),7.99(1H,d,J=8.8Hz),8.10(1H,d,J=8.1Hz),8.48(1H, d,J=7.8Hz),8.64(1H,d,J=7.8Hz).

### Example 47-4: Synthesis of N-(1-naphthylmethyl)-5-(3-methylpyridin-2-yl)methylamino-2-(S)-[4-[N-{imidazol-2-ylmethyl)aminomethyl]benzoyl]aminopentanoylamide [Compound No. 47]

The compound (0.050 g) obtained in Example 47-3 was dissolved in methanol (2 ml) and then added with 3-methyl-2-pyridinecarboxyaldehyde (0.016 g), followed by stirring at room temperature for 1 hour. Once the solvent was distilled off under reduced pressure, the residue was dried using a vacuum pump and then dissolved in methanol (1 ml). After cooling to 0°C, sodium borohydride (0.010 g) was added to the methanol solution followed by stirring at room temperature for 1 hour. After completion of reaction, the solvent was distilled off under reduced pressure, and the residue was dissolved in chloroform and further added with water. The aqueous layer was extracted with chloroform three times, and the obtained organic layer was washed with brine and dried with anhydrous sodium sulfate. After filtration, the solvent was distilled off under reduced pressure. The residue was added with chloroform (2 ml) and methanesulfonate (55 µl) and stirred at room temperature for 3 hours. After completion of reaction, the solvent was distilled off under reduced pressure, and the residue was purified by silica gel column chromatography (chloroform/methanol/water=7/3/0.5), to thereby obtain a methanesulfonate (0.070 g) of the subject compound as a white solid.
MS(FAB,Pos.):m/z=590[M+1]⁺
¹H-NMR(500MHz,DMSO-d₆) : δ=1.80-1.87(4H,m) , 2.27(3H,s) ,2.38(12H,s) ,3.06(2H,brs),4.33(2H,s),4.38(2H,sH),3.24(1H,mH),4.55(3H,brs), 4.77(2H,d,J=5.6Hz),7.35(1H,dd,J=7.6,4.9Hz),7.45-7.48(2H,m),7.5 5(2H,t,J=4.9Hz),7.63(2H,d,J=8.1Hz),7.70(1H,d,J=7.6Hz),7.75(2H, s),7.85(1H,d,J=9.3Hz),7.95(1H,d,J=9.5Hz),8.00(3H,d,J=8.1Hz),8. 06(1H,d,J=9.0Hz),8.44(1H,d,J=4.4Hz),8.64(1H,t,J=5.6Hz),8.68(1H ,d,J=8.1Hz),9.00(2H,brs).

### Production Example 48: Synthesis of N-(1-naphthylmethyl)-5-diisobutylamino-2-(S)-[4-[N-(imidazol-2-ylmethyl)aminomethyl]benzoyl]aminopentanoylamide [Compound No. 48]

The compound (0.051 g) obtained in Example 47-3 was dissolved in methanol (2 ml), and the solution was added with isobutyl aldehyde (0.017 ml) and 0.108 mg of MP-cyano borohydride (manufactured by Argonaut Technologies Inc., 2.42 mmol/g) and stirred at room temperature overnight. After completion of reaction, the solution was filtered, and the solvent in the filtrate was distilled off under reduced pressure. The residue was dissolved in chloroform, and 0.5 mol/l sodium hydroxide aqueous solution was added to the solution. The aqueous layer was subjected to extraction with chloroform three times, and the organic layer was washed with brine and dried with anhydrous sodium sulfate. After filtration, the solvent was distilled off under reduced pressure, and the residue was purified by silica gel column chromatography (chloroform/methanol = 10/1). The purified product was dissolved in chloroform (2 ml), and the solution was added with methanesulfonic acid (10 µl) and stirred at room temperature for 4 hours. After completion of reaction, the solvent was distilled off under reduced pressure, and the residue was purified by silica gel column chromatography (chloroform/methanol/water = 7/3/0.5), to thereby obtain a methanesulfonate (26 mg) of the subject compound as a white solid.
MS(FAB,Pos.):m/z=597(M+1]⁺
¹H-NMR(500MHz,DMSO-d₆): δ=0.89-1.07(12H,m) ,1.68-1.84(4H,m) ,1.96-2.05(2H,m),2.35(15H,s),2.84-2.91(4H,m),3.10(2H,br),4.30(2H,s), 4.39(2H,s),4.56(1H,dd,J=8.8,5.6Hz),4.76(1H,dd,J=9.6,5.6Hz),7.4 3(2H,d,J=6.3Hz),7.53-7.57(4H,m),7.61(2H,d,J=8.3Hz),7.86(1H,d,J =6.3Hz),7.96(1H,d,J=6.1Hz),7.99(2H,d,J=8.3Hz),8.07(1H,d,J=6.1H z),8.34(1H,brs),8.65(1H,t,J=5.7Hz),8.68(1H,d,J=8.1Hz).

### Production Example 49: Synthesis of N-(1-naphthylmethyl)-5-(N-methyl-cyclohexylamino)-2-(S)-[4-[N-(imidazol-2-ylmethyl)aminomethyl]benzoyl]aminopentanoylamide [Compound No. 49]

The compound (0.285 g) obtained in Example 47-3 was dissolved in methanol (5 ml), and the solution was added with cyclohexanone (0.055 ml), acetic acid (0.487 ml), and 604 mg of MP-cyano borohydride (manufactured by Argonaut Technologies Inc., 2.42 mmol/g) and stirred at room temperature overnight. After completion of reaction, the solution was filtered, and the solvent in the filtrate was distilled off under reduced pressure. The residue was dissolved in chloroform, and 0.5 mol/l sodium hydroxide aqueous solution was added to the solution. The aqueous layer was subj ected to extraction with chloroform three times, and the organic layer was washed with brine and dried with anhydrous sodium sulfate. After filtration, the solvent was distilled off under reduced pressure, and the residue was dissolved in THF (4 ml). Subsequently, the solution was added with 36% formaldehyde aqueous solution (27 µl), 261 mg of MP-cyano borohydride (manufactured by Argonaut Technologies Inc., 2.42 mmol/g), acetic acid (0.420 ml) and stirred at room temperature for 3 hours. After completion of reaction, the solution was filtered, and the solvent in the filtrate was distilled off under reduced pressure. The residue was dissolved in chloroform, and 0.5 mol/l sodium hydroxide aqueous solution was added to the solution. The aqueous layer was subjected to extraction with chloroform three times, and the organic layer was washed with brine and dried with anhydrous sodium sulfate. After filtration, the solvent was distilled off under reduced pressure, and the residue was purified by silica gel column chromatography (chloroform/methanol = 20/1). The purified product was dissolved in chloroform (4 ml), and the solution was added with methanesulfonic acid (0.176 ml) and stirred at room temperature for 4 hours. After completion of reaction, the solvent was distilled off under reduced pressure, and the residue was purified by silica gel column chromatography (chloroform/methanol/water = 7/3/0.5), to thereby obtain a methanesulfonate (0.290 g) of the subject compound as a white solid.
MS (FAB,Pos.) :m/z=581 [M+1]⁺
¹H-NMR(500MHz, DMSO-d₆) : δ=1.22 (2H,br) , 1.33 (2H,br) , 1.57-1.91(10H, m) ,2.59(3H,s) ,3.09-3.16(3H,m) ,3.83(2H,s) ,3.90(2H,s) ,4.56(1H,dd ,J=8.1,5.9Hz) ,4.75(1H,dd,J=9.5,5.9Hz) ,4.80(1H,dd,J=9.5,5.9Hz), 7.04(2H,s),7.45-7.50(4H,m),7.53-7.56(2H,m),7.85(1H,d,J=6.3Hz), 7.91(2H,d,J=8.1Hz),7.95(1H,d,J=6.3Hz),8.07(1H,d,J=6.6Hz),8.57-8.61(2H,m).

### Production Example 50: Synthesis of N-[(S)-1-(1-naphthyl)ethyl]-5-(2-ethoxybenzyl)amino-2-(S)-[4-[N-(imidazol-2-ylmethyl)aminomethyl]benzoyl]aminopentanoylamide [Compound No. 50]

### Example 50-1: Synthesis of N-[(1S)-1-(1-naphthyl)ethyl]-4-(benzyloxycarbonyl)-2-(S)-(N-Boc-amino)butylamide (Compound XIV-1)

Commercially available N"-Boc-L-glutamic acid-benzyl ester (1.05 g) was dissolved in DMF (21.0 ml), and then added with commercially available (S)-1-(1-naphty)ethylamine (0.799 g) , WSCI hydrochloride (0.894 g), and HOBt (0.631 g), followed by allowing to stand at room temperature for one day. After assuring the termination of the reaction using TLC, the reaction systemwas in-situ evaporated under reduced pressure. Then, 1 mol/l hydrochloric acid aqueous solution was added thereto and the resultant solution was subjected to separatory extraction with chloroform. The obtained organic layer was washed with a saturated sodium hydrogen carbonate solution and dried with anhydrous sodium sulfate, followed by concentrating under reduced pressure. The residue was purified by silica gel column chromatography (50 g, chloroform/ethyl acetate=5/1), to thereby obtain the subject compound (1.42 g) as a white solid.
MS(FAB,Pos.):m/z=491[M+1]⁺
¹H-NMR(500MHz,CDCl₃):δ=1.40(9H,s),1.63(3H,d,J=6.8Hz),1.84-1.91(1H,m),2.01-2.10(1H,m),2.32-2.36(1H,m),2.45-2.51(1H,m),4.05-4.1 5(1H,m),5.07(2H,s),5.22-5.30(1H,m),5.90(1H,t,J=7.3Hz),6.42-6.5 0(1H,m),7.23-7.53(9H,m),7.78(1H,d,J=8.1Hz),7.84(1H,d,J=7.8Hz), 8.04(1H,d,J=8.5Hz).

### Example 50-2: Synthesis of N-[(1S)-1-(1-naphthyl)ethyl]-4-(methoxycarbonyl)-2-(S)-[4-[N-Boc-N-(imidazol-2-ylmethyl)aminomethyl]benzoylamino]butyrylamide (Compound XVI-1)

The compound (7.44 g) obtained in Example 50-1 was dissolved in methanol (70 ml) and then added with 4 mol/l hydrochloric acid/dioxane solution followed by stirring at room temperature for 3 hours. After completion of reaction, the solvent was distilled off and then azeotropically distilled usingmethanol. The resultant was dissolved in DMF (100 ml), and added with WSCI hydrochloride (4.36 g) and HOBt (3.08 g). Then, the resultant solution was added with a solution obtained by dissolving the compound (5.30 g) obtained in Example 1-1 in DMF (40 ml) and stirred at room temperature overnight. After completion of reaction, the solvent was distilled off, and the residue was dissolved in chloroform and washed with a hydrochloric acid aqueous solution, a saturated sodium hydrogen carbonate solution and a brine, followed by drying with anhydrous sodium sulfate. The solvent was distilled off and the residue was purified by silica gel column chromatography (chloroform/methanol=20/1), to thereby obtain the subject compound (6.44 g) as a white solid.
MS(FAB,Pos.):m/z=628[M+1]⁺
¹H-NMR(500MHz,CDCl₃):δ=1.34and1.37(9H,2s),1.51(3H,d,J=6.8Hz),1. 93-1.98(2H,m),2.33(2H,m),3.55(3H,s),4.33(1H,m),4.42(2H,s),4.50 (2H,s) ,5.71(1H,quint. ,J=6.8Hz),6.85(1H,s) ,7.04(1H,brs) ,7.23(2H, m) ,7.31(3H,m) ,7.46-7.56(4H,m) ,7.85(2H,m) ,7.93(1H,m) ,8.11(1H,m ) ,8.40(1H,brs) ,8.68(1H,brs) .

### Example 50-3: Synthesis of N-[(1S)-1-(1-naphthyl)ethyl]-5-hydroxy-2-(S)-[4-[N-Boc-N-(imidazol-2-ylmethyl)aminomethyl]benzoylamino]pentanoylamide (Compound XVII-1)

The compound (5.41 g) obtained in Example 50-2 was dissolved in ethanol (75 ml) and then added with THF (37.5 ml). Under ice-cooling, the solution was added with sodium borohydride (1.30 g) and calcium chloride (1.91 g), and stirred at room temperature overnight. After completion of reaction, the resultant solution was added with a saturated ammonium chloride aqueous solution, and extracted with chloroform. The solvent was distilled off and the residue was purified by silica gel column chromatography (chloroform/methanol=10/1), to thereby obtain the subject compound (3.86 g) as a white solid.
MS(FAB,Pos.) :m/z=600[M+1]⁺
¹H-NMR(500MHz,CDCl₃):δ=1.35and1.37(9H,2s),1.50(3H,d,J=7.1Hz),1. 65-1.78(2H,m),3.35-3.39(2H,m),4.35(1H,m),4.42-4.49(3H,s),4.51(2H,s),5.70(1H,quint. ,J=7.1Hz) ,6.84(1H,s) ,7.05(1H,s) ,7.26(2H,m), 7.47-7.56(4H,m), 7.83(3H,m), 7.93(1H,m), 8.10(1H,m), 8.35(1H,brs), ,8.62(1H,brs) .

### Example 50-4: Synthesis of N-[(1S)-1-(1-naphthyl)ethyl]-5-(p-toluenesulfonyl)oxy-2-(S)-[4 -[N-Boc-N-(1-(p-toluenesulfonyl)imidazol-2-ylmethyl)aminomethyl]benzoylamino]pentanoylamide (Compound XVIII-1)

The compound (499.7 mg) obtained in Example 50-3 was dissolved in methylene chloride (5 ml), and added with triethylamine (0.586 ml). Then, chlorotoluenesulfonic acid (795 mg) was gradually added under ice-cooling, followed by stirring at room temperature for 17 hours. After completion of reaction, the reaction solution was washed with a 0.5 mol/l sodium hydroxide aqueous solution and brine followed by drying with anhydrous sodium sulfate. The residue was purified by silica gel column chromatography (chloroform/ethyl acetate=1/1) , to thereby obtain the subject compound (544 mg) as a white solid.
MS (FAB,Pos.) :m/z=908 [M+1]⁺

### Example 50-5: Synthesis of N-[(S)-1-(1-naphthyl)ethyl]-5-(2-ethoxybenzyl)amino-2-(S)-[4-[N-Boc-N-(imidazol-2-ylmethyl)aminomethyl]benzoyl]aminopentanoylamide (Compound XIIa-10)

The compound (61.3 mg) obtained in Example 50-4 was dissolved in toluene (1.2 ml), and the solution was added with 2-ethoxybenzylamine (0.0204 ml) and stirred at 70°C for 3 hours. After completion of reaction, the solution was cooled to room temperature. Subsequently, the solvent was distilled off, and the residue was purified by silica gel column chromatography (2.2 g, chloroform/methanol = 15/1), to thereby obtain the subject compound (30.0 mg) as a white solid.
MS (FAB,Pos.) :m/z=887 [M+1]⁺

### Example 50-6: Synthesis of N-[(S)-1-(1-naphthyl)ethyl]-5-(2-ethoxybenzyl)amino-2-(S)-[4-[N-(imidazol-2-ylmethyl)aminomethyl]benzoyl]aminopentanoylamide [Compound No. 50]

The compound (14.7 mg) obtained in Example 50-5 was dissolved in methanol (0.15 ml), and the solution was added with 4 mol/l hydrochloric acid/dioxane (0.15 ml) and stirred at room temperature for 1 hour. After completion of reaction, the solvent was distilled off, and the residue was purified by silica gel column chromatography (0.35 g, chloroform/methanol/water = 7/3/0.5), to thereby obtain a hydrochloride (10.1 mg) of the subject compound as a white solid.
MS(FAB,Pos.):m/z=633[M+1]⁺
¹H-NMR(500MHz,DMSO-d₆): δ=1.32(3H,t,J=7.1Hz) ,1.52(2H,d,J=6.8Hz), 1.68-1.95(4H,m),2.85-2.95(2H,m),4.00-4.03(2H,m),4.05(2H,q,J=7. 1Hz),4.34(2H,s),4.46(2H,s),4.55-4.65(1H,m),5.71(1H,quint.,J=6. 8Hz),6.96(1H,t,J=7.3Hz),7.05(1H,d,J=7.8Hz),7.37(1H,t,J=7.8Hz), 7.41(1H,d,J=7.5Hz),7.45-7.59(4H,m),7.64(2H,s),7.66(2H,d,J=8.3H z),7.82(1H,d,J=8.3Hz),7.94(1H,d,J=6.6Hz),7.98(2H,d,J=8.3Hz),8. 10(1H,d,J=8.1Hz),8.60(1H,d,J=8.3Hz),8.82(1H,d,J=7.8Hz),8.80-8. 97(2H,br).

### Production Example 51: Synthesis of N-[(S)-1-(1-naphthyl)ethyl]-5-diethylamino-2-(S)-[4-[N-(imidazol-2-ylmethyl)aminomethyl]benzoyl]aminopentanoylamide [Compound No. 51]

The compound (51.4 mg) obtained in Example 50-4 was dissolved in toluene (2.5 ml) , and the solution was added with diethylamine (0.0071 ml) and stirred at 60°C for 2 days. After completion of reaction, the solution was cooled to room temperature. Subsequently, the solvent was distilled off, and the residue was purified by silica gel column chromatography (2.5 g, chloroform/methanol = 20/1). The purified product was dissolved in methanol (0.2 ml), and 4 mol/l hydrochloric acid/dioxane (0.2 ml) was added, followed by stirring at room temperature for 18 hours. After completion of reaction, the solvent was distilled off, and the residue was purified by silica gel column chromatography (1.5 g, chloroform/methanol/water = 7/3/0.5), to thereby obtain a hydrochloride (11.8 mg) of the subject compound as a white solid.
MS(FAB,Pos.):m/z=555[M+1]⁺
¹H-NMR(500MHz,DMSO-d₆):δ=1.13(3H,t,J=7.3Hz),1.14(3H,t,J=7.3Hz), 1.52(3H,d,J=6.8Hz),1.57-1.88(4H,m),2.81-3.05(8H,m),4.35(2H,s), 4.48(2H,s),4.55-4.61(1H,m),5.73(1H,quint.),7.46-7.58(4H,m),7.6 5(2H,s),7.67(2H,d,J=8.3Hz),7.83(1H,d,J=8.1Hz),7.96(1H,d,J=8.3H z),7.98(2H,d,J=8.3Hz),8.12(1H,d,J=8.3Hz),8.63(1H,d,J=8.1Hz),8. 83(1H,d,J=8.1Hz),10.03(1H,brs).

### Production Example 52: Synthesis of N-[(S)-1-(1-naphthyl)ethyl]-5-(2-phenylpropyl-2-ylamino)-2-(S)-[4-[N-(imidazol-2-ylmethyl)aminomethyl]benzoyl]aminopentanoylamide [Compound No. 52]

The compound (51.6 mg) obtained in Example 50-4 was dissolved in toluene (1.0 ml) and then added with cumylamine (0.0159 ml), followed by stirring at 70°C for 3 days. After completion of reaction, the resultant solution was cooled to room temperature, and the solvent was distilled off. The residue was added with chloroform, and the solution was washed with a 0.5 mol/l sodiumhydroxide aqueous solution and brine and dried with anhydrous sodium sulfate. The solvent was distilled off and the residue was roughly purified by silica gel column chromatography (3g, chloroform/methanol=30/1), to thereby obtain a fraction containing a methanesulfonate of the subject compound.

The fraction was dissolved in methanol (0.1 ml) and added with 4 mol/l hydrochloric acid/dioxane solution (0.1 ml) and stirred at room temperature for one hour. After completion of reaction, the solvent was distilled off, and the residue was purified by silica gel column chromatography (0.6 g, chloroform/methanol/water =7/3/0.5), to thereby obtain a hydrochloride (6.0 mg, 15%) of the subject compound as a white solid.
MS(FAB,Pos.):m/z=619[M+1]⁺
¹H-NMR(500MHz,DMSO-d₆) : δ=1.49(3H,d,J=6.8Hz), 1.61-1.79(4H,m), 1.7 0(6H,s),2.45-2.60(2H,m),4.34(2H,s),4.44(2H,s),4.48-4.57(1H,m), 4.72(1H,sex,J=5.9Hz),5.67(1H,quint.,J=6.8Hz),7.38-7.58(7H,m),7 .50-7.71(5H,m),7.81(1H,d,J=8.3Hz),7.92-7.95(1H,m),7.96(2H,d,J= 8.3Hz),8.07(1H,d,J=9.0Hz),8.53(1H,d,J=8.3Hz),8.76(1H,d,J=7.6Hz ),9.32(1H,brs),9.45(1H,brs).

### Production Example 53: Synthesis of N-[(S)-1-(1-naphthyl)ethyl]-5-[2-(2-methoxyphenyl)ethyl]amino-2-(S)-[4-[N-(imidazol-2-ylmethyl)aminomethyl]benzoyl]aminopentanoylamide [Compound No. 53]

The compound (50 mg) obtained in Example 50-4 was dissolved in toluene (1.0 ml), and the solution was added with 2-(2-methoxyphenyl)ethylamine (20 mg) and stirred at 70°C for 3 hours. After completion of reaction, the solution was cooled to room temperature, and the solvent was distilled off, and the residue was purified by silica gel column chromatography (chloroform/methanol = 15/1). The purified product was dissolved in methanol (0.6 ml), and the solution was added with 4 mol/l hydrochloric acid/dioxane (0.6 ml) and stirred at room temperature for 4 hours. After completion of reaction, the solvent was distilled off, and the residue was purified by silica gel column chromatography (chloroform/methanol/water = 7/3/0.5), to thereby obtain a hydrochloride (9.0 mg) of the subject compound as a white solid.
MS(FAB,Pos.):m/z=633[M+1]⁺
¹H-NMR(500MHz,DMSO-d₆) : δ=1.53(3H,d,J=6.8Hz) ,1.63-1.81(4H,m) ,2.8 8(4H,m),3.00(2H,s),3.78(3H,s),4.33(2H,s),4.42(2H,s),4.57-4.60(1H,m),5.71(1H,quint.,J=6.8Hz),6.91(1H,t,J=7.5Hz),7.00(1H,d,J=8 .1Hz) ,7.15(1H,d,J=7.3Hz) ,7.24(1H,t,J=8.1Hz) ,7.45-7.57 (6H,m) ,7. 59(1H,brs),7.65(1H,brs),7.80(1H,d,J=8.1Hz),7.93(1H,d,J=7.8Hz), 7.98(2H,d,J=8.3Hz),8.10(1H,d,J=8.3Hz),8.60(1H,d,J=7.1Hz),8.70-8.90(3H,m).

### Production Example 54: Synthesis of N-[(S)-1-(1-naphthyl)ethyl]-5-(1,2,3,4-tetrahydroisoquinolin-2-yl)-2-(S)-[4-[N-(imidazol-2-ylmethyl)aminomethyl]benzoyl]aminopentanoylamide [Compound No. 54]

The compound (49.7 mg) obtained in Example 50-4 was dissolved in toluene (1.0 ml), and the solution was added with 1,2,3,4-tetrahydroisoquinoline (0.0084 ml) and stirred at 70°C for 22 hours. After completion of reaction, the solution was cooled to room temperature, and the solvent was distilled off, and the residue was dissolved in methanol (0.6 ml). Subsequently, the solution was added with 4 mol/l hydrochloric acid/dioxane (0.6 ml) and stirred at room temperature for 12 hours. After completion of reaction, the solvent was distilled off, and the residue was purified by silica gel column chromatography (1.5 g, chloroform/methanol/water = 7/3/0.5), to thereby obtain a hydrochloride (23.8 mg) of the subject compound as a white solid.
MS(FAB,Pos.):m/z=615[M+1]⁺
¹H-NMR(500MHz,DMSO-d₆+D₂O) : δ=1.54(3H,d,J=6.8Hz),1.71-1.93(4H,m) ,2.95-3.03(1H,m),3.08-3.27(3H,m),3.38-3.48(1H,m),3.53-3.61(1H, m) ,4.19(1H,dd,J=15.3,7.2Hz) ,4.33(2H,s) ,4.38-4.45(1H,m) ,4.46(2H ,s) ,4.57-4.62(1H,m) ,5.72(1H,q,J=6.8Hz) ,7.14-7.17(1H,m) ,7.23-7. 33(3H,m),7.46-7.68(8H,m),7.83-7.85(1H,m),7.93-7.99(3H,m),8.11(1H,d,J=8.3Hz) .

### Production Example 55: Synthesis of N-[(S)-1-(1-naphthyl)ethyl]-5-(hexamethyleneimin-1-yl)-2-(S)-[4-[N-(imidazol-2-ylmethyl)aminomethyl]benzoyl]aminopentanoylamide [Compound No. 55]

The compound (63.5 mg) obtained in Example 50-4 was dissolved in toluene (3 ml), and the solution was added with hexamethyleneimine (0.0197 ml) and stirred at 70°C for 2 days. After completion of reaction, the solution was cooled to room temperature, and the solvent was distilled off, and the residue was dissolved in methanol (3 ml). Subsequently, the solution was added with 1 mol/l sodium hydroxide aqueous solution (0.3 ml) and stirred at room temperature for 15 minutes. After completion of reaction, the solvent was distilled off, and the residue was dissolved in chloroform. The resultant solution was washed with distilled water and brine and dried with anhydrous sodium sulfate. The solvent was distilled off, and the residue was purified by silica gel column chromatography (2.5 g, chloroform/methanol = 20/1), to thereby obtain an intermediate.

The intermediate was dissolved in chloroform (1 ml), and the solution was added withmethanesulfonic acid (0.0205ml) andmethanol (0.0205 ml) and stirred at room temperature for 20 hours. After completion of reaction, the solvent was distilled off, and the residue was purified by silica gel column chromatography (1.5 g, chloroform/methanol/water = 7/3/0.5), to thereby obtain a methanesulfonate (20.8 mg) of the subject compound as a white solid.
MS(FAB,Pos.) :m/z=581[M+1]⁺
¹H-NMR (500MHz,DMSO-d₆) : δ=1.53 (3H,d,J=6.8Hz) , 1.51-1.80 (12H,m) ,2. 36(9H,s) ,2.87-2.96(2H,m) ,2.97-3.06(2H,m) ,3.13-3.25(2H,m) ,4.32(2H,s),4.43(2H,s) ,4.52-4.59(1H,m) ,5.74(1H,quint. ,J=6.8Hz) ,7.46-7.65(8H,m) ,7.84(1H,d,J=8.1Hz) ,7.96(1H,d,J=7.6Hz) ,7.98(2H,d,J=8 .3Hz) ,8.12(1H,d,J=8.1Hz) ,8.56(1H,d,J=7.8Hz) ,8.75(1H,d,J=8.1Hz) ,9.06 (1H,brs) .

### Production Example 56: Synthesis of N-[(S)-1-(1-naphthyl)ethyl]-5-(heptamethyleneimin-1-yl)-2-(S)-[4-[N-(imidazol-2-ylmethyl)aminomethyl]benzoyl]aminopentanoylamide [Compound No. 56]

The compound (60.8 mg) obtained in Example 50-4 was dissolved in toluene (3ml), and the solution was added with hexamethyleneimine (0.0212 ml) and stirred at 70°C for 2 days. After completion of reaction, the solution was cooled to room temperature, and the solvent was distilled off, and the residue was dissolved in methanol (3 ml). Subsequently, the solution was added with 1 mol/l sodium hydroxide aqueous solution (0.3 ml) and stirred at room temperature for 15 minutes. After completion of reaction, the solvent was distilled off, and the residue was dissolved in chloroform. The resultant solution was washed with distilled water and brine and dried with anhydrous sodium sulfate. The solvent was distilled off, and the residue was purified by silica gel column chromatography (2.5 g, chloroform/methanol = 20/1), to thereby obtain an intermediate.

The intermediate was dissolved in chloroform (1 ml), and the solution was added with methanesulfonic acid (0.0160ml) and methanol (0.0160 ml) and stirred at room temperature for 20 hours. After completion of reaction, the solvent was distilled off, and the residue was purified by silica gel column chromatography (1.5 g, chloroform/methanol/water = 7/3/0.5), to thereby obtain a methanesulfonate (17.3 mg) of the subject compound as a white solid.
MS(FAB,Pos.):m/z=595[M+1]⁺
¹H-NMR(500MHz,DMSO-d₆) : δ=1.53(3H,d,J=6.8Hz) ,1.39-1.83(14H,m) ,2. 40(9H,s),2.90-3.00(2H,m),2.99-3.10(2H,m),3.14-3.23(2H,m),4.30(2H,s),4.38(2H,s),4.52-4.59(1H,m),5.74(1H,quint.),7.45-7.62(8H, m),7.84(1H,d,J=8.3Hz),7.96(1H,d,J=8.3Hz),7.97(2H,d,J=8.5Hz),8. 12(1H,d,J=8.1Hz),8.56(1H,d,J=7.8Hz),8.75(1H,d,J=7.8Hz),8.85(1H,brs).

### Production Example 57: Synthesis of N-[(S)-1-(1-naphthyl)ethyl]-5-morpholino-2-(S)-[4-[N-(imidazol -2-ylmethyl)aminomethyl]benzoyl]aminopentanoylamide [Compound No. 57]

### Example 57-1: Synthesis of N-[(1S)-1-(1-naphthyl)ethyl]-5-(methanesulfonyl)oxy-2-(S)-[4-[N-Boc-N-(1-(methanesulfonyl)imidazol-2-ylmethyl)aminomethyl]benzoylamino]pentanoylamide (Compound XVIII-2)

The compound (878.7 mg) obtained in Example 50-3 was added to methylene chloride (10 ml) and triethylamine (1.64 ml), and the solution was ice-cooled. The resultant solution was added with methanesulfonyl chloride (0.68 ml) and stirred for 2 hours at room temperature. After completion of reaction, the resultant solution was washed with water and dried with anhydrous magnesium sulfate. The solvent was distilled off under reduced pressure, and the residue was purified by silica gel column chromatography (chloroform/methanol=19/1), to thereby obtain the subj ect compound (952.5 mg) as white crystals.
MS(FAB,Pos.):m/z=756[M+1]⁺

### Example 57-2: Synthesis of N-[(S)-1-(1-naphthyl)ethyl]-5-morpholino-2-(S)-[4-[N-(imidazol-2-ylmethyl)aminomethyl]benzoyl]aminopentanoylamide [Compound No. 57]

The compound (169.7 mg) obtained in Example 57-1 andmorpholine (0.058 ml) were dissolved in toluene (2.5 ml), followed by stirring at 70°C for 14 hours. After completion of reaction, the solvent was distilled off under reduced pressure, and the residue was dissolved in chloroform, followed by washing with water. The resultant solution was dried with anhydrous magnesium sulfate, and the solvent was distilled off under reduced pressure. Then, the residue was dissolved in chloroform (3 ml), and the solution was added with methanesulfonic acid (0.143 ml) and stirred for 3 hours at room temperature. The solvent was distilled off under reduced pressure, and the residue was purified by silica gel column chromatography (chloroform/methanol/water = 7/3/0.5), to thereby obtain a methanesulfonate (106.7 mg) of the subject compound as white crystals.
MS(FAB,Pos.):m/z=569[M+1]⁺
¹H-NMR(500MHz,DMSO-d₆):δ=1.53(3H,d,J=6.8Hz),1.57-1.73(4H,m),2.3 2(9H,s),2.92(2H,t,J=9.5Hz),3.06(2H,m),3.24-3.27(2H,m),3.55-3.6 5(2H,m),3.93(2H,d,J=12.5Hz),4.25(4H,brs),4.56(1H,dd,J=7.8,14.6 Hz),5.74(1H,quint.,J=6.8Hz),7.45-7.59(8H,m),7.85(1H,d,J=8.1Hz) ,7.95-7.98(3H,m),8.12(1H,d,J=8.1Hz),8.55(1H,d,J=8.1Hz),8.75(1H ,d,J=8.1Hz),9.46(1H,brs).

### Production Example 58: Synthesis of N-[(S)-1-(1-naphthyl)ethyl]-5-piperidino-2-(S)-[4-[N-(imidazol-2-ylmethyl)aminomethyl]benzoyl]aminopentanoylamide [Compound No. 58]

The compound (147.7 mg) obtained in Example 57-1 and piperidine (0.059 ml) were dissolved in toluene (2.2 ml), followed by stirring at 70°C for 12 hours. After completion of reaction, the solvent was distilled off under reduced pressure, and water (2.5 ml) and chloroform (3 ml) were added to the residue. The solution was added to a diatomite column (Chem Elut CE 1003, manufactured by Varian), and washed with chloroform (2 ml). The solution was added with methanesulfonic acid (0.130 ml) and stirred for 3 hours at room temperature. The solvent was distilled off under reduced pressure, and the residue was purified by silica gel column chromatography (chloroform/methanol/water = 7/3/0.5), to thereby obtain a methanesulfonate (99.2mg) of the subj ect compound as white crystals.
MS(FAB,Pos.):m/z=567[M+1]⁺
¹H-NMR(500MHz,DMSO-d₆):δ=1.14-1.35(1H,m),1.53(3H,d,J=6.8Hz),1.5 7-1.60(3H,m),1.64-1.76(6H,m),2.35(9H,s),2.64-2.74(2H,m),2.97(2 H,dd,J=8.1,13.4Hz),3.24(2H,m),4.30(2H,brs),4.38(2H,brs),4.56(1 H,dd,J=8.1,14.3Hz),5.74(1H,quint.,J=6.8Hz),7.48-7.60(8H,m),7.8 5(1H,d,J=8.3Hz),7.95-7.99(3H,m),8.12(1H,d,J=8.1Hz),8.56(1H,d,J =7.8Hz),8.76(1H,d,J=7.8Hz),8.89(1H,brs).

### Production Example 59: Synthesis of N-[(S)-1-(1-naphthyl)ethyl]-5-(pyrrolidin-1-yl)-2-(S)-[4-[N-(imidazol-2-ylmethyl)aminomethyl]benzoyl]aminopentanoylamide [Compound No. 59]

The compound (142.6 mg) obtained in Example 57-1 and pyrrolidine (0.047 ml) were dissolved in toluene (2.2 ml), followed by stirring at 70°C for 12 hours. After completion of reaction, the solvent was distilled off under reduced pressure, and water (2.5ml) and chloroform (3ml) were added to the residue. The solution was added to a diatomite column (Chem Elut CE 1003, manufactured by Varian), and washed with chloroform (2 ml). The solution was added with methanesulfonic acid (0.130 ml) and stirred for 3 hours at room temperature. The solvent was distilled off under reduced pressure, and the residue was purified by silica gel column chromatography (chloroform/methanol/water = 7/3/0.5), to thereby obtain a methanesulfonate (115.4 mg) of the subject compound as white crystals.
MS(FAB,Pos.):m/z=553[M+1]⁺
¹H-NMR(500MHz,DMSO-d₆) : δ=1.53(3H,d,J=6.8Hz),1.56-1.66(2H,m),1.7 1(4H,m) , 1.91-1.96(2H,m) ,2.80-2.85(2H,m) ,3.43-3.45(2H,m) ,4.31(2 H,brs),4.41(2H,brs),4.55(1H,dd,J=8.3,13.9Hz),5.73(1H,quint.,J= 6.8Hz),7.49-7.61(8H,m),7.84(1H,d,J=8.1Hz),7.95-7.99(3H,m),8.12 (1H,d,J=8.1Hz),8.56(1H,d,J=7.8Hz),8.74(1H,d,J=7.8Hz),9.37(1H,b rs).

### Production Example 60: Synthesis of

### N-[(S)-1-(1-naphthyl)ethyl]-5-bis(2-methoxyethyl)amino-2-(S)-[4-[N-(imidazol-2-ylmethyl)aminomethyl]benzoyl]aminopentanoylamide [Compound No. 60]

The compound (108.9 mg) obtained in Example 57-1 and bis(2-methoxyethyl)amine (0.062 ml) were dissolved in anhydrous toluene (1.5 ml), followed by stirring at 70°C for 19 hours. After completion of reaction, the solvent was distilled off under reduced pressure, and the residue was dissolved in chloroform, followed by washing with water. The resultant solution was dried with anhydrous magnesium sulfate, and the solvent was distilled off under reduced pressure. Then, the residue was dissolved in chloroform (2 ml) , and the solution was added with methanesulfonic acid (0.091 ml) and stirred for 14 hours at room temperature. The solvent was distilled off under reduced pressure, and the residue was purified by silica gel column chromatography (chloroform/methanol/water = 7/3/0.5), to thereby obtain a methanesulfonate (22.6 mg) of the subject compound as white crystals.
MS(FAB,Pos.) :m/z=615[M+1]⁺
¹H-NMR(500MHz,DMSO-d₆) : δ=1.53(3H, d, J=7.1Hz) ,1.58-1.72 (4H,m) ,2.3 1 (9H,s) ,3.09-3.22 (6H,m) ,3.24 (6H,s) ,3.56-3.58 (4H,m) ,4.20 (4H,brs ) ,4.56 (1H,dd,J=8.5,14.3Hz) ,5.73 (1H,quint. ,J=7.1Hz) ,7.36 (2H,m), 7.48-7.58(6H,m) ,7.85(1H,d,J=8.1Hz) ,7.95-7.97(3H,m) ,8.11(1H,d,J =7.8Hz) ,8.73(1H,d,J=7.8Hz) ,9.21(1H,brs)

### Production Example 61: Synthesis of N-[(S)-1-(1-naphthyl)ethyl]-5-bis(2-hydroxyethyl)amino-2-(S)-[4-[N-(imidazol-2-ylmethyl)aminomethyl]benzoyl]aminopentanoylamide [Compound No. 61]

The compound (199.4 mg) obtained in Example 57-1 and diethanolamine (75.6 mg) were dissolved in toluene (3 ml), followed by stirring at 70°C for 19 hours. After completion of reaction, the solvent was distilled off under reduced pressure, and the residue was dissolved in chloroform. The resultant solution was washed with distilled water and brine. The organic layer was dried with anhydrous magnesium sulfate, and the solvent was distilled off under reduced pressure. The residue was dissolved in chloroform (3ml) andmethanol (1 ml), and the solution was added with methanesulfonic acid (0.143 ml) and stirred for 21 hours at room temperature. The solvent was distilled off under reduced pressure, and the residue was purified by silica gel column chromatography (chloroform/methanol/water = 7/3/0.5), to thereby obtain a methanesulfonate (72.6 mg) of the subject compound as white crystals.
MS(FAB,Pos.):m/z=587[M+1]⁺
¹H-NMR(500MHz,DMSO-d₆) : δ=1.52(3H,d,J=6.8Hz) ,1.60-1.73(4H,m) ,2.3 1(9H,s) ,3.06-3.17(6H,m) ,3.69-3.70(4H,m) ,4.21(4H,brs) ,4.56(1H,d d,J=8.1,13.6Hz),5.28(2H,brs),5.72(1H,quint.,J=6.8Hz),7.37(2H,b r) ,7.49-7.58(6H,m) ,7.84(1H,d,J=8.1Hz) ,7.94-7.96(3H,m) ,8.11(1H, d,J=8.3Hz),8.52(1H,d,J=8.1Hz),8.73(1H,d,J=7.8Hz),8.99(1H,br)

### Production Example 62: Synthesis of N-[(S)-1-(1-naphthyl)ethyl]-5-(N-n-propyl-(2-methoxyethyl)amino)-2-(S)-[4-[N-(imidazol-2-ylmethyl)aminomethyl]benzoyl]aminopentanoylamide [Compound No. 62]Example 62-1: Synthesis of N-[(S)-1-(1-naphthyl)ethyl]-5-(2-methoxyethyl)amino-2-(S)-[4-[N-Boc-N-(imidazol-2-ylmethyl)aminomethyl]benzoyl]aminopentanoylamide (Compound XIIa-11)

The compound (276.4 mg) obtained in Example 57-1 and 2-methoxyethylamine (0.095 ml) were dissolved in anhydrous toluene (4.5ml), followed by stirring at 70 ° C for 19 hours . After completion of reaction, the solvent was distilled off under reduced pressure, and the residue was purified by silica gel column chromatography (chloroform/methanol=10/1), to thereby obtain the subject compound (188.4 mg) as white crystals.
MS(FAB,Pos.):m/z=657[M+1]⁺
¹H-NMR(500MHz,DMSO-d₆) : δ=1.25-1.45(11H,m) ,1.51(3H,d,J=6.8Hz) ,1. 68-1.75 (2H,m) , 2.52-2.54 (2H,m) , 2.60-2.64 (2H,m) , 3.20-3.21 (3H,m), 3.34-3.36 (2H,m) ,4.32 (1H,m) ,4.42 (2H,brs) , 4.49 (2H,brs) ,5.71 (1H,q uint. ,J=6.8Hz) ,6.84 (1H,brs) ,7.05 (1H,brs) ,7.25-7.28 (3H,m) ,7.47-7.56(4H,m) ,7.82-7.86(3H,m) ,7.94(1H,d,J=7.3Hz) ,8.11(1H,d,J=7.6H z) ,8.46(1H,d,J=8.5Hz) ,8.64(1H,d,J=6.1Hz) .

### Example 62-2: Synthesis of N-[(S)-1-(1-naphthyl)ethyl]-5-(N-n-propyl-(2-methoxyethyl)amino)-2-(S)-[4-[N-(imidazol-2-ylmethyl)aminomethyl]benzoyl]aminopentanoylamide [Compound No. 62]

The compound (56.7 mg) obtained in Example 62-1 was dissolved in methanol (2.3 ml). Propionaldehyde (0.009 ml) and sodium cyano borohydride (16.2 mg) were added to the solution, and pH of the resultant solution was adjusted to 5 with acetic acid, followed by stirring at room temperature for 14 hours. After completion of reaction, the solvent was distilled off under reduced pressure, and the residue was dissolved in chloroform. The resultant solution was washed with a saturated sodium hydrogen carbonate aqueous solution and dried with anhydrous magnesium sulfate. The solvent was distilled off under reduced pressure, and chloroform (2 ml) and methanesulfonic acid (0.056 ml) were added to the residue, followed by stirring for 5 hours at room temperature. The solvent was distilled off under reduced pressure, and the residue was purified by silica gel column chromatography (chloroform/methanol/water = 7/3/0.5), to thereby obtain a methanesulfonate (61.7 mg) of the subject compound as white crystals.
MS(FAB,Pos.):m/z=599[M+1]⁺
¹H-NMR(500MHz,DMSO-d₆): δ=0.82(3H,dt,J=2.4,7.3Hz),1.53(3H,d,J=6. 8Hz),1.55-1.73(6H,m),2.33(9H,s),2.89-3.21(6H,m),3.26(3H,s),3.5 6(2H,m),4.26(2H,brs),4.32(2H,brs),4.56(1H,dd,J=8.5,14.6Hz),5.7 3(1H,quint.,J=6.8Hz),7.48-7.63(8H,m),7.85(1H,d,J=7.8Hz),7.95-7 .97(3H,m) ,8.10-8.14(1H,m) ,8.55(1H,d,J=8.3Hz) ,8.73(1H,d,J=7.6Hz ),9.11(1H,brs).

### Production Example 63: Synthesis of N-[(S)-1-(1-naphthyl)ethyl]-5-(N-isobutyl-(2-methoxyethyl)amino)-2-(S)-[4-[N-(imidazol-2-ylmethyl)aminomethyl]benzoyl]aminopentanoylamide [Compound No. 63]

The compound (51.7 mg) obtained in Example 62-1 was dissolved in anhydrous methanol (2ml). Isobutylaldehyde (0.011ml) and sodium cyano borohydride (15.2 mg) were added to the solution, and pH of the resultant solution was adjusted to 5 with acetic acid, followed by stirring at room temperature for 4 days. After completion of reaction, the solvent was distilled off under reduced pressure, and the residue was dissolved in chloroform. The resultant solution was washed with a saturated sodium hydrogen carbonate aqueous solution. After the organic layer was dried with anhydrous magnesium sulfate, the solvent was distilled off under reduced pressure, and the residue was purified by silica gel column chromatography (chloroform/methanol = 10/1). The purified product was dissolved in chloroform (1 ml), and the solution was added withmethanesulfonic acid (0.033 ml) and stirred for 22 hours at room temperature. The solvent was distilled off under reduced pressure, and the residue was purified by silica gel column chromatography (chloroform/methanol/water = 7/3/0.5), to thereby obtain a methanesulfonate (28.7mg) of the subj ect compound as white crystals. MS(FAB,Pos.):m/z=613[M+1]⁺
¹H-NMR(500MHz,DMSO-d₆):δ=0.84-0.90(6H,m),1.53(3H,d,J=6.8Hz),1.60 -1.73(4H,m) ,1.94(1H,dt,J=6.8,13.1Hz) ,2.34(9H,s) ,2 .77-2.79(1H,m ) ,2.88-2.89(1H,m) ,3.08(2H,m) ,3.17-3.18(2H,m) ,3.25(3H,d,J=3.4Hz ) ,3.59(2H,brs) ,4.28(4H,brs) ,4.57(1H,m) ,5.73(1H,quint. ,J=6.8Hz) ,7.48-7.60(8H,m) ,7.85(1H,d,J=8.1Hz) ,7.95-7.98(3H,m) ,8.11(1H,d, J=7.5Hz) ,8.56(1H,d,J=6.6Hz) ,8.74(2H,t,7.1Hz) .

### Production Example 64: Synthesis of N-[(S)-1-(1-naphthyl)ethyl]-5-(N-isopropyl-(2-methoxyethyl)amino)-2-(S)-[4-[N-(imidazol-2-ylmethyl)aminomethyl]benzoyl]aminopentanoylamide [Compound No. 64]

The compound (74.8 mg) obtained in Example 62-1 was dissolved in methanol (3 ml). Acetone (0.132 ml) and sodium cyano borohydride (21.5mg) were added to the solution, and pH of the resultant solution was adjusted to 5 with acetic acid, followed by stirring at room temperature for 4 days. After completion of reaction, the solvent was distilled off under reduced pressure, and the residue was dissolved in chloroform. The resultant solution was washed with a saturated sodium hydrogen carbonate aqueous solution. After the organic layer was dried with anhydrous magnesium sulfate, the solvent was distilled off under reduced pressure, and the residue was purified by silica gel column chromatography (chloroform/methanol = 10/1). The purified product was dissolved in chloroform (1.2 ml), and the solution was added with methanesulfonic acid (0. 047 ml) and stirred for 21 hours at room temperature. The solvent was distilled off under reduced pressure, and the residue was purified by silica gel column chromatography (chloroform/methanol/water = 7/3/0.5), to thereby obtain a methanesulfonate (50.5 mg) of the subject compound as white crystals.
MS(FAB,Pos.):m/z=599(M+1]⁺
¹H-NMR(500MHz,DMSO-d₆):δ=1.14-1.18(6H,m),1.53(3H,d,J=6.8Hz),1.6 1-1.74(4H,m) ,2.34(9H,s) ,3.02(3H,m) ,3.17-3.28(5H,m) ,3.53(2H,m), 4.29(4H,brs),4.59(1H,m),5.74(1H,quint.,J=6.8Hz),7.49-7.60(8H,m ),7.85(1H,d,J=7.8Hz),7.95-7.98(3H,m),8.12(1H,d,J=7.6Hz),8.56(1 H,brs),8.73(2H,d,J=8.1Hz).

### Production Example 65: N-[(S)-1-(1-naphthyl)ethyl]-5-(N-n-propyl-(2-hydroxyethyl)amino)-2-(S)-[4-[N-(imidazol-2-ylmethyl)aminomethyl]benzoyl]aminopentanoylamide [Compound No. 65]

### Example 65-1: Synthesis of N-[(S)-1-(1-naphthyl)ethyl]-5-(2-hydroxyethyl)amino-2-(S)-[4-[N-(imidazol-2-ylmethyl)aminomethyl]benzoyl]aminopentanoylamide (Compound XIIa-12)

The compound (458.7 mg) obtained in Example 57-1 and ethanolamine (0.110 ml) were dissolved in toluene (7 ml), followed by stirring at 70°C for 16 hours. After completion of reaction, the solvent was distilled off under reduced pressure, and the residue was purified by silica gel column chromatography (chloroform/methanol = 5/1) , to thereby obtain the subject compound (109.1 mg) as white crystals.
MS(FAB,Pos.):m/z=643[M+1]⁺
¹H-NMR(500MHz,DMSO)-d₆) : δ=1.35(9H,brs) ,1.42-1.48(2H,m) ,1.51(3H,d ,J=6.8Hz),1.70-1.73(2H,m),3.35(4H,m),3.40(2H,m),4.32(1H,m),4.4 2-4.52(5H,m),5.71(1H,quint.,J=6.8Hz),6.84(1H,brs),7.04(1H,brs) ,7.25(2H,m),7.47-7.56(4H,m),7.81-7.86(3H,m),7.93-7.95(1H,m),8. 11(1H,d,J=8.1Hz),8.44(1H,d,J=7.8Hz),8.64(1H,d,J=8.1Hz).

### Example 65-2: Synthesis of N-[(S)-1-(1-naphthyl)ethyl]-5-(N-n-propyl-(2-hydroxyethyl)amino)-2-(S)-[4-[N-(imidazol-2-ylmethyl)aminomethyl]benzoyl]aminopentanoylamide [Compound No. 65]

The compound (43.2 mg) obtained in Example 65-1 was dissolved in methanol (1.7 ml). Propionaldehyde (0.007 ml) and sodium cyano borohydride (12.6 mg) were added to the solution, and pH of the resultant solution was adjusted to 5 with acetic acid, followed by stirring at room temperature for 14 hours. After completion of reaction, the solvent was distilled off under reduced pressure, and the residue was dissolved in chloroform. The resultant solution was washed with a saturated sodium hydrogen carbonate aqueous solution. The organic layer was dried with anhydrous magnesium sulfate, and the solvent was distilled off under reduced pressure. The residue was dissolved in chloroform (5 ml), and the solution was added with methanesulfonic acid (0.043 ml) and stirred for 3 hours at room temperature. The solvent was distilled off under reduced pressure, and the residue was purified by silica gel column chromatography (chloroform/methanol/water = 7/3/0.5), to thereby obtain a methanesulfonate (18.5 mg) of the subject compound as white crystals.
MS(FAB,Pos.):m/z=585[M+1]⁺
¹H-NMR(500MHz,DMSO-d₆) : δ=0.82(3H,dt,J=3.2,7.3Hz) ,1.52(3H,d,J=7. 1Hz),1.55-1.75(6H,m),2.34(9H,s),2.89-2.97(2H,m),3.06(4H,m),3.6 7(2H,brs),4.23(2H,brs),4.34(2H,brs),4.57(1H,dd,J=7.8,13.9Hz),5 .73(1H,quint.,J=7.1Hz),7.48-7.60(8H,m),7.84(1H,d,J=8.3Hz),7.94 -7.97(3H,m),8.11(1H,d,J=8.1Hz),8.55(1H,d,J=7.8Hz),8.73(1H,d,J= 7.8Hz),9.03(1H,brs)

### Production Example 66: Synthesis of N-[(S)-1-(1-naphthyl)ethyl]-5-(N-isobutyl-(2-hydroxyethyl)amino)-2-(S)-[4-[N-(imidazol-2-ylmethyl)aminomethyl]benzoyl]aminopentanoylamide [Compound No. 66]

The compound (32.1 mg) obtained in Example 65-1 was dissolved in methanol (1.2 ml). Isobutyl aldehyde (0.013 ml) and sodium cyano borohydride (9.4 mg) were added to the solution, and pH of the resultant solution was adjusted to 5 with acetic acid, followed by stirring at room temperature for 14 hours. After completion of reaction, the solvent was distilled off under reduced pressure, and a saturated sodium hydrogen carbonate aqueous solution (1 ml) and chloroform (3 ml) were added to the residue. The solution was added to a diatomite column (Chem Elut CE 1003, manufactured by Varian), and washed with chloroform (2 ml). The solution was added with methanesulfonic acid (0.032 ml) and stirred for 2.5 hours at room temperature. The solvent was distilled off under reduced pressure, and the residue was purified by silica gel column chromatography (chloroform/methanol/water = 7/3/0.5), to thereby obtain a methanesulfonate (24.6 mg) of the subj ect compound as white crystals.
MS (FAB,Pos.) :m/z=599 [M+1]⁺
¹H-NMR(500MHz,DMSO-d₆) : δ=0.84-0.91(6H,m) ,1.53(3H,d,J=6.8Hz) ,1.6 3-1.73(4H,m) ,1.91-2.00(1H,m) ,2.34(9H,s) ,2.76(1H,dt,J=5.9,14.6H z) ,2.91-2.94(1H,m) ,3.08(4H,m) ,3.68-3.69(2H,m) ,4.29(2H,brs) ,4.3 7(2H,brs) ,4.59(1H,m) ,5.73(1H,quint. ,J=6.8Hz) ,7.48-7.60(8H,m) ,7 .84(1H,d,J=8.3Hz) ,7.95-7.98(3H,m) ,8.11(1H,d,J=8.1Hz) ,8.56(1H,d ,J=7.8Hz) ,8.65(1H,brs) ,8.74(1H,t,J=8.1Hz) .

### Production Example 67: Synthesis of N-[(S)-1-(1-naphthyl)ethyl]-5-(N-isopropyl-(2-hydroxyethyl)amino)-2-(S)-[4-[N-(imidazol-2-ylmethyl)aminomethyl]benzoyl]aminopentanoylamide [Compound No. 67]

The compound (28.71 mg) obtained in Example 65-1 was dissolved inmethanol (1.2ml). Acetone (0.033ml) and sodium cyano borohydride (8.4 mg) were added to the solution, and pH of the resultant solution was adjusted to 5 with acetic acid, followed by stirring at room temperature for 14 hours. After completion of reaction, the solvent was distilled off under reduced pressure, and a saturated sodium hydrogen carbonate aqueous solution (1 ml) and chloroform (3 ml) were added to the residue. The solution was added to a diatomite column (Chem Elut CE 1003, manufactured by Varian) , and washed with chloroform (2 ml) . The solution was added with methanesulfonic acid (0.028ml) and stirred for 2.5 hours at room temperature. The solvent was distilledoff under reduced pressure, and the residue was purif ied by silica gel column chromatography (chloroform/methanol/water = 7/3/0.5), to thereby obtain a methanesulfonate (10.6 mg) of the subject compound as white crystals.
MS(FAB,Pos.):m/z=585[M+1]⁺
¹H-NMR(500MHz,DMSO-d₆) : δ=1.13-1.19(6H,m) ,1.53(3H,d,J=7.1Hz) ,1.6 5-1.73(4H,m),2.33(9H,s),2.92-3.15(5H,m),3.66(2H,brs),4.27(2H,b rs),4.32(2H,brs),4.58(1H,m),5.73(1H,quint.,J=7.1Hz),7.48-7.60(8H,m),7.84(1H,d,J=8.1Hz),7.95-7.98(3H,m),8.11(1H,d,J=7.3Hz),8. 55(1H,d,J=7.6Hz),8.69-8.74(2H,m).

### Production Example 68: Synthesis of N-[(S)-1-(1-naphthyl)ethyl]-5-(hexamethyleneimin-1-yl)-2-(S)-[4-[N-(pyridin-2-ylmethyl)aminomethyl]benzoyl]aminopentanoylamide [Compound No. 68]

### Example 68-1: Synthesis of N-[(1S)-1-(1-naphthyl)ethyl]-4-(methoxycarbonyl)-2-(S)-[4-[N-Boc-N-(pyridin-2-ylmethyl)aminomethyl]benzoylamino]butyrylamide (Compound XVI-2)

The compound (3.0726 g) obtained in Example 50-1 was dissolved in anhydrous methanol (30 ml), and the solution was added with 4 mol/l hydrochloric acid/dioxane (15 ml) and stirred at room temperature for 4 hours. After completion of reaction, the solvent was distilled off under reduced pressure. Then, the resultant residue, the compound (2.2554 g) obtained in Example 45-2, and DMAP (1.1681 g) were dissolved in chloroform (30 ml). A solution of DCC (2.0902 g) in chloroform (10 ml) was slowly added to the solution, followed by stirring at room temperature for 16 hours. The resultant solution containing precipitates was filtered, and the filtrate was acidified by adding 1 mol/l hydrochloric acid. Subsequently, extraction was performed with chloroform, and the resultant solution was washed with a saturated sodium hydrogen carbonate aqueous solution and brine and dried with anhydrous magnesium sulfate. The solvent was distilled off under reduced pressure, and the residue was purified by silica gel column chromatography (157.8 g, hexane/ethyl acetate = 1/2) , to thereby obtain the subject compound (3.4691 g) as a white solid.
MS(FAB,Pos.):m/z=639[M+1]⁺
¹H-NMR(500MHz,CDCl₃):δ=1.45(9H,brs),1.66(3H,d,J=6.8Hz),2.02-2.0 8(1H,m)2.10-2.18(1H,m),2.31-2.37(1H,-m),2.55-2.60(1H,m),3.62(3H ,s),4.49(2H,brs),4.60(2H,m),4.63-4.67(1H,m),5.93(1H,quint.,J=6 .8Hz),6.90(1H,d,J=8.3Hz),7.17-7.19(1H,m),7.31(1H,d,J=7.3Hz),7. 35(1H,d,J=7.6Hz),7.46-7.56(4H,m),7.64-7.67(1H,dt,J=1.7,6.0Hz), 7.76(2H,d,J=8.3Hz),7.80(1H,d,J=8.3Hz),7.88(1H,d,J=8.1Hz),8.09(1H,d,J=8.6Hz),8.53(1H,d,J=4.2Hz).

### Example 68-2: Synthesis of N-[(1S)-1-(1-naphthyl)ethyl]-5-hydroxy-2-(S)-[4-[N-Boc-N-(pyridin-2-ylmethyl)aminomethyl]benzoylamino]pentanoylamide (Compound XVII-2)

The compound (3.4477g) obtained in Example 68-1, sodium borohydride (821.4 mg), and calcium chloride (1.2154 g) were dissolved in a mixed solvent of THF (30 ml) and ethanol (40 ml), followed by stirring at room temperature for 2 hours. After completion of reaction, 1 mol/l citric acid aqueous solution was added to the solution, and extraction was performed with ethyl acetate. The resultant solution was washed with a saturated sodium hydrogen carbonate aqueous solution and dried with anhydrous magnesium sulfate. The solvent was distilled off under reduced pressure, and the resultant residue was purified by silica gel column chromatography (170 g, ethyl acetate) , to thereby obtain the subject compound (2.6203 g) as a white solid.
MS (FAB,Pos.) :m/z-611 [M+1]⁺
¹H-NMR(500MHz,CDCl₃) : δ=1.45 (9H,2s) , 1.52-1.61 (2H,m) 1.65 (3H,d,J= 7.3Hz) ,1.69-1.87(1H,m) ,1.88-1.95(1H,m) ,3.55-3.59(1H,m) ,3.63-3. 67(1H,m) ,4.49(2H,br) ,4.59(2H,br) ,4.78(1H,q,J=7.1Hz) ,5.93(1H,qu int. ,J=7.3Hz) ,6.95(1H,d,J=8.1Hz) ,7.17-7.19(2H,m) ,7.30-7.34(3H, m) ,7.45-7.56(4H,m) ,7.66(1H,dt,J=7.6,1.7Hz) ,7.74-7.76(2H,m) ,7.8 0(1H,d,J=8.1Hz) ,7.88(1H,d,J=7.8Hz) ,8.10(1H,d,J=8.5Hz) ,8.53(1H, d,J=4.2Hz).

### Example 68-3: Synthesis of N-[(1S)-1-(1-naphthyl)ethyl]-5-methanesulfoxy-2-(S)-[4-[N-Boc-N-(pyridin-2-ylmethyl)aminomethyl]benzoylamino]pentanoylamide (Compound XVIII-3)

The compound (2.6203 g) obtained in Example 68-2 was dissolved in anhydrous methylene chloride (35 ml) , and triethylamine (0.251 ml) was added to the solution. Methanesulfonyl chloride (0. 139 ml) was added to the solution with stirring, followed by stirring at room temperature for 1 hour. After completion of reaction, methylene chloride was added to the solution, and the resultant solution was washed with a saturated sodium hydrogen carbonate aqueous solution and brine and dried with anhydrous magnesium sulfate. The solvent was distilled off, and the residue was purified by silica gel column chromatography (chloroform/ethyl acetate = 1/1) , to thereby obtain the subject compound (2.5453 g) as a pale brown solid.
MS (FAB,Pos.) :m/z=765 [M+1]⁺
¹H-NMR(500MHz,CDCl₃) : δ=1.45(9H,2s) ,1.66(3H,d,J=6.9Hz) ,1.74-1.76 (3H,m) ,1.98-2.00(1H,m) ,2.85(3H,s) ,4.18-4.22(1H,m) ,4.42-4.55(3H ,m) ,4.59(2H,s) ,4.7-4.76(1H,m) ,5.91(1H,quint. ,J=6.8Hz) ,6.67(1H, d,J=8.1Hz) ,7.01-7.05(1H,brs) ,7.17-7.21(1H,m) ,7.29-7.31(2H,m) ,7 .35(1H,d,J=6.3Hz) ,7.47-7.57(4H,m) ,7.66(1H,t,J=7.5Hz) ,7.75(2H,d ,J=8.1Hz) ,7.81(1H,d,J=8.2Hz) ,7.88(1H,d,J=8.1Hz) ,8.09(1H,d,J=8. 7Hz) ,8.53(1H,d,J=4.4Hz).

### Example 68-4: Synthesis of N-[(S)-1-(1-naphthyl)ethyl]-5-(hexamethyleneimin-1-yl)-2-(S)-[4-[N-(pyridin-2-ylmethyl)aminomethyl]benzoyl]aminopentanoylamide [Compound No. 68]

The compound (66.8 mg) obtained in Example 68-3 was dissolved in toluene (3 ml) , and the solution was added with hexamethyleneimine (0.025 ml) and stirred at 70°C for 2 days. After completion of reaction, chloroform was added to the solution, and the resultant solution was washed with 0.5 mol/l sodium hydroxide aqueous solution and brine and dried with anhydrous sodium sulfate. Subsequently, the solvent was distilled off, and the residue was dissolved in chloroform (1.8 ml), and the solution was added with methanesul fonic acid (0.034ml) andmethanol (0.034ml) andstirredatroomtemperature for 1 day. After completion of reaction, the solvent was distilled off, and the residue was purified by silica gel column chromatography (chloroform/methanol/water=7/3/0.5), to thereby obtain the subject compound (44.1 mg) as a white solid.
MS(FAB,Pos.):m/z=592[M+1]⁺
¹H-NMR(500MHz,DMSO-d₆) : δ=1.53 (3H,d,J=7.1Hz) ,.50-1.81(12H,m) ,2. 33(9H,s) ,2.88-2.97(2H,m) ,2.99-3.08(2H,m) ,3.16-3.23(2H,m),4.32(2H,s),4.33(2H,s),4.52-4.60(1H.m),5.74(1H,quint.J=7.1Hz),7.46-7 .60(6H,m),7.63(2H,d,J=8.3Hz),7.84(1H,d,J=8.3Hz),7.90(1H,td,J=7 .5,1.5Hz) ,7.96(1H,d,J=7.8Hz) ,7.98(2H,d,J=8.3Hz) ,8.12(1H,d,J=8. 3Hz),8.56(1H,d,J=7.8Hz),8.67(1H,ddd,J=4.6,1.5,0.7Hz),8.75(1H,d ,J=8.1Hz),9.07(1H,brs),9.56(2H,brs).

### Production Example 69: Synthesis of N-[(S)-1-(1-naphthyl)ethyl]-5-(bis(2-hydroxyethyl)amino)-2-(S)-[4-[N-(pyridin-2-ylmethyl)aminomethyl]benzoyl]aminopentanoylamide [Compound No. 69]

The compound (64.3 mg) obtained in Example 68-3 and diethanolamine (34.8 mg) were dissolved in toluene (1 ml), followed by stirring at 70°C for 20 hours. After completion of reaction, the solvent was distilled off under reduced pressure, and the residue was dissolved in chloroform, followed by washing with water. The organic layer was dried with anhydrous magnesium sulfate, and the solvent was distilled off under reduced pressure. Then, the residue was dissolved in chloroform (2 ml) and methanol (0.5 ml), and the solution was added with methanesulfonic acid (0.060 ml) and stirred for 4 days at room temperature. The solvent was distilled off under reduced pressure, and the residue was purified by silica gel column chromatography (chloroform/methanol/water = 7/3/0.5), to thereby obtain a methanesulfonate (61.8 mg) of the subject compound as white crystals.
MS(FAB,Pos.):m/z=598[M+1]⁺
¹H-NMR(500MHz,DMSO-d₆) : δ=1.53 (3H,d,J=6.8Hz) , 1.56-1.74 (4H,m) ,2.3 5(9H,s) , 3.09-3.20(4H,m) ,3.65-3.70(6H,m) ,4.33(4H,brs) ,4.55(1H,d d,J=8.5,13.4Hz),5.72(1H,quint.,J=6.8Hz),7.45-7.58(6H,m),7.63(2 H,d,J=8.3Hz),7.83(1H,d,J=8.3Hz),7.91(1H,dt,J=1.7,7.8Hz),7.94-7 .96(1H,m),7.97(2H,d,J=8.5Hz),8.11(1H,d,J=8.1Hz),8.56(1H,d,J=8. 1Hz),8.67(1H,d,J=2.2Hz),8.73(1H,d,J=7.8Hz),9.01(1H,br),9.57'1H,br).

### Production Example 70: N-[(S)-1-(1-naphthyl)ethyl]-5-(N-isobutyl(2-hydroxyethyl)amino)-2-(S)-[4-[N-(pyridin-2-ylmethyl)aminomethyl]benzoyl]aminopentanoylamide [Compound No. 70]

### Example 70-1: Synthesis of N-[(S)-1-(1-naphthyl)ethyl]-5-(N-isobutyl(2-hydroxyethyl)amino)-2-(S)-[4-[N-(pyridin-2-ylmethyl)aminomethyl]benzoyl]aminopentanoylamide (Compound XIIa-13)

The compound (150.6 mg) obtained in Example 68-3 and ethanolamine (0. 040 ml) were dissolved in toluene (2.3 ml) , followed by stirring at 70°C for 21 hours. After completion of reaction, the solvent was distilled off under reduced pressure, and the residue was dissolved in chloroform, followed by washing with water. The organic layer was dried with anhydrous magnesium sulfate, and the solvent was distilled off under reduced pressure, to thereby obtain the subject compound (154.7 mg).
MS(FAB,Pos.):m/z=654[M+1]⁺
¹H-NMR(500MHz,DMSO-d₆): δ=1.31(9H,brs) ,1.52(3H,d,J=6.8Hz) ,1.63-1 .80(4H,m) ,2.83-2.95(4H,m) ,3.62(2H,m) ,4.41(1H,m) ,4.49 (2H,brs) ,4 .57(2H,brs),5.22(1H,quint.,J=6.8Hz),7.21-7.36(4H,m),7.47-7.58(4H,m),7.78(1H,dt,J=1.7,7.8Hz),7.82(1H,d,J=8.1Hz),7.89(2H,d,J=8 .3Hz),7.94(1H,d,J=7.8Hz),8.11(1H,d,J=8.1Hz),8.47(1H,d,J=8.3Hz) ,8.52(1H,d,J=4.2Hz),8.78(1H,d,J=7.6Hz)

### Example 70-2: Synthesis of N-[(S)-1-(1-naphthyl)ethyl]-5-(N-isobutyl(2-hydroxyethyl)amino)-2-(S)-[4-[N-(pyridin-2-ylmethyl)aminomethyl]benzoyl]aminopentanoylamide [Compound No. 70]

The compound (135.5 mg) obtained in Example 70-1 was dissolved in methanol (5.4 ml), and sodium cyano borohydride (40.1 mg) was added to the solution. Subsequently, pH of the resultant solution was adjusted to 5 with acetic acid, and isobutyl aldehyde (0.058 ml) was gradually added to the solution, followed by stirring at room temperature for 14 hours. After completion of reaction, the solvent was distilled off under reduced pressure, and the residue was dissolved in chloroform, followed by washing with water. The organic layer was dried with anhydrous magnesium sulfate, and the solvent was distilled off under reduced pressure. The residue was dissolved in chloroform (10ml) andmethanol (0.5 ml), and the solution was added with methanesulfonic acid (0.136 ml) and stirred for 2 hours at room temperature. After completion of reaction, the solvent was distilled off under reduced pressure, and the residue was purified by silica gel column chromatography (chloroform/methanol/water = 7/3/0.5), to thereby obtain a methanesulfonate (92.3 mg) of the subject compound as white crystals.
MS (FAB,Pos.) :m/z=610 [M+1]⁺
¹H-NMR(500MHz,DMSO-d₆) : δ=0.84-0.91 (6H,m) ,1 .53 (3H,d,J=6. 8Hz) ,1.6 1-1.73 (4H,m) ,1.96-2.02 (1H,m) ,2.32 (9H,s) ,2.75-2.81 (1H,m) ,2.90-2 .95(1H,m) ,3.08-3.17(4H,m) ,3.68-3.69(2H,m) ,4.32(4H,brs) ,4.58(1H ,dd,J=8.1,12.1Hz) ,5.73 (1H,quint.,J=6.8Hz) ,7.45-7.61 (6H,m) ,7.63 (2H,d,J=8.5Hz) ,7.84 (1H,d,J=8.1Hz) ,7.88-7.91 (1H,m) ,7.92-7.99 (3H ,m) ,8.11(1H,d,J=8.3Hz) ,8.57(1H,d,J=7.8Hz) ,8.66-8.68(2H,m) ,8.74 (1H,t,J=8.1Hz) ,9.56 (2H,brs).

### Production Example 71: Synthesis of N-[(S)-1-(1-naphthyl)ethyl]-5-ureide-2-(S)-[4-[N-(imidazol-2-ylmethyl)aminomethyl]benzoyl]aminopentanoylamide [Compound No. 71]

The compound (100.4 mg) obtained in Example 1-5 was dissolved in DMF (2 ml), and the solution was added with 3,5-dimethylpyrazole-1-carboxyamide (28.1 mg) and stirred at 80°C for 3 hours. After completion of reaction, the solvent was distilled off, and the residue was purified by silica gel column chromatography (chloroform/methanol = 5/1). The purified product was dissolved in chloroform, and the resultant solution was washed with distilled water and brine. After the solution was filtered with cerite, the solvent was distilled off, and the residue was dissolved in methanol (1 ml). Subsequently, the solution was added with 4 mol/l hydrochloric acid/dioxane solution (2 ml) and stirred at room temperature for 4.5 hours. After completion of reaction, the solvent was distilled off. The residue was azeotropically distilled with methanol, to thereby obtain a hydrochloride (51.5 mg) of the subject compound as a white solid.
MS(FAB,Pos.):m/z=542[M+1]⁺
¹H-NMR(500MHz,DMSO-d₆) : δ=1.33-1.47(2H,m),1.51(3H,d,J=6.8Hz),1.6 3-1.79 (2H,m) ,2.93-3.02(2H,m) ,4.40(2H,s) ,4.54-4.61(1H,m) ,4.56(2 H,s),4.8-6.2(3H,br),5.70(1H,quint.,J=6.8Hz),7.45-7.58(4H,m),7. 69(2H,d,J=8.3Hz) ,7.75(2H,s) ,7.81(1H,d,J=8.1Hz) ,7.94(1H,d,J=7.8 Hz) ,7.97(2H,d,J=8.3Hz) ,8.10(1H,d,J=8.3Hz) ,8.57(1H,d,J=8.1Hz), 8 .76(1H,d,J=7.6Hz).

### Production Example 72: N-[(S)-1-(1-naphthyl)ethyl]-5-(3-phenylthioureide)-2-(S)-[4-[N-(pyridin-2-ylmethyl)aminomethyl]benzoyl]aminopentanoylamide [Compound No. 72]

### Example 72-1: Synthesis of N-[(S)-1-(1-naphthyl)ethyl]-5-(3-phenylthioureide)-2-(S)-[4-[N-Boc-N-(pyridin-2-ylmethyl)aminomethyl)benzoyl]aminopentanoylamide (Compound XIIc-1)

The compound (377.2 mg) obtained in Example 45-3 and phenylisothiocyanate (0.074ml) were dissolved inmethylene chloride (12.4 ml), followed by stirring at room temperature for 3 hours, but the raw materials remained in the solution. Then, the solution was added with phenylisothiocyanate (0.023 ml) and stirred for 18 hours. After completion of reaction, the solvent was distilled off under reduced pressure, and the residue was purified by silica gel column chromatography (ethyl acetate) , to thereby obtain the subject compound (413.3 mg) as white crystals.
MS(FAB,Pos.):m/z=745[M+1]⁺
¹H-NMR(500MHz,DMSO-d₆): δ=1.34(9H,br) ,1.51(3H,d,J=6.8Hz) ,1.54-1. 63(2H,m),1.68-1.78(2H,m),3.46(2H,brs),4.41(1H,brs),4.49(4H,br) ,5.68-5.74(1H,quint.,J=6.8Hz),7.09(1H,t,J=7.3Hz),7.20-7.40(8H, m) ,7.47-7.58(4H,m) ,7.78(1H,td,J=1.7,7.6Hz) ,7.82(1H,d,J=8.1Hz), 7.87(2H,d,J=8.3Hz),7.94(1H,d,J=8.1Hz),8.10(1H,d,J=8.5Hz),8.40(1H,d,J=8.2Hz),8.52(1H,d,J=4.4Hz),8.67(1H,d,J=7.5Hz),9.55(1H,brs).

### Example 72-2: Synthesis of N-[(S)-1-(1-naphthyl)ethyl]-5-(3-phenylthioureide)-2-(S)-[4-[N-Boc-N-(pyridin-2-ylmethyl)aminomethyl]benzoyl]aminopentanoylamide [Compound No. 72]

The compound (43.0 mg) obtained in Example 72-1 was added to chloroform (1.0 ml) and methanesulfonic acid (0.0132 ml), followed by stirring at room temperature for 7 hours. After completion of reaction, the solvent was distilled off under reduced pressure, and the residue was purified by silica gel column chromatography (chloroform/methanol/water = 7/3/0.5), to thereby obtain a methanesulfonate (48.9mg) of the subject compound as white crystals.
MS (FAB,Pos.) :m/z=645 [M+1]⁺
¹H-NMR(500MHz,DMSO-d₆+D₂O) : δ=1.53(3H,d,J=7.1Hz) ,1.56-1.60(2H,m) ,1.72-1.81 (2H,m) ,2.42 (6H,s) ,3.48 (2H,brs) ,4.32 (4H,d,J=7.6Hz) ,4. 58(1H,dd,J=5.4,9.3Hz) ,5.70(1H,q,J=7.1Hz) ,7.14(1H,t,J=7.1Hz) ,7. 30-7.36(4H,m) ,7.45-7.63(6H,m) ,7.62(2H,d,J=8.3Hz) ,7.83(1H,d,J=8 .1Hz) ,7.86-790(1H,m) ,7.91-7.95(3H,m) ,8.09(1H,d,J=8.3Hz) ,8.65-8 .66(1H,m) .

### Production Example 73: Synthesis of N-[(S)-1-(1-naphthyl)ethyl]-5-sulfinamidino-2-(S)-[4-[N-(pyridin-2-ylmethyl)aminomethyl]benzoyl]aminopentanoylamide [Compound No. 73]

### Example 73-1: Synthesis of N-[(1S)-1-(1-naphthyl)ethyl]-5-toluenesulfoxy-2-(S)-[4-[N-Boc-N-(pyridin-2-ylmethyl)aminomethyl]benzoylamino]pentanoylamide (Compound XVIII-4)

The compound (365.6 mg) obtained in Example 68-2 was dissolved in anhydrous methylene chloride (4 ml), and triethylamine (1.79 ml) was added to the solution. Toluenesulfonyl chloride (5.07 g) was added to the solution with stirring, followed by stirring at room temperature for 29 hours. After completion of reaction, methylene chloride was added to the solution. The resultant solution was washed with a saturated sodium hydrogen carbonate aqueous solution and brine and dried with anhydrous magnesium sulfate. The solvent was distilled off, and the residue was purified by silica gel column chromatography (chloroform/ethyl acetate = 1/3), to thereby obtain the subject compound (368 mg) as a white solid.
MS (FAB,Pos.) :m/z=689 [M+1]⁺
¹H-NMR(500MHz,CDCl₃) : δ=1.45(9H,2s) ,1.66(3H,d,J=6.8Hz) ,1.68-1.83 (3H,m) ,1.93-1.99(1H,m) ,2.42(3H,s), 3.95-3.99(1H,m) ,4.29(1H,m) ,4 .49(2H,br) ,4.59(2H,s) ,4.71(1H,brs) ,5.88-5.94(1H,m) ,6.66(1H,d,J =8.3Hz) ,6.96(1H,brs) ,7.17-7.19(1H,m) ,7.28-7.30(4H,m) ,7.35(1H,d ,J=7.1Hz) ,7.47-7.57(4H,m) ,7.64-7.67(1H,m) ,7.69(2H,d,J=8.5Hz) ,7 .73(2H,d,J=8.3Hz) ,7.82(1H,d,J=8.1Hz) ,7.90(1H,d,J=7.8Hz) ,8.08(1 H,d,J=8.5Hz) ,8.53(1H,d,J=3.9Hz) .

### Example 73-2: Synthesis of N-[(S)-1-(1-naphthyl)ethyl]-5-sulfinamidino-2-(S)-[4-[N-Boc-N-(pyridin-2-ylmethyl)aminomethyl]benzoyl]aminopentanoylamide (Compound XIIc-2)

The compound (242.0 mg) obtained in Example 73-1 and thiourea (63.1 mg) were added to acetone (1.5 ml) , followed by reflux with heating for 17 hours. After completion of reaction, the solvent was distilled off under reducedpressure, and the residue was purified by silica gel column chromatography (chloroform/methanol = 7/1). The purified product was dissolved in chloroform (20 ml) , and the resultant solution was washed with 1 mol/l sodium hydroxide aqueous solution and dried with anhydrous magnesium sulfate. The solvent was distilled off under reduced pressure, to thereby obtain the subject compound (40.7 mg) as white crystals.
MS (FAB,Pos.) :m/z=669 [M+1]⁺
¹H-NMR(500MHz,DMSO-d₆) : δ=1.34 (9H,br) , 1.51 (3H,d,J=6.8Hz) , 1.54-1. 61 (2H,m) ,1.70-1.83 (2H,m) ,2.80 (2H,t,J=7.3Hz) ,4.40 (1H,brs) ,4.48-4.56(4H,br) ,5.70(1H,quint. ,J=6.8Hz) ,7.23-7.35(4H,m) ,7.46-7.57(4H,m),7.78(1H,td,J=1.7,7.6Hz) ,7.82(1H,d,J=7.1Hz) ,7.86(2H,d,J=8 .3Hz) ,7.93-7.95(1H,m) ,8.10(1H,d,J=8.1Hz) ,8.40(1H,d,J=8.1Hz) ,8. 52(1H,d,J=4.8Hz) ,8.69(1H,d,J=7.3Hz)

### Example 73-3: Synthesis of N-[(S)-1-(1-naphthyl)ethyl]-5-sulfinamidino-2-(S)-[4-[N-(pyridin-2-ylmethyl)aminomethyl]benzoyl]aminopentanoylamide [Compound No. 73]

The compound (38.4 mg) obtained in Example 73-1 was dissolved in methanol (0.1 ml) and chloroform (1.0 ml), and the solution was added with methanesulfonic acid (0.0131 ml) and stirred at room temperature for 22 hours. After completion of reaction, the solvent was distilled off under reduced pressure, and the residue was purified by silica gel column chromatography (chloroform/methanol/water = 7/3/0.5), to thereby obtain a methanesulfonate (39.4 mg) of the subject compound as white crystals.
MS (FAB,Pos.) :m/z=569 [M+1]⁺
¹H-NMR(500MHz,DM50-d₆) : δ=1.52 (3H,d,J=6.8Hz) ,1.57-1.63 (2H,m) ,1.7 5-1.84 (2H,m) ,2.33(9H,s) ,3.11(2H,t,J=7.2Hz) ,4.32(4H,brs) ,4.55-4 .59 (1H,m) ,5.72 (1H,quint. ,J=6. 8Hz) , 7.45-7.58 (6H,m) 7.62 (2H,d,J= 8.4Hz) ,7.84(1H,d,J=8.1Hz) ,7.89-7.97(3H,m) ,8.11(1H,d,J=8.2Hz) ,8 .53(1H,d,J=7.7Hz) ,8.66-8.68(1H,m) ,8.75(1H,d,J=7.7Hz) ,9.00(4H,b rs) ,9.55(2H,brs) .

### Production Example 74: Synthesis of N-[(S)-1-(1-naphthyl)ethyl]-5-(N-methylsulfinamidino)-2-(S)-[4-[N-(pyridin-2-ylmethyl)aminomethyl]benzoyl]aminopentanoylamide [Compound No. 74]

The compound (123.1 mg) obtained in Example 68-3 and methylthiourea (45.5mg) were dissolved in acetone (2.5ml), followed by reflux with heating for 49 hours. The solvent was distilled off under reduced pressure, and the residue was dissolved in chloroform (2.5ml) andmethanol (0.2 ml). Subsequently, the solution was added with methanesulfonic acid (0.117 ml) and stirred at room temperature for 22 hours . After completion of reaction, the solvent was distilled off under reduced pressure, and the residue was purified by silica gel column chromatography (chloroform/methanol/water = 7/3/0.5), to thereby obtain a methanesulfonate (143.3 mg) of the subject compound as white crystals.
MS (FAB,Pos.) :m/z=583 [M+1]⁺
^{1H}-NMR(500MHz, DMSO-d₆) : δ=1.51 (3H,d,J=6.8Hz) , 1.56-1.62 (2H,m) , ,1.7 5-1.82(2H,m),2.31(9H,s),2.87(3H,d,J=4.9Hz),3.11-3.14(2H,m),4.3 2(4H,brs) ,4.57(1H,dd,J=8.1,14.4Hz) ,5.72(1H,quint. ,J=6.8Hz) ,7.4 5-7.56(6H,m) ,7.62(2H,d,J=8.3Hz) ,7.84(1H,d,J=7.8Hz) ,7.91(1H,dt, J=1.7,7.6Hz) ,7.94-7.97(3H,m) ,8.10(1H,d,J=7.8Hz) ,8.53(1H,d,J=8. 1Hz),8.67(1H,d,J=4.2Hz),8.74(1H,d,J=7.8Hz),8.97(1H,brs),9.20(1 H,brs),9.43(1H,br),9.55(2H,brs).

### Production Example 75: Synthesis of N-[(S)-1-(1-naphthyl)ethyl]-5-(N,N'-dimethylsulfinamidino)-2-(S)-[4-[N-(pyridin-2-ylmethyl)aminomethyl]benzoyl]aminopentanoylamide [Compound No. 75]

The compound (100.7 mg) obtained in Example 73-1 and N,N'-dimethylthiourea (98.0 mg) were dissolved in acetone (3 ml), followed by reflux with heating for 72 hours. After completion of reaction, the solvent was distilled off under reduced pressure, and the residue was dissolved in chloroform (2 ml) and methanol (0.2ml). Subsequently, the solution was added withmethanesulfonic acid (0.097 ml) and stirred at room temperature for 22 hours. The solvent was distilled off under reduced pressure, and the residue was purified by silica gel column chromatography (chloroform/methanol/water = 7/3/0.5), to thereby obtain a methanesulfonate (66.8 mg) of the subj ect compound as white crystals.
MS(FAB,Pos.):m/z=597[M+1]⁺
¹H-NMR(500MHz,DMSO-d₆):δ=1.49-1.65(2H,m),1.52(3H,d,J=7.1Hz),1.7 3-1.88(2H,m),2.31(9H,s),2.86(3H,d,J=4.6Hz),2.92(3H,d,J=4.4Hz), 3.15(2H,t,J=7.3Hz) ,4.32(4H,s) ,4.56(1H,dd,J=8.5,13.9Hz) ,5.73(1H, ,quint.,J=7.1Hz),7.45-7.57(6H,m),7.62(2H,d,J=8.5Hz),7.84(1H,d, J=8.5Hz),7.89-7.95(1H,m),7.96-7.97(2H,m),8.11(1H,d,J=7.6Hz),8. 54(1H,d,J=7.8Hz),8.66-8.68(1H,m),8.73(1H,d,J=7.8Hz),8.82(1H,d, J=4.9Hz),9.07(1H,d,J=4.4Hz),9.55(2H,brs).

### Production Example 76: Synthesis of N-[(S)-1-(1-naphthyl)ethyl]-5-sulfinamidino-2-(S)-[4-[N-(imidazol-2-ylmethyl)aminomethyl]benzoyl]aminopentanoylamide [Compound No. 76]

The compound (230.1 mg) obtained in Example 50-4 and thiourea (77.1 mg) were dissolved in acetone (2.0 ml), followed by reflux with heating for 3 hours. After completion of reaction, the solvent was distilled off under reduced pressure, and the residue was dissolved in chloroform. The resultant solution was washed with 1 mol/l sodium hydroxide aqueous solution and dried with anhydrous magnesium sulfate.

The solvent was distilled off under reduced pressure. The residue was dissolved in chloroform (1.1 ml) and methanol (0.5 ml), and the solution was added with methanesulfonic acid (0.034 ml) and stirred at room temperature for 24 hours. The solvent was distilled off under reduced pressure, and the residue was purified by silica gel column chromatography (chloroform/methanol/water = 7/3/0.5), to thereby obtain a methanesulfonate (26.2 mg) of the subject compound as white crystals.
MS (FAB,Pos.) :m/z=558 [M+1]⁺
¹H-NMR(500MHz,DMSO-d₆) : δ=1.52 (3H,d,J=6.8Hz) ,2.55-1.62 (2H,m) ,1.7 5-1.83(2H,m) ,2.34(9H,s) ,3.11(2H,t,J=7.1Hz) ,4.27(2H,s) ,4.32(2H, s) ,4.57(1H,dd,J=8.1,13.9Hz) ,5.71(1H,qint. ,J=6.8Hz) ,7.47-7.59(8 H,m) ,7.84(1H,d,J=8.1Hz) ,7.94-7.97(3H,m) ,8.10(1H,d,J=7.1Hz) ,8.5 2(1H,d,J=7.8Hz) ,8.75(1H,J=7.8Hz) ,8.99(3H,brs) .

### Production Example 77: Synthesis of N-[(S)-1-(1-naphthyl)ethyl]-5-(N-methylsulfinamidino)-2-(S)-[4-[N-(imidazol-2-ylmethyl)aminomethyl]benzoyl]aminopentanoylamide [Compound No. 77]

The compound (577.0 mg) obtained in Example 57-1 and methylthiourea (414.9mg) were dissolved in acetone (17 ml), followed by reflux with heating for 4 days. After completion of reaction, the solvent was distilled off under reduced pressure, and the residue was dissolved in chloroform (10 ml) and methanol (0.5 ml). Subsequently, the solution was added with methanesulfonic acid (0.49 ml) and stirred at room temperature for 22 hours. The solvent was distilled off under reduced pressure, and the residue was purified by silica gel column chromatography (chloroform/methanol/water = 7/3/0.5), to thereby obtain a methanesulfonate (42.0 mg) of the subject compound as white crystals.
MS (FAB,Pos.) :m/z=572 [M+1]⁺
¹H-NMR(500MHz,DMSO-d₆) : δ=1.52 (3H,d,J=6.8Hz) , 1.54-1.64 (2H,m) , 1.7 5-1.82(2H,m) ,2.33(9H,s) ,2.87(3H,d,J-4.6Hz) ,3.12-3.14(1H,m) ,4.2 8(2H,brs) ,4.34(2H,brs) ,4.56(1H,dd,J=8.1,13.9Hz) ,5.72(1H,quint. ,J=6.8Hz) ,7.47-7.59(8H,m) ,7.84(1H,d,J=8.3Hz) ,7.94-7.97(3H,m) ,8 .11(1H,d,J=7.8Hz) ,8.73(1H,d,J=7.8Hz) ,8.97(1H,br) ,9.20(1H,br) ,9 .43(1H,d,J=4.9Hz) .

### Production Example 78: Synthesis of N-[(S)-1-(1-naphthyl)ethyl]-5-(N,N'-dimethylsulfinamidino)-2-S)-[4-[N-(imidazol-2-ylmethyl)aminomethyl]benzoyl]aminopentanoylamide [Compound No. 78]

The compound (253.1 mg) obtained in Example 57-1 and N,N'-dimethylthiourea (213.2 mg) were dissolved in acetone (2.5 ml), followed by reflux with heating for 3 days. After completion of reaction, the solvent was distilled off under reduced pressure, and the residue was dissolved in chloroform (3 ml) and methanol (0.5ml). Subsequently, the solution was added with methanesulfonic acid (0.214 ml) and stirred at room temperature for 17 hours. The solvent was distilled off under reduced pressure, and the residue was purified by silica gel column chromatography (chloroform/methanol/water = 7/3/0.5), to thereby obtain a methanesulfonate (56.5mg) of the subj ect compound as white crystals.
MS (FAB,Pos.) :m/z=586 [M+1]⁺
¹H-NMR(500MHz,DMSO-d₆) : δ=1.52 (3H,d,J=7.1Hz) , 1.55-1.64 (2H,m) , 1.7 6-1.87(2H,m) ,2.33(9H,s) ,2.86(3H,d,J=4.4Hz) ,2.92(3H,d,J=4.4Hz), 4.27(4H,br) ,4.56(1H,dd,J=8.1,14.2Hz) ,5.72(1H,quint. ,J=7.1Hz) ,7 .47-7.59(8H,m) ,7.84(1H,d,J=8.3Hz) ,7.94-7.97(3H,m) ,8.11(1H,d,J= 7.1Hz) , 8.52 (1H,d,J=7.6Hz) ,8.73 (1H,d,J=7.8Hz) , 8. 81 (1H,br) , 9.06 (1H,br).

### Production Example 78: Synthesis of N-[(S)-1-(1-naphthyl)ethyl]-5-(N,N'-dimethylsulfinamidino)-2-(S)-[4-(N-(imidazol-2-ylmethyl)aminomethyl)benzoyl]aminopentanoylamide [Compound No. 78]

The compound (253.1 mg) obtained in Example 57-1 and N,N'-dimethylthiourea (213.2 mg) were dissolved in acetone (2.5 ml), followed by reflux with heating for 3 days. After completion of reaction, the solvent was distilled off under reduced pressure, and the residue was dissolved in chloroform (3 ml) and methanol (0.5ml). Subsequently, the solution wasadded with methanesulfonic acid (0.214 ml) and stirred at room temperature for 17 hours. The solvent was distilled off under reduced pressure, and the residue was purified by silica gel column chromatography (chloroform/methanol/water = 7/3/0.5), to thereby obtain a methanesulfonate (56.5mg) of the subject compound as white crystals.
MS(FAB,Pos.):m/z=586[M+1]⁺
¹H-NMR(500MHz,DMSO-d₆) : δ=1.52(3H,d,J=7.1Hz),1.55-1.64(2H,m),1.7 6-1.87(2H,m) ,2.33(9H,s) ,2.86(3H,d,J=4.4Hz) ,2.92(3H,d,J=4.4Hz), 4.27(4H,br),4.56(1H,dd,J=8.1,14.2Hz),5.72(1H,quint.,J=7.1Hz),7 .47-7.59(8H,m) ,7.84(1H,d,J=8.3Hz) ,7.94-7.97(3H,m) ,8.11(1H,d,J= 7.1Hz) ,8.52(1H,d,J=7.6Hz) ,8.73 (1H,d,J=7.8Hz) ,8.81(1H,br) ,9.06(1H,br).

### Production Example 79: Synthesis of N-[(S)-1-(naphthyl)ethyl]-5-[N-methyl-(pentan-3-yl)amino]-2-(S)-[4-[N-(imidazol-2-ylmethyl)aminomethyl]benzoyl]aminopentanoylamide [Compound No. 79]

The compound (50.0 mg) obtained in Example 1-5 was dissolved in methanol (1 ml). Then, the solution was added with sodium cyano borohydride (8 mg), acetic acid (10 µl), and 3-pentanone (10 µl) and stirred at room temperature for 1.5 hours. Further, the solution was added with sodium cyano borohydride (8 mg), acetic acid (40 µl), and 3-pentanone (10 µl) and stirred at room temperature for 18 hours. After completion of reaction, the solution was added with sodium cyano borohydride (15 mg) and 36% formaldehyde aqueous solution (20 µl) and stirred at room temperature for 1.5 hours. After completion of reaction, the solvent was distilled off under reduced pressure, and the residue was dissolved in chloroform. The resultant solution was washed with 0.5 mol/l sodium hydroxide aqueous solution and brine and dried with anhydroussodiumsulfate. After filtration, the solvent was distilled off under reduced pressure, and the residue was purified by silica gel column chromatography (chloroform/methanol = 10/1). The purified product was dissolved in chloroform (1 ml) , and the solution was added with methanesulfonic acid (17 µl) and stirred at room temperature for 14 hours. After completion of stirring, the solvent was distilled off under reduced pressure, and the residue was purified by silica gel column chromatography (chloroform/methanol/water = 7/3/0.5), to thereby obtain a methanesulfonate (45 mg) of the subject compound as a white solid.
MS(FAB,Pos.):m/z=583[M+1)⁺
¹H-NMR (500MHz,DMSO-d₆) : δ=0.83-0.95(6H,m) ,1.40-1.82(11H,m),2.35(9H,s),2.55-2.69(3H,m),2.92-3.00(2H,m),3.01-3.17(1H,m),4.31(2H, s),4.41(2H,s),4.55-4.63(1H,m),5.73(1H,quint.,J=7.1Hz),7.46-7.6 4(8H,m),7.84(1H,d,J=8.1Hz),7.98(2H,d,J=8.1Hz),8.11(1H,d,J=8.1H z)8.65(1H,brs),8.70-8.77(1H,m)

### Production Example 80: Synthesis of N-[(S)-1-(naphthyl)ethyl]-5-[N-ethyl-(pentan-3-yl)amino]-2-(S)-[4-[N-(imidazol-2-ylmethyl)aminomethyl]benzoyl]aminopentanoylamide [Compound No. 80]

The compound (50.0 mg) obtained in Example 1-5 was dissolved in methanol (1 ml). Then, the solution was added with sodium cyano borohydride (8 mg), acetic acid (10 µl), and 3-pentanone (10 µl) and stirred at room temperature for 1.5 hours. Further, the solution was added with sodium cyano borohydride (8 mg), acetic acid (40 µl) , and 3-pentanone (10 µl) and stirred at room temperature for 18 hours. After completion of reaction, the solution was added with sodium cyano borohydride (16 mg) and acetaldehyde (3 drops, by using a Pasteur pipet) and stirred at room temperature for 1.5 hours. After completion of reaction, the solvent was distilled off under reduced pressure, and the residue was dissolved in chloroform. The resultant solution was washed with 0.5 mol/l sodium hydroxide aqueous solution and brine and dried with anhydrous sodium sulfate. After filtration, the solvent was distilled off under reduced pressure, and the residue was purified by silica gel column chromatography (chloroform/methanol = 10/1). The purified product was dissolved in chloroform (1ml), and the solution was added with methanesulfonic acid (15 µl) and stirred at room temperature for 14 hours. After completion of stirring, the solvent was distilled off under reduced pressure, and the residue was purified by silica gel column chromatography (chloroform/methanol/water = 7/3/0.5), to thereby obtain a methanesulfonate (47 mg) of the subject compound as a white solid.
MS (FAB,Pos.) :m/z=597 [M+1]⁺
¹H-NMR(500MHz,DMSO-d₆, 80°C) : δ=0. 85-0.95 (6H,m) ,1.4-1.21 (3H,m) ,1. 48-1.61 (2H,m) ,1.54 (2H,d,J=7.1Hz) ,1.60-1.89 (6H,m) ,2.37 (9H,s) ,2. 92-3.13(5H,m) ,4.18(2H,s) 4.27(2H,s) ,4.57-4.63(1H,m) ,5.75(1H,quint. , 7.1Hz) , 7.41 (2H,s) 7.44-7.59 (6H,m) 7.81 (1H,d, J=8.3Hz) , 7.91 (3H,d,J=8.1Hz) ,8.12(1H,d,J=7.8Hz) ,8.25(2H,d,J=7.8Hz) ,8.39-8.43 (1H,m)

### Production Example 81: Synthesis of N-[(S)-1-(naphthyl)ethyl]-5-[N-n-propyl-(pentan-3-yl)amino]-2-(S)-[4-[N-(imidazol-2-ylmethyl)aminomethyl]benzoyl]aminopentanoylamide [Compound No. 81]

The compound (50.0 mg) obtained in Example 1-5 was dissolved in methanol (1 ml). Then, the solution was added with sodium cyano borohydride (8 mg), acetic acid (10 µl), and 3-pentanone (10 µl) and stirred at room temperature for 1.5 hours. Further, the solution was added with sodium cyano borohydride (12 mg), acetic acid (40 µl), and 3-pentanone (10 µl) and stirred at room temperature for 18 hours. After completion of reaction, the solution was added with sodium cyano borohydride (16 mg) and propionaldehyde (68 µl) and stirred at room temperature for 17 hours. After completion of reaction, the solvent was distilled off under reduced pressure, and the residue was dissolved in chloroform. The resultant solution was washed with 0.5 mol/l sodium hydroxide aqueous solution and brine and dried with anhydrous sodium sulfate. After filtration, the solvent was distilled off under reduced pressure, and the residue was purified by silica gel column chromatography (chloroform/methanol = 10/1). The purified product was dissolved in chloroform (1 ml), and the solution was added with methanesulfonic acid (17 µl) and stirred at room temperature for 23 hours. After completion of stirring, the solvent was distilled off under reduced pressure, and the residue was purified by silica gel column chromatography (chloroform/methanol/water = 7/3/0.5), to thereby obtain a methanesulfonate (41 mg) of the subject compound as a white solid.
MS(FAB,Pos.) :m/z=611[M+1]⁺
¹H-NMR(500MHz,DMSO-d₆,80°C) : δ=0.83-0.94(9H,m) ,1.51-1.80(10H,m), 1.52(1H,d,J=6.6Hz) ,2.36(9H,s) ,2.80-2.93(1H,m) ,2.95-3.14(4H,m), 4.31(2H,s) ,4.41(2H,s) ,4.57-4.65(1H,m)5.70-5.80(1H,brs) ,7.48-7. 61(8H,m) ,7.84(1H,d,J=8.3Hz) ,7.95-7.98(1H,m) ,7.98(1H,d,J=8.3Hz) ,8.11(1H,d,J=8.1Hz)8.41(1H,brs) ,8.58(1H,d,J=5.4Hz) ,8.70-8.75(1H ,m) .

The following compounds were obtainedby a similar synthesizing method.
N-[(S)-1-(naphthyl)ethyl]-5-[N-carboxymethyl(isobutyl)amino]-2 -(S)-{4-[N-(1H-imidazol-2-ylmethyl)aminomethyl]benzoyl}aminopentanoylamide [Compound No. 82]
MS(FAB,Pos.):m/z=613[M+H]⁺
¹H-NMR(500MHz,DMSO-d₆) : δ=0.84-0.93(6H,m), 1.52(3H,d,J=6.8Hz), 1.5 3-1.75(4H,m),1.97(1H,sext.,d=6.6Hz),2.32(6H,s),2.89(2H,d,J=6.6 Hz) ,3.08-3.21(2H,m,4.02(2H,s) ,4.22(4H,brs) ,4.56-4.62(1H,m) ,5. 72(1H,quint.,J=6.8Hz) ,7.39(2H,brs) ,7.47-7.61(6H,m) ,7.83(1H,d,J =8.1Hz),7.93-7.99(3H,m),8.11(1H,d,J=8.3Hz),8.53(1H,d,J=8.1Hz), 8.73(1H,d,J=7.8Hz).
N-[(S)-1-(naphthyl)ethyl]-5-[N-carbamoylmethyl(isobutyl)amino] -2-(S)-{4-[N-(1H-imidazol-2-ylmethyl)aminomethyl]benzoyl}aminopentanoylamide [Compound No. 83]
MS(FAB,Pos.):m/z=612[M+H]⁺
¹H-NMR(500MHz,DMSO-d₆) : δ=0.79-0.95(6H,m),1.52(3H,d,J=7.1Hz),1.5 7-1.77(4H,m),1.8-2.01(1H,m),2.35(9H,s),2.80-2.95(2H,m),3.06-3. 21(2H,m) ,3.83(2H,s) ,4.31(2H,brs) ,4.40(2H,brs) ,4.51-4.61(1H,m), 5.71(1H,quint.,J=7.1Hz),7.48-7.63(8H,m),7.83(1H,d,J=7.8Hz),7.9 1-8.01(3H,m),8.10(1H,d,J=8.3Hz),8.56(1H,d,J=8.1Hz),8.71-8.79(1 H,m).8.93(1H,br).
N-[(S)-1-(naphthyl)ethyl]-5-[N-methoxycarbonylmethyl(isobutyl) amino]-2-(S)-{4-[N-(1H-imidazol-2-ylmethyl)aminomethyl]benzoyl }aminopentanoylamide [Compound No. 84]
MS(FAB,Pos.):m/z= 627 [M+H]⁺
N-[(1S)-4-[(2-hydroxyethyl)isobutylamino]-1-{[(S)-1-(naphthalen-1-yl)ethyl]carbamoyl}butyl]-4-{[(1-methyl-1H-imidazol-2-ylmethyl)amino]methyl}benzamide [Compound No. 85]
MS (FAB,Pos.) :m/z=613 [M+H]⁺
¹H-NMR (500MHz,DMSO-d₆) : δ=0.90(2H,d,J=6.6Hz) ,1.53(3H,d,J=6.8Hz), 1.57-1.79(4H,m) ,1.93-2.00(1H,m) ,2.35(9H,s) ,2.76-2.79(1H,m) ,2.9 0-2.94 (1H,m) ,3.04-3.14(4H,m) ,3.66-3.73 (2H,m) ,3.85 (3H,s) ,4.36 (2 H,brs) ,4.52 (2H,brs) ,4.55-4.62 (1H,m) ,5.73 (1H,quint. ,J=7.1Hz) ,7. 48-7.58(5H,m) ,7.61(2H,d,J=8.1Hz) ,7.68(1H,brs) ,7.84(1H,d,J=8.1H z) ,7.96(1H,d,J=7.8Hz) ,7.99(2H,dd,J=2.0,8.5Hz) ,8.11(1H,d,J=8.1H z) ,8.57(1H,d,J=8.1Hz) ,8.66(1H,brs) ,8.75(1H,t,J=8.1Hz) .
N-[(1S)-4-dipropylamino-1-{[(S)-1-(naphthalen-1-yl)ethyl]carbamoyl}butyl-4-{[(1-methyl-1H-imidazol-2-ylmethyl)amino]methyl} benzamide [Compound No. 86]
MS (FAB,Pos.) :m/z=597 [M+H]⁺
¹H-NMR(500MHz,DMSO-d₆) : δ=0.83(6H,dt,J=2.2, 7.3Hz) ,1.52(3H,d,J=6. 8Hz) ,1.55-1.70 (6H,m) ,1.74-1.76 (2H,m) ,2.33 (9H,s) ,2.86-2.92 (4H,m ) ,3.02-3.03 (2H,m) ,3.81 (3H,s) ,4.32 (2H,br) ,4.44 (2H,br) ,4.58 (1H,d d,J=8.5,14.6Hz) ,5.73(1H,quint. ,J=6.8Hz) ,7.48-7.66(8H,m) ,7.84(1 H,d,J=8.1Hz) ,7.95-7.99(3H,m) ,8.11(1H,d,J=8.1Hz) ,8.57(1H,d,J=7. 8Hz) ,8.74(1H,d,J=7.8Hz) ,9.01(1H,br) .
N-[(1S)-4-dipropylamino-1-{[(S)-1-(naphthalen-1-yl)ethyl]carbamoyl}butyl]-4-{[(1-methyl-1H-benzoimidazol-2-ylmethyl)amino]methyl}benzamide [Compound No. 87]
MS(FAB,Pos.):m/z=647[M+H]⁺
¹H-NMR(500MHz,DMSO-d₆):δ=0.84(6H,dt,J=2.2,7.3Hz),1.53(3H,d,J=6. 8Hz),1.56-1.66(6H,m),1.75(2H,m),2.32(9H,s),2.86-2.93(4H,m),3.0 3(2H,m),3.82(3H,s),4.44(2H,s),4.57-4.59(1H,m),4.62(2H,s),5.73(1H,quint.,J=6.8Hz),7.30(1H,dt,J=1.0,8.0Hz),7.35(1H,dt,J=0.7,7. 8Hz),7.48-7.58(4H,m),7.65-7.68(3H,m),7.71(1H,d,J=8.1Hz),7.84(1 H,d,J=7.8Hz),7.96(1H,d,J=7.6Hz),7.99(2H,d,J=8.3Hz),8.12(1H,d,J =8.1Hz) ,8.59(1H,d,J=8.1Hz) ,8.74(1H,d,J=7.8Hz) ,9.02(1H,br).
N-[(1S)-4-dipropylamino-1-{[(S)-1-(naphthalen-1-yl)ethyl]carbamoyl}butyl]-4-{[(1-methyl-1H-imidazol-2-ylmethyl)methylamino]methyl}benzamide [Compound No. 88]
MS(FAB,Pos.):m/z=597[M+H]⁺
¹H-NMR(500MHz,DMSO-d₆) : δ=0.83(6H,dt,J=2.4,7.3Hz) ,1.52(3H,d,J=6. 8Hz),1.55-1.69(6H,m) ,1.72-1.77 (2H,m) ,2.33(9H,s) ,2.86-2.92 (4H,m ) ,3.02-3.03(2H,m) ,3.67(7H,m) ,4.58(1H,dd,J=8.3,14.2Hz) ,5.73(1H, quint.,J=6.8Hz),7.48-7.58(6H,m),7.65(2H,s),7.84(1H,d,J=8.3Hz), 7.91-7.96(3H,m) ,8.11(1H,d,J=8.3Hz) ,8.50(1H,d,J=8.3Hz) ,8.71(1H, d,J=8.1Hz),8.97(1H,br).
N-[(1S)-4-[(2-hydroxyethyl)isobutylamino]-1-{[(S)-1-(naphthalen-1-yl)ethyl]carbamoyl}butyl]-4-{[(1-methyl-1H-imidazol-2-ylmethyl)methylamino]methyl}benzamide [Compound No. 89]
MS(FAB,Pos.):m/z=613[M+H]⁺
¹H-NMR(500MHz,DMSO-d₆) : δ=0.83-0.90(6H,m) ,1.52(3H,d,J=6.8Hz) ,1.6 1-1.73(4H,m),1.93-2.01(1H,m),2.32(9H,s),2.64-2.92(2H,m),2.94-3 .08(4H,m),3.68-3.69(9H,m),4.57(1H,m),5.73(1H,quint.,J=6.8Hz),7 .48-7.58(6H,m),7.65(2H,s),7.84(1H,d,J=8.1Hz),7.92-7.96(3H,m),8 .11(1H,d,J=8.1Hz),8.49-8.64(1H,m),8.72(1H,t,J=7.8Hz).
N-[(1S)-4-[(2-hydroxyethyl)isobutylamino]-1-{[(S)-1-(naphthalen-1-yl)ethyl]carbamoyl}butyl]-4-{[(1-methyl-1H-benzoimidazol-2-ylmethyl)amino]methyl}benzamide [Compound No. 90]
MS(FAB,Pos.):m/z=663[M+H]⁺.
¹H-NMR(500MHz,DMSO-d₆):δ=0.84-0.91(6H,m),1.52(3H,d,J=6.8Hz),1.5 9-1.79(4H,m),1.96-2.01(1H,m),2.31(9H,s),2.76-2.81(1H,m),2.91-2 .95(1H,m),3.04-3.12(4H,m),3.67-3.70(1H,m),3.81(3H,s),4.43(2H,s ) ,4.57-4.61(3H,m) ,5.71-5.75(1H,m) ,7.27-7.36(2H,m) ,7.48-7.56(4H ,m) ,7.64-7.67 (3H,m) ,7.70 (1H,d,J=7.8Hz) ,7.84 (1H,d,J=8.3Hz) ,7.95 (1H,d,J=7.8Hz) ,7.99(1H,d,J=8.3Hz) ,7.99(1H,d,J=8.3Hz) ,8.11(1H,d ,J=8.5Hz) ,8.57(1H,d,J=7.5Hz) ,8.66(1H,brs) ,8.73-8.76(1H,m) .
N-[(1S)-4-[(2-hydroxyethyl)isobutylamino]-1-{[(S)-1-(naphthalen-1-yl)ethyl]carbamoyl}butyl]-4-{[(1-methyl-1H-imidazol-2-ylmethyl)methylamino]methyl}benzamide [Compound No. 91]
MS(FAB,Pos.):m/z=627[M+H]⁺
¹H-NMR(500MHz,DMSO-d₆) : δ=0.83-0.90(6H,m),1.15-2.25(1H,m),1.52(3 H,d,J=6.8Hz),1.54-1.75(4H,m),1.91-2.02(1H,m),2.07-2.30(3H,m),2 .50(9H,s),2.75-2.82(1H,m),2.90-2.97(1H,m),3.05-3.13(4H,m),3.67 -4.05(7H,m) ,4.56-4.58(1H,m) ,5.71-5.76(1H,m) ,7.47-7.68(8H,m) ,7. 84(1H,d,J=8.3Hz),7.91-7.97(3H,m),8.11(1H,d,J=7.8Hz),8.49(1H,d, J=5.8Hz),8.65(1H,brs),8.72(1H,t,J=7.8Hz).
N-[(1S)-4-dipropylamino-1-{[(S)-1-(naphthalen-1-yl)ethyl]carbamoyl}butyl]-4-{[(1-methyl-1H-imidazol-2-ylmethyl)methylamino]methyl}benzamide [Compound No. 92]
MS (FAB,Pos.) :m/z=611 [M+H]⁺
¹H-NMR (500MHz,DMSO-d₆) :δ=0.83 (6H,dt,J=2.4,7.3Hz) ,1.52 (3H,d,J=6. 8Hz) ,1.55-1.65 (6H,m) ,1.72-1.75 (2H,m) ,2.32 (9H,s) ,2.85-2.92 (4H,m ) ,3.02-3.03 (2H,m) ,3.81 (3H,s) ,3.93 (7H,br) ,4.57 (1H,dd,J=7.1,14.9 Hz) ,5.73 (1H,quint.,J=6.8Hz) ,7.48-7.66 (8H,m) ,7.84 (1H,d,J=8.1Hz) ,7.91-7.96 (3H,m) ,8.11 (1H,d,J=8.1Hz).
N-[(1S)-4-[(2-hydroxyethyl)isobutylamino]-1-{[(S)-1-(naphthalen-1-yl)ethyl]carbamoyl}butyl]-4-{[(furan-2-ylmethyl)amino]methyl}benzamide [Compound No. 93]
MS(FAB,Pos.):m/z=599[M+H]⁺
¹H-NMR(500MHz,DMSO-d₆) : δ=0.85(3H,d,J=6.6Hz) ,0.90(3H,d,J=6.6H_{z}), 1.53(3H,d,J=7.1Hz),1.58-1.63(1H,m),1.63-1.78(3H,m),1.93-2.01(1 H,m),2.32(6H,s),2.76-2.80(1H,m),2.91-2.94(1H,m),3.17(4H,brs),3 .68(2H,brs),4.23(2H,t,J=5.9Hz),4.27(2H,t,J=4.6Hz),4.53-4.60(1H ,m),5.73(1H,quint.,J=7.3Hz),6.56(1H,dd,J=2.0,3.4Hz),6.66(1H,d, J=3.4Hz),7.48-7.59(6H,m),7.82(1H,dd,J=0.7,1.7Hz),7.84(1H,d,J=8 .5Hz),7.95(1H,d,J=2.0Hz),7.97(2H,dd,J=1.7,8.3Hz),8.11(1H,d,J=7 .8Hz) ,8.57(1H,d,J=7.6Hz) ,8.65(1H,brs) ,8.74(1H,t,J=7.8Hz) .
N-[(1S)-4-isobutylmethanesulfonylamino-1-{[(S)-1-(naphthalen-1 -yl)ethyl]carbamoyl}butyl]-4-{[(1H-imidazol-2-ylmethyl)amino]methyl}benzamide [Compound No. 94]
MS(FAB,Pos.):m/z=633[M+H]⁺
¹H-NMR(500MHz,DMSO-d₆) : δ=0.76(3H,d,J=6.5Hz),0.76(3H,d,J=6.5Hz), 1.51(3H,d,J=6.8Hz),1.50-1.79(5H,m),2.32(6H,s),2.75-2.77(2H,m), 2.81(3H,s) ,3.03-3.07(2H,m) ,4.30(2H,s) ,4.37(2H,s) ,4.54-4.59(1H, m) ,5.70(1H,dt,J=6.6,7.3Hz) ,7.47-7.60(8H,m) ,7.83(1H,d,J=8.0Hz), 7.95(3H,m) ,8.10(1H,d,J=7.8Hz) ,8.48(1H,d,J=8.0Hz) ,8.69(1H,d,J=7.8Hz) .
N-[(1S)-4-dipropylamino-1-{[(S)-1-(naphthalen-1-yl)ethyl]carbamoyl}butyl]-4-{[(1-ethyl-1H-imidazol-2-ylmethyl)amino]methyl}benzamide [Compound No. 95]
MS (FAB,Pos.) :m/z=611 [M+H]⁺
¹H-NMR(500MHz,DMSO-d₆) : δ=0.83 (6H,t,J=7.2Hz) ,1.39 (3H,t,J=7.2Hz), 1.49-1.80(8H,m) ,1.52(3H,d,J=7.1Hz) ,2.34(9H,s) ,2.83-2.97(4H,br) ,3.03(2H,brs),4.19(2H,brs)4.33(2H,brs),4.50(2H,brs),4.59(1H,dd ,J=8.4,14.4Hz),5.73(1H,t,J=7.1Hz),7.48-7.60(7H,m),7.73(1H,brs) 7.84(1H, d, J=8.1Hz) ,7.95-7.99(3H,m) ,8.11(1H,d,J=8.1Hz) ,8.58(1H, d,J=7.9Hz), 8.74(1H,d,J=7.9Hz), 9.01(1H,brs).
N-((1S)-4-dipropylamino-1-{[(S)-1-(naphthalen-1-yl)ethyl]carbamoyl}butyl]-4-{[(1-propyl-1H-imidazol-2-ylmethyl)amino]methyl} benzamide [Compound No. 96]
MS(FAB,Pos.):m/z=625[M+H]⁺
¹H-NMR(500MHz,DMSO-d₆) : δ=0.84(6H,t,J=7.3Hz) ,0.89(3H,t,J=7.3Hz), 1.49-1.71(6H,m),1.53(3H,d,J=7.0Hz),1.71-1.80(4H,m),2.35(9H,s), 2.83-2.97(4H,br),3.03(2H,brs),4.09-4.17(2H,brs),4.34(2H,brs),4 .52(2H,brs),4.58(1H,dd,J=8.2,14.3Hz),5.73(1H,t,J=7.0Hz),7.48-7 .62(7H,m),7.72(1H,brs),7.84(1H,d,J=8.1Hz),7.94-7.99(3H,m),8.12 (1H,d,J=8.1Hz),8.58(1H,d,J=8.1Hz),8.74(1H,d,J=7.8Hz),9.03(1H,b rs).
5-{[(1-methyl-1H-imidazol-2-ylmethyl)amino]methyl}thiophene-2-carboxylic-[(1S)-4-[(3-methylpyridin-2-ylmethyl)amino]-1-{[(S)-1-(naphthalen-1-yl)ethyl]carbamoyl}butyl]amide[Compound No.97]
MS (FAB,Pos.) :m/z=624 [M+H]⁺
¹H-NMR(500MHz,DMSO-d₆+D₂O) : δ=1.52 (3H,d,J=6.8Hz) ,1.75-1.93 (4H,m) ,2.25(3H,s) ,2.40(12H,s) ,2.97-3.08(2H,m) ,3.86(3H,s) ,4.27(2H,s), 4.40(4H,s) ,4.51-4.56(1H,m) ,5.70(1H,q,J=6.8Hz) ,7.28(1H,d,J=3.7H z) ,7.34-7.37(1H,m) ,7.50-7.59(4H,m) ,7.61(1H,s) ,7.66(1H,s),7.69(1H,d,J=7.5Hz) ,7.82-7.86(2H,m) ,7.95(1H,d,J=8.1Hz) ,8.09(1H,d,J=8 .5Hz) ,8.22-8.30(2H,m) ,8.43(1H,d,J=3.9Hz) .
5-{[(1-methyl-1H-imidazol-2-ylmethyl)amino]methyl}thiophene-2-carboxylic-[(1S)-4-[(2-methyoxybenzylamino)-1-{[(S)-1-(naphthalen-1-yl)ethyl]carbamoyl}butyl]amide [Compound No. 98]
MS(FAB,Pos.):m/z=639[M+H]⁺
¹H-NMR(500MHz,DMSO-d₆+D₂O) : δ=1.52(3H,d,J=6.8Hz) ,1.59-1.80(4H,m) ,2.39(9H,s),2.87-2.94(2H,m),3.79(3H,s),3.83(3H,s),4.01(2H,s),4 .37(4H,br),4.49-4.54(1H,m),5.70(1H,q,J=6.8Hz),6.98(1H,dt,J=1.0 ,7.3Hz),7.08(1H,d,J=7.8Hz),7.24(1H,d,J=3.9Hz),7.34(1H,dd,J=1.7 ,7.6Hz),7.43(1H,dt,J=1.7,7.6Hz),7.47-7.59(4H,m),7.61(1H,d,J=1. 7Hz) ,7.80-7.84(2H,m) ,7.94(1H,d,J=7.8Hz) ,8.09(1H,d,J=8.3Hz) .
5-{[(1-methyl-1H-imidazol-2-ylmethyl)amino]methyl}thiophene-2-carboxylic-[(1S)-4-(1-ethylpropylamino)-1-{[(S)-1-(naphthalen-1-yl)ethyl]carbamoyl}butyl]amide [Compound No. 99]
MS (FAB,Pos.) :m/z=589 [M+H]⁺
¹H-NMR (500MHz, DMSO-d₆+D₂O) : δ=0.84 (3H,t,J=7.5Hz) ,0.85 (3H,t,J=7.5 Hz) ,1.51(3H,d,J=7.1Hz) ,1.53-1.65(4H,m) ,1.65-1.81(4H,m) ,2.40(9H ,s) ,2.83-2.93(3H,m) ,3.83(3H,s) ,4.37(4H,br) ,4.50-4.57(1H,m) ,5.7 0(1H,q,J=7.1Hz) ,7.25(1H,d,J=3.7Hz) ,7.45-7.58(5H,m) ,7.62(1H,d,J =1.7Hz) ,7.81-7.84(2H,m) ,7.95(1H,d,J=7.8Hz) ,8.09(1H,d,J=8.3Hz).
N-{(1S)-4-dipropylamino-1-[2-(1H-indol-3-yl)ethylcarbamoyl]butyl}-4-{[(1H-imidazol-2-ylmethyl)amino]methyl}benzamide [Compound No. 100]
MS(FAB,Pos.):m/z=572[M+H]⁺
¹H-NMR(500MHz,DMSO-d₆):δ=0.88(6H,dt,J=2.0,7.3Hz),1.58-1.74(8H,m ),2.32(9H,s),2.77(2H,t,J=1.2Hz),2.82-3.05(6H,m),3.36-3.39(2H,m ),4.28(4H,br),4.47-4.49(1H,m),6.97(1H,t,J=7.8Hz),7.06(1H,t,J=7 .8Hz),7.16(1H,s),7.34(1H,d,J=8.1Hz),7.54(1H,d,J=7.8Hz),7.59(1H ,d,J=7.9Hz),7.97(2H,d,J=8.5Hz),8.21(1H,t,J=5.5Hz),8.58(1H,d,J= 8.1Hz),10.8(1H,br). 4-{[(1H-imidazol-2-ylmethyl)amino]methyl}naphthalen-1-carboxylic-{(1S)-4-dipropylamino-1-[2-(1H-indol-3-yl)ethylcarbamoyl]butyl}amide [Compound No. 101]
MS(FAB,Pos.):m/z=622[M+H]⁺
¹H-NMR(500MHz,DM50-d₆) : δ=0. 89 (6H, t, J=7. 3Hz) , 1.59-1.74 (8H,m) ,2.3 4(9H,s) ,2.89(2H,t,J=7.6Hz) ,2.98-3.13(6H,m) ,3.30-3.48(2H,m) ,4.5 4 (2H,br) ,4.55 (1H,m) ,4.78(2H,br) ,7.00(1H,dt,J=1.0,8.0Hz) ,7.08(1 H,dt,J=1.0,8.0Hz),7.21(1H,s),7.36(1H,d,J=8.1Hz),7.54-7.73(6H,m ),8.25-8.31(2H,m),8.76(1H,d,J=7.6Hz),9.05(1H,br),10.86(1H,brs)
N-{(1S)-4-dipropylamino-1-[2-(1H-indol-3-yl)ethylcarbamoyl]butyl)-4-{[(1-methyl-1H-imidazol-2-ylmethyl)amino]methyl}benzamide [Compound No. 102]
MS (FAB,Pos.) :m/z=586 [M+H]⁺
¹H-NMR (500MHZ, DMSO-d₆) : δ=0.88(6H,dt,J=1.7,7.3Hz) ,1.57-1.76(8H,m ) ,2.33(9H,s) ,2.83(2H,t,J=7.6Hz) ,2.97-2.98(4H,m) ,3.07(2H,br) ,3. 30-3.40(2H,m) ,3.81(3H,s) ,4.33(4H,br) ,4.46-4.51(1H,m) ,6.97(1H,d t,J=1.0,7.1Hz) ,7.06(1H,dt,J=1.2,8.0Hz) ,7.16(1H,d,J=2.0Hz) ,7.34 (1H,d,J=8.1Hz) ,7.54(2H,d,J=7.6Hz) ,7.60(2H,d,J=8.1Hz) ,7.99(3H,d ,J=8.3Hz) ,8.21(1H,t,J=5.9Hz) ,8.59(1H,d,J=7.6Hz) ,9.01(1H,br) ,10 .84 (1H,br) .
5-{[(1-methyl-1H-imidazol-2-ylmethyl)amino]methyl}thiophene-2-carboxylic-{(1S)-4-dipropylamino-1-[2-(1H-indol-3-yl)ethylcarbamoyl]butyl}amide [Compound No. 103]
MS(FAB,Pos.):m/z=586[M+H]⁺
¹H-NMR(500MHz,DMSO-d₆):δ=0.88(6H,dt,J=2.4,7.3Hz),1.56-1.72(8H,m ),2.33(9H,s),2.83(2H,t,J=7.3Hz),2.96-2.97(4H,br),3.02-3.08(2H, br) ,3.31-3.39(2H,m) , 3.80(3H,s) ,4.31(4H,br) ,4.40-4.42(1H,m) ,6.9 7(1H,dt,J=0.7,7.8Hz),7.06(1H,dt,J=1.0,8.0Hz),7.16(1H,s),7.24(1 H,br) ,7.34(1H,d,J=8.3Hz) ,7.54(1H,d,J=7.8Hz) ,7.61-7.66(1H,br) ,7. 86(1H,d,J=3.2Hz) ,8.23(1H,t,J=5.9Hz) ,8.65(1H,br) ,9.00(1H,br) ,1 0.84(1H,br).
N-{(1S)-4-dipropylamino-1-[2-(1H-indol-3-yl)ethylcarbamoyl]butyl}-4-{[(pyridin-2-ylmethyl)amino]methyl}benzamide [Compound No. 104]
MS (FAB,Pos.) :m/z=583 [M+H]⁺
¹H-NMR(500MHz,DMSO-d₆) : δ=0. 88 (6H,dt,J=1.7,7.3Hz) ,1.57-1.76 (8H,m ) ,2.33(9H,s) ,2.84(2H,t,J=7.5Hz) ,2.97-3.07(6H,m) ,3.33-3.46(2H,m ) ,4.33(4H,s) ,4.46-4.51(1H,m) ,6.96(1H,dt,J=1.0,7.8Hz) ,7.06(1H,d t,J=1.0,7.9Hz) ,7.17(1H,s) ,7.34(1H,d,J=7.3Hz) ,7.45-7.57 (4H,m) ,7 .63(2H,d,J=8.3Hz) ,7.88-7.93(1H,m) ,7.99(2H,d,J=8.3Hz) ,8.20(1H,t ,J=5.4Hz) ,8.60(1H,d,J=8.1Hz) ,8.66-8.68(2H,m) ,9.02(1H,br) ,9.62(1H,br) ,10.84(1H,brs).
4-{[(pyridin-2-ylmethyl)amino]methyl}naphthalen-1-carboxylic-{ (1S)-4-dipropylamino-1-[2-(1H-indol-3-yl)ethylcarbamoyl]butyl} amide [Compound No. 105]
MS(FAB,Pos.):m/z=633[M+H]⁺
¹H-NMR(500MHz,DMSO-d₆): δ=0.98(6H,t,J=7.1Hz) ,1.59-1.67(4H,m) ,1.6 8-1.72(4H,m),2.31(9H,s),2.89(2H,t,J=7.6Hz),2.98-3.08(6H,m),3.3 8-3.48(2H,m) ,4.50(2H,brs) ,4.55-4.56(1H,m) ,4.80(2H,brs) ,6.99(1H, dt,J=0.7,7.8Hz) ,7.08(1H,dt,J=J.1.0,8.0Hz) ,7.21(1H,s) ,7.35(1H,d, J=8.3Hz),7.47-7.50(1H,m),7.54-7.58(2H,m),7.65(1H,dt,J=1.2,8.0H z),7.69-7.74(2H,m),7.77(1H,d,J=7.3Hz),7.93(1H,dt,J=1.9,7.8Hz), 8.30(2H,t,J=9.5Hz),8.71-8.72(1H,m),8.77(1H,d,J=7.8Hz),9.04(1H, br) ,9.61(1H,br) ,9.66(1H,br) ,10.86(1H,brs) .
N-{(1S)-4-dipropylamino-1-[2-(1H-indol-3-yl)ethylcarbamoyl]butyl}-6-([(pyridin-2-ylmethyl)amino]methyl}nicotinamide [Compound No. 106]
MS(FAB,Pos.):m/z=584[M+H]⁺
¹H-NMR(500MHz,DMSO-d₆) : δ=0.88(6H,dt,J=1.4,7.3H2) ,1.57-1.65(4H,m ) ,1.68-1.76(4H,m) ,2.32(12H,s) ,2.84(2H,t,J=7.3Hz) ,2.97-3.06(6H, m) ,3.35-3.40(1H,m) ,4.44(2H,brs) ,4.48-4.49(1H,m) ,4.52(2H,brs) ,6 .95-6.83(1H,dt,J=1.0,7.1Hz),7.06(1H,dt,J=1.2,8.0Hz),7.16(1H,d, J=2.4Hz),7.34(1H,d,J=8.3Hz),7.45-7.48(1H,m),7.53(2H,t,J=8.5Hz) ,7.64(1H,d,J=8.0Hz),7.90-7.93(1H,m),8.25(1H,t,J=5.6Hz),8.34-8. 37(1H,m),8.66-8.68(1H,m),8.87(1H,d,J=7.8Hz),9.11(1H,d,J=2.2Hz) ,9.67(2H,br),10.84(1H,brs).
8-{[(pyridin-2-ylmethyl)amino]methyl}isoquinoline-5-carboxylic -{(1S)-4-dipropylamino-1-[2-(1H-indol-3-yl)ethylcarbamoyl]butyl}amide [Compound No. 107]
MS(FAB,Pos.):m/z=634[M+H]⁺
¹H-NMR(500MHz,DMSO-d₆) : δ=0.89(6H,t,J=7.3Hz) ,1.59-1.72(8H,m) ,2.3 2(12H,s),2.88(2H,t,J=7.3Hz),2.98-3.07(6H,m),3.39-3.48(2H,m),4. 43(2H,brs),4.51-4.54(1H,m),4.87(2H,brs),6.99(1H,dt,J=1.2,7.1Hz ),7.08(1H,dt,J=1.0,7.1Hz),7.20(1H,d,J=2.2Hz),7.34-7.36(1H,m),7 .43-7.47(1H,m),7.53(1H,d,J=7.8Hz),7.57(1H,d,J=8.1Hz),7.71-7.74 (1H,m),7.85(1H,d,J=7.3Hz),7.89-7.93(1H,m),7.97(1H,d,J=7.3Hz),8 .27-8.30(1H,m),8.66-8.67(1H,m),8.78(1H,dd,J=1.7,8.5Hz),8.88(1H ,d,J=7.8Hz),9.05(2H,dd,J=1.7,4.1Hz),9.63(2H,br),10.86(1H,brs).
N-[(1S)-4-dipropylamino-1-{[(S)-1-(naphthalen-1-yl)ethyl]carbamoyl}butyl]-4-({[1-{2-hydroxyethyl)-1H-imidazol-2-ylmethyl]ami no}methyl)benzamide [Compound No. 108]
MS (FAB,Pos.) :m/z=627 [M+H]⁺
¹H-NMR(500MHz,DMSO-d₆) : δ=0.84(6H,dt,J=7.3,2.4Hz) ,1.49-1.80(8H,m ) ,1.52 (3H,d,J=6.8Hz) ,1.71-1.80 (4H,m) ,2.35 (12H,s) ,2.80-2.96 (4H, m) ,3.03 (2H,brs) ,3.73(2H,t,J=4.8Hz) ,4.36(4H,s) ,4.58 (1H,dd,J=15. 1,8.3Hz) ,4.63 (2H,s) ,5.73(1H,t,J=6.8Hz) ,7.48-7.66 (6H,m) ,7.69 (1H ,s) ,7.76 (1H,s) ,7.84 (1H,d,J=8.3Hz) ,7.94-7.99 (3H,m) ,8.12(1H,d,J= 8.1Hz) ,8.59(1H,d,J=8.1Hz) ,8.75(1H,d,J=7.8Hz) ,9.12(1H,s) .
4-{[(1-methyl-1H-imidazol-2-ylmethyl)amino]methyl}naphthalen-1 -carboxylic-[(1S)-4-[(3-methylpyridin-2-ylmethyl)amino]-1-{[(S)-1-(naphthalen-1-yl)ethyl]carbamoyl}butyl]amide [Compound No. 109]
MS (FAB,Pos.) :m/z=668 [M+H]⁺
¹H-NMR (500MHz,DMSO-d₆+D₂O) : δ=1.57(3H,d,J=6.8Hz) ,1.73-1.90(4H,m) ,2.25(3H,s) ,2.39(12H,s) ,3.04-3.13(2H,m) ,3.89(3H,s) ,4.30(2H,s), 4.61-4.65(1H,m) ,4.69(2H,s) ,4.83(2H,s) ,5.76(1H,q,J=6.8Hz) ,7.35-7.38(1H,m) ,7.50-7.62 (4H,m) ,7.63-7.75 (7H,m) ,7.86 (1H,d,J=8.1Hz), 7.97(1H,d,J=8.1Hz) ,8.14(1H,d,J=8.3Hz) ,8.22-8.30(2H,m) ,8.44(1H, d,J=3.4Hz).
4-{[(1-methyl-1H-imidazol-2-ylmethyl)amino]methyl}naphthalen-1 -carboxylic-[(1S)-4-(2-methoxybenzylamino)-1-{[(S)-1-(naphthalen-1-yl)ethyl]carbamoyl}butyl]amide [Compound No. 110]
MS(FAB,Pos.):m/z=683[M+H]⁺
¹H-NMR(500MHz,DMSO-d₆+D₂O) : δ=1.57(3H,d,J=6.8Hz) ,1.70-1.86(4H,m) ,2.40(9H,s),2.90-3.00(2H,m),3.80(3H,s),3.86(3H,s),4.04(2H,s),4 .55-4.65(1H,m),4.64(2H,s),4.77(1H,s),5.77(1H,q,J=6.8Hz),7.00(1 H,dt,J=1.0,7.3Hz),7.09(1H,d,J=7.1Hz),7.35(1H,dd,J=1.7,7.6Hz),7 .44(1H,dt,J=1.0,7.6Hz),7.45-7.68(8H,m),7.69-7.74(2H,m),7.85(1H ,d,J=8.3Hz),7.96(1H,d,J=8.1Hz),8.14(1H,d,J=8.3Hz),8.24-8.27(2H ,m).
4-{[(1-methyl-1H-imidazol-2-ylmethyl)amino]methyl}naphthalen-1 -carboxylic-[(1S)-4-(1-ethylpropylamino)-1-{[(S)-1-(naphthalen -1-yl)ethyl]carbamoyl}butyl]amide [Compound No. 111]
MS (FAB,Pos.) :m/z=633 [M+H]⁺
¹H-NMR(500MHz, DMSO-d₆+D₂O) : δ=0.86(6H,t,J=7.3Hz) ,1.53-1.67(4H,m) ,1.57(3H,d,J=6.8Hz) ,1.67-1.79(3H,m) ,1.79-1.88(1H,m) ,2.39(9H,s) ,2.86-2.98(3H,m) ,3.84(3H,s) ,4.58(2H,s) ,4.60-4.67(1H,m) ,4.66(2H ,s) ,5.78(1H,q,J=6.8Hz) ,7.45-7.67(8H,m) ,7.68-7.73(2H,m) ,7.86(1H ,d,J=8.3Hz) ,7.97(1H,d,J=7.8Hz) ,8.14(1H,d,J=8.5Hz) ,8.24-8.27(2H ,m) .
4-{[bis(pyridin-2-ylmethyl)amino]methyl}-N-[(1S)-4-[2-hydroxyethyl)isobutylamino]-1-{[(S)-1-naphthalen-1-yl)ethyl]carbamoyl} butyl]benzamide [Compound No. 112]
MS(FAB,Pos.):m/z=701[M+H]⁺
¹H-NMR(500MHz,DMSO-d₆) : δ=0.88(6H,d,J=6.6Hz) ,1.52(3H,d,J=6.8Hz), 1.56-1.78(2H,m),1.92-2.02(1H,m),2.09(3H,s),2.35(12H,s),2.75-2. 84(1H,m),2.88-2.95(1H,m),3.02-3.11(4H,m),3.65-3.73(2H,m),4.29(4H,s),5.72(1H,quint.,J=6.6Hz),7.48-7.63(8H,m),7.73(2H,d,J=7.6H z),7.84(1H,d,J=8.1Hz),7.88-7.90(2H,m),7.95(1H,dd,J=2.0,7.6Hz), 8.11(3H,d,J-8.1Hz),8.50(1H,d,J=8.3Hz),8.65(1H,m),8.71(1H,d,J=7 .8Hz),8.74(2H,d,J=6.3Hz).
N-[(1S)-4-dipropylamino-1-(3-isopropoxypropylcarbamoyl)butyl]-4-{[(1-methyl-1H-imidazol-2-ylmethyl)amino]methyl}benzamide [Compound No. 113]
MS (FAB,Pos.) :m/z=543 [M+H]⁺
¹H-NMR(500MHz,DMSO-d₆) : δ=0.90(6H,t,J=7.3Hz) ,1.07(6H,dd,J=1.5,6. 1Hz) ,1.60-1.85(10H,m) ,2.36(12H,s) ,3.00-3.17(8H,m) ,3.37(2H,t,J= 6.3Hz) ,3.50(1H,quint. ,J=6.1Hz) ,4.35(2H,brs) ,4.43-4.47(1H,m) ,4. 48 (2H,brs) 6.08 (2H,br) ,7.57-7.65(1H,br) ,7.62 (2H,d,J=8.3Hz) , 7.9 9(2H,d,J=8.3Hz) ,8.05(1H,t,J=5.6Hz) ,8.61(1H,d,J=8.1Hz) ,9.06(1H, br) ,9.18(1H,br) ,9.32(1H,br) .
4-{[(1-methyl-1H-imidazol-2-ylmethyl)amino]methyl}naphthalen-1 -carboxylic-[(1S)-4-dipropylamino-1-(3-isopropoxypropylcarbamoyl)butyl]amide [Compound No. 114]
MS(FAB,Pos.):m/z=593[M+H]⁺
¹H-NMR(500MHz,DMSO-d₆) : δ=0.89(6H,dt,J=2.0,7.1Hz) ,1.07(6H,d,J=6. 1Hz),1.60-1.72(10H,m),2.32(12H,s),2.98-3.05(8H,m),3.40(2H,t,J= 6.6Hz),3.51(1H,quint.,J=6.1Hz),3.80(3H,brs),4.50-4.51(1H,m),4. 53(2H,br),6.01-6.04(2H,br),7.62-7.71(4H,m),8.10(1H,t,J=5.4Hz), 8.27(2H,t,J=8.1Hz),8.74(1H,d,J=7.8Hz),9.04(1H,br),9.28(1H,br).
N-[(1S)-4-dipropylamino-1-(3-isopropoxypropylcarbamoyl)butyl]-4-{[(1H-imidazol-2-ylmethyl)amino]methyl}benzamide [Compound No. 115]
MS (FAB,Pos.) :m/z=529 [M+H]⁺
¹H-NMR(500MHz,DMSO-d₆) : δ=0.88(6H,t,J=7.1Hz) ,1.05(6H,dd,J=1.5,6. 1Hz) ,1.59-1.72(10H,m) ,2.32(12H,s) ,2.98-3.15(8H,m) ,3.35(2H,t,J= 6.3Hz) ,3.48(1H,quint. ,J=6.1Hz) ,4.26(4H,br) ,4.44(1H,dd,J=9.0,14 .1Hz) ,6.00-6.04(4H,br) ,7.46(1H,br) ,7.57(3H,d,J=8.3Hz) ,7.96(3H, d,J=8.1Hz) ,8.03(1H,t,J=6.3Hz) ,8.57(1H,d,J=7.3Hz) ,9.00(1H,br) ,9 .27(2H,br).
5-{[(1H-imidazol-2-ylmethyl)amino]methyl}thiophene-2-carboxylic-{(1S)-4-dipropylamino-1-[2-(1H-indol-3-yl)ethylcarbamoyl]butyl}amide [Compound No. 116]
MS (FAB,Pos.) :m/z=578 [M+H]⁺
5-{[(pyridin-2-ylmethyl)amino]methyl}thiophene-2-carboxylic-{(1S)-4-dipropylamino-1-[2-(1H-indol-3-yl)ethylcarbamoyl]butyl}amide [Compound No. 117]
MS (FAB,Pos.) :m/z=589 (M+H]⁺
¹H-NMR(500MHz,DMSO-d₆) : δ=0.88(6H,dt,J=2.2,7.3Hz) ,1.58-1.72(8H,m ) ,2.32 (9H,s) ,2.83 (2H,t,J=7.6Hz) ,2.94-3.06 (6H,m) ,3.34-3.39 (2H,m ) ,4.33(2H,brs) ,4.43(1H,dd,J=8.5,14.1Hz) ,4.51(2H,brs) ,6.97(1H,d t,J=0.7,7.8Hz) ,7.06(1H,dt,J=1.0,8.2Hz) ,7.16(1H,d,J=2.2Hz) ,7.33 -7.34(2H,m) ,7.45-7.48(1H,m) ,7.50(1H,d,J=8.1Hz) ,7.54(1H,d,J=7.8 Hz) ,7.89-7.92(2H,m) ,8.24(1H,t,J=5.4Hz) ,8.66(1H,d,J=4.2Hz) ,8.71 (1H,d,J=8.1Hz) ,9.02(1H,br) ,9.61(2H,br) ,10.83(1H,brs) .
N-{(1S)-4-dipropylamino-1-[2-(1H-indol-3-yl)ethylcarbamoyl]butyl}-4-{[(pyridin-2-ylmethyl)amino]methyl}benzamide [Compound No. 118]
MS(FAB,Pos.):m/z=583[M+H]⁺
¹H-NMR(500MHz,DMSO-d₆) : δ=0.87(6H,dt,J=1.7,7.3Hz), 1.58-1.82(8H,m ),2.33(9H,s),2.84(2H,t,J=7.8Hz),2.97-3.06(6H,m),3.36(2H,m),4.3 3-4.34(4H,br),4.49(1H,dd,J=8.5,14.4Hz),6.97(1H,dt,J=0.7,7.8Hz) ,7.06(1H,dt,J=1.0,8.0Hz),7.16(1H,d,J=2.2Hz),7.34(1H,d,J=8.1Hz) ,7.44-7.46(1H,m),7.50(1H,d,J=7.8Hz),7.54(1H,d,J=8.3Hz),7.57(1H ,t,J=7.8Hz),7.70(1H,d,J=7.8Hz),7.90(1H,dt,J=1.7,7.8Hz),7.99(1H ,d,J=7.8Hz),8.08(1H,s),8.23(1H,t,J=5.6Hz),8.54(1H,d,J=8.3Hz),8 .65-8.66(1H,m), 9.01(1H,br) ,9.53(2H,br) ,10.84(1H,brs).
4-{[(1-methyl-1H-imidazol-2-ylmethyl)amino]methyl}naphthalen-1 -carboxylic-{(1S)-4-dipropylamino-1-[2-(1H-indol-3-yl)ethylcarbamoyl]butyl}amide [Compound No. 119]
MS(FAB,Pos.):m/z=636[M+H]⁺
¹H-NMR(500MHz,DMSO-d₆) : δ=0.89(6H,t,J=7.3Hz) ,1.59-1.71(8H,m) ,2.3 3(9H,s) ,2.89(2H,t,J=7.6Hz) ,2.98-3.00(4H,m) ,3.08(2H,br) ,3.39-3. 47(2H,m),3.83(3H,s),4.54-4.56(3H,m),4.76(2H,br),6.99(1H,dt,J=0 .7,7.8Hz),7.08(1H,dt,J=1.2,8.0Hz),7.20(1H,d,J=1.9Hz),7.35(1H,d ,J=8.1Hz),7.57-7.71(9H,m),8.25-8.31(3H,m),8.75(1H,d,J=7.8Hz),9 .05(1H,br),10.86(1H,brs).
N-[(1S)-4-(4,5-dihydro-1H-imidazol-2-ylsulfanyl)-1-{[(S)-1-(naphthalen-1-yl)ethyl]carbamoyl}butyl]-4-{[(pyridin-2-ylmethyl)amino]methyl}benzamide [Compound No. 120]
MS(FAB,Pos.):m/z=595[M+H]⁺
¹H-NMR(500MHz,DMSO-d₆): δ=1.52(3H,d,J=6.8Hz) ,1.59-1.69(2H,m) ,1.7 5-1.87(2H,m),2.32(9H,s),3.16(2H,t,J=7.3Hz),3.82(4H,brs),4.32(2 H,brs),4.33(2H,brs),4.52(1H,dd,J=8.1,13.9Hz),5.72(1H,quint.,J= 6.8Hz),7.45-7.57(6H,m),7.62(2H,d,J=8.5Hz),7.84(1H,d,J=7.8Hz),7 .89-7.97(4H,m),8.11(1H,d,J=8.0Hz),8.53(1H,d,J=7.8Hz),8.67(1H,d dd,J=1.0,1.7,3.9Hz),8.73(1H,d,J=8.1Hz),9.55(2H,brs),10.04(2H,b rs) .
N-[(1S)-4-(1H-imidazol-2-ylsulfanyl)-1-{[(S)-1-(naphthalen-1-yl)ethyl]carbamoyl]butyl]-4-{[(1H-imidazol-2-ylmethyl)amino]methyl}benzamide [Compound No. 121]
MS (FAB,Pos.) :m/z=582 [M+H]⁺
¹H-NMR(500MHz, DMSO-d₆+D₂O) : δ=1.50(3H, d,J=7.1Hz) , ,1.48-1.62 (2H,m) ,1.72-1.88 (2H,m) ,2.36 (9H, s) ,3.13-3.24(2H,m) ,4.31(2H,s) ,4.40(2H ,s) ,4.50-4.59(1H,m) ,5.70(1H,quint. ,J=7.1Hz) ,7.43-7.62(8H,m) ,7. 70 (2H,s) ,7.83 (1H,d,J=8.1Hz) ,7.88-7.97 (3H,m) ,8.09 (1H,d,J=7.0Hz) ,8.49(1H,d,J=7.9Hz) ,8.70(1H,d,J=7.8Hz) .
N-[(1S)-4-dipropylamino-1-(isopropylcarbamoyl)butyl]-4-{[(1-methyl-1H-imidazol-2-ylmethyl)amino]methyl}benzamide [Compound No. 122]
MS (FAB,Pos.) :m/z=485 [M+H]⁺
¹H-NMR(500MHz,DMSO-d₆) : δ=0. 87 (3H, d, J=7.3Hz) ,0.89 (3H,d,J=7.3Hz), 1.06 (3H,d,J=6.6Hz) ,1.08 (3H,d,J=6.6Hz) ,1.59-1.82 (8H,m) ,2.32 (9H, s) ,2.90-3.00(4H,m) ,3.03-3.12(2H,m) ,3.78(3H,s) ,3.78-3.90(1H,m), 4.22-4.48 (5H,m) 7. 59 (2H,d,J=7.8Hz) , 7.94-7.98 (2H,m) 8.51 (1H,.d,J =7.8Hz) ,9.04(1H,s) .
N-[(1S)-4-dipropylamino-1-(isopropylcarbamoyl)butyl]-4-{[(pyridin-2-ylmethyl)amino]methyl}benzamide [Compound No. 123]
MS(FAB,Pos.):m/z=482[M+H]⁺
¹H-NMR(500MHz,DMSO-d₆) : δ=0.87(3H,d,J=7.3Hz) ,0.89(3H,d,J=7.3H),1 .06(3H,d,J=6.6Hz),1.08(3H,d,J=6.6Hz),1.58-1.81(8H,m),2.30(9H,m ),2.80-3.12(6H,m),3.80-3.88(1H,m),4.321(4H,s),4.43-4.46(1H,m), 7.46(1H,dd,J=4.8,7.5Hz),7.49(1H,d,J=8.0Hz),7.62(2H,d,J=8.5H),7 .88-7.98(4H,m),8.52(1H,d,J=8.5Hz),8.66(1H,d,J=4.8Hz),9.03(1H,b rs),9.51-9.62(1H,m).
4-{[bis(1H-imidazol-2-ylmethyl)amino]methyl}-N-[(1S)-4-[(2-hydroxyethyl)isobutylamino]-1-{[(S)-1-(naphthalen-1-yl)ethyl]carbamoyl}butyl]benzamide [Compound No. 124]
MS (FAB,Pos.) :m/z=679 [M+H]⁺
¹H-NMR(500MHz,DMSO-d₆) δ=0.83-0.90 (6H,m) ,1.52 (3H,d,J=6.7Hz) ,1.5 5-1.63(1H,m) ,1.63-1.76(3H,m) ,1.94-1.98(1H,m) ,2.38-2.39(18H,m), 2.73-2.80 (1H,m) ,2.86-2.97 (1H,m) ,3.07 (4H,brs) ,3.69 (2H,s) ,3.76 (2 H,s) ,4.09(4H,s) ,4.54-4.56(1H,m) ,5.72(1H,quint. ,J=7.3Hz) ,7.46-7 .57(6H,m) ,7.64(4H,d,J-2.0Hz) ,7.83-7.86(3H,m) ,7.95(1H,dd,J=1.7, 7.8Hz) ,8.11(1H,d,J=7.6Hz) ,8.44 (1H,d,J=7.6Hz) ,8.67(1H,brs) ,8.72 (1H,t,J=7.8Hz) .
N-[(1S)-4-dipropylamino-1-(isopropylcarbamoyl)butyl]-4-{[(1H-imidazol-2-ylmethyl)amino]methyl}benzamide [Compound No. 125]
MS (FAB,Pos.) :m/z=471 [M+H]⁺
¹H-NMR(500MHz, DMSO-d₆) : δ=0.86-0.92 (6H,m) ,1.06(3H,d,J=6.6Hz) ,1.0 8(3H,d,J=6.6Hz) ,1.59-1.80(8H,m) ,2.32(9H,s) ,2.97-3.16(6H,m) ,3.8 2-3.90(1H,m) ,4.10-4.2(4H,m) ,4.27-4.47(1H,m) ,7.42-7.61(4H,m) ,7. 93-7.98(2H,m) ,8.10-8.20(1H,m) ,8.36(1H,d,3=7.OHz) ,8.51(1H,d,J=8.3Hz) .
4-{[(1H-imidazol-2-ylmethyl)amino]methyl)-N-[(1S)-2-(3H-imidazol-4-yl)-1-{[(S)-1-(naphthalen-1-yl)ethyl]carbamoyl}ethyl]benzamide [Compound No. 126]
MS (FAB,Pos. ) m/z= 522 [M+H]⁺
¹H-NMR (500MHz,DMSO-d₆) : δ=1.51(3H,t,J=6.8Hz) ,2.34(9H,s) ,3.05-3.1 3(2H,m) ,4.20-4.32(4H,br) ,4.88(1H,dd,J=14.6,8.1Hz) ,5.71(1H,t,J= 7.3Hz) ,7.32(1H,s) ,7.35(1H,d,J=7.1Hz) ,7.44-7.59(7H,m) ,7.83(1H,d ,J=8.3Hz) ,7.92-7.96 (3H,m) ,8.07 (1H,d,J=9.8Hz) ,8.59 (1H,brs) ,8.76 (2H,d,J=8.1Hz) ,8.95(1H,s) .
4-{[(1H-imidazol-2-ylmethyl)amino]methyl}-N-[ (1S)-2-(1H-indol-2-yl)-1-{[(S)-1-(naphthalen-1-yl) ethyl]carbamoyl}ethyl]benzamide [Compound No. 127]
MS (FAB,Pos.) :m/z 522 [M+H]⁺
¹H-NMR(500MHz,DMSO-d₆) : δ=1.54 (3H,t,J=6.8Hz) ,2.34 (9H,s) ,3.07-3.1 5 (2H,m) , 4 .28 (2H, s) , 4 .35 (2H, s) , 4.88 (1H, dd,J=14.1, 8.8Hz) , 5.73 (1H ,quint. ,J=7.1Hz) ,6.96(1H,t,J=7.1Hz), 7.04(1H,t,7.1Hz) ,7.31(1H,d ,J=8.3Hz),7.45-7.57(8H,m),7.73(1H,d,J=7.6Hz),7.82(1H,d,J=9.0Hz ) ,7.89(2H,d,J=8.1Hz) ,7.95(1H,d,J=7.6Hz) ,8.12(1H,d,J=7.8Hz) ,8.5 5(1H,d, J=8.3Hz) ,8.89(1H,d,J=7.6Hz) ,8.89(1H,d,J=7.6Hz) ,10.79(1H ,s).
N-[(1S)-4-dipropylamino-1-(3-phenylpropylcarbamoyl)butyl]-4-{[ (1H-imidazol-2-ylmethyl)amino]methyl}benzamide[Compound No. 128]
MS(FAB,Pos.):m/z=547[M+H]⁺
¹H-NMR(500MHz,DMSO-d₆) : δ=0.87(6H,dt,J=0.7,7.3Hz),1.58-1.91(10H, m) ,2.33(9H,s) ,2.57(2H,t,J=8.1Hz) ,2.97-2.99(4H,m) ,3.06-3.13(4H, m) ,4.27(4H,br) ,4.46 (1H,dd,J=8.5,13.9Hz) ,7.16-7.20(2H,m) ,7.28-7 .31(2H,m) ,7.48-7.54(1H,br) ,7.58(2H,d,J=8.1Hz) ,7.97(2H,d,J=8.3H z),8.11(1H,t,J=5.6Hz),8.58(1H,d,J=8.1Hz),9.01(1H,br).
N-[(1S)-4-dipropylamino-1-(3-phenylpropylcarbamoyl)butyl]-4-{[ (1-methyl-1H-imidazol-2-ylmethyl)amino]methyl}benzamide [Compound No. 129]
MS(FAB,Pos.):m/z=561[M+H]⁺
¹H-NMR(500MHz,DMSO-d₆) : δ=0.87(6H,t,J=7.3Hz) ,1.57-1.83(10H,m) ,2. 33(9H,s),2.57(2H,t,J=8.3Hz),2.97-2.99(4H,m),3.05-3.14(4H,m),3. 78(3H,s) ,4.29(2H,br) ,4.39(2H,br) ,4.46(1H,dd,J=8.5,13.9Hz) ,7.17 -7.20(3H,m),7.26(2H,d,J=7.3Hz),7.59(3H,d,J=8.1Hz),7.98(2H,d,J= 8.3Hz),8.12(1H,t,J=5.6Hz),9.01(1H,br).
N-[(1S)-4-dipropylamino-1-(3-phenylpropylcarbamoyl)butyl]-4-{[ (pyridin-2-ylmethyl)amino]methyl}benzamide [Compound No. 130]
MS(FAB,Pos.):m/z=558[M+H]⁺
¹H-NMR(500MHz,DMSO-d₆) : δ=0.87(6H,t,J=7.1Hz) ,1.57-1.83(10H,m) ,2. 32(9H,s),2.57(2H,t,J=8.1Hz),4.32(4H,br),4.46(1H,dd,J=8.5,14.1H z),7.16-7.20(3H,m),7.26-7.29(2H,m),7.38(1H,d,J=8.1Hz),7.44-7.5 1(4H,m),7.62(2H,d,J=8.3Hz),7.88-7.92(2H,m),7.97(2H,d,J=8.5Hz), 8.11(1H,t,J=5.6Hz),8.60(1H,d,J=8.1Hz),8.66-8.67(2H,m),9.01(1H, br),9.42(1H,br),9.55(2H,br).
4-{[(1H-imidazol-2-ylmethyl)amino)methyl}-N-({[(S)-1-(naphthalen-1-yl)ethyl]carbamoyl}methyl)benzamide [Compound No. 131]
MS(FAB,Pos.):m/z=442[M+H]⁺
¹H-NMR(500MHz,DMSO-d₆+D₂O) : δ=1.51(3H,d,J=7.1Hz),3.92(1H,dd,J=5. 9,16.3Hz),3.98(1H,dd,J=5.9,16.3Hz),4.35(2H,s),4.46(2H,s),5.71(1H,quint.,J=7.1Hz),7.45-7.61(4H,m),7.62-7.68(4H,m),7.83(1H,d,J =8.3Hz),7.92(2H,d,J=8.5Hz),7.93(1H,d,J=8.1Hz),8.11(1H,d,J=8.8H z),8.63(1H,d,H=7.6Hz),8.80(1H,br).
4-{[(1H-imidazol-2-ylmethyl)amino]methyl}-N-[(1S)-2-methyl-1-{ [(S)-1-(naphthalen-1-yl)ethyl]carbamoyl}propyl]benzamide [Compound No. 132]
MS (FAB,Pos.) :m/z 484[M+H]⁺
¹H-NMR(500MHz,DMSO-d₆) : δ=0.84(6H,dd,J=15.9,6.8Hz) ,1.51(3H,t,J=6 .8Hz) ,2.08(1H,q,J=6.8Hz) ,2.41(6H,s) ,4.36(2H,s) ,4.41(1H,t,J=8.3 Hz) ,4.50(2H,s) ,5.75(1H,quint. ,J=6.8Hz) ,6.84(1H,s) ,7.46-7.61(6H ,m) ,7.71 (2H,s) ,7.82 (1H,d,J=8.1Hz) ,7.93 (2H,d,J=6.8Hz) ,7.97 (1H,d ,J=8.3Hz) ,8.14(1H,d,J=7.6Hz) ,8.31(1H,d,J=8.3Hz) ,8.77(1H,d,J=8. 1Hz) .
N-[(1S)-4-dipropylamino-1-{[(naphthalen-1-ylmethyl)carbamoyl]butyl}-4-{[(1H-imidazol-2-ylmethyl)amino]methyl}benzamide [Compound No. 133]
MS (FAB,Pos.) :m/z=569 [M+H]⁺
¹H-NMR(500MHz,DMSO-d₆} : δ=0. 86 (6H,t,J=7.3Hz) ,1.55-1.65(6H,m) ,1.7 1-1.84 (2H,m) ,2.34 (9H, s) ,2.92-2.95 (4H,m) ,3.06-3.07 (2H,m) ,4.29(2 H,br) ,4.36(2H,br) ,4.56(1H,dd,J=8.3,14.4Hz) ,4.73-4.82(2H,m) ,7.4 5-7.49(2H,m),7.53-7.57(4H,m),7.59(2H,d,J=8.6Hz),7.85-7.87(1H,m ),7.94-7.99(3H,m),8.06-8.08(1H,m),8.63(1H,t,J=5.6Hz),8.67(1H,d ,J=7.6Hz),9.00(1H,br).
4-{[(1H-imidazol-2-ylmethyl)amino]methyl}-N-[(1S)-4-[(1H-imidazol-2-ylmethyl)amino]-1-{[(S)-1-(naphthalen-1-yl)ethyl]carbamoyl}butyl]benzamide [Compound No. 134]
MS(FAB,Pos.):m/z=579[M+H]⁺
¹H-NMR(500MHz,DMSO-d₆) : δ=1.52(3H,d,J=6.8Hz), 1.60-1.83 (4H,m) ,2.3 7(12H,s),3.02(2H,d,J=5.6Hz),4.32(2H,s),4.39(2H,s),4.43(2H,s),4 .51-4.59(1H,m),5.71(1H,quint.J=6.8Hz),7.48-7.63(10H,m),7.83(1H ,d,J=7.8Hz),7.95(1H,d,J=7.6Hz),7.99(2H,d,J=8.3Hz),8.10(1H,d,J= 7.8Hz),8.55(1H,d,J=8.3Hz),8.70(1H,d,J=7.8Hz).
4-{[(pyridin-2-ylmethyl)amino]methyl}-N-[(1S)-4-[(1H-imidazol-2-ylmethyl)amino]-1-{[(S)-1-(naphthalen-1-yl)ethyl]carbamoyl}butyl]benzamide [Compound No. 135]
MS(FAB,Pos.):m/z=590[M+H)⁺
¹H-NMR(500MHz,DMSO-d₆):δ=1.52(3H,d,J=6.8Hz),1.64-1.79(4H,m),2.3 7(12H,s) ,3.04(2H,d,J=6.8Hz) ,4.33(4H,brs) ,4.46(2H,s) ,4.55-4.59(1H,m),5,71(1H,quint. ,J=6.8Hz) ,7.45-7.58(6H,m) ,7.63(2H,d,J=8.3H z) ,7.72 (2H,s) ,7.83 (1H,d,J=8.3Hz) ,7.89-7.99 (4H,m) ,8.10 (1H,d,J=8 .1Hz) ,8.55(1H,d,J=8.3Hz) ,8.67(1H,d,J=7.6Hz) ,8.70(1H,d,J=7.8Hz)
N-[(1S)-4-(2-hydroxyethylamino)-1-{[(S)-1-(naphthalen-1-yl)ethyl]carbamoyl}butyl]-4-{[(1-methyl-1H-imidazol-2-ylmethyl)amino ]methyl}benzamide [Compound No. 136]
MS(FAB,Pos.):m/z=557[M+H]⁺
¹H-NMR(500MHz,DMSO-d₆+D₂O) : δ=1.52(3H,d,J=7.0Hz) ,1.58-1.77(4H,m) ,2,37(9H,s) ,2.88-2.93(3H,m) ,3.59-3.62(3H,m) ,3.76(3H,s) ,4.27(2H, s) ,4.36(2H,s) ,4.54-4.58(1H,m) ,5:69-5.73(1H,m) ,7.36(1H,s) ,7.48 -7.60(7H,m),7.84(1H,d,J=8.3Hz),7.95(3H,m),8.09(1H,d,J=8.3Hz).
N-[(1S)-4-(2-hydroxyethylamino)-1-{[(S)-1-(naphthalen-1-yl)ethyl]carbamoyl}butyl]-4-{[(1H-imidazol-2-ylmethyl)amino]methyl}benzamide [Compound No. 137]
MS(FAB,Pos.):m/z=543[M+H]⁺
¹H-NMR(500MHz,DMSO-d₆+D₂O) : δ=1.52(3H,d,J=6.8Hz) ,1.58-1.80(4H,m), 2.38(9H,s) ,2.88-2.93(3H,m) ,3.59-3.62(3H,m) ,4.26(2H,s) ,4.30(2H ,s) ,4.54-4.58(1H,m) ,5.69-5.72(1H,m) ,7.45(1H,s) ,7.48-7.60(7H,m), 7.84(1H,d,J=8.5Hz) ,7.94-7.96(3H,m) ,8.09(1H,d,J=8.3Hz) .
4-{[(pyridin-2-ylmethyl)amino]methyl}naphthalen-1-carboxylic-[ (1S)-4-(4,5-dihydro-1H-imidazol-2-ylamio)-1-{[(S)-1-(naphthalen-1-yl)ethyl]carbamoyl}butyl]amide [Compound No. 138]
MS (FAB,Pos.) :m/z=709 [M+H]⁺
¹H-NMR(500MHz,DMSO-d₆) : δ=1.51-1.63(2H,m) ,1.57 (3H,d,J=6.8Hz) ,1.6 6-1.69(1H,m),1.75-1.78(1H,m),3.13(1H,dd,J=6.8,13.2Hz),3.56(4H, s) ,4.50(2H,br) ,4.62(1H,dt,J=5.6,8.1Hz) ,4.79(2H,br) ,5.79(1H,quint.,J=7.3Hz),7.47-7.60(7H,m),7.62-7.65(2H,m),7.70-7.73(1H,m),7 .77(1H,d,J=7.3Hz),7.86(1H,d,J=8.1Hz),7.93(1H,dt,J=1.7,7.6Hz),7 .96-7.98(1H,m),8.14(1H,d,J=8.1Hz)8.23(1H,t,J=5.9Hz),8.25(1H,dd ,J=0.7,8.5Hz),8.31(1H.d,J=8.8Hz),8.69-8.75(3H,m),9.62(2H,br).
N-[(1S)-4-(4,5-dihydro-1H-imidazol-2-ylamio)-1-{[(S)-1-(naphthalen-1-yl)ethyl]carbamoyl}butyl]-4-({[1-methyl-1H-imidazol-2-ylmethyl]amino}methyl)benzamide [Compound No. 139]
MS(FAB,Pos.):m/z=662[M+H]⁺
¹H-NMR(500MHz,DMSO-d₆) : δ=1.46-1.53 (2H,m),1.52(3H,d,J=7.1Hz),3.1 0(2H,dd,J=6.6,12.7Hz) ,3.56(4H,s) ,3.85(3H,s) ,4.36(2H,br) ,4.48(2 H,br),4.56(1H,dd,J=9.0,14.1Hz),5.72(1H,quint.,J=7.1Hz),7.47-7. 57(6H,m) ,7.62(3H,d,J=8.3Hz) ,7.89(1H,d,J=8.1Hz) ,7.95-7.99(3H,m) ,8.10(1H,dd,J=2.2Hz),8.19(1H,t,J=5.9Hz),8.52(1H,d,J=7.8Hz),8.7 3(1H,d,J=7.8Hz).
4-{[(1H-imidazol-2-ylmethyl)amino]methyl}naphthalen-1-carboxylic-[(1S)-4-cyclohexylamino-1-{[(S)-1-(naphthalen-1-yl)ethyl]carbamoyl}butyl]amide [Compound No. 140]
MS(FAB,Pos.):m/z=631[M+H]⁺
¹H-NMR(500MHz,DMSO-d₆) : δ=1.04-1.12(1H,m) ,1.13-1.28(4H,m) ,1.56(3 H,d,J=6.8Hz),1.58-2.02(7H,m),2.34(9H,s),2.85-2.98(3H,m),4.42-4 .83(5H,m),5.76-5.81(1H,m),7.50-7.73(10H,m),7.85(1H,d,J=8.0Hz), 7.97(1H,d,J=7.8Hz),8.14(1H,d,J=8.5Hz),8.24-8.32(3H,m),8.70-8.7 5(2H,m).
N-[(1S)-4-cyclohexylamino-1-{[(S)-1-(naphthalen-1-yl)ethyl]carbamoyl}butyl]-4-({[1-(2-hydroxyethyl)-1H-imidazol-2-ylmethyl]amino}methyl)benzamide [Compound No. 141]
MS(FAB,Pos.):m/z=625[M+H]⁺
¹H-NMR(500MHz,DMSO-d₆) : δ=1.15-1.23(6H,m) ,1.51(3H,d,J=6.8Hz) ,1.5 7-1.75(7H,m),1.91-1.95(2H,m),2.31(9H,s),2.83-2.94(2H,brs),3.67 -3.70(2H,m),4.17-4.42(6H,m),4.58-4.62(1H,m),5.70-5.74(1H,m),7. 48-7.61(7H,m),7.84(1H,d,J=7.5Hz),7.94-7.97(3H,m),8.10(1H,d,J=7 .8Hz),8.19-8.30(1H,m),8.54(1H,d,J=8.3Hz),8.72(1H,d,J=8.3Hz).
4-{[(1-methyl-1H-imidazol-2-ylmethyl)amino]methyl)naphthalen-1 -carboxylic-[(1S)-4-methylcarbamimidoylsulfanyl-1-{[(S)-1-(naphthalen-1-yl)ethyl]carbamoyl}butyl]amide [Compound No. 142]
MS(FAB,Pos.):m/z=636[M+H]⁺
¹H-NMR(500MHz,DMSO-d₆) : δ=1.56(3H,d,J=7.1Hz) ,1.61-1.69(2H,m) ,1.7 5-1.78(1H,m),1.83-1.88(1H,m),2.33(9H,s),2.87(3H,d,J=4.9Hz),3.1 0-3.20(3H,m),3.85(3H,s),4.55-4.66(3H,m),4.77(1H,brs),5.78(1H,q uint.,J=7.1Hz),7.53-7.61(6H,m),7.63(2H,d,J=7.3Hz),7.68-7.72(2H ,m),7.86(1H,d,J=8.1Hz),7.97(1H,dd,J=1.7,7.8Hz),8.15(1H,d,J=8.3 Hz),8.25(1H,d,J=8.5Hz),8.28(1H,d,J=8.8Hz),8.71(1H,d,J=7.6Hz),8 .76(1H,d,J=7.8Hz),8.99(1H,s),9.22(1H,s),9.45(1H,d,J=4.2Hz).
4-{[(1H-imidazol-2-ylmethyl)amino]methyl}-N-[(1S)-1-{[(S)-1-(naphthalen-1-yl)ethyl]carbamoyl}-(2-phenyl)ethyl]benzamide [Compound No. 143]
MS(FAB,Pos.):m/z=532[M+H]⁺
¹H-NMR(500MHz,DMSO-d₆):δ=1.54(3H,d,J=6.8Hz),2.38(6H,s),2.93-3.1 7(2H,m),4.32(2H,brs),4.45(2H,brs),4.81-4.86(1H,m),5.72(1H,t,J= 7.1Hz)),7.16(1H,d,J=7.1Hz),7.22(2H,t,J=7.6Hz),7.32(2H,d,J=7.1H z),7.43-7.49(2H,m),7.51-7.60(4H,m),7.67(2H,brs),7.83(1H,d,J=7. 6Hz) ,7.89(2H,d,J=8.3Hz) ,7.95(1H,d,J=7.6Hz) ,8.11(1H,d,J=8.1Hz), 8.64(1H,d,J=8.5Hz),8.85(1H,d,J=7.8Hz).
4-{[(1-methyl-1H-imidazol-2-ylmethyl)amino]methyl}-N-[(1S)-4-[ (1H-imidazol-2-ylmethyl)amino]-1-{[(S)-1-(naphthalen-1-yl)ethy1]carbamoyl}butyl]benzamide [Compound No. 144]
MS (FAB,Pos.) :m/z=593 [M+H]⁺
¹H-NMR(500MHz,DMSO-d₆) : δ=1.51 (3H,d,J=6.8Hz) , 1.65-1.79 (4H,m) ,2.3 8 (12H,s) ,3.03-3.09 (2H,br) 3.87(3H,s) , 4.36 (2H,brs) ,4.43 (2H,brs) ,4.51-4.60 (3H,m) , 5.71 (1H,quint. J=6. 8Hz) ,7.47-7.57(4H,m) ,7.60-7 .71(6H,m) ,7.83(1H,d,J=7.8Hz) ,7.95(1H,d,J=7.6Hz) ,7.99(2H,d,J=8. 3Hz) ,8.09(1H,d,J=8.1Hz) ,8.55(1H,d,J=8.1Hz) ,8.70(1H,d,J=7.6Hz) .
4-{[(1-methyl-1H-imidazol-2-ylmethyl)amino]methyl}-N-[(1S)-4-[ (1-methyl-1H-imidazol-2-ylmethyl)amino]-1-{[(S)-1-(naphthalen-1-yl)ethyl]carbamoyl}butyl]benzamide [Compound No. 145]
MS(FAB,Pos.):m/z=607[M+H]⁺
¹H-NMR(500MHz,DMSO-d₆) : δ=2.52(3H,d,J=6.8Hz),1.63-1.80(4H,m),2.3 6(12H,s),3.05(2H,brs),3.82(3H,s),3.85(3H,s),4.35(2H,brs),4.45(2H,brs),4.51(2H,brs),4.55-4.60(1H,m),5.71(1H,quint.),7.47-7.69 (10H,m),7.83(1H,d,J=8.1Hz),7.95(1H,d,J=7.6Hz),7.99(2H,d,J=7.6H z),8.10(1H,d,J=8.3Hz),8.56(1H,d,J=8.3Hz),8.71(1H,d,J=7.6Hz).
4-({[1-(2-hydroxyethyl)-1H-imidazol-2-ylmethyl]amino}methyl)naphthalen-1-carboxylic-[(1S)-4-cyclohexylamino-1-{[(S)-1-(naphthalen-1-yl)ethyl]carbamoyl}butyl]amide [Compound No. 146]
MS(FAB,Pos.):m/z=675[M+H]⁺
¹H-NMR(500MHz,DMSO-d₆) : δ=1.04-1.13(1H,m) ,1.15-1.26(4H,m) ,1.56(3 H,d,J=7.0Hz),1.60-2.00(7H,m),2.37(9H,s),2.85-2.97(3H,m),3.70-3 .72(2H,m) ,4.25-4.27(2H,m) ,4.53(2H,s) ,4.62-4.65(1H,m) ,4.69(2H,s ),5.75-5.80(1H,m),7.49-7.70(10H,m),7.85(1H,d,J=8.5Hz),7.97(1H, d,J=8.0Hz),8.14(1H,d,J=8.3Hz),8.22-8.33(3H,m),8.69-8.75(2H,m).
4-{[(1-methyl-1H-imidazol-2-ylmethyl)amino]methyl}-N-[(1S)-4-methylcarbamimidoylsulfanyl-1-{[(S)-1-(naphthalen-1-yl)ethyl]carbamoyl}butyl]benzamide [Compound No. 147]
MS(FAB,Pos.):m/z=586[M+H]⁺
¹H-NMR(500MHz,DMSO-d₆) : δ=1.53(3H,d,J=6.8Hz) ,1.53-1.65(2H,m) ,1.7 4-1.86(2H,m),2.31(9H,s),2.87(3H,d,J=4.9Hz),3.10-3.16(1H,m),3.7 1(4H,brs) ,4.24(4H,br) ,4.51(1H,q,J=5.6Hz) ,5.72(1H,quint.,J=6.8H z) ,7.24(1H,brs) ,7.42 (1H,brs) ,7.47-7.59 (6H,m) ,7.84 (1H,d,J=8.1Hz ) ,7.94-7.96(3H,m) ,8.10(1H,d,J=7.3Hz) ,8.51(1H,d,J=8.5Hz) ,8.74(1 H,d,J=7.8Hz) ,8.97 (1H,s) ,9.20 (1H,s) ,9.43 (1H,brs) .
4-{[(1H-imidazol-2-ylmethyl)amino]methyl}-N-[(1S)-4-[(1-methyl -1H-imidazol-2-ylmethyl)amino]-1-{[(S)-1-(naphthalen-1-yl)ethyl]carbamoyl}butyl]benzamide [Compound No. 148]
MS (FAB,Pos.) :m/z=593 [M+H]⁺
¹H-NMR(500MHz,DMSO-d₆) : δ=1. 52 (3H, d, J=6.8Hz) ,1.63-1.83 (4H,m) ,2.3 8 (12H, s) 3.05 (2H,brs) ,3.82 (3H,s) ,4.34 (2H,brs) ,4.46 (4H,brs) ,4.55 -4.60(1H,m) ,5.70(1H,quint. ,J=6.8Hz) ,7.47-7.66(10H,m) ,7.83(1H,d ,J=8.1Hz) ,7.95(1H,d,J=7.6Hz) ,7.99(2H,d,J=8.4Hz) ,8.10(1H,d,J=8. 2Hz) ,8.56 (1H,d,J=8.2Hz) ,8.71 (1H,d,J=7.8Hz) .
N-[(1S)-4-[(3-methylpyridin-2-ylmethyl)amino-1-{[(S)-1-(naphthalen-1-yl)ethyl]carbamoyl}butyl]-4-({[1-(2-hydroxyethyl)-1H-imidazol-2-ylmethyl]amino}methyl)benzamide [Compound No. 149]
MS(FAB,Pos.) :m/z=648[M+H]⁺
¹H-NMR(500MHz,DMSO-d₆+D₂O) : δ=1. 52 (3H,d,J=7.0Hz) ,1.72-1.84 (4H,m) ,2.24(3H,s) ,2.37(9H,s) ,3.02-3.04(2H,m) ,3.69-3.71(2H,m) ,4.21-4. 24 (2H,m) ,4.28 (4H,s) ,4.43(2H,s) ,4.57-4.60(1H,m) ,5.70(1H,q,J=6.8 Hz) ,7.34-7.36(1H,m) ,7.41(1H,s) ,7.47-7.59(7H,m) ,7.69(1H,d,J=7.8 Hz) ,7.84 (1H,d,J=8.3Hz) ,7.95 (3H,d,J=8.3Hz) ,8.09 (1H,d,J=8.3Hz) ,8 .42(1H,d,J=3.9Hz) .
N-{(1S)-4-dipropylamino-1-[(1H-indol-3-ylmethyl)carbamoyl]buty1}-4-{[(1H-imidazol-2-ylmethyl)amino]methyl}benzamide [Compound No. 150]
MS (FAB,Pos.) :m/z=558 [M+H]⁺
¹H-NMR(500MHz,DMSO-d₆) : δ=0.78(6H,t,J=7.3Hz) ,1.28-1.44(6H,m) ,1.6 6-1.74(2H,m) ,2.23(4H,t,J=7.3Hz) ,2.31(2H,t,J=6.8Hz) ,3.67(2H,s), 3.73(2H,s) ,4.38-4.47(3H,m) ,6.94(1H,t,J=7.3Hz) ,7.01(1H,br) ,7.07 (1H,t,J=8.1Hz) ,7.23 (1H,d,J=2.4Hz) ,7.34 (1H,d,J=8.1Hz) ,7.42 (2H,d ,J=8.3Hz) ,7.53(1H,d,J=7.8Hz) ,7.83(2H,d,J=8.1Hz) ,8.18(1H,t,J=5. 1Hz) ,8.35(1H,d,J=8.3Hz) ,10.90(1H,d,J=0.5Hz) .
N-[(1S)-4-dipropylamino-1-{[(S)-1-(naphthalen-1-yl)ethyl]carbamoyl}butyl]-4-{[(1-oxypyridin-2-ylmethyl)amino]methyl}benzamide [Compound No. 151]
MS(FAB,Pos.):m/z=610[M+H]⁺
N-[(1S)-4-(4,5-dihydro-1H-imidazol-2-ylamino)-1-{[(S)-1-(naphthalen-1-yl)ethyl]carbamoyl}butyl]-4-({[1-(2-hydroxyethyl)-1H-imidazol-2-ylmethyl]amino}methyl)benzamide [Compound No. 152]
MS(FAB,Pos.):m/z=611[M+H]⁺
¹H-NMR(500MHz,DMSO-d₆):δ=1.43-1.56(2H,m),1.52(3H,d,J=7.0Hz),1.6 6-1.78(2H,m),3.10(1H,dd,J=6.9,13.0Hz),3.56(4H,s),3.70(2H,t,J=5 .2Hz) ,4.25(2H,br) ,4.30(2H,br) ,4.45(2H,br) ,4.56(1H,dd,J=8.6,14. 2Hz),5.72(1H,quint.,J=7.0Hz),7.47-7.60(8H,m),7.84(1H,d,J=7.9Hz ),7.94-7.98(3H,m),8.10(1H,dd,J=2.1,9.6Hz),8.19(1H,t,J=5.8Hz),8 .51(1H,d,J=7.9Hz),8.72(1H,d,J=7.9Hz).
(2S)-5-dipropylamino-2-(4-{[(1-methyl-1H-imidazol-2-ylmethyl)amino]methyl}benzenesulfonylamino)pentanoylic-[(S)-1-(naphthalen-1-yl)ethyl]amide [Compound No. 153]
MS(FAB,Pos.):m/z=633[M+H]⁺
¹H-NMR(500MHz,DMSO-d₆) : δ=0.83-0.88(6H,m) ,1.23-1.26(5H,m) ,1.47-1 .61(6H,m) ,2,31(9H,s) ,2,84-2.92(4H,m) ,2.92-3.03(2H,m) ,3.77(3H,m ) ,3.82-3.92(1H,m) ,4.20-4.60(4H,m) ,5.35-5.36(1H,m) ,7.43-7.59(6H ,m) ,7.65-7.72 (2H,m) ,7.81-7.89 (2H,m) ,7.91-7.98 (2H,m) ,8.17-8.21 (1H,m),8.50-8.58(1H,m) ,8.92-9.00(1H,brs) .
(2S)-5-dipropylamino-2-(4-{[(1H-imidazol-2-ylmethyl)amino]methyl}benzenesulfonylamino)pentanoylic-[(S)-1-(naphthalen-1-yl)ethyl]amide [Compound No. 154]
MS (FAB,Pos.) :m/z=619 [M+H]⁺
¹H-NMR (500MHz, DMSO-d₆) : δ=0.84 (3H,t,J=7.3Hz) ,0.85(3H,m) , 1.22-1.2
5(4H,m) ,1.49-1.60(7H,m) ,2.33(9H,s) ,2.82-2.92(4H,m) ,2.92-3.03(2 H,m) ,3.82-3.92(1H,m) ,4.21-4.42(4H,m) ,5.34-5.38(1H,m) ,7.43-7.59 (6H,m) ,7.65-7.72(2H,m) ,7.78-7.89(3H,m) ,7.91-7.95(2H,m) ,8.17-8. 21(1H,m) ,8.48-8.55(1H,m) ,8.96(1H,brs) .
N-[(1S)-4-dipropylamino-1-([1-(4-fluoronaphthalen-1-yl)ethyl]carbamoyl}butyl]-4-{[(1H-imidazol-2-ylmethyl)amino]methyl}benzamide [Compound No. 155]
MS(FAB,Pos.):m/z=601[M+H]⁺
¹H-NMR (500MHz,DMSO-d₆) : δ=0.83 and 0.89(6H,t,J=7.3 and 7.3Hz) ,1.50an d1.53(3H,d,J=7.1and7.1Hz) ,1.53-1.70(5H,m) ,1.70-1.77(2H,m) ,1.77 -1.89(1H,m) ,2.37(9H,s) ,2.84-2.93(2H,m) ,2.96-3.05(3H,m) ,3.06-3. 14(1H,m) ,4.31(2H,brs) ,4.42(2H,brs) ,4.51-4.59(1H,m) ,5.67 and 5.69 (1H,quint. ,J=7.6and7.6Hz) ,7.31and7.33(1H,dd,J=2.4,8.1and2.9,8. 1Hz),7.57-7.69(7H,m),7.94(1H,d,J=8.3Hz),7.97(1H,d,J=8.3Hz),8.0 8-8.11(1H,m),8.14-8.19(1H,m),8.57and8.59(1H,d,J=8.3and7.8Hz),8 .68and8.74(1H,d,J=7.8and7.6Hz),9.02and9.08(1H,brs).
N-[(1S)-4-dipropylamino-1-{[1-(4-fluoronaphthalen-1-yl)ethyl]carbamoyl}butyl]-4-{[(1-methyl-1H-imidazol-2-ylmethyl)amino]methyl}benzamide [Compound No. 156]
MS(FAB,Pos.):m/z=615[M+H]⁺
¹H-NMR(500MHz,DMSO-d₆): δ=0.83 and 0.89(6H,t,J=7.3and7.3Hz) ,1.50an d1.52(3H,d,J=7.1and8.5Hz),1.53-1.69(5H,m),1.69-1.77(2H,m),1.77 -1.89(1H,m) ,2.34(9H,s) ,2.86-2.92(2H,m) ,2.98-3.00(3H,m) ,3.10-3. 12(1H,m),3.81(3H,brs),4.32(2H,brs),4.45(2H,brs),4.51-4.59(1H,m ),5.64-5.70(1H,m),7.30-7.34(1H,m),7.52-7.69(7H,m),7.94(1H,d,J= 8.3Hz),7.97(1H,d,J=8.3Hz),8.08-8.11(1H,m),8.15-8.19(1H,m),8.57 and8.59(1H,d,J=8.1and8.3Hz),8.68and8.74(1H,d,J=7.8and7.8Hz),9. 01and9.07(1H,brs).
N-[(1S)-4-dipropylamino-1-((S)-1-naphthalen-2-ylethylcarbamoyl)butyl]-4-{[(1H-imidazol-2-ylmethyl)amino]methyl}benzamide [Compound No. 157]
MS(FAB,Pos.):m/z=583[M+H]⁺
¹H-NMR(500MHz,DMSO-d₆+D₂O) : δ=0.80(3H,t,J=7.3Hz),0.83(3H,t,J=7.3 Hz),1.48(3H,d,J=7.1Hz),1.50-1.62(5H,m),1.62-1.84(3H,m),2.84-2. 94(4H,m),2.98-3.09(2H,m),4.30(2H,brs),4.39(2H,brs),4.55-4.61(1 H,m) ,5.10 (1H,quint. ,J=7.3Hz) ,7.46-7.53 (3H,m) ,7.53-7.61 (4H,m) ,7 .79 (1H,s) ,7.86 (2H,d,J=8.1Hz) ,7.87 (1H,s) ,7.99 (1H,d,J=8.1Hz) ,8.5 7 (1H,d,J=7.8Hz) ,8.67 (1H,d,J=7.8Hz) ,8.99 (1H,brs) .
4-{[bis(1H-imidazol-2-ylmethyl)amino]methyl}-N-{(1S)-4-dipropylamino-1-[2-(1H-indol-3-yl)ethylcarbamoyl]butyl}benzamide [Compound No. 158]
MS (FAB,Pos.) :m/z=652 [M+H]⁺
¹H-NMR(500MHz,DMSO-d₆) : δ=0.86(6H,t,J=7.1Hz) ,1.63-1.64(4H,m) ,1.7 0-1.77(4H,m) ,2.82(2H,t,J=7.1Hz) ,2.92-2.93(2H,m) ,2.97-3.05(6H,m ) ,3.73 (2H,s) ,4.11 (4H,s) ,4.46 (1H,d,J=6.1Hz) ,6.01 (1H,m) ,6.08 (1H, m) 6.96(1H,t,J=8.1Hz) ,7.04-7.17 (2H,m) ,7.33 (2H,d,J=8.3Hz) ,7.51-7.58(3H,m),7.86(1H,d,J=8.5Hz),8.23(1H,t,J=5.9Hz),8.52(1H,d,J=8.1Hz),10.85(1H,br) .
N-{(1S)-4-dipropylamino-1-[(2-methoxynaphthalen-1-ylmethyl)carbamoyl]butyl}-4-{[(1H-imidazol-2-ylmethyl)amino]methyl}benzamide [Compound No. 159]
MS (FAB,Pos.) :m/z=599 [M+H]⁺
¹H-NMR(500MHz,DMSO-d₆) : δ=0.82(6H,dt,J=2.9,7.3Hz) ,1.54-1.80(8H,m ) ,2.83 (4H,br) ,2.96 (2H,br) ,3.95 (3H,s) ,4.35 (4H,s) ,4.49 (3H,br) ,4. 66(2H,dq,J=5.4,13.9Hz) ,7.37(1H,d,J=7.6Hz) ,7.46-7.50(2H,m) ,7.67 (4H,br) ,7.89(1H,d,J=8.3Hz) ,7.94-7.99(4H,m) ,8.23(1H,s) ,8.59(1H, d,J=7.8Hz) .
N-{(1S)-4-dipropylamino-1-[(4-methoxynaphthalen-1-ylmethyl)carbamoyl]butyl}-4-{[(1H-imidazol-2-ylmethyl)amino]methyl}benzamide [Compound No. 160]
MS(FAB,Pos.):m/z=599[M+H]⁺
¹H-NMR(500MHz,DMSO-d₆) : δ=0.85(6H,dt,J=1.5,7.3Hz),1.60-1.91(8H,m ) ,2.88(4H,br) ,3.01(2H,br) ,3.96(3H,s) ,4.38(4H,s) ,4.51-4.54(3H,b r),4.66(2H,dq,J=5.4,15.1Hz),6.92(1H,d,J=8.1Hz),7.40(1H,d,J=8.1 Hz),7.51-7.58(2H,m),7.69(2H,d,J=8.1Hz),7.73(2H,d,J=5.4Hz),7.98 (1H,d,J=8.5Hz),8.01(2H,d,J=7.6Hz),8.19(1H,d,J=8.1Hz),8.59(1H,b r),8.70(1H,d,J=7.8Hz).
4-{[bis(1H-imidazol-2-ylmethyl)amino]methyl}-N-((1S)-4-dipropylamino-1-isopropylcarbamoylbutyl)benzamide [Compound No. 161]
MS (FAB,Pos.) :m/z=551 [M+H]⁺
¹H-NMR(500MHz,DMSO-d₆) : δ=0.87 (6H,t,J=7.3Hz) ,1.07 (6H,d,J=6.6Hz) , 1.57-1.77(8H,d,m) ,2.37(12H,s) ,2.94-3.00(4H,m) ,3.06-3.09(2H,m) , 3.76(2H,s) ,3.83(1H,sext. ,J=6.6Hz) ,4.08(4H,s) ,4.42(1H,q,J=5.6Hz ) ,7.45(2H,d,J=8.3Hz) ,7.63(4H,s) ,7.84(2H,d,J=8.3Hz) ,7.91(1H,d,J =7.6Hz) ,8.40(1H,d,J=8.3Hz) ,9.04(1H,brs) .

Next, structural formulae of the respective compounds produced in the above Production Examples are shown in Table 1.

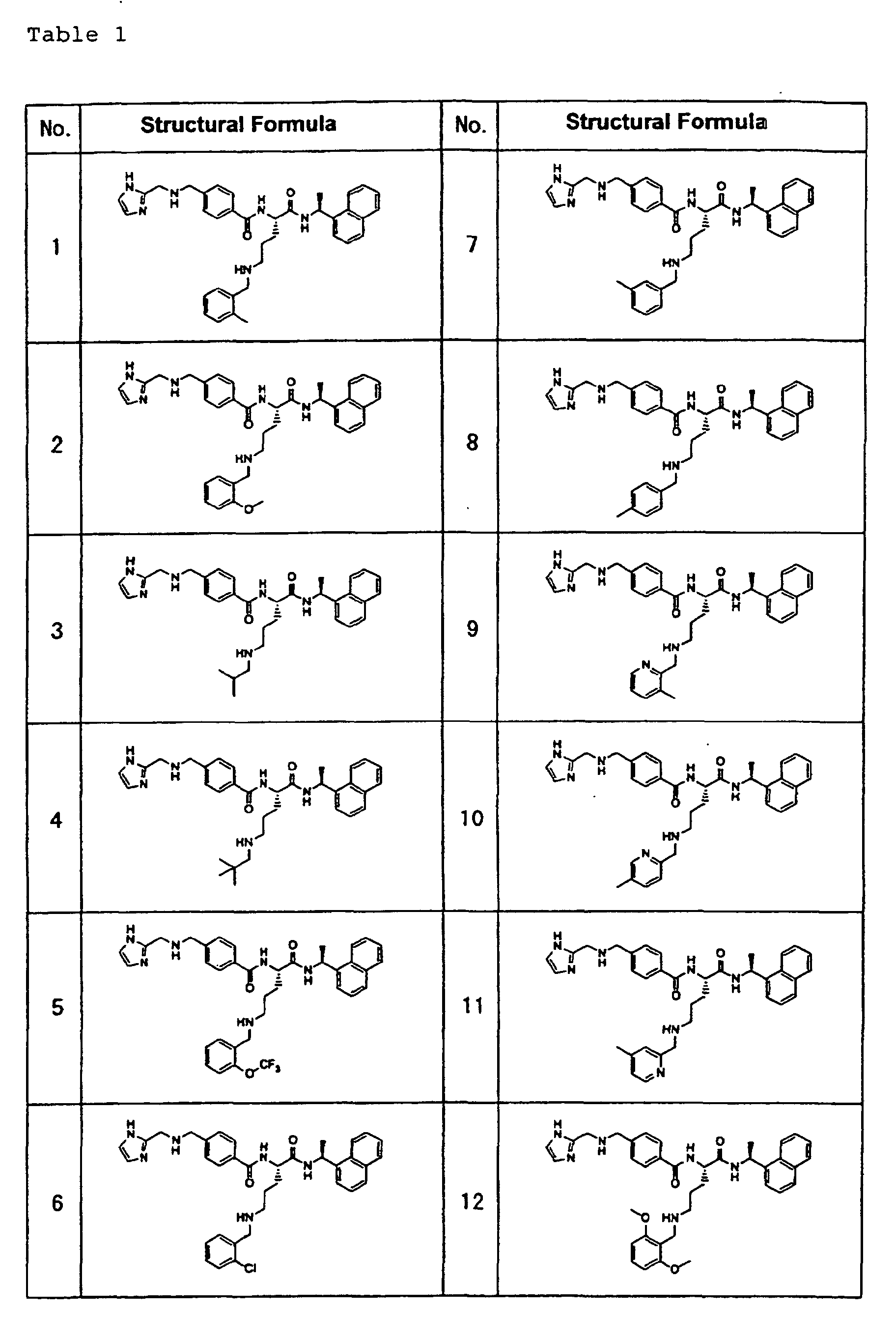

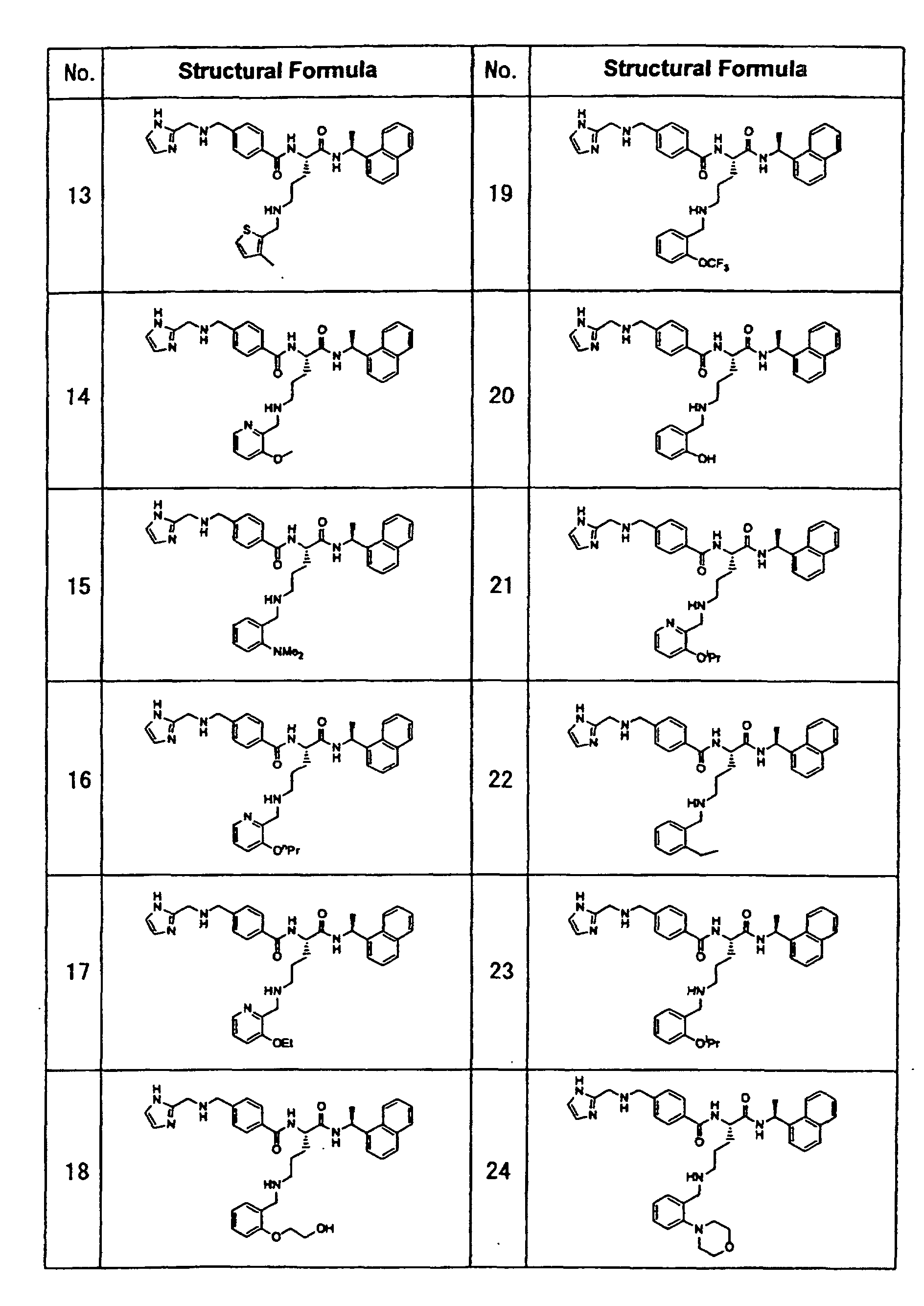

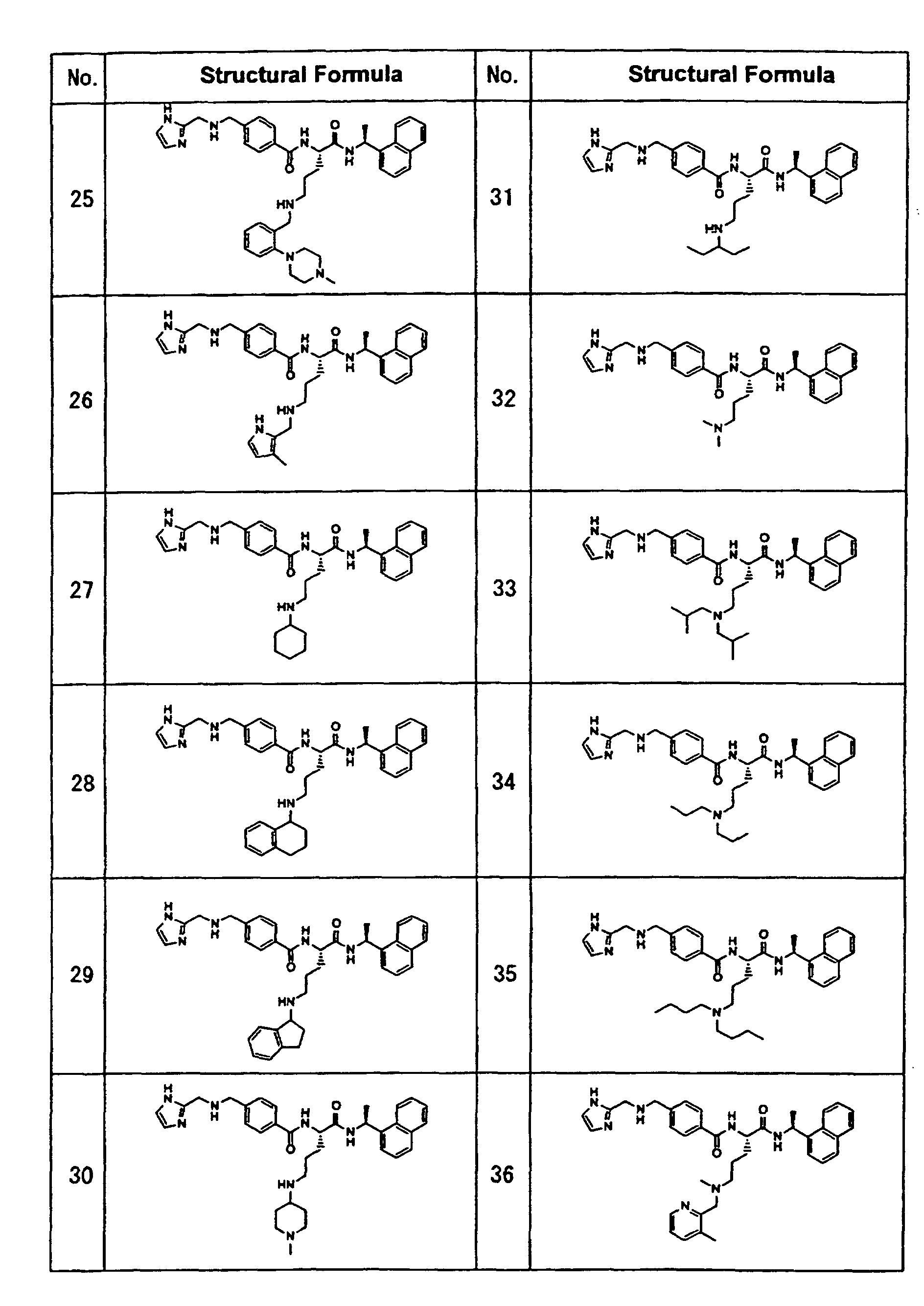

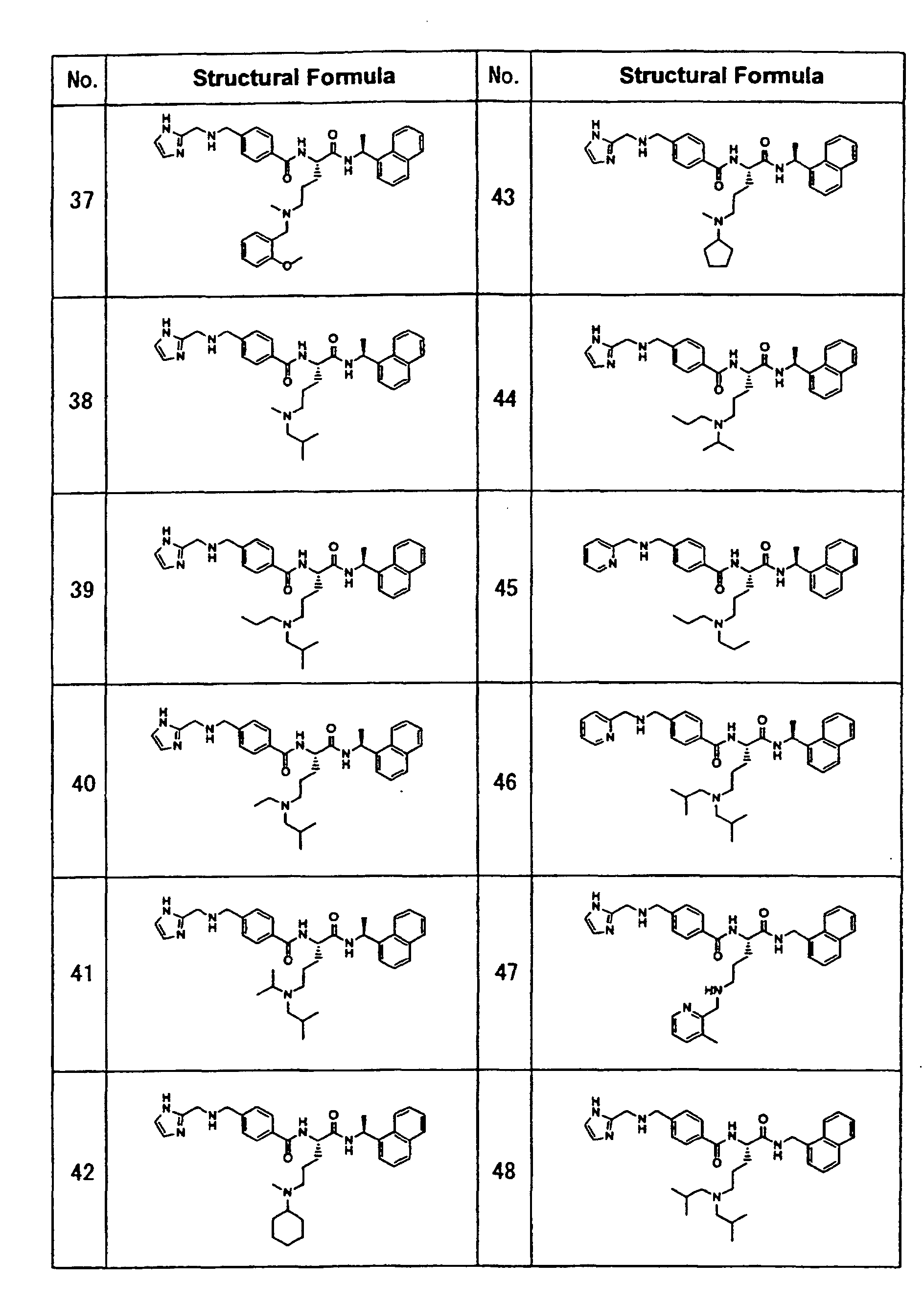

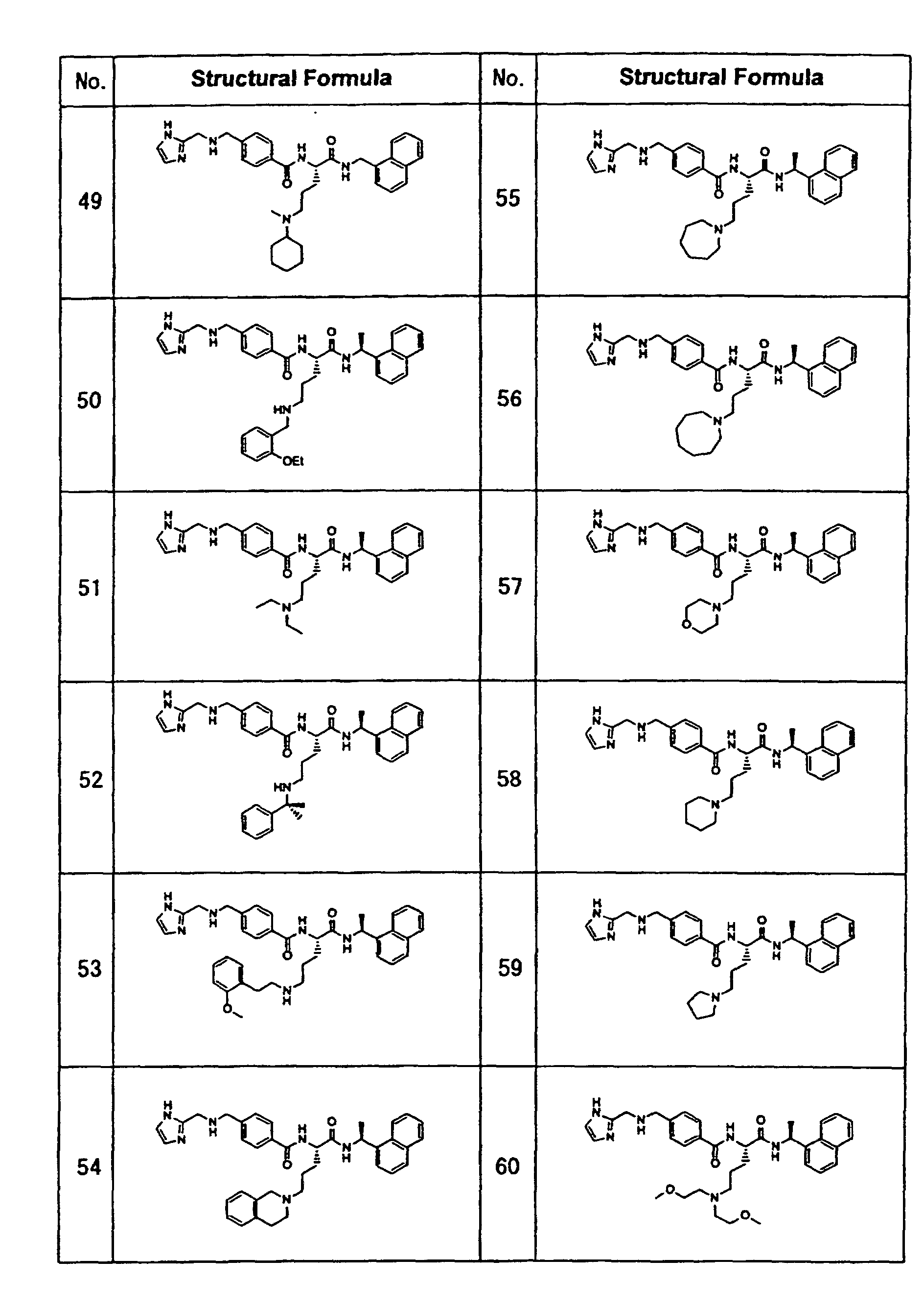

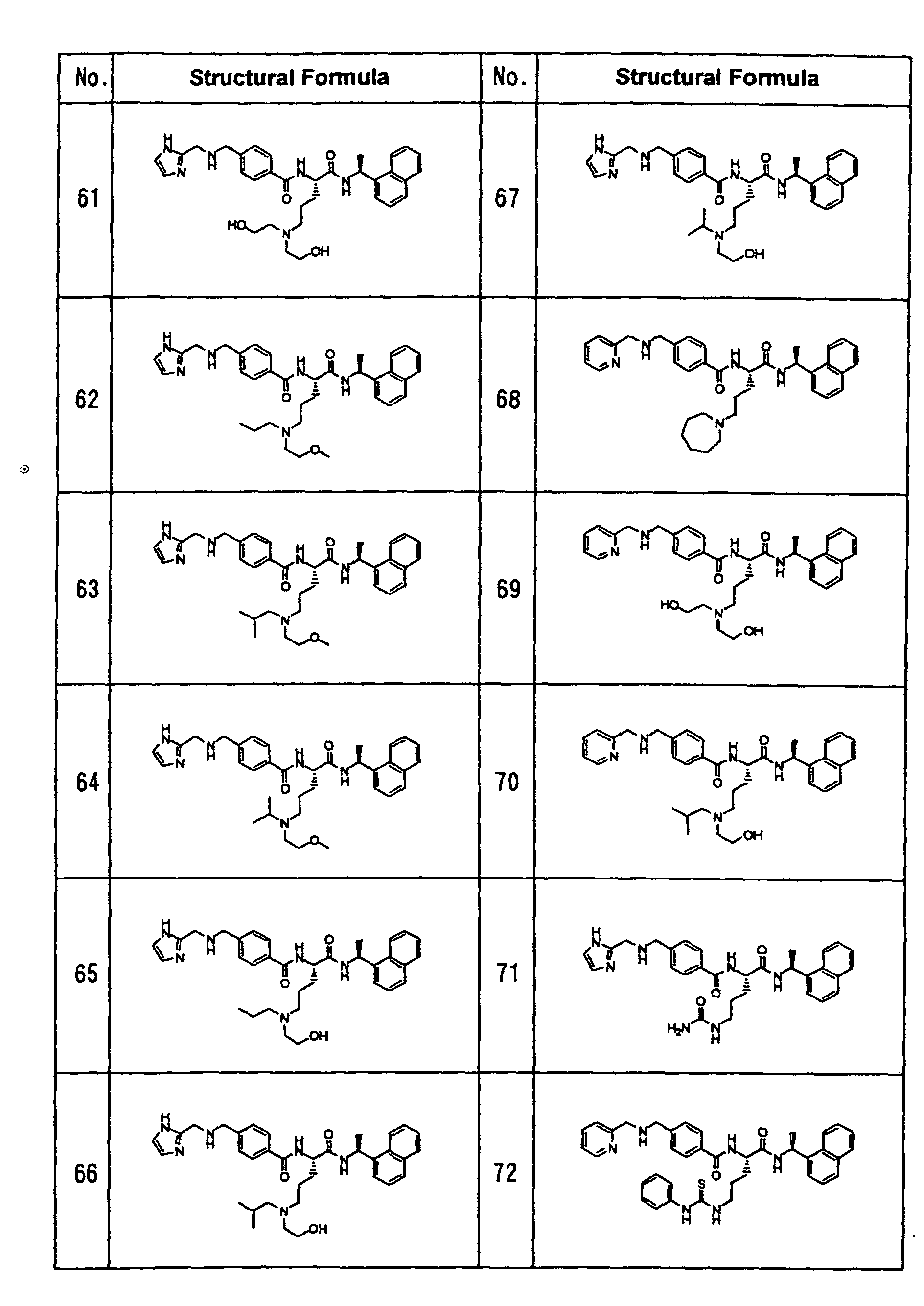

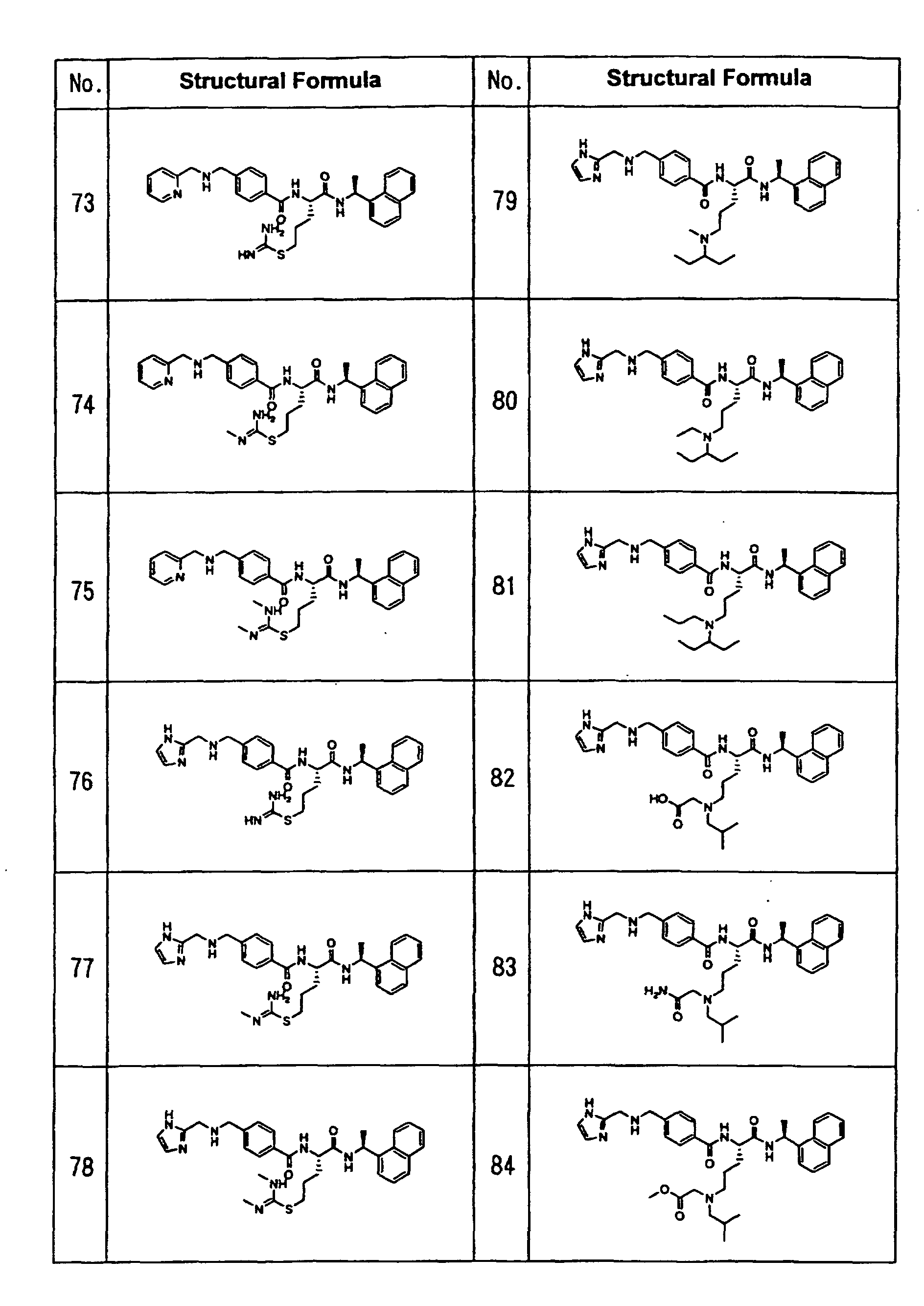

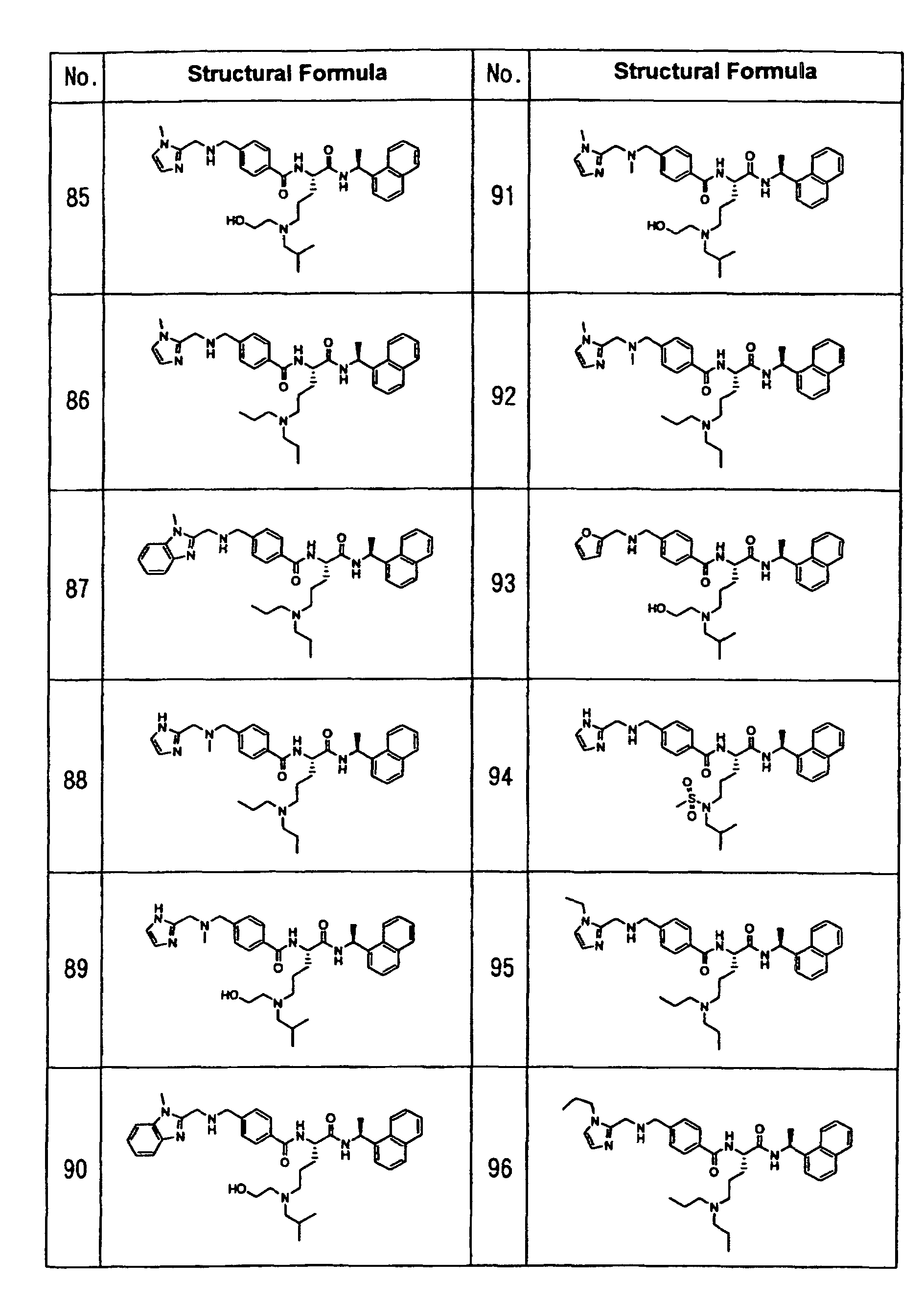

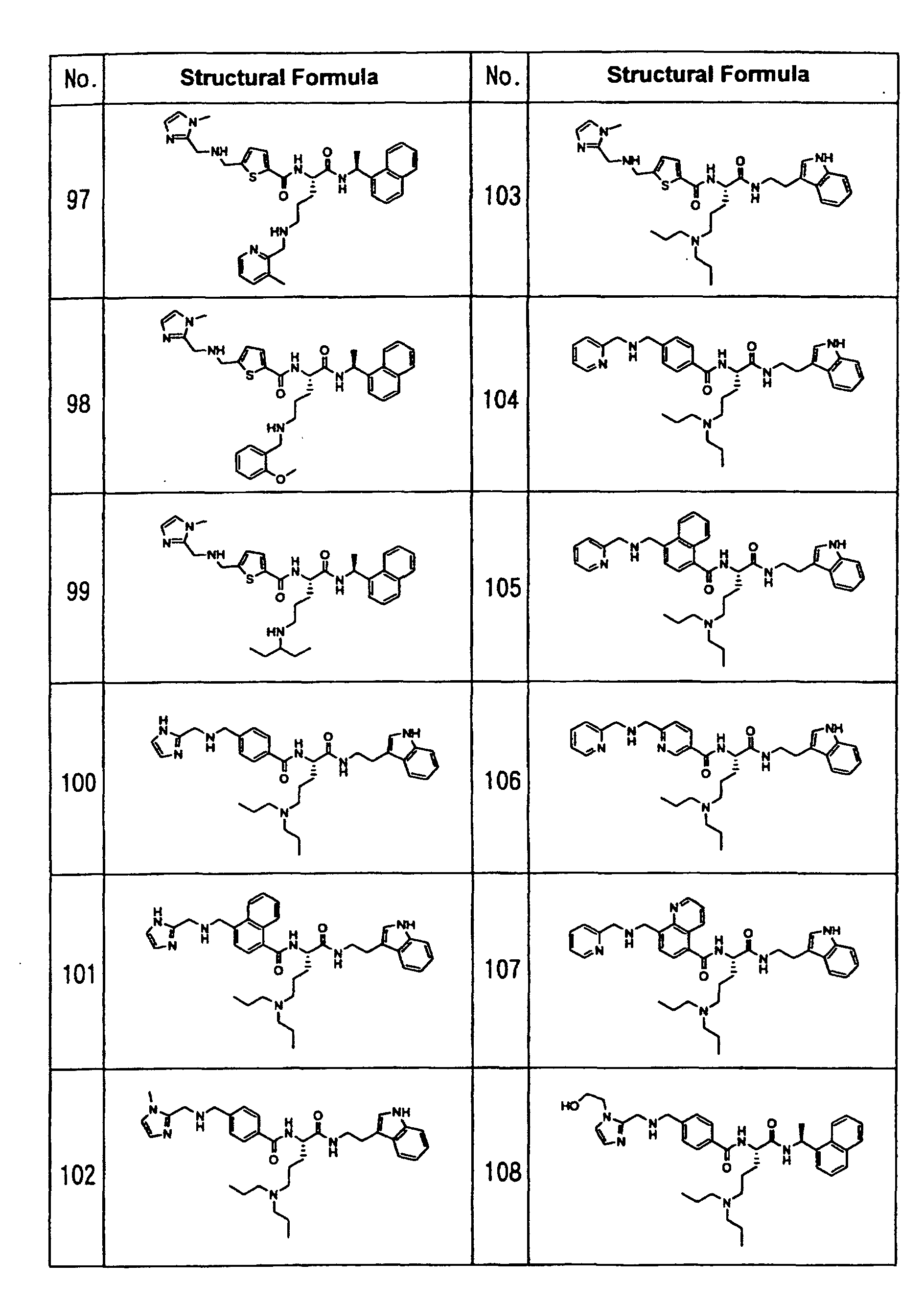

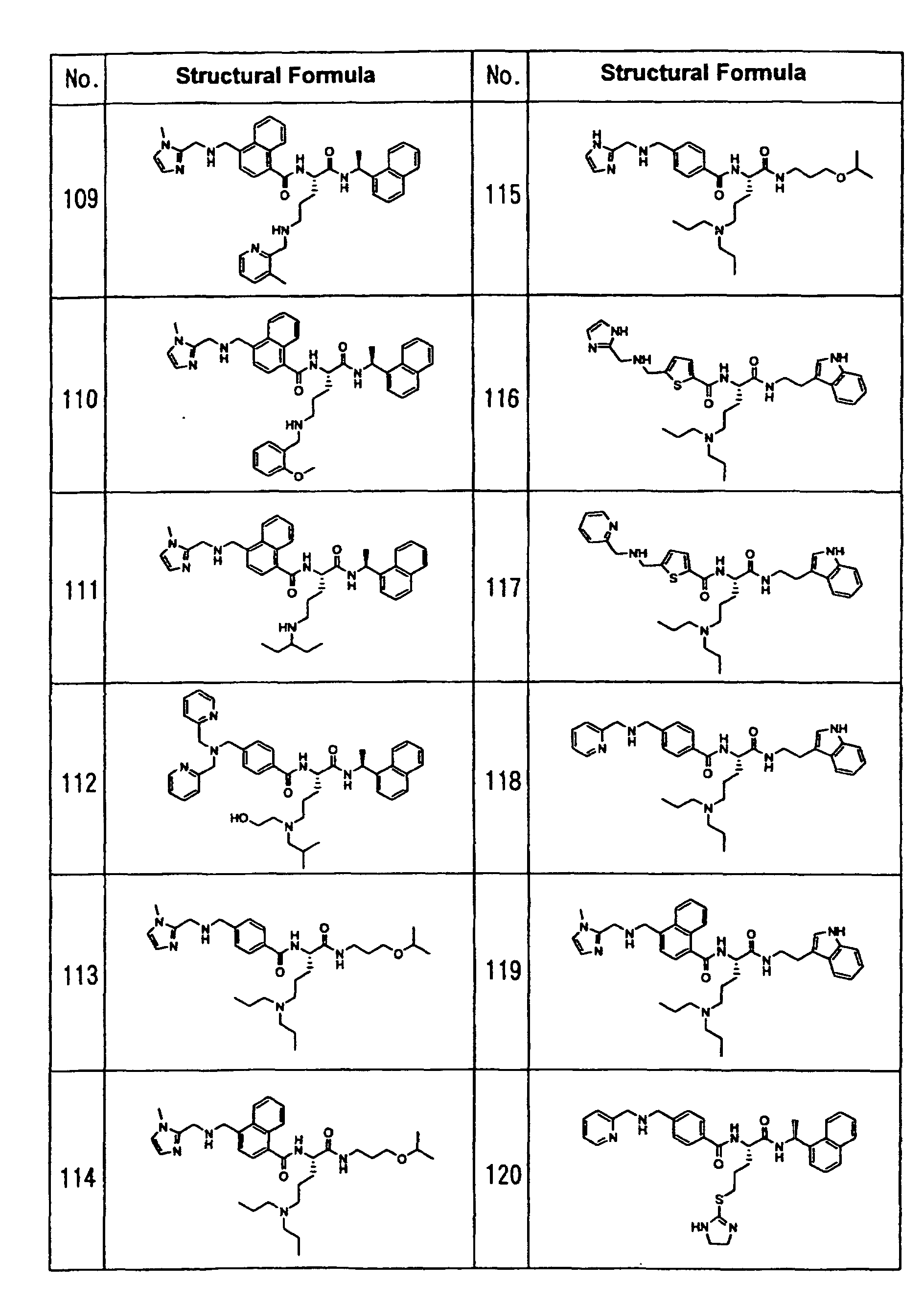

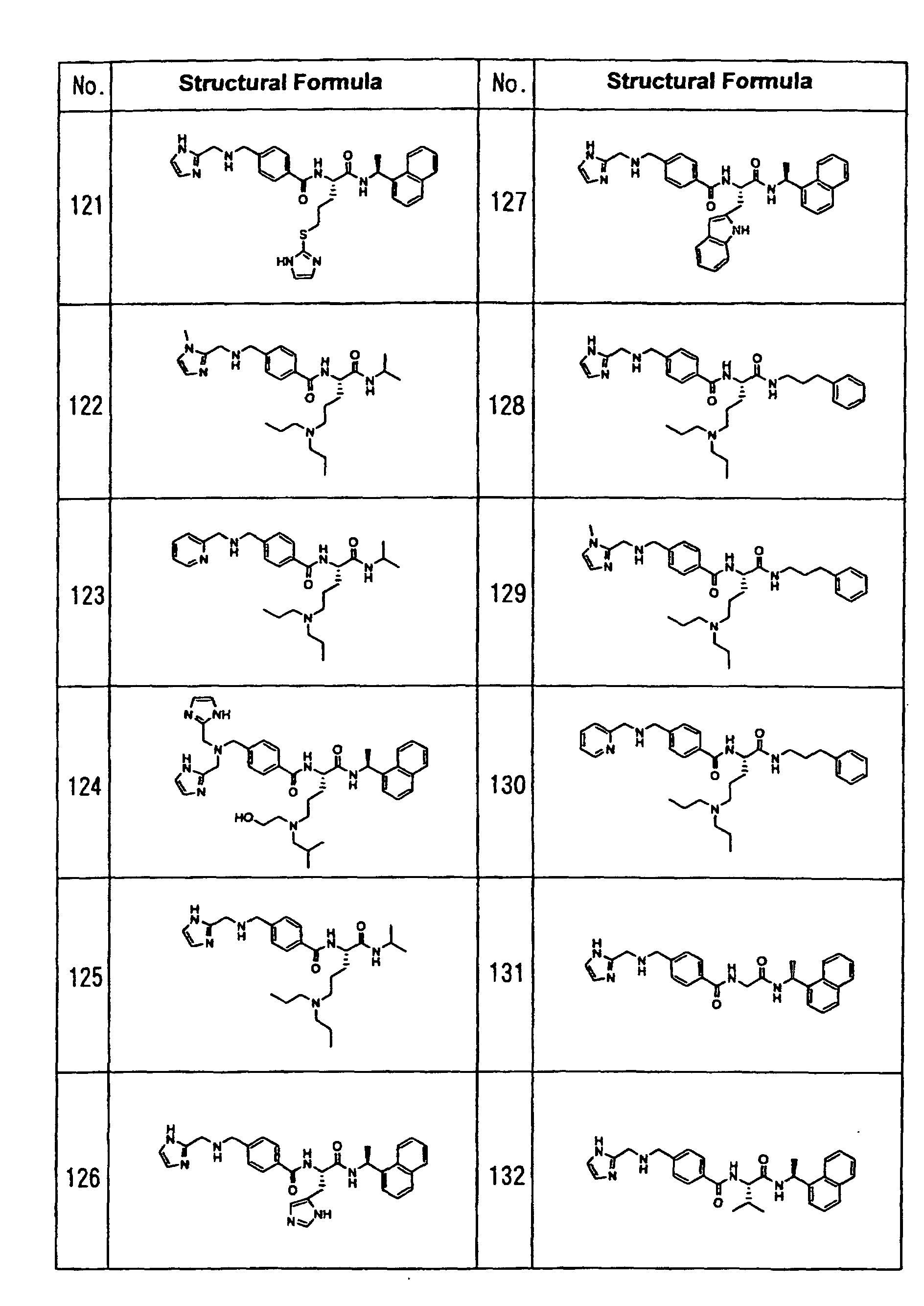

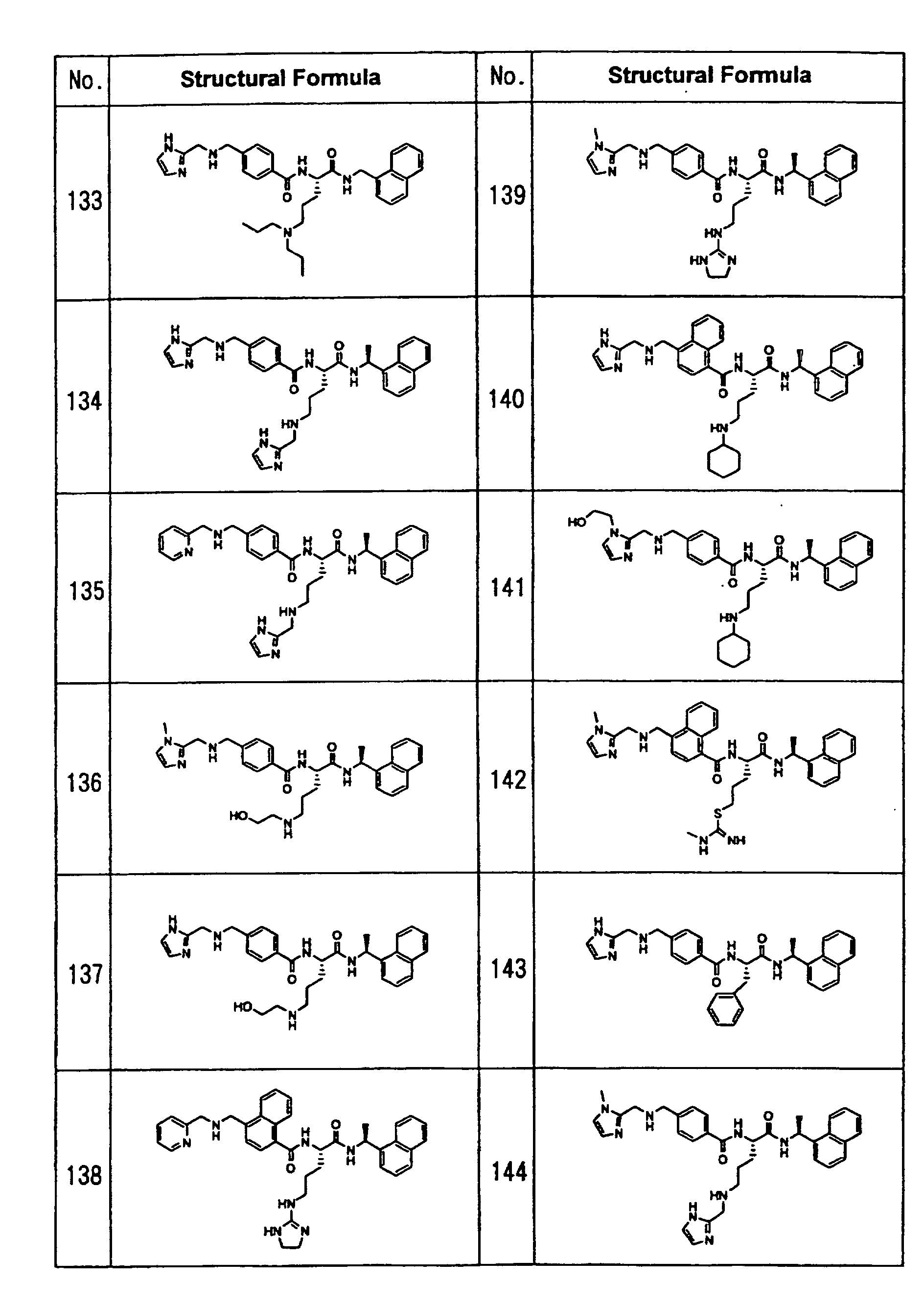

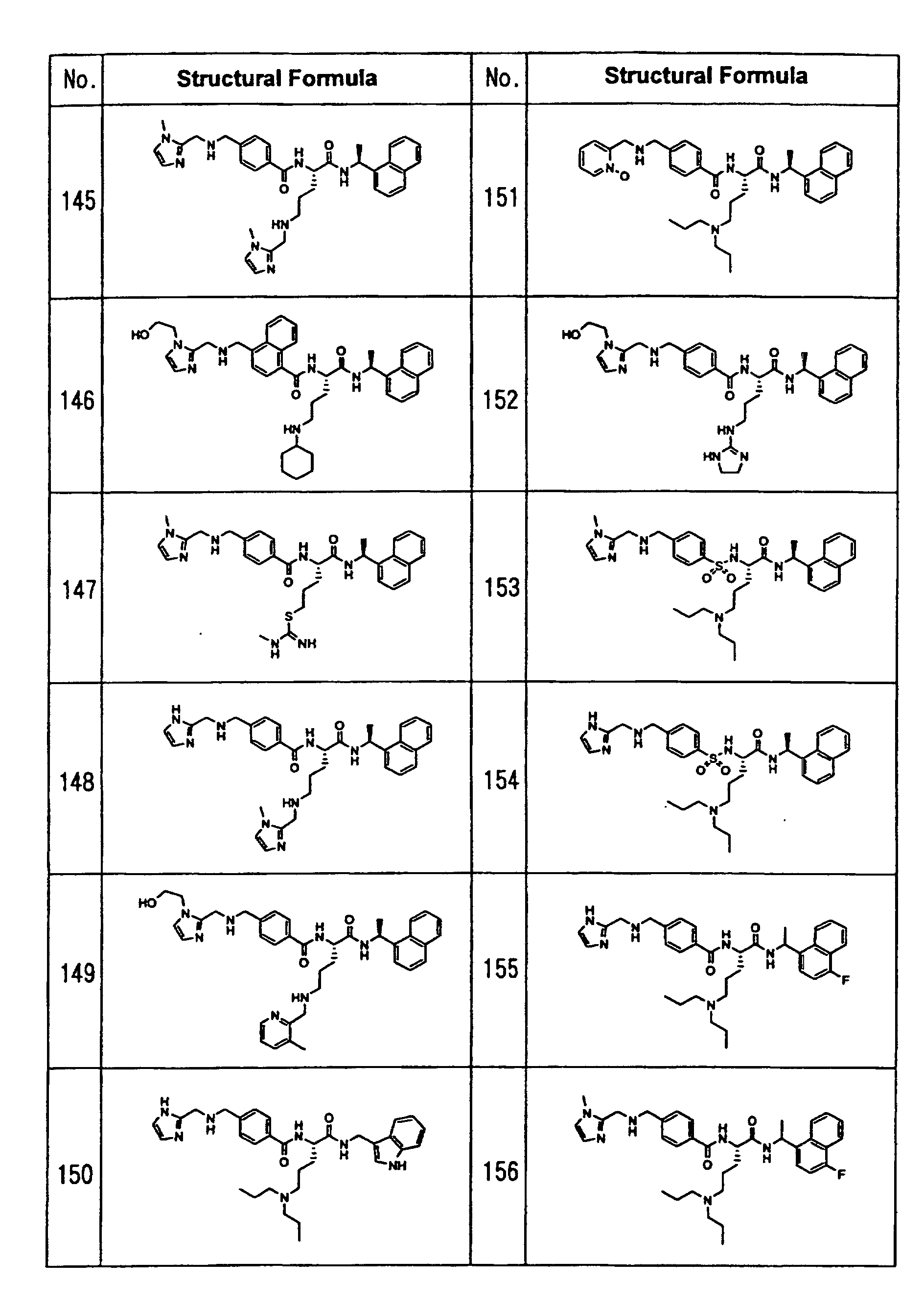

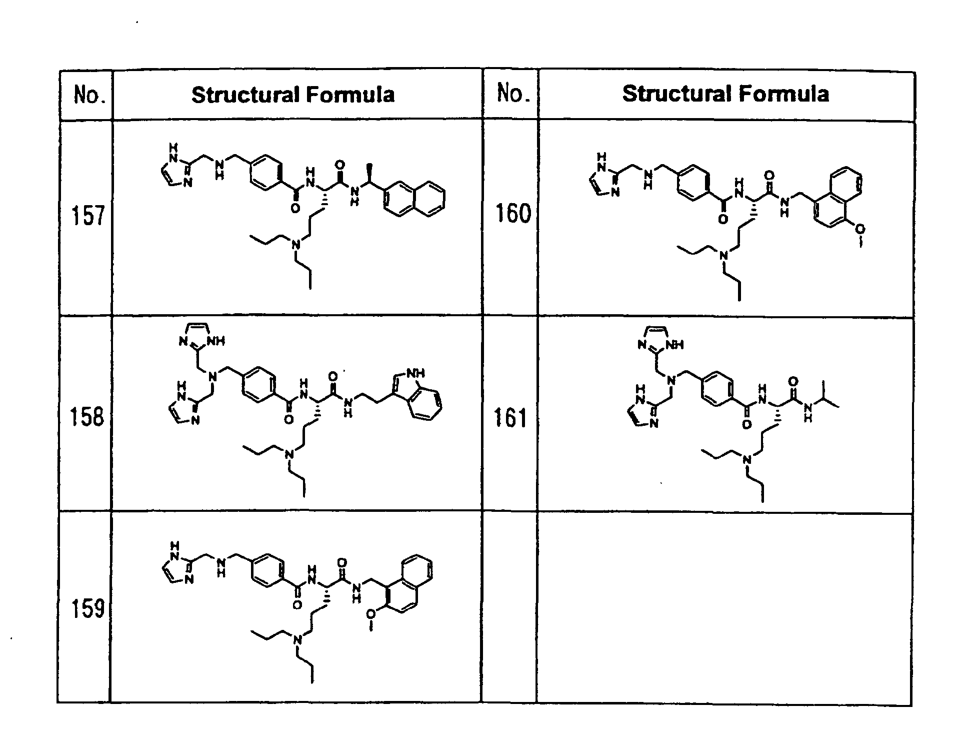

The results of the activity test for the compound of the present invention will be shown below.

### Test Example 1

Immediately after infection, HIV-1_{IIIB} infected MT-4 cells (3.0 × 10⁴/well, MOI (Multiplicity of infection): 0.01) were added to a 96-well microtiter plate together with the test compounds having different concentrations. The cells were cultured in a carbon dioxide incubator at 37°C for 5 days, and the number of living cells was measured in accordance with the MTT (tetrazolium) method (Pawels et al., J. of Virol. Method, 20, 309-321 (1988)). The antiviral activity is represented the concentration required for inhibition of cell disorder due to HIV infection by 50% (EC₅₀: 50% Effective Concentration) , and expressed using µM, and the results are shown in Table 2.

**Table 2**

| Compound No. | EC₅₀ [µM] | Compound No. | EC₅₀[µM] |
|---|---|---|---|
| 1 | 0.0580 | 39 | 0.0145 |
| 2 | 0.0996 | 42 | 0.0145 |
| 9 | 0.0553 | 55 | 0.0290 |
| 22 | 0.0300 | 66 | 0.0200 |
| 33 | 0.0067 | 73 | 0.0691 |
| 34 | 0.0150 | 74 | 0.0461 |

### Test Example 2

MT-4 cells (5 × 10⁶/0.2 ml/well) were cultured on a 24-well microtiter plate. After the cells were incubated for 24 hours at 37°C in a carbon dioxide gas incubator, a culture medium was replaced with a buffer solution (0.1% BSA-containing RPMI-1640). Together with a ligand (¹²⁵ I-SDF-1", specific activity: 2,200 Ci/mmol; manufactured by Daiichi Chemicals Co. , Ltd. (Tokyo)) , test materials of various concentrations were subjected to a binding reaction for 2 hours under ice-cooling. Ligands that did not bind in cold PBS were washed out, and then the radioactivities of bound ligands were measured by a scintillation counter (manufactured by Nihon Packard K.K. (Tokyo)) and a rate of inhibiting the binding between radio-active ligands and receptors CXCR4 by a test material (a binding-inhibition % at 0.1 µM) was calculated.

The results are shown in Table 3.

**Table 3**

| Compound No. | Inhibition rate (%) | Compound No. | Inhibition rate (%) |
|---|---|---|---|
| 1 | 100.0 | 33 | 100 |
| 2 | 97.6 | 34 | 98.0 |
| 27 | 97.0 | 55 | 89.3 |
| 28 | 100.0 | 66 | 90.6 |
| 29 | 100.0 | 73 | 93.5 |

### Test Example 3

The acute toxicity of the aforementioned compound was examined. Specifically, 7-week-old ICR mice (male) were divided into several groups (4 or 5 mice in each group), and the mice were bred for 1 week for habituation. Subsequently, each of the compounds of Examples was dissolved in distilled water or physiological saline, and the solution was administered to the mice via tail vein (dose: 15 mg/kg) twice a day for 4 days. On the fifth day, dead mice were counted. The results are shown in Table 4.

As shown in Table 4, it was confirmed that the administration of each compound did not cause any death and did not involve acute toxicity.

**Table 4**

| Compound No. | Dead mice/ test mice | Compound No. | Dead mice/ test mice |
|---|---|---|---|
| 9 | 0/5 | 42 | 0/5 |
| 29 | 0/5 | 55 | 0/5 |
| 33 | 0/5 | 66 | 0/5 |
| 34 | 0/5 | 73 | 0/5 |
| 39 | 0/5 | 74 | 0/5 |

### Preparation Example

34.6% of the compound No. 2, 34.6% of lactose (Japanese Pharmacopoeia: hereinafter simply refered to as JP), 17.3% of corn starch (JP), 7.3% of hydroxypropylcellulose (JP), and 6.2% of low-substitution hydroxypropylcellulose (JP) were sieved and mixed well in a vinyl bag. Purified water (JP) in an amount equal to those compounds was added thereto and then a wet cake was obtained by kneading the mixture for 20 minutes by a biaxial kneader. The wet cake was granulated using an extrusion granulating machine (cylinder pore size: 1 mm) , and then the granulated product was dried using a fluidized-bed dryer (40°C, 30 minutes). The dried granules were sieved. Subsequently, magnesium stearate was added to the sieved product in the proportion of 1% of magnesium stearate to 99% of sieved product and then the whole was mixed well, followed by making tablets having an average weight of 292 mg therefrom using a tableting machine.

In addition, an undercoat solution was prepared by dissolving 8% of hydroxypropylcellulose (JP) and 1.6% of macrogol 6000 (JP) in purified water (JP) so as to be 100% in total. An under coat tablet was prepared by spraying the undercoat solution using a hicoater in a ratio of 5% with respect to the weight of the tablet which was previously made and subjecting the sprayed tablet to drying for 20 minutes after the spraying.

Furthermore, an enteric coating solution was prepared by dissolving 10% of hydroxypropylcellulose acetate succinate (Pharmaceutical additive specification), 3% of triethyl citrate (JP), 2% of titanium oxide (JP), and 0.05% of hydroxypropylcellulose (JP) in purified water (JP) so as to be 100% in total. The enteric coating solution was sprayed using a hicoater in a ratio of 10% with respect to the tablet weight. After the spraying, the tablet was dried for 30 minutes, resulting in an enteric tablet. This enteric tablet had properties of not allowing a main component to be eluted within 2 hours in one liquid (JP), and allowing 80% or more of the main component to be eluted within 30 minutes in 2 liquids (JP).

### Industrial Applicability

The present invention provides a novel nitrogen-containing compound. The novel nitrogen-containing compound of the present invention or a pharmacologically acceptable salt thereof can provide a novel CXCR4 antagonist. The novel CXCR4 antagonist of the present invention has a CXCR4 antagonism, and shows, based on the CXCR4 antagonism, excellent effects as a therapeutic or preventive for disease such as a viral infectious disease such as HIV, rheumatism, or cancer metastasis.

## Claims

1. A compound represented by the following general formula (1) or a pharmacologically acceptable salt thereof: 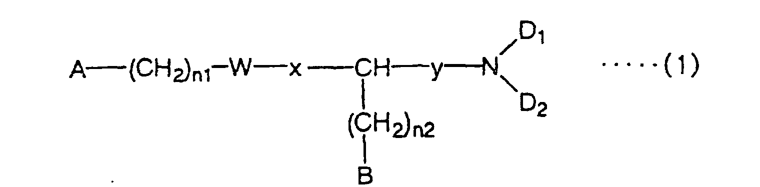 (in the general formula (1),
n₁ represents an integer of 0 to 3 and n₂ represents an integer of 0 to 4;
A represents a group represented by the following general formula (2): 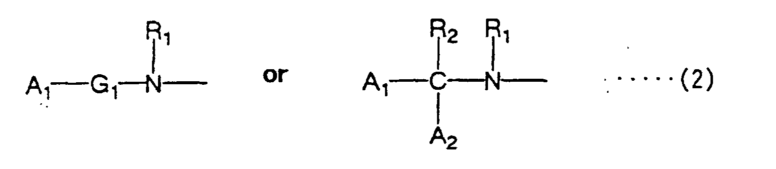 in the general formula (2),
A₁ and A₂ each independently represent a hydrogen atom, an optionally substituted mono- or polycyclic heteroaromatic ring, or an optionally substituted mono- or polycyclic aromatic ring;
G₁ represents a single bond or a group represented by the following general formula (3); and 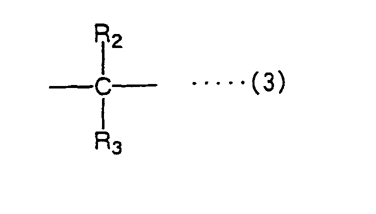 R₁, R₂ and R₃ represent an optionally substituted alkyl group having 1 to 6 carbon atoms, an optionally substituted alkenyl group having 2 to 6 carbon atoms, an optionally substituted alkynyl group having 2 to 6 carbon atoms, or an optionally substituted cyclic alkyl group having 3 to 6 carbon atoms;
W represents an optionally substituted alkylene group having 1 to 7 carbon atoms, an optionally substituted alkenylene group having 2 to 7 carbon atoms, an optionally substituted alkynylene group having 2 to 7 carbon atoms, an optionally substituted cyclic alkylene group having 3 to 10 carbon atoms, an optionally substituted mono- or polycyclic aromatic ring, an optionally substituted mono-or polycyclic heteroaromatic ring, or an optionally substituted mono- or polycyclic saturated heterocyclic ring;
D₁ and D₂ each independently represent a hydrogen atom or a group represented by the following general formula (4):
―G₂―R₄ (4)
in the general formula (4),
G₂ represents an optionally substituted alkylene group having 1 to 15 carbon atoms, an optionally substituted alkenylene group having 2 to 7 carbon atoms, or an optionally substituted alkynylene group having 2 to 7 carbon atoms; and
R₄ represents a hydrogen atom, an optionally substituted cyclic alkyl group having 3 to 10 carbon atoms, an optionally substituted mono- or polycyclic aromatic ring, an optionally substituted and partly saturated polycyclic aromatic ring, an optionally substituted mono- or polycyclic heteroaromatic ring, an optionally substituted and partly saturated polycyclic heteroaromatic ring, or an optionally substituted mono- or polycyclic saturated heterocyclic ring;
B represents a group represented by the following general formula (5): 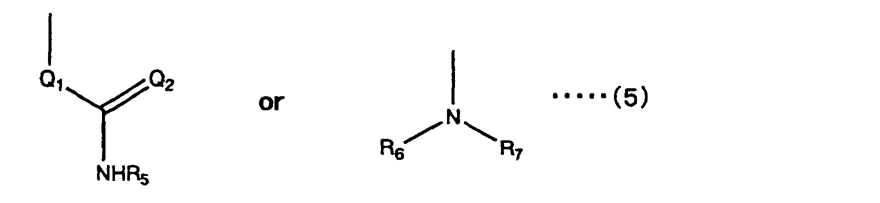 in the general formula (5),
Q₁ represents S, O, or NH and Q₂ represents S, O, or NR₈ (except for a case of Q₁=NH and Q₂=NR₈);
R₅ and R₈ each independently represent a hydrogen atom, an optionally substituted lower alkyl group, an optionally substituted cyclic alkyl group, or an optionally substituted aromatic ring, and R₅ and R₈ optionally form a ring; and
R₆ and R₇ each independently represent a hydrogen atom, a substituent represented by the following general formula (6), an optionally substituted alkyl group having 1 to 15 carbon atoms, an optionally substituted cyclic alkyl group having 3 to 15 carbon atoms, an optionally substituted alkenyl group having 1 to 3 double bonds and 2 to 15 carbon atoms, or an optionally substituted alkynyl group having 1 to 3 triple bonds and 2 to 15 carbon atoms, and R₆ and R₇ optionally form a ring, wherein R₆ and R₇ are optionally bonded with each other via a heteroatom, a cyclic alkyl group, an aromatic ring, a heteroaromatic ring, or a heterocyclic ring to form the ring: 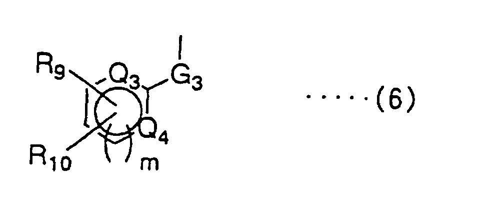 in the formula (6),
m represents 0 or 1, when m=0, Q₃ represents CH or N and Q₄ represents N, S, or O, and when m=1, Q₃ and Q₄ each independently represent CH or N;
G₃ represents an optionally subsituted alkylene group having 1 to 4 carbon atoms, or an optionally substituted alkenylene group having 2 to 4 carbon atoms;
R₉ represents a lower alkyl group, an alkoxy group, a haloalkyl group, a haloalkoxy group, a hydroxyalkoxy group, a halogen atom, an amino group, an alkylamino group, a carboxyl group, an alkoxycarbonyl group, a carbamoyl group, an alkylcarbamoyl group, a saturated heterocyclic ring, or a heteroaromatic ring, which is substituted at any position in a ring other than that of a nitrogen atom optionally existing in the ring, and when m=1 and Q₃ and Q₄ simultaneously represent CH, R₉ optionally represents a hydrogen atom; and
R₁₀ represents a hydrogen atom, or a same group as R₅, and optionally bonds with G₃ to form a ring);
x represents a group represented by the following general formula (7): 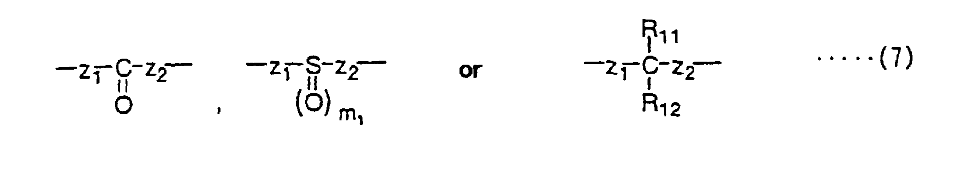 in the general formula (7),
z₁ and z₂ each independently represent a single bond, S, O, or NR₁₃, and m₁ represents an integer of 1 or 2; and
R₁₁, R₁₂, and R₁₃ each independently represent a hydrogen atom, an optionally substituted alkyl group having 1 to 6 carbon atoms, an optionally substituted alkenyl group having 2 to 6 carbon atoms, an optionally substituted alkynyl group having 2 to 6 carbon atoms, or an optionally substituted cyclic alkyl group having 3 to 6 carbon atoms;
y represents a group represented by the following general formula (8): 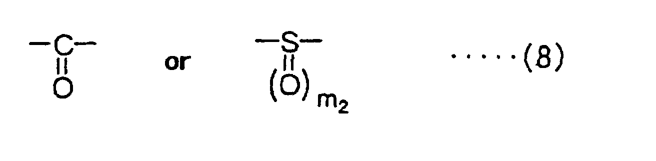 in the general formula (8),
m₂ represents an integer of 1 or 2; and
when there is one asymmetric carbon atom optionally existing in the compound represented by the general formula (1), the compound is in any form of a pure optical isomer represented as absolute configuration of R or S, a mixture thereof in any ratio, and a racemic mixture thereof,
and when there are two or more of the asymmetric carbon atoms in the compound, the compound is in any form of an optically pure diastereomer, a racemic mixture thereof, and a combination thereof in any ratio).

2. A compound or a pharmacologically acceptable salt thereof according to claim 1, wherein
x is a group represented by the following general formula (9) : 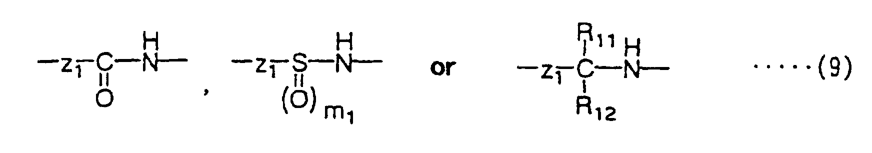 z1, ml, R11, and R12 are the same as described in claim 1; and
y is a group represented by the following general formula (10).

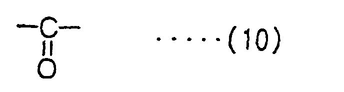

3. A compound or a pharmacologically acceptable salt thereof according to claim 1 or 2, wherein
A is a group represented by the following general formula (11) : 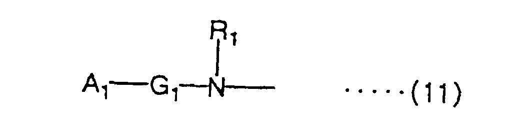 A1, G1, and R1 are the same as described in claim 1.

4. A compound or a pharmacologically acceptable salt thereof according to any one of claims 1 to 3, wherein D1 represents a hydrogen atom and D2 represents a group represented by the following general formula (12):
―G₄―R₁₄ (12)
(wherein G4 represents an optionally substituted alkylene group having 1 to 4 carbon atoms; and R14 represents an optionally substituted mono- or polycyclic aromatic ring, or an optionally substituted mono- or polycyclic heteroaromatic ring).

5. A compound or a pharmacologically acceptable salt thereof according to any one of claims 1 to 4, wherein
x is represented by the following general formula (13).

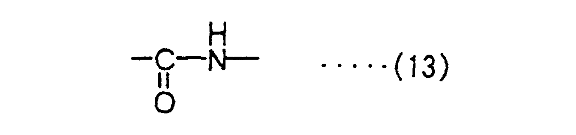

6. A compound or a pharmacologically acceptable salt thereof according to any one of claims 1 to 5, wherein
B is represented by the following general formula (14): 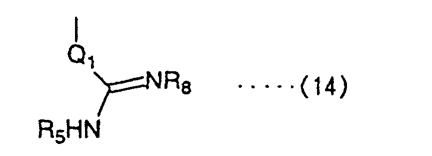 Q1, R5, and R8 are the same as described in claim 1 (except for when Q1 = NH).

7. A compound or a pharmacologically acceptable salt thereof according to any one of claims 1 to 5, wherein
B is represented by the following general formula (15): 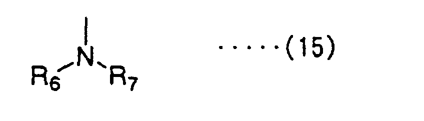 (wherein R6 and R7 are the same as described in claim 1).

8. A compound or a pharmacologically acceptable salt thereof selected from the group consisting of:
N-[(S)-1-(1-naphthyl)ethyl]-5-(2-methylbenzyl)amino-2-(S)-[4-[ N-(imidazol-2-ylmethyl)aminomethyl]benzoyl]aminopentanoylamide;
N-[(S)-1-(1-naphthyl)ethyl]-5-(2-methoxybenzyl)amino-2-(S)-[4-[N-(imidazol-2-ylmethyl)aminomethyl]benzoyl]aminopentanoylamide;
N-[(S)-1-(1-naphthyl)ethyl]-5-isobutyl-2-(S)-[4-[N-(imidazol-2-ylmethyl)aminomethyl]benzoyl]aminopentanoylamide;
N-[(S)-1-(1-naphthyl)ethyl]-5-(2,2-dimethylpropyl)amino-2-(S) -[4-[N-(imidazol-2-ylmethyl)aminomethyl]benzoyl]aminopentanoylamide;
N-[(S)-1-(2-naphthyl)ethyl]-5-(2-trifluoromethylbenzyl)amino-2-(S)-[4-[N-(imidazol-2-ylmethyl)aminomethyl]benzoyl]aminopentanoylamide;
N-[(S)-1-(1-naphthyl)ethyl]-5-(2-chlorobenzyl)amino-2-(S)-(4-[N-(imidazol-2-ylmethyl)aminomethyl]benzoyl]aminopentanoylamide;
N-[(S)-1-(1-naphthyl)ethyl]-5-(3-methylbenzyl)amino-2-(S)-[4-[N-(imidazol-2-ylmethyl)aminomethyl]benzoyl]aminopentanoylamide;
N-[(S)-1-(1-naphthyl)ethyl]-5-(4-methylbenzyl)amino-2-(S)-[4-[N-(imidazol-2-ylmethyl)aminomethyl]benzoyl]aminopentanoylamide;
N-[(S)-1-(1-naphthyl)ethyl]-5-(3-methylpyridin-2-yl)methylamino-2-(S)-[4-[N-(imidazol-2-ylmethyl)aminomethyl]benzoyl]aminopentanoylamide;
N-[(S)-1-(1-naphthyl)ethyl]-5-(5-methylpyridin-2-yl)methylamino-2-(S)-[4-[N-(imidazol-2-ylmethyl)aminomethyl]benzoyl]aminopentanoylamide;
N-[(S)-1-(1-naphthyl)ethyl]-5-(4-methylpyridin-2-yl)methylamino-2-(S)-[4-[N-(imidazol-2-ylmethyl)aminomethyl]benzoyl]aminopentanoylamide;
N-[(S)-1-(1-naphthyl)ethyl]-5-(2,6-dimethoxybenzyl)amino-2-(S)-[4-[N-(imidazol-2-ylmethyl)aminomethyl]benzoyl]aminopentanoylamide;
N-[(S)-1-(1-naphthyl)ethyl]-5-(3-methylthiophen-2-yl)methylamino-2-(S)-[4-[N-(imidazol-2-ylmethyl)aminomethyl]benzoyl]aminopentanoylamide;
N-[(S)-1-(1-naphthyl)ethyl]-5-(3-methoxypyridin-2-yl)methylamino-2-(S)-[4-[N-(imidazol-2-ylmethyl)aminomethyl]benzoyl]aminopentanoylamide;
N-[(S)-1-(1-naphthyl)ethyl]-5-(2-dimethylaminobenzyl)amino-2-(S)-[4-[N-(imidazol-2-ylmethyl)aminomethyl]benzoyl]aminopentanoylamide;
N-[(S)-1-(1-naphthyl)ethyl]-5-(3-n-propyloxypyridin-2-yl)methylamino-2-(S)-[4-[N-(imidazol-2-ylmethyl)aminomethyl]benzoyl]aminopentanoylamide;
N-[(S)-1-(1-naphthyl)ethyl]-5-(3-ethoxypyridin-2-yl)methylamino-2-(S)-[4-[N-(imidazol-2-ylmethyl)aminomethyl]benzoyl]aminopentanoylamide;
N-[(S)-1-(1-naphthyl)ethyl]-5-[2-(2-hydroxyethoxy)benzyl]amino-2-(S)-[4-[N-(imidazol-2-ylmethyl)aminomethyl]benzoyl]aminopentanoylamide;
N-[(S)-1-(1-naphthyl)ethyl]-5-(2-trifluoromethoxybenzyl)amino-2-(S)-[4-[N-(imidazol-2-ylmethyl)aminomethyl]benzoyl]aminopentanoylamide;
N-[(S)-1-(1-naphthyl)ethyl]-5-(2-hydroxybenzyl)amino-2-(S)-[4-[N-(imidazol-2-ylmethyl)aminomethyl]benzoyl]aminopentanoylamide;
N-[(S)-1-(1-naphthyl)ethyl]-5-(3-isopropyloxypyridin-2-yl)methylamino-2-(S)-[4-[N-(imidazol-2-ylmethyl)aminomethyl]benzoyl] aminopentanoylamide;
N-[(S)-1-(1-naphthyl)ethyl)-5-(2-ethylbenzyl)amino-2-(S)-[4-[N-(imidazol-2-ylmethyl)aminomethyl]benzoyl]aminopentanoylamide;
N-[(S)-1-(1-naphthyl)ethyl]-5-(2-isopropyloxybenzyl)amino-2-(S)-[4-[N-(imidazol-2-ylmethyl)aminomethyl]benzoyl]aminopentanoylamide;
N-[(S)-1-(1-naphthyl)ethyl]-5-(2-morpholinobenzyl)amino-2-(S)-[4-[N-(imidazol-2-ylmethyl)aminomethyl]benzoyl]aminopentanoylamide;
N-[(S)-1-(1-naphthyl)ethyl]-5-(2-(4-methylpiperazino)benzyl)amino-2-(S)-[4-[N-(imidazol-2-ylmethyl)aminomethyl]benzoyl]aminopentanoylamide;
N-[(S)-1-(1-naphthyl)ethyl]-5-(3-methylpyrrol-2-yl)methylamino-2-(S)-[4-[N-(imidazol-2-ylmethyl)aminomethyl]benzoyl]aminopentanoylamide;
N-[(S)-1-(1-naphthyl)ethyl]-5-cyclohexylamino-2-(S)-[4-[N-(imidazol-2-ylmethyl)aminomethyl]benzoyl]aminopentanoylamide;
N-[(S)-1-(1-naphthyl)ethyl]-5-(1,2,3,4-tetrahydronaphthalen-1-yl)amino-2-(S)-[4-[N-(imidazol-2-ylmethyl)aminomethyl]benzoyl]aminopentanoylamide;
N-[(S)-1-(1-naphthyl)ethyl]-5-(indan-1-yl)amino-2-(S)-[4-[N-(imidazol-2-ylmethyl)aminomethyl]benzoyl]aminopentanoylamide;
N-[(S)-1-(1-naphthyl)ethyl]-5-(1-methylpiperidin-4-yl)amino-2-(S)-[4-[N-(imidazol-2-ylmethyl)aminomethyl]benzoyl]aminopentanoylamide;
N-[(S)-1-(1-naphthyl)ethyl]-5-(pentan-3-yl)amino-2-(S)-[4-[N-(imidazol-2-ylmethyl)aminomethyl]benzoyl]aminopentanoylamide;
N-[(S)-1-(1-naphthyl)ethyl]-5-dimethylamino-2-(S)-[4-[N-(imidazol-2-ylmethyl)aminomethyl]benzoyl]aminopentanoylamide;
N-[(S)-1-(1-naphthyl)ethyl]-5-diisobutylamino-2-(S)-[4-[N-(imidazol-2-ylmethyl)aminomethyl]benzoyl]aminopentanoylamide;
N-[(S)-1-(1-naphthyl)ethyl]-5-di-n-propylamino-2-(S)-[4-[N-(imidazol-2-ylmethyl)aminomethyl]benzoyl]aminopentanoylamide;
N-[(S)-1-(1-naphthyl)ethyl]-5-di-n-butylamino-2-(S)-[4-[N-(imidazol-2-ylmethyl)aminomethyl]benzoyl]aminopentanoylamide;
N-[(S)-1-(1-naphthyl)ethyl]-5-[N-methyl-(3-methylpyridin-2-yl)methylamino]-2-(S)-[4-[N-(imidazol-2-ylmethyl)aminomethyl]benzoyl]aminopentanoylamide;
N-[(S)-1-(1-naphthyl)ethyl]-5-[N-methyl-(2-methoxybenzyl)amino]-2-(S)-[4-[N-(imidazol-2-ylmethyl)aminomethyl]benzoyl]aminopentanoylamide;
N-[(S)-1-(1-naphthyl)ethyl]-5-(N-methyl-isobutylamino)-2-(S)-[4-[N-(imidazol-2-ylmethyl)aminomethyl]benzoyl]aminopentanoylamide;
N-[(S)-1-(1-naphthyl)ethyl]-5-(N-n-propyl-isobutylamino)-2-(S)-[4-[N-(imidazol-2-ylmethyl)aminomethyl]benzoyl]aminopentanoylamide;
N-[(S)-1-(1-naphthyl)ethyl]-5-(N-ethyl-isobutylamino)-2-(S)-[4-[N-(imidazol-2-ylmethyl)aminomethyl]benzoyl]aminopentanoylamide;
N-[(S)-1-(1-naphthyl)ethyl]-5-(N-isopropyl-isobutylamino)-2-(S)-[4-[N-(imidazol-2-ylmethyl)aminomethyl]benzoyl]aminopentanoylamide;
N-[(S)-1-(1-naphthyl)ethyl]-5-(N-methyl-cyclohexylamino)-2-(S)-[4-[N-(imidazol-2-ylmethyl)aminomethyl]benzoyl]aminopentanoylamide;
N-[(S)-1-(1-naphthyl)ethyl]-5-(N-methyl-cyclopentylamino)-2-(S)-[4-[N-(imidazol-2-ylmethyl)aminomethyl]benzoyl]aminopentanoylamide;
N-[(S)-1-(1-naphthyl)ethyl]-5-(N-n-propyl-isopropylamino)-2-(S)-[4-[N-(imidazol-2-ylmethyl)aminomethyl)benzoyl]aminopentanoylamide;
N-[(S)-1-(1-naphthyl)ethyl]-5-di-n-propylamino-2-(S)-[4-[N-(pyridin-2-ylmethyl)aminomethyl]benzoyl]aminopentanoylamide;
N-[(S)-1-(1-naphthyl)ethyl]-5-diisobutylamino-2-(S)-[4-[N-(pyridin-2-ylmethyl)aminomethyl]benzoyl]aminopentanoylamide;
N-(1-naphthylmethyl)-5-(3-methylpyridin-2-yl)methylamino-2-(S)-[9-[N-(imidazol-2-ylmethyl)aminomethyl]benzoyl]aminopentanoylamide;
N-(1-naphthylmethyl)-5-diisobutylamino-2-(S)-[4-[N-(imidazol-2-ylmethyl)aminomethyl]benzoyl]aminopentanoylamide;
N-(1-naphthylmethyl)-5-(N-methyl-cyclohexylamino)-2-(S)-[4-[N-(imidazol-2-ylmethyl)aminomethyl)benzoyl]aminopentanoylamide;
N-[(S)-1-(1-naphthyl)ethyl]-5-(2-ethoxybenzyl)amino-2-(S)-[4-[N-(imidazol-2-ylmethyl)aminomethyl]benzoyl]aminopentanoylamide;
N-[(S)-1-(1-naphthyl)ethyl]-5-diethylamino-2-(S)-[4-[N-(imidazol-2-ylmethyl)aminomethyl]benzoyl]aminopentanoylamide;
N-[(S)-1-(1-naphthyl)ethyl]-5-(2-phenylpropan-2-ylamino)-2-(S)-[4-[N-(imidazol-2-ylmethyl) aminomethyl]benzoyl]aminopentanoylamide;
N-[(S)-1-(1-naphthyl)ethyl]-5-[2-(2-methoxyphenyl)ethyl]amino-2-(S)-[4-[N-(imidazol-2-ylmethyl)aminomethyl]benzoyl]aminopentanoylamide;
N-[(S)-1-(1-naphthyl)ethyl]-5-(1,2,3,4-tetrahydroisoquinolin-2-yl)-2-(S)-[4-[N-(imidazol-2-ylmethyl)aminomethyl]benzoyl]aminopentanoylamide;
N-[(S)-1-(1-naphthyl)ethyl]-5-(hexamethyleneimin-1-yl)-2-(S)-[4-[N-(imidazol-2-ylmethyl)aminomethyl]benzoyl]aminopentanoylamide;
N-[(S)-1-(1-naphthyl)ethyl]-5-(heptamethyleneimin-1-yl)-2-(S)-[4-[N-(imidazol-2-ylmethyl)aminomethyl]benzoyl]aminopentanoylamide;
N-[(S)-1-(1-naphthyl)ethyl]-5-morpholino-2-(S)-[4-[N-(imidazol-2-ylmethyl)aminomethyl]benzoyl]aminopentanoylamide;
N-[(S)-1-(1-naphthyl)ethyl]-5-piperidino-2-(S)-[4-[N-(imidazol-2-ylmethyl)aminomethyl]benzoyl]aminopentanoylamide;
N-[(S)-1-(1-naphthyl)ethyl]-5-(pyrrolidin-1-yl)-2-(S)-[4-[N-(imidazol-2-ylmethyl)aminomethyl]benzoyl]aminopentanoylamide;
N-[(S)-1-(1-naphthyl)ethyl]-5-bis (2-methoxyethyl)amino-2-(S)-[4-[N-(imidazol-2-ylmethyl)aminomethyl]benzoyl]aminopentanoylamide;
N-[(S)-1-(1-naphthyl)ethyl]-5-bis(2-hydroxyethyl)amino-2-(S)-[4-[N-(imidazol-2-ylmethyl)aminomethyl]benzoyl]aminopentanoylamide;
N-[(S)-1-(1-naphthyl)ethyl]-S-(N-n-propyl-(2-methoxyethyl)amino)-2-(S)-[4-[N-(imidazol-2-ylmethyl)aminomethyl]benzoyl]aminopentanoylamide;
N-[(S)-1-(1-naphthyl)ethyl]-5-(N-isobutyl-(2-methoxyethyl)amino)-2-(S)-[4-[N-(imidazol-2-ylmethyl}aminomethyl]benzoyl]aminopentanoylamide;
N-[(S)-1-(1-naphthyl)ethyl]-5-(N-isopropyl-(2-methoxyethyl)amino)-2-(S)-[4-[N-(imidazol-2-ylmethyl)aminomethyl]benzoyl]aminopentanoylamide;
N-[(S)-1-(1-naphthyl)ethyl]-5-(N-n-propyl-(2-hydroxyethyl)amino)-2-(S)-[4-[N-(imidazol-2-ylmethyl)aminomethyl]benzoyl]aminopentanoylamide;
N-[(S)-1-(1-naphthyl)ethyl]-5-(N-isobutyl-(2-hydroxyethyl)amino)-2-(S)-[4-[N-(imidazol-2-ylmethyl)aminomethyl]benzoyl]aminopentanoylamide;
N-[(S)-1-(1-naphthyl)ethyl]-5-(N-isopropyl-(2-hydroxyethyl)amino)-2-(S)-[4-[N-(imidazol-2-ylmethyl)aminomethyl]benzoyl]aminopentanoylamide;
N-[(S)-1-(1-naphthyl)ethyl]-5-(hexamethyleneimin-1-yl)-2-(S)-[4-[N-(pyridin-2-ylmethyl)aminomethyl]benzoyl]aminopentanoylamide;
N-[(S)-1-(1-naphthyl)ethyl]-5-(bis(2-hydroxyethyl)amino)-2-(S)-[4-[N-(pyridin-2-ylmethyl)aminomethyl]benzoyl]aminopentanoylamide;
N-[(S)-1-(1-naphthyl)ethyl]-5-(N-isobutyl(2-hydroxyethyl)amino)-2-(S)-[4-[N-(pyridin-2-ylmethyl)aminomethyl]benzoyl]aminopentanoylamide;
N-[(S)-1-(1-naphthyl)ethyl]-5-ureide-2-(S)-[4-[N-(imidazol-2-ylmethyl)aminomethyl]benzoyl]aminopentanoylamide;
N-[(S)-1-(1-naphthyl)ethyl)-5-(3-phenylthioureide)-2-(S)-[4-[N-(pyridin-2-ylmethyl)aminomethyl]benzoyl]aminopentanoylamide;
N-[(S)-1-(1-naphthyl)ethyl]-5-sulfinamidino-2-(S)-[4-[N-(pyridin-2-ylmethyl)aminomethyl)benzoyl]aminopentanoylamide;
N-[(S)-1-(1-naphthyl)ethyl]-5-(N-methylsulfinamidino)-2-(S)-[4-[N-(pyridin-2-ylmethyl)aminomethyl]benzoyl]aminopentanoylamide;
N-[(S)-1-(1-naphthyl)ethyl]-5-(N,N'-dimethylsulfinamidino)-2-(S)-[4-[N-(pyridin-2-ylmethyl)aminomethyl]benzoyl]aminopentanoylamide;
N-[(S)-1-(1-naphthyl)ethyl]-5-sulfinamidino-2-(S)-[4-[N-(imidazol-2-ylmethyl)aminomethyl]benzoyl]aminopentanoylamide;
N-[(S)-1-(1-naphthyl)ethyl]-5-(N-methylsulfinamidino)-2-(S)-[4-[N-(imidazol-2-ylmethyl)aminomethyl]benzoyl]aminopentanoylamide;
N-[(S)-1-(1-naphthyl)ethyl]-5-(N,N'-dimethylsulfinamidino)-2-(S)-[4-[N-(imidazol-2-ylmethyl)aminomethyl]benzoyl]aminopentanoylamide;
N-[(S)-1-(naphthyl)ethyl]-5-[N-n)ethyl-(pentan-3-yl)amino]-2-(S)-[4-[N-(imidazol-2-ylmethyl)aminomethyl]benzoyl]aminopentanoylamide;
N-[(S)-1-(naphthyl)ethyl]-5-[N-ethyl-(pentan-3-yl)amino]-2-(S)-[4-[N-(imidazol-2-ylmethyl)aminomethyl]benzoyl]aminopentanoylamide;
N-[(S)-1-(naphthyl)ethyl]-5-[N-n-propyl-(pentan-3-yl)amino]-2-(S)-[4-[N-(imidazol-2-ylmethyl)aminomethyl]benzoyl]aminopentanoylamide;
N-[(S)-1-(naphthyl)ethyl]-5-[N-carboxymethyl(isobutyl)amino]-2-(S)-{4-[N-(1H-imidazol-2-ylmethyl)aminomethyl]benzoyl}aminopentanoylamide;
N-[(S)-1-(naphthyl)ethyl]-5-[N-carbamoylmethyl(isobutyl)amino]-2-(S)-{4-[N-(1H-imidazol-2-ylmethyl)aminomethyl]benzoyl}aminopentanoylamide;
N-[(S)-1-(1-naphthyl)ethyl]-5-[N-methoxycarbanylmethyl(isobutyl)amino]-2-(S)-{4-[N-(1H-imidazol-2-ylmethyl)aminomethyl]benzoyl}aminopentanoylamide;
N-[(1S)-4-[(2-hydroxyethyl)isobutylamino]-1-{[(S)-1-(naphthalen-1-yl)ethyl]carbamoyl}butyl]-4-{[(1-methyl-1H-imidazol-2-ylmethyl)amino]methyl}benzamide;
N-((1S)-4-dipropylamino-1-{[(S)-1-(naphthalen-1-yl)ethyl]carbamoyl}butyl]-4-{[(1-methyl-1H-imidazol-2-ylmethyl)amino]methyl}benzamide;
N-[(1S)-4-dipropylamino-1-{[(S)-1-(naphthalen-1-yl)ethyl]carbamoyl}butyl]-4-{[(1-methyl-1H-benzoimidazol-2-ylmethyl}amino]methyl}benzamide;
N-[(1S)-4-dipropylamino-1-{[(S)-1-(naphthalen-1-yl)ethyl]carbamoyl}butyl]-4-{[(1-methyl-1H-imidazol-2-ylmethyl)methylamino] methyl}benzamide;
N-[(1S)-4-[(2-hydroxyethyl)isobutylamino]-1-{[(S)-1-(naphthalen-1-yl)ethyl]carbamoyl}butyl]-4-{[(1-methyl-1H-imidazol-2-ylmethyl)methylamino]methyl}benzamide;
N-[(1S)-4-[(2-hydroxyethyl)isobutylamino]-1-{[(S)-1-(naphthalen-1-yl)ethyl]carbamoyl}butyl]-4-{[(1-methyl-1H-benzoimidazol-2-ylmethyl)amino]methyl}benzamide;
N-[(1S)-4-[(2-hydroxyethyl)isobutylamino]-1-{[(S)-1-(naphthalen-1-yl)ethyl]carbamoyl}butyl]-4-{[(1-methyl-1H-imidazol-2-ylmethyl)methylamino]methyl}benzamide;
N-[(1S)-4-dipropylamino-1-{[(S)-1-(naphthalen-1-yl)ethyl]carbamoyl}butyl]-4-{[(1-methyl-1H-imidazol-2-ylmethyl)methylamino] methyl}benzamide;
N-[(1S)-4-[(2-hydroxyethyl)isobutylamino]-1-{[(S)-1-(naphthalen-1-yl)ethyl]carbamoyl}butyl]-4-{[(furan-2-ylmethyl)amino]methyl}benzamide;
N-[(1S)-4-isobutylmethanesulfonylamino-1-{[(S)-1-(naphthalen-1-yl)ethyl]carbamoyl}butyl]-4-{[(1H-imidazol-2-ylmethyl)amino] methyl}benzamide;
N-[(1S)-4-dipropylamino-1-{[(S)-1-(naphthalen-1-yl)ethyl]carbamoyl}butyl]-4-{[(1-ethyl-1H-imidazol-2-ylmethyl)amino]methyl} benzamide;
N-[(1S)-4-dipropylamino-1-{[(S)-1-(naphthalen-1-yl)ethyl]carbamoyl)butyl]-4-{[(1-propyl-1H-imidazol-2-ylmethyl)amino]methyl }benzamide;
S-{[(1-methyl-1H-imidazol-2-ylmethyl)amino]methyl}thiophene-2-carboxylic-[(1S)-4-[(3-methylpyridin-2-ylmethyl)amino]-1-{[(S)-1-(naphthalen-1-yl)ethyl]carbamoyl}butyl]amide;
5-{[(1-methyl-1H-imidazol-2-ylmethyl)amino]methyl)thiophene-2-carboxylic-[(1S)-4-[(2-methyoxybenzylamino)-1-{[(S)-1-(naphthalen-1-yl)ethyl]carbamoyl}butyl]amide;
5-{[(1-methyl-1H-imidazol-2-ylmethyl)amino]methyl}thiophene-2-carboxylic-[(1S)-4-(1-ethylpropylamino)-1-{[(S)-1-(naphthalen-1-yl)ethyl]carbamoyl}butyl]amide;
N-{(1S)-4-dipropylamino-1-[2-(1H-indol-3-yl)ethylcarbamoyl]butyl)-4-{[(1H-imidazol-2-ylmethyl)amino]methyl}benzamide;
4-{[(1H-imidazol-2-ylmethyl)amino]methyl}naphthalen-1-carboxylic-{(1S)-4-dipropylamino-1-[2-(1H-indol-3-yl)ethylcarbamoyl]butyl}amide;
N-{(1S)-4-dipropylamino-1-[2-(1H-indol-3-yl)ethylcarbamoyl]butyl}-4-{[(1-methyl-1H-imidazol-2-ylmethyl)amino]methyl}benzamide;
5-{[(1-methyl-1H-imidazol-2-ylmethyl)amino]methyl)thiophene-2-carboxylic-{(1S)-4-dipropylamino-1-[2-(1H-indol-3-yl)ethylcarbamoyl]butyl}amide;
N-{(1S)-4-dipropylamino-1-[2-(1H-indol-3-yl)ethylcarbamoyl]butyl}-4-{[(pyridin-2-ylmethyl)amino]methyl}benzamide;
4-{[(pyridin-2-ylmethyl)amino]methyl}naphthalen-1-carboxylic-{(1S)-4-dipropylamino-1-[2-(1H-indol-3-yl)ethylcarbamoyl]butyl}amide;
N-{(1S)-4-dipropylamino-1-[2-(1H-indol-3-yl)ethylcarbamoyl]butyl}-6-{[(pyridin-2-ylmethyl)amino]methyl}nicotinamide;
8-{[(pyridin-2-ylmethyl)amino]methyl}isoquinoline-5-carboxylic-{(1S)-4-dipropylamino-1-[2-(1H-indal-3-yl)ethylcarbamoyl]butyl}amide;
N-[(1S)-4-dipropylamino-1-{[(S)-1-(naphthalen-1-yl)ethyl]carbamoyl}butyl]-4-({[1-(2-hydroxyethyl)-1H-imidazol-2-ylmethyl]amino}methyl)benzamide;
4-{[(1-methyl-1H-imidazol-2-ylmethyl)amino]methyl}naphthalen-1-carboxylic-[(1S)-4-[(3-methylpyridin-2-ylmethyl)amino]-1-{[(S)-1-(naphthalen-1-yl)ethyl]carbamoyl}butyl]amide;
4-{[(1-methyl-1H-imidazol-2-ylmethyl)amino]methyl}naphthalen-1-carboxylic-[(1S)-4-(2-methoxybenzylamino)-1-{[(S)-1-(naphthalen-1-yl)ethyl]carbamoyl}butyl]amide;
4-{[(1-methyl-1H-imidazol-2-ylmethyl)amino]methyl}naphthalen-1-carboxylic-[(1S)-4-(1-ethylpropylamino)-1-{[(S)-1-(naphthalen-1-yl)ethyl]carbamoyl}butyl]amide;
4-{[bis(pyridin-2-ylmethyl)amino]methyl}-N-[(1S)-4-[2-hydroxy ethyl]isobutylamino]-1-{[(S)-1-(naphthalen-1-yl)ethyl]carbamoyl)butyl]benzamide;
N-[(1S)-4-dipropylamino-1-(3-isopropoxypropylcarbamoyl)butyl]-4-{[(1-methyl-1H-imidazol-2-ylmethyl)amino]methyl}benzamide;
4-{[(1-methyl-1H-imidazol-2-ylmethyl)amino]methyl}naphthalen-1-carboxylic-[(1S)-4-dipropylamino-1-(3-isopropoxypropylcarbamoyl)butyl]amide;
N-[(1S)-4-dipropylamino-1-(3-isopropoxypropylcarbamoyl)butyl]-4-{[(1H-imidazol-2-ylmethyl)amino]methyl}benzamide;
5-{[(1H-imidazol-2-ylmethyl)amino]methyl}thiophene-2-carboxylic-((1S)-4-dipropylamino-1-[2-(1H-indol-3-yl)ethylcarbamoyl]butyl}amide;
5-{[(pyridin-2-ylmethyl)amino]methyl}thiophene-2-carboxylic-{(1S)-4-dipropylamino-1-[2-(1H-indol-3-yl)ethylcarbamoyl]butyl}amide;
N-{(1S)-4-dipropylamino-1-[2-(1H-indol-3-yl)ethylcarbamoyl]butyl}-4-{[(pyridin-2-ylmethyl)amino]methyl}benzamide;
4-{[(1-methyl-1H-imidazol-2-ylmethyl)amino]methyl}naphthalen-1-carboxylic-{(1S)-4-dipropylamino-1-[2-(1H-indol-3-yl)ethylcarbamoyl]butyl}amide;
N-[(1S)-4-(4,5-dihydro-1H-imidazol-2-ylsulfanyl)-1-{[(S)-1-(naphthalen-1-yl)ethyl]carbamoyl)butyl}-4-{[(pyridin-2-ylmethyl) amino]methyl}benzamide;
N-[(1S)-4-(1H-imidazol-2-ylsulfanyl)-1-{[(S)-1-(naphthalen-1-yl)ethyl]carbamoyl)butyl]-4-([(lH-imidazol-2-ylmethyl)amino]methyl}benzamide;
N-[(1S)-4-dipropylamino-1-(isopropylcarbamoyl)butyl]-4-{[(1-methyl-1H-imidazol-2-ylmethyl)amino]methyl}benzamide;
N-[(1S)-4-dipropylamino-1-(isopropylcarbamoyl)butyl]-4-{[(pyridin-2-ylmethyl)amino]methyl}benzamide;
4-{[bis(1H-imidazol-2-ylmethyl)amino]methyl}-N-[(1S)-4-[(2-hydroxyethyl)isobutylamino]-1-{[(S)-1-(naphthalen-1-yl)ethyl]carbamoyl}butyl]benzamide;
N-[(1S)-4-dipropylamino-1-(isopropylcarbamoyl)butyl]-4-{[(1H-imidazol-2-ylmethyl)amino]methyl}benzamide;
4-{[(1H-imidazol-2-ylmethyl)amino]methyl}-N-[(1S)-2-(3H-imidazol-4-yl)-1-{[(S)-1-(naphthalen-1-yl)ethyl]carbamoyl}ethyl]benzamide;
4-{[(1H-imidazol-2-ylmethyl)amino]methyl}-N-[(1S)-2-(1H-indol-2-yl)-1-{[(S)-1-(naphthalen-1-yl)ethyl]carbamoyl}ethyl]benzamide;
N-[(1S)-4-dipropylamino-1-(3-phenylpropylcarbamoyl)butyl]-4-{[(1H-imidazol-2-ylmethyl)amino]methyl}benzamide;
N-[(1S)-4-dipropylamino-1-(3-phenylpropylcarbamoyl)butyl]-4-{[(1-methyl-1H-imidazol-2-ylmethyl)amino]methyl}benzamide;
N-[(1S)-4-dipropylamino-1-(3-phenylpropylcarbamoyl)butyl]-4-{[(pyridin-2-ylmethyl)amino]methyl}benzamide;
4-{[(1H-imidazol-2-ylmethyl)amino]methyl}-N-({[(S)-1-(naphthalen-1-yl)ethyl]carbamoyl}methyl)benzamide;
4-{[(1H-imidazol-2-ylmethyl)amino]methyl)-N-[(1S)-2-methyl-1-{[(S)-1-(naphthalen-1-yl)ethyl]carbamoyl}propyl]benzamide;
N-[(1S)-4-diprapylamino-1-{[(naphthalen-1-ylmethyl)carbamoyl]butyl}-4-{[(1H-imidazol-2-ylmethyl)amino]methyl}benzamide;
4-{[(1H-imidazol-2-ylmethyl)amino]methyl}-N-[(1S)-4-[(1H-imidazol-2-ylmethyl)amino]-1-{[(S)-1-(naphthalen-1-yl)ethyl]carbamoyl}butyl]benzamide;
4-{[(pyridin-2-ylmethyl)amino]methyl}-N-[(1S)-4-[(1H-imidazol-2-ylmethyl)amino]-1-{[(S)-1-(naphthalen-1-yl)ethyl]carbamoyl}butyl]benzamide;
N-[(1S)-4-(2-hydroxyethylamino)-1-{[(S)-1-(naphthalen-1-yl)ethyl]carbamoyl}butyl)-4-{[(1-methyl-1H-imidazol-2-ylmethyl)amino]methyl}benzamide;
N-[(1S)-4-(2-hydroxyethylamino)-1-{[(S)-1-(naphthalen-1-yl)ethyl]carbamoyl}butyl]-4-{[(1H-imidazol-2-ylmethyl)amino]methyl}benzamide;
4-{[(pyridin-2-ylmethyl)amino]methyl}naphthalen-1-carboxylic-[(1S)-4-(4,5-dihydro-1H-imidazol-2-ylamio)-1-{[(S)-1-naphthalen-1-yl)ethyl]carbamoyl}butyl]amide;
N-[(1S)-4-(4,5-dihydro-1H-imidazol-2-ylamio)-1-{[(S)-1-(naphthalen-1-yl)ethyl]carbamoyl}butyl]-4-({[1-methyl-1H-imidazol-2-ylmethyl]amino}methyl)benzamide;
4-{[(1H-imidazol-2-ylmethyl)amino]methyl}-naphthalen-1-carboxylic-[(1S)-4-cyclohexylamino-1-{[(S)-1-(naphthalen-1-yl)ethyl]carbamoyl}butyl]amide;
N-[(1S)-4-cyclohexylamino-1-{[(S)-1-(naphthalen-1-yl)ethyl]carbamoyl}butyl]-4-({[1-(2-hydroxyethyl)-1H-imidazol-2-ylmethyl]amino}methyl)benzamide;
4-{[(1-methyl-1H-imidazol-2-ylmethyl)amino]methyl}naphthalen-1-carboxylic-[(1S)-4-methylcarbamimidoylsulfanyl-1-{[(S)-1-(naphthalen-1-yl)ethyl]carbamoyl}butyl]amide;
4-{[(1H-imidazol-2-ylmethyl)amino]methyl}-N-[(1S)-1-{((S)-1-(naphthalen-1-yl)ethyl]carbamoyl}-(2-phenyl)ethyl]benzamide;
4-{[(1-methyl-1H-irnidazol-2-ylmethyl)amino]methyl}-N-[(1S)-4-[(1H-imidazol-2-ylmethyl)amino]-1-([(S)-1-(naphthalen-1-yl)ethyl]carbamoyl}butyl]benzamide;
4-{[(1-methyl-1H-imidazol-2-ylmethyl)amino]methyl}-N-[(1S)-4-[(1-methyl-1H-imidazol-2-ylmethyl)amino]-1-{[(S)-1-(naphthalen-1-yl)ethyl]carbamoyl}butyl]benzamide;
4-({[1-(2-hydroxyethyl)-1H-imidazol-2-ylmethyl]amino}methyl)naphthalen-1-carboxylic-[(1S)-4-cyclohexylamino-1-{[(S)-1-(naphthalen-1-yl)ethyl]carbamoyl}butyl]amide;
4-{[(1-methyl-1H-imidazol-2-ylmethyl)amino]methyl}-N-[(1S)-4-methylcarbamimidoylsulfanyl-1-{[(S)-1-(naphthalen-1-yl)ethyl]carbamoyl}butyl]benzamide;
4-{[(1H-imidazol-2-ylmethyl)amino]methyl}-N-[(1S)-4-[(1-methyl-1H-imidazol-2-ylmethyl)amino]-1-{[(S)-1-(naphthalen-1-yl)ethyl]carbamoyl}butyl]benzamide;
N-[(1S)-4-[(3-methylpyridin-2-ylmethyl)amino-1-{[(S)-1-(naphthalen-1-yl)ethyl]carbamoyl}butyl]-4-({[1-(2-hydroxyethyl)-1H-imidazol-2-ylmethyl]amino}methyl)benzamide;
N-{(1S)-4-dipropylamino-1-[(1H-indol-3-ylmethyl)carbamoyl]butyl}-4-{[(1H-imidazol-2-ylmethyl)amino]methyl}benzamide;
N-[(1S)-4-dipropylamino-1-{[(S)-1-(naphthalen-1-yl)ethyl]carbamoyl}butyl]-4-{[(1-oxypyridin-2-ylmethyl)amino]methyl}benzamide;
N-[(1S)-4-(4,5-dihydro-1H-imidazol-2-ylamino)-1-{[(S)-1-naphthalen-1-yl)ethyl]carbamoyl}butyl]-4-({[1-(2-hydroxyethyl)-1H-imidazol-2-ylmethyl]amino}methyl)benzamide;
(2S)-5-dipropylamino-2-(4-{[(1-methyl-1H-imidazol-2-ylmethyl)amino]methyl}benzenesulfonylamino)pentanoylic-[(S)-1-(naphthalen-1-yl)ethyl]amide;
(2S)-5-dipropylamino-2-(4-{[(1H-imidazol-2-ylmethyl)amino]methyl}benzenesulfonylamino)pentanoylic-[(S)-1-(naphthalen-1-yl)ethyl]amide;
N-[(1S)-4-dipropylamino-1-{[1-(4-fluoronaphthalen-1-yl)ethyl]carbamoyl}butyl]-4-{[(1H-imidazol-2-ylmethyl)amino]methyl}benzamide;
N-[(1S)-4-dipropylamino-1-{[1-(4-fluoronaphthalen-1-yl)ethyl]carbamoyl}butyl]-4-{[(1-methyl-1H-imidazol-2-ylmethyl)amino]methyl}benzamide;
N-[(1S)-4-dipropylamino-1-((S)-1-naphthalen-2-ylethylcarbamoyl)butyl]-4-{[(1H-imidazol-2-ylmethyl)amino]methyl}benzamide;
4-{[bis(1H-imidazol-2-ylmethyl)amino]methyl}-N-{(1S)-4-dipropylamino-1-[2-(1H-indol-3-yl)ethylcarbamoyl]butyl}benzamide;
N-{(1S)-4-dipropylamino-1-[(2-methoxynaphthalen-1-ylmethyl)carbamoyl]butyl}-4-{[(1H-imidazol-2-ylmethyl)amino]methyl}benzamide;
N-{(1S)-4-dipropylamino-1-[(4-methoxynaphthalen-1-ylmethyl)carbamoyl]butyl}-4-{[(1H-imidazol-2-ylmethyl)amino]methyl}benzamide; and
4-{[bis(1H-imidazol-2-ylmethyl)amino]methyl)-N-((1S)-4-dipropylamino-1-isopropylcarbamoylbutyl)benzamide.

9. A CXCR4 antagonist comprising the compound or the pharmacologically acceptable salt thereof according to any one of claims 1 to 8 as an active ingredient.

10. An antiviral agent comprising the compound or the pharmacologically acceptable salt thereof according to any one of claims 1 to 8 as an active ingredient.

11. A rheumatic disease improvingagent comprising the compound or the pharmacologically acceptable salt thereof according to any one of claims 1 to 8 as an active ingredient based on a CXCR4 antagonism.

12. A cancer metastatic disease improving agent comprising the compound or the pharmacologically acceptable salt thereof according to any one of claims 1 to 8 as an active ingredient based on a CXCR4 antagonism.
